# EUROPEAN PATENT APPLICATION

(11) **EP 4 786 521 A1**
(43) Date of publication of application: **05.08.2026**
(21) Application number: 24871016.2
(22) Date of filing: 27.09.2024
(51) Int. Cl.: C08G 65/333, A61K 47/54, A61P 35/00

(54) **PHOSPHOLIPID DRUG CONJUGATE, AND PREPARATION METHOD AND USE THEREFOR**

(30) Priority: 27.09.2023 CN 202311261706
(71) Applicant: Shanghai Best-Link Bioscience, LLC, Shanghai 201203 (CN)
(72) Inventor: ZHANG, Fuyao, Shanghai 201203 (CN); CAI, Shengyun, Shanghai 201203 (CN); CHEN, Xianjie, Shanghai 201203 (CN)
(74) Representative: Murgitroyd & Company
(86) International application number: PCT/CN2024/121906
(87) International publication number: WO 2025/067481

(57) **Abstract**

Disclosed in the present invention are a phospholipid drug conjugate and a preparation method and use therefor. Disclosed are the phospholipid drug conjugate as shown in formula (I), or a stereoisomer, prodrug, pharmaceutically acceptable salt or pharmaceutically acceptable solvate thereof. The phospholipid drug conjugate disclosed by the invention has good water solubility, can self-assemble to form a nanostructure, and has an excellent tumor inhibition effect.

## Description

This application claims priority to Chinese Patent Application No. 2023112617069, filed on September 27, 2023. The entire content of the aforementioned Chinese patent application is incorporated herein by reference.

### TECHNICAL FIELD

The present invention relates to the field of drug delivery, and specifically, to a phospholipid drug conjugate, a preparation method, and a use thereof.

### BACKGROUND

The incidence and mortality of cancer have been increasing year by year, making it a major public health problem threatening human health. In recent years, despite tremendous efforts and significant progress made in cancer treatment, therapeutic outcomes remain limited. Among the three major cancer treatment methods (surgery, chemotherapy, radiotherapy), chemotherapy plays a pivotal role in the treatment of various tumors. However, conventional chemotherapeutic drugs are mainly administered intravenously, which has some undesirable limitations, including uncontrolled distribution of the drug in tissues leading to cytotoxicity to normal cells, poor drug permeability, poor retention due to passive diffusion, and frequent administration potentially inducing drug resistance, thereby exhibiting a narrow therapeutic window and poor drug safety. All of these issues stem from poor drug delivery in vivo.

To address these obstacles, various strategies have been developed. These include: (1) Utilizing nanocarriers to physically package drug molecules, thereby improving drug properties, enhancing the solubility of poorly soluble drugs, and increasing drug enrichment in lesion tissues to improve bioavailability while reducing toxicity. However, nanodrugs based on physical interactions have poor in vivo stability and lack active targeting, which compromises their delivery and release efficiency. (2) Polymer-drug conjugates: Polymer-drug conjugates are prodrug-like products conjugated via chemical bonds, which can deliver drugs with improved drug solubility, slowly released active pharmaceutical ingredients, and long-circulating pharmacokinetic characteristics. However, their inherent defects still exist, including cumbersome and complex synthesis procedures, difficulty in controlling drug loading, poor membrane permeability due to large molecular weight, lack of active targeting, and potential systemic toxicity. These shortcomings further limit their clinical development.

Compared with traditional polymer prodrugs and nanocarriers, small molecule lipids (e.g., phospholipids, fatty acids, cholesterol, glycerides) are readily available, non-toxic, biocompatible, and biodegradable, making them ideal building blocks for designing drug conjugates and nanocarriers.

The present invention provides a novel class of phospholipid conjugates, which are conjugated to drugs via stimuli-responsive linkers and can self-assemble into nanoparticles. These conjugates improve the in vivo stability of nanodrugs and enable the release of drug molecules in response to specific stimuli in the disease microenvironment, thereby achieving enhanced therapeutic efficacy.

### BRIEF SUMMARY OF THE INVENTION

The technical problem to be solved by the present invention is to overcome the shortcomings of drug conjugates in the prior art. For this purpose, the present invention provides a phospholipid drug conjugate and its preparation method and use. The phospholipid drug conjugate of the present invention has one or more of the following effect advantages: (1) excellent anti-tumor effect; (2) effective release of bioactive molecules/drugs; (3) having nano-size; (4) good water solubility.

The technical problem of the present invention is solved by the following technical solution:

The present invention provides a phospholipid drug conjugate having nano-size and comprising a stimuli-responsive linker as shown in formula (I), 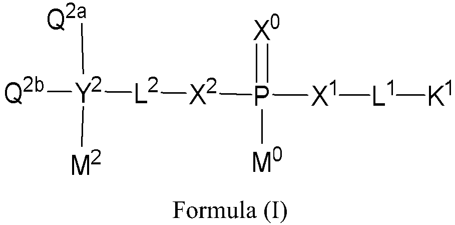
or a stereoisomer, a prodrug, a pharmaceutically acceptable salt, or a pharmaceutically acceptable solvate thereof,
wherein:
   X⁰ is O or S;
   X¹ is O, S, or NR^{1a};
   X² is O, S, or NR^{2a};
   L¹ and L² are absent or are linking units;
   M⁰ is OR^{0a}, SR^{0b}, NR^{0c}R^{0d}, or T⁰-(L-D)ₙ;
   K¹ is P¹ or 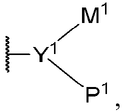 Y¹ is a branching center, P¹ is a pharmacokinetic modulating moiety unit, M¹ is T¹-(L-D)ₙ;
   Y² is a branching center;
   M² is absent or is T²-(L-D)ₙ;
   at least one of M⁰, M¹, and M² contains an L-D fragment;
   Q^{2a} is T^{2a}-R¹ or is absent, Q^{2b} is T^{2b}-R² or is absent, at least one of Q^{2a} and Q^{2b} is present;
   T⁰, T¹, and T² are each independently selected from absent or an extension moiety;
   T^{2a} and T^{2b} are absent or are linking units;
   L is L³-L^{s}, L³ is an environment-responsive linking unit, L^{s} is present or absent, and when present, it is a self-immolative linker moiety;
   D is a bioactive molecule fragment, and when the conjugate contains multiple D, D may be the same or different;
   R^{1a} and R^{2a} are each independently selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or an amino protecting group;
   R^{0a} is each independently selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or a hydroxyl protecting group;
   R^{0b} is selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or a thiol protecting group;
   R^{0c} and R^{0d} are each independently selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or an amino protecting group;
   R¹ is selected from saturated or unsaturated C₈-C₅₀ alkyl;
   R² is selected from saturated or unsaturated C₁-C₅₀ alkyl;
   n is an integer from 1 to 10, and when n>1, L-D may be the same or different;

In the compound represented by formula (I), one or more hydrogens bonded to carbon may be substituted by deuterium;

In the compound represented by formula (I), one or more hydrogens bonded to carbon may be substituted by any substituent;

Phospholipid isomers include compounds with phosphorus as a chiral center, selected from: 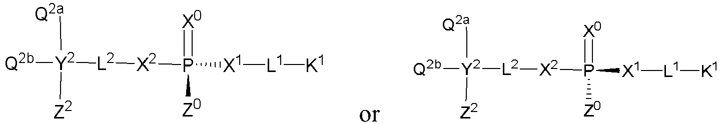 or chiral mixtures thereof.

In some embodiments, R¹ and R² are each independently selected from saturated or unsaturated C₁-C₅₀ alkyl, cholesterol analogs, retinoids, and tocopherol analogs, wherein the C₁-C₅₀ alkyl, cholesterol analogs, retinoids and tocopherol analogs are optionally substituted by one or more substituents selected from hydroxyl, halogen, C₁₋₁₀ alkyl, C₁₋₁₀ alkoxy, C₃₋₁₂ cycloalkyl, and 3-12 membered heterocycloalkyl, and the C₁₋₁₀ alkyl, C₁₋₁₀ alkoxy, C₃₋₁₂ cycloalkyl, and 3-12 membered heterocycloalkyl are optionally substituted by one or more substituents selected from hydroxyl, halogen, C₁₋₃ alkyl, C₁₋₃ alkoxy, and C₃₋₆ cycloalkyl.

In some embodiments, the C₁-C₅₀ alkyl is each independently preferably C₈-C₅₀ alkyl, more preferably C₁₀-C₃₀ alkyl, and further preferably C₁₀-C₂₀ alkyl.

In some embodiments, the phospholipid drug conjugate having nano-size and comprising a stimuli-responsive linker provided by the present invention, or a stereoisomer, prodrug, pharmaceutically acceptable salt, or pharmaceutically acceptable solvate thereof, is characterized in that the phospholipid core 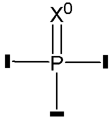 in formula (I) satisfies one or more of the following conditions:
(1) 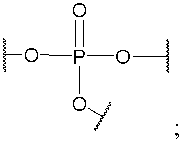
(2) 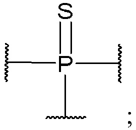
(3) 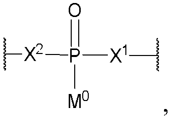 wherein at least one of the atoms connecting X², X¹, and M⁰ to P is not an oxygen atom.

In some embodiments, the phospholipid drug conjugate having nano-size and comprising a stimuli-responsive linker provided by the present invention, or a stereoisomer, prodrug, pharmaceutically acceptable salt, or pharmaceutically acceptable solvate thereof, is characterized in that the nano-size is an average nanoparticle size range of 1-500 nanometers; preferably, the average particle size range is 5-100 nanometers.

In some embodiments, the phospholipid drug conjugate having nano-size and comprising a stimuli-responsive linker provided by the present invention, or a stereoisomer, prodrug, pharmaceutically acceptable salt, or pharmaceutically acceptable solvate thereof, satisfies one or more of the following conditions:
(1) L¹ and L² are absent or present, and when present, L¹ and L² are each independently alkane, heterocycloalkane, aromatic hydrocarbon, heterocycloalkane, substituted or unsubstituted amino acids or polypeptides composed thereof, or various combinations thereof, wherein the functional group connecting portions at both ends of L¹ and L² are each independently selected from: 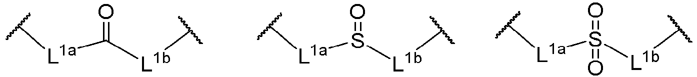 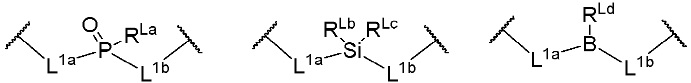 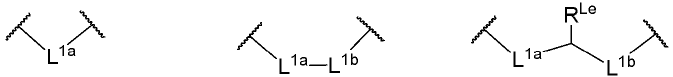
   wherein, R^{La}, R^{Lb}, R^{Lc}, and R^{Ld} are each independently selected from hydroxyl, C1-C5 alkyl, or C1-C5 alkoxy;
   R^{Le}, is hydrogen, hydroxyl, C1-C5 alkyl, or C1-C5 alkoxy;
   L^{1a} and L^{1b} are each independently selected from O, NR^{Lf}, S, S-S, 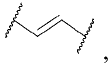 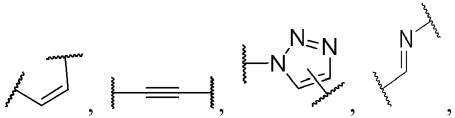 or directly bonded (absent), R^{Lf} is hydrogen or C1-C5 alkyl;
(2) T⁰, T¹, and T² are each independently absent or present, and when present, T⁰, T¹, and T² are each independently alkane, heterocycloalkane, aromatic hydrocarbon, heterocycloalkane, substituted or unsubstituted amino acids or polypeptides composed thereof, or various combinations thereof, wherein the functional groups at both ends of T⁰, T¹, and T² are each independently selected from: 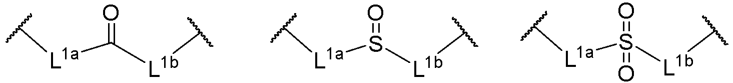 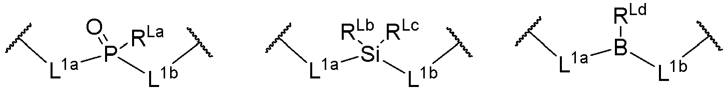 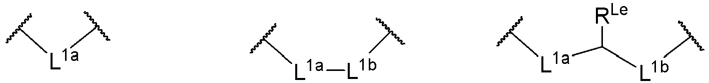
   wherein, R^{La}, R^{Lb}, R^{Lc}, and R^{Ld} are each independently selected from hydroxyl, C1-C5 alkyl, or C1-C5 alkoxy;
   R^{Lc}, is hydrogen, hydroxyl, C1-C5 alkyl, or C1-C5 alkoxy;
   L^{1a} and L^{1b} are each independently selected from O, NR^{Lf}, S, S-S, 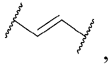 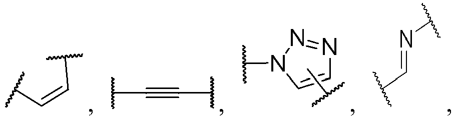 or directly bonded (absent), R^{Lf} is hydrogen or C1-C5 alkyl;
(3) T^{2a} and T^{2b} are absent or present, when present, T^{2a} and T^{2b} are each independently alkane, heterocycloalkane, aromatic hydrocarbon, heterocycloalkane, substituted or unsubstituted amino acids or polypeptides composed thereof, or various combinations thereof, wherein the functional groups at both ends of T^{2a} and T^{2b} are each independently selected from: 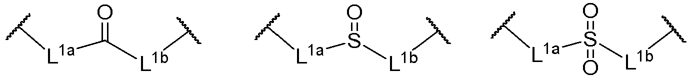 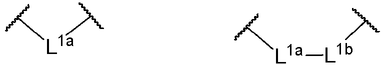 wherein, L^{1a} and L^{1b} are each independently selected from O, NR^{Lf}, S, S-S, 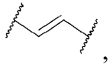 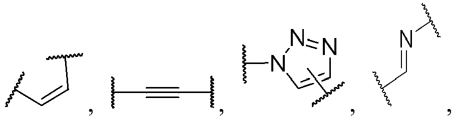 or directly bonded (absent), R^{Lf} is hydrogen or C1-C5 alkyl;
(4) L³ is selected from an enzyme-responsive linking unit, a pH-sensitive linking unit, a reduction-responsive linking unit, a reactive oxygen species (ROS) stimulus-responsive linking unit, or a photo-responsive linking unit;
(5) Y¹ and Y² are at least trifunctional branching centers, selected from trifunctional amino acids, polypeptides, or the following structures: 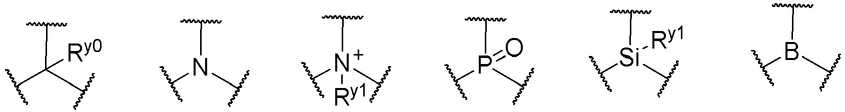 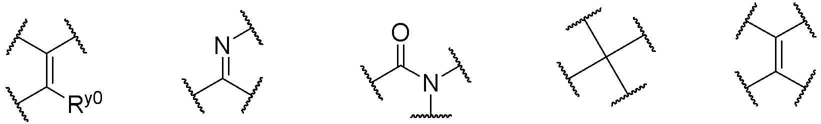 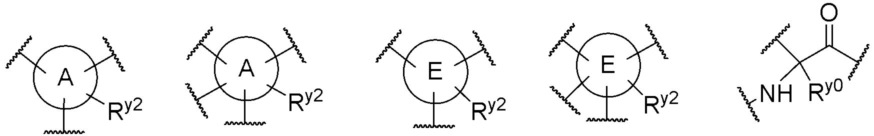
   wherein, R^{y0} is selected from hydrogen, deuterium, halogen, nitro, cyano, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or a group containing primary amine, secondary amine, tertiary amine, hydroxyl, thiol, carboxyl, ester, amide, boronic acid, boronic ester, phosphoric acid, sulfonic acid, sulfoxide, aldehyde, ketone functional groups;
   R^{y1} is selected from C1-C10 alkyl, C1-C10 alkoxy, C3-C8 cycloalkyl, and C2-C8 heterocycloalkyl;
   R^{y2} is selected from hydrogen, deuterium, halogen, hydroxyl, amino, nitro, cyano, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or a group containing primary amine, secondary amine, tertiary amine, hydroxyl, thiol, carboxyl, ester, amide, boronic acid, boronic ester, phosphoric acid, sulfonic acid, sulfoxide, aldehyde, ketone functional groups;
   Ring A is C₆-C₂₀ aryl or C₅-C₂₀ heteroaryl; the heteroatom(s) in the C₅-C₂₀ heteroaryl are selected from O, S, or N, the number of the heteroatom(s) is one or more, and when multiple heteroatoms are present, they may be the same or different;
   Ring E is C2-C8 heterocycloalkyl; the heteroatom(s) in the heterocycloalkyl are selected from O, S, or N, the number of heteroatom(s) is one or more, and when multiple heteroatoms are present, they may be the same or different.
(6) P¹ is a pharmacokinetic modulating moiety unit, selected from polyethylene glycol, sugars, oligosaccharides, and polysaccharides (such as hyaluronic acid, chondroitin sulfate, chitosan, heparin, tea polysaccharide, glucan, polysialic acid, sodium alginate, cellulose, polysucrose cyclodextrin), polysarcosine, polyphosphoester, polyglycerol, polyvinylpyrrolidone, polyoxazoline, polyamino acids, polyacrylic acid residues, polymethacrylic acid residues, quaternary ammonium salt derivatives, carboxyl-containing derivatives, sulfonic acid-containing derivatives, phosphoric acid-containing derivatives, zwitterionic derivatives, or zwitterionic polymers.

In some embodiments, L³ is an environment-responsive linking unit, selected from an enzyme-responsive linker, a pH-responsive linker, a photo-responsive linker, and a redox-responsive linker, preferably selected from an enzyme-responsive linker, a pH-responsive linker, and a redox-responsive linker, more preferably an enzyme-responsive linker.

In some embodiments, the phospholipid drug conjugate having nano-size and comprising a stimuli-responsive linker provided by the present invention, or a stereoisomer, prodrug, pharmaceutically acceptable salt, or pharmaceutically acceptable solvate thereof, is characterized in that it satisfies one or more of the following conditions:
(1) The L³ is an enzyme-responsive linker, capable of being cleaved by one or more of the following enzymes: secretory phospholipase A2, acid phosphatase, serum alkaline phosphatase, cytochrome P450, sulfatase, prostate-specific antigen, phospholipase A1, phospholipase A2, phospholipase B, phospholipase C, phospholipase D, neutrophil elastase, caspase-3, cathepsin, matrix metalloproteinase, β-glucuronidase, β-galactosidase, nitroreductase, NADPH, aminopeptidase N, carboxylesterase, diaphorase, histone deacetylase, legumain, urokinase-type plasminogen activator, urokinase-type plasminogen activator receptor, collagenase; preferably cleaved by one or more of the following enzymes: caspase-3, cathepsin, matrix metalloproteinase, elastase, or β-glucuronidase;
(2) The L³ is a pH-responsive linker, comprising one or more of the following structures: hydrazone, imine, oxime, carboxylic ester, thioester, sulfate ester, sulfonate ester, orthoester, carbonate, carbamate, substituted carbamate, ketal, acetal, silyl ether, phosphate ester, boronic ester, phosphoramidate, or cis-aconitic acid group;
(3) The L³ is a photo-responsive linker, comprising one or more of the following structures: o-nitrobenzyl, coumarin, benzoin, BODIPY, or cyanine group;
(4) The L³ is a redox-responsive linker, comprising one or more of the following structures: thioketal, phenylboronic ester, phenylboronic acid, oxalate ester, vinyl ether, thioether, aminoacrylate, disulfide, diselenide, 2,4-dinitrobenzenesulfonate, 2-azidomethylbenzoate, 4-azidobenzyl, unsaturated acid ester, or azobenzene group.

In some embodiments, the phospholipid drug conjugate having nano-size and comprising a stimuli-responsive linker provided by the present invention, or a stereoisomer, prodrug, pharmaceutically acceptable salt, or pharmaceutically acceptable solvate thereof, is characterized in that it satisfies one or more of the following conditions:
(1) L³ is an enzyme-responsive linker, selected from short peptides composed of 2-10 amino acid residues Val, D-Val, Cit, Phe, Lys, Leu, Gly, Ala, Asn, Asp, Arg, Pro, Tyr, Ile, His, Ser, Gln, Glu, Met, preferably short peptides composed of 2-10 amino acids selected from Val, Cit, Phe, Lys, Ala, Leu, Gly, Asn, Asp; preferably, L³ is selected from the following structures: 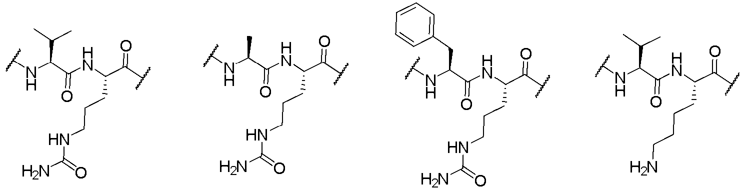 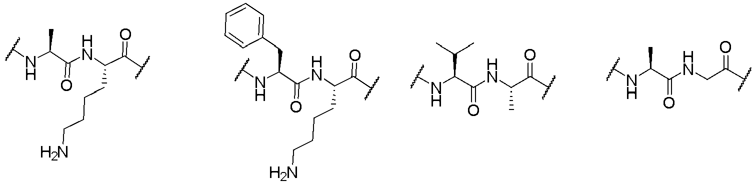 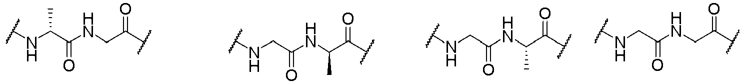 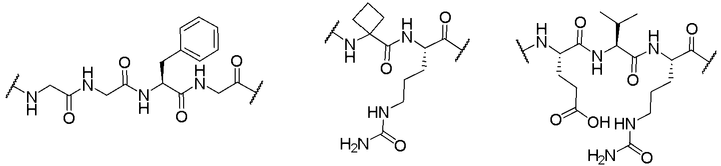 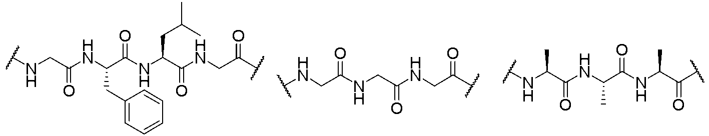 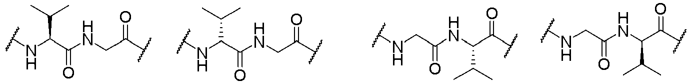 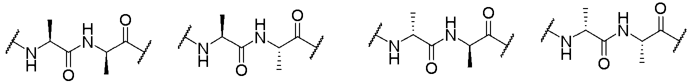
(2) When L^{s} is present, it is selected from the following structures:

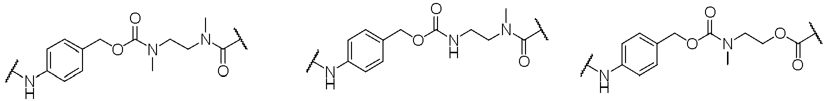

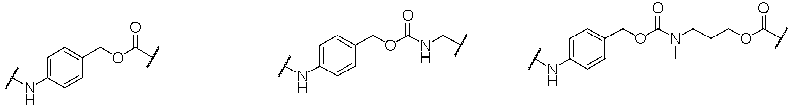

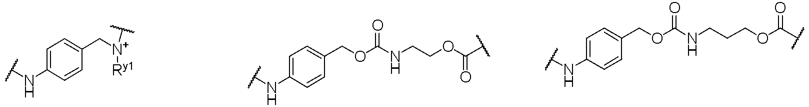

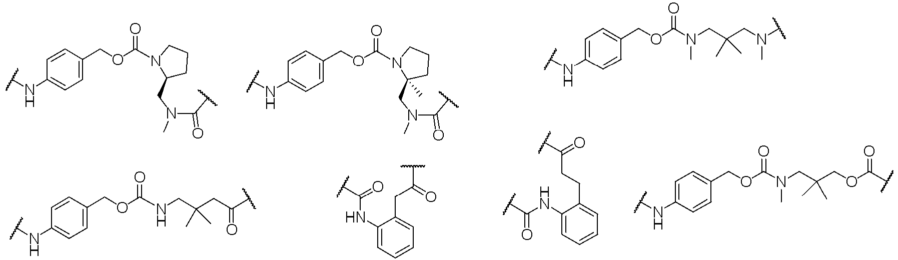

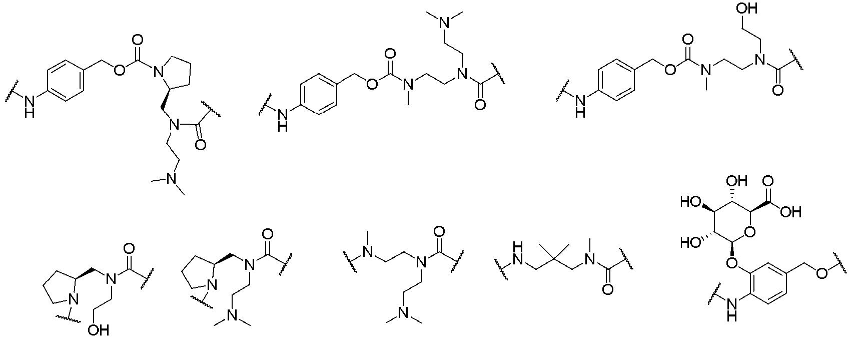

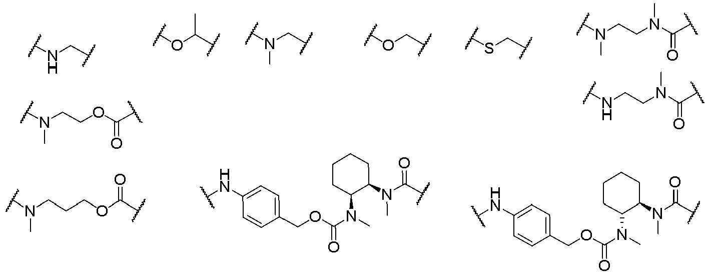

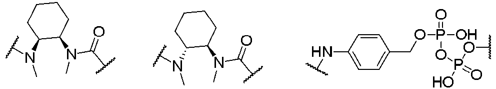

Preferably, L^{s} is selected from the following structures:

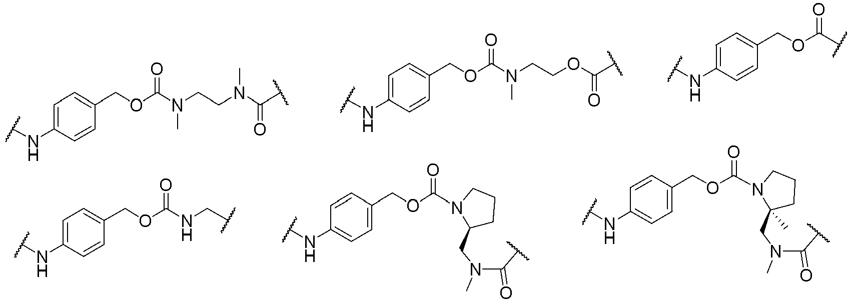

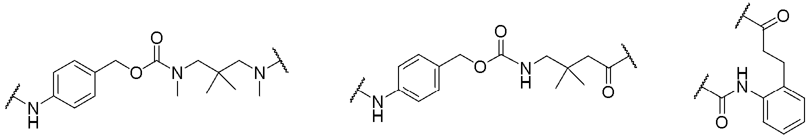

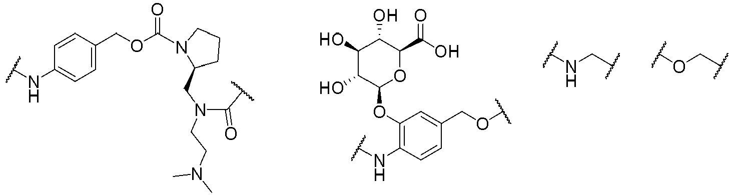

(3) L³ is a pH-responsive linker, selected from the following structures: 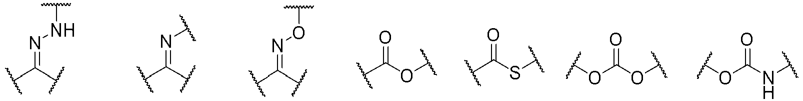 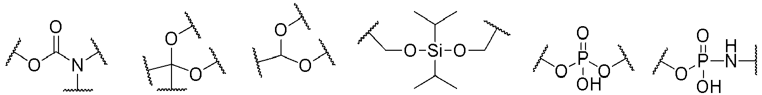 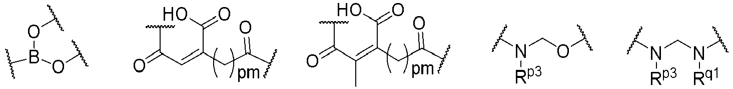 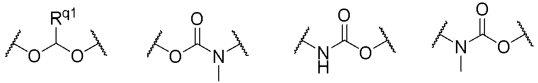
wherein, pm = 0-4; R^{p3} and R^{q1} are selected from H, substituted or unsubstituted C1-C10 alkyl;
Preferably, L³ is selected from the following structures:

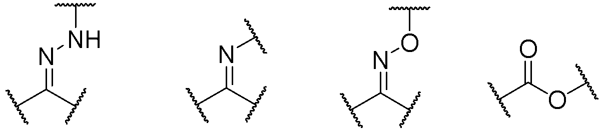

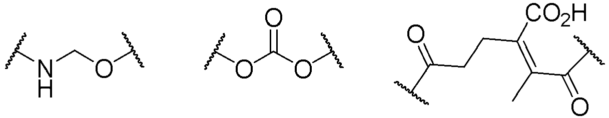

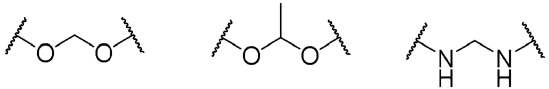

(4) The L³ is a photo-responsive linker, selected from the following structures:

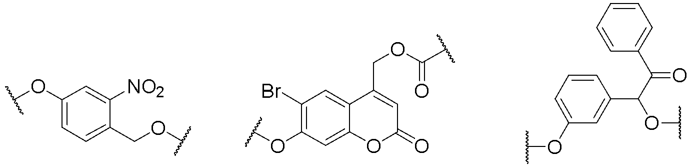

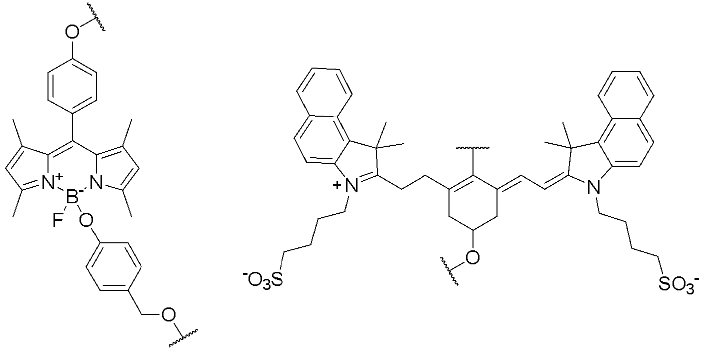

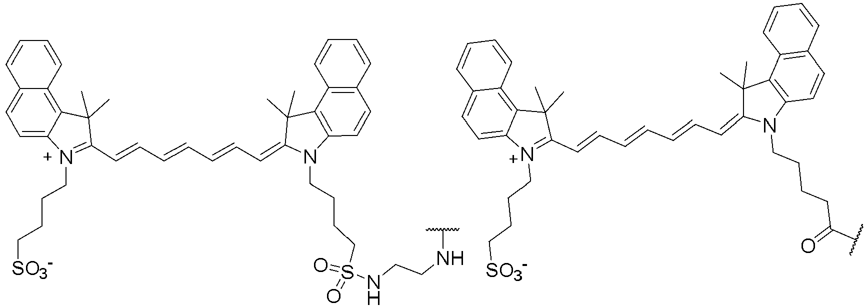

(5) L³ is a redox-responsive linker, selected from the following structures:

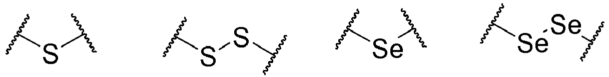

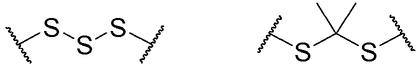

In some embodiments, when L³ is a redox-responsive linker, it comprises the following structure:

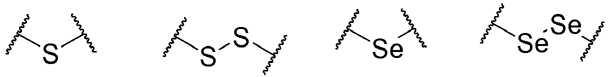

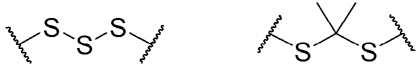

In some embodiments, when L³ is a redox-responsive linker, it is selected from the following structures:

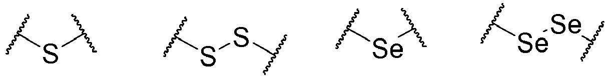

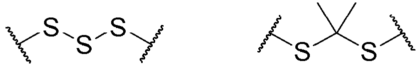

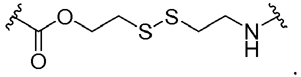

In some embodiments, the phospholipid drug conjugate having nano-size and comprising a stimuli-responsive linker provided by the present invention, or a stereoisomer, prodrug, pharmaceutically acceptable salt, or pharmaceutically acceptable solvate thereof, is characterized in that P¹ is selected from:
(1) Polyethylene glycol derivative residues are selected from the following structures: 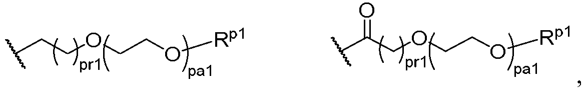
   wherein, pa1 is an integer from 5 to 250, pr1 is an integer from 0 to 8, R^{p1} is hydrogen, C1-C10 alkyl, C1-C10 heteroalkyl, C3-C10 cycloalkyl, C3-C10 alkenyl, C3-C10 alkynyl, or a hydroxyl protecting group;
   preferably, pa1 is an integer from 5 to 150, pr1 is an integer from 0 to 8, R^{p1} is hydrogen, C1-C10 alkyl, or a hydroxyl protecting group;
   more preferably, pa1 is an integer from 10 to 60, pr1 is an integer from 1 to 2, R^{p1} is C1-C10 alkyl;
(2) Polysarcosine derivative residues are selected from the following structures: 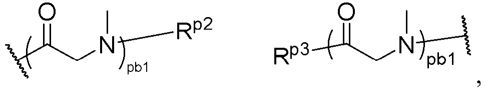
   wherein, pb1 is an integer from 5 to 250, R^{p2} is selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or an amino protecting group; R^{p3} is selected from OR^{p3a} or NR^{p3b}R^{p3c}, wherein R^{p3a} is selected from hydrogen, C1-C10 alkyl, C3-C10 alkenyl, C3-C10 alkynyl, C3-C8 cycloalkyl, C2-C8 heterocycloalkyl, C6-C10 aryl, or C5-C10 heteroaryl; R^{p3b} and R^{p3c} are each independently selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, an amino protecting group, or R^{p3b} and R^{p3c} together with the nitrogen atom to which they are attached form a C3-C10 cyclic structure;
   Preferably, pb1 is an integer from 5 to 150;
(3) Quaternary ammonium salt derivative residues are selected from the following structures: 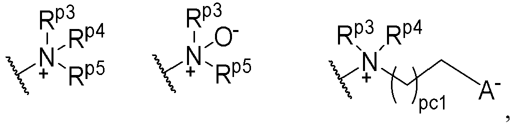 wherein, pc1 is an integer from 0 to 10, A⁻ is selected from 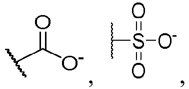 or 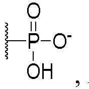 R^{p3}, R^{p4}, and R^{p5} are each independently selected from C1-C10 alkyl, C1-C10 alkoxy, C3-C8 cycloalkyl, or C2-C8 heterocycloalkyl;
(4) zwitterionic derivatives or zwitterionic polymers: 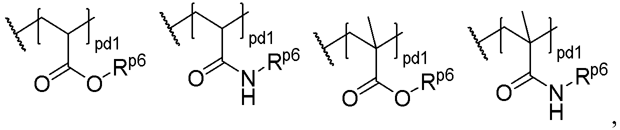 wherein,
   pd1 is an integer from 5 to 250;
   R^{p6} is selected from: 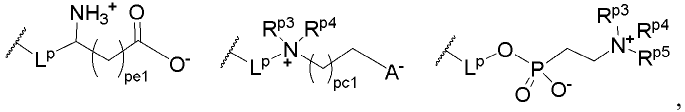
   pc1 and pe1 are integers from 0 to 10, A⁻ is selected from 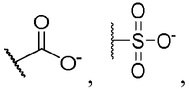 or 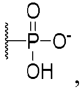 R^{p3}, R^{p4}, and R^{p5} are each independently selected from C1-C10 alkyl, C1-C10 alkoxy, C3-C8 cycloalkyl, or C2-C8 heterocycloalkyl; L^{p} is absent or a linking unit, selected from alkane, heterocycloalkane, aromatic hydrocarbon, heterocycloalkane, or various combinations thereof;
(5) polysaccharide polymers: 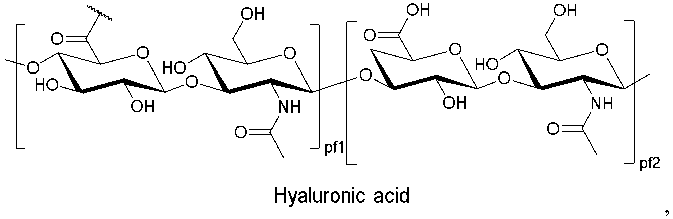 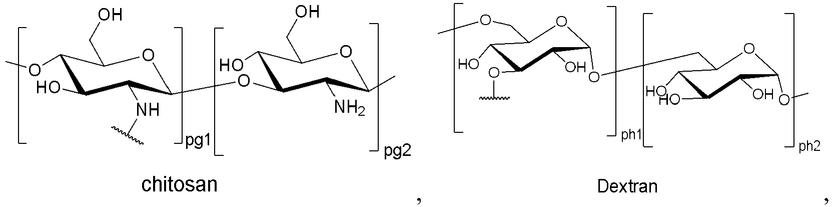 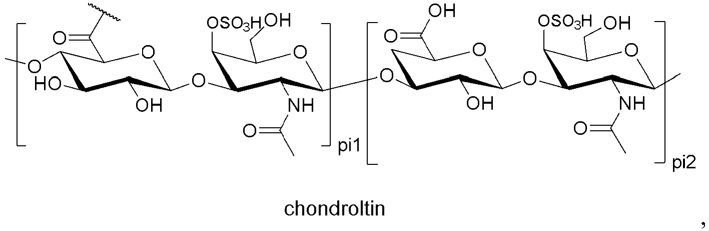 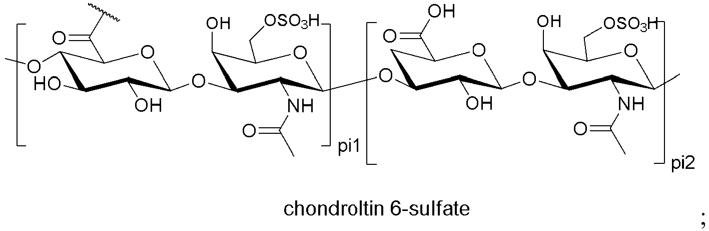 wherein, pf1, pf2, pg1, pg2, ph1, ph2, pi1, and pi2 are integers from 1 to 250.

In some embodiments, the polysaccharide polymer is selected from: 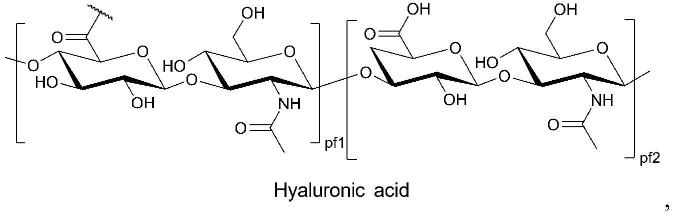 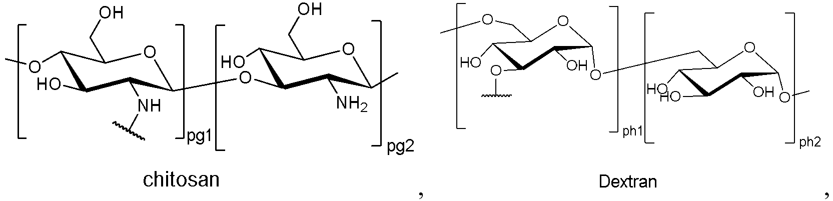 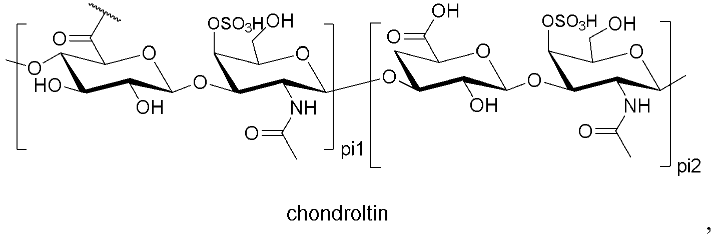 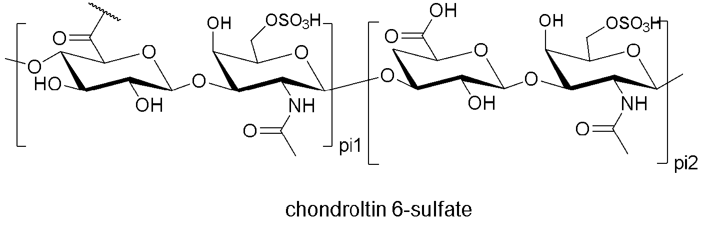 and 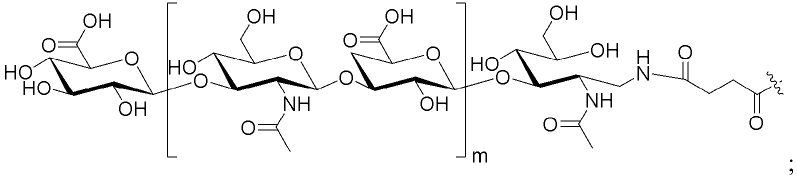 wherein, pf1, pf2, pg1, pg2, ph1, ph2, pi1, pi2, and m are each independently integers from 1 to 250, preferably integers from 1 to 50, for example integers from 1 to 5, integers from 4 to 24, or integers from 5 to 25.

In some embodiments, pf1 is an integer from 1 to 250, preferably an integer from 1 to 50, more preferably an integer from 1 to 20, for example an integer from 1 to 5.

In some embodiments, pf2 is an integer from 1 to 250, preferably an integer from 1 to 50, for example an integer from 4 to 24.

In some embodiments, m is an integer from 1 to 250, preferably an integer from 1 to 50, for example an integer from 5 to 25.

In some embodiments, the phospholipid drug conjugate having nano-size and comprising a stimuli-responsive linker, or a stereoisomer, prodrug, pharmaceutically acceptable salt, or pharmaceutically acceptable solvate thereof provided by the present invention is characterized in that the bioactive molecule fragment D is selected from: small molecule drugs, peptides, proteins, nucleic acids, sugars, specifically can be anesthetics, ophthalmic drugs, central nervous system drugs, pain therapeutic agents, anti-arthritis drugs, anticonvulsants, antihistamines, anti-ulcer drugs, behavioral correction drugs, respiratory drugs, antispasmodics, muscle relaxants, anti-inflammatory drugs, appetite suppressants, growth promoters, hemostatic agents, immunostimulants, immunomodulators, muscle relaxant drugs, obesity therapeutic agents, osteoporosis drugs, sedatives, tranquilizers, migraine therapeutic agents, muscle contractants, anti-infective drugs, antirheumatic drugs, antimalarial drugs, antiemetics, tracheal dilators, antithrombotic drugs, antihypertensive drugs, cardiovascular drugs, antiarrhythmic drugs, antioxidants, antiasthmatic drugs, diuretics, lipid regulators, antiandrogens, antiparasitic drugs, anticoagulants, antitumor drugs, hypoglycemic drugs, nutritional agents, growth supplements, anti-enteritis agents, vaccines, antibodies, radionuclides, diagnostic agents, and contrast agents; preferably, selected from small molecule drugs or peptide drugs; more preferably, selected from antitumor drugs, such as cytotoxic drugs, small molecule targeted drugs, hormones, biological response modifiers (e.g., immunomodulators), tumor adjuvant drugs, and radionuclide drugs.

In some embodiments, the phospholipid drug conjugate having nano-size and comprising a stimuli-responsive linker, or a stereoisomer, prodrug, pharmaceutically acceptable salt, or pharmaceutically acceptable solvate thereof provided by the present invention has a structure represented by formula (II): 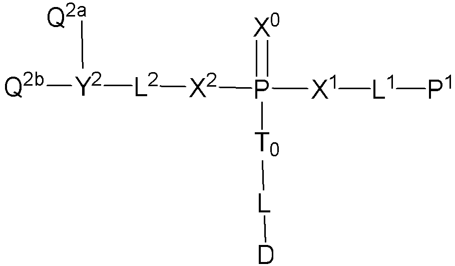 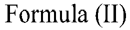
wherein, X⁰, X¹, L¹, P¹, X², L², Y², Q^{2a} Q^{2b}, Y², D are as defined in compound formula (I);
wherein T⁰ is present or absent, and when present, the connection point to the P end is selected from 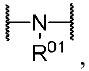 wherein R⁰¹ is hydrogen, C1-C10 alkyl, C1-C10 alkoxy, cycloalkyl, heterocycloalkyl, aryl, or heteroaryl;
L is L³-L^{s}, L³ is an environment-responsive linking unit, L^{s} is present or absent, when present it is a self-immolative linking moiety; when T⁰ is absent, L³ is directly bonded to the P end, and the functional group at the connection point is a nitrogen atom.

In some embodiments, the phospholipid drug conjugate having nano-size and a stimuli-responsive linker, or a stereoisomer, prodrug, pharmaceutically acceptable salt, or pharmaceutically acceptable solvate thereof provided by the present invention is characterized in that:
X⁰, X¹, X² are O;
L¹, L² are selected from: 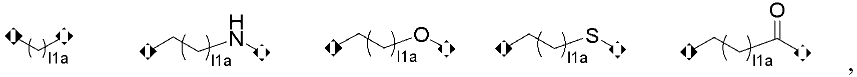 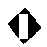 is the connection point to the P end, 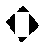 is the connection point to P¹ or Y²; l1a is an integer from 0 to 10, preferably an integer from 1 to 3;
Y² is selected from 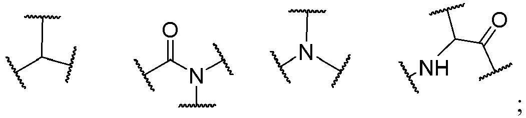 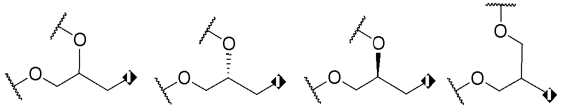
preferably, selected from 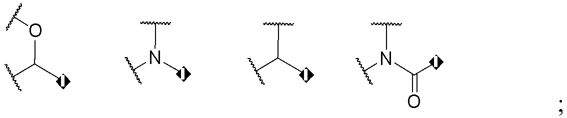
more preferably, selected from 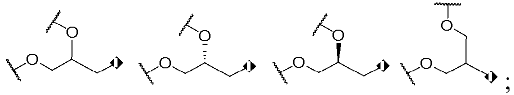 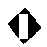 is the connection point to L²;
P¹ is selected from a polyethylene glycol derivative residue with a repeating unit number of pa1 and an end group of R^{p1}, wherein, pa1 is an integer from 10 to 60, preferably an integer from 15 to 50, for example 21, 22, 44, or 45; R^{p1} is C1-C6 alkyl, preferably C1-C3 alkyl, for example methyl, ethyl, n-propyl, or isopropyl;
or P¹ is selected from a polysarcosine derivative residue with a repeating unit number of pb1 and an end group of R^{p2}: 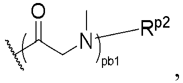
wherein, pb1 is an integer from 10 to 60, preferably an integer from 15 to 50; R^{p2} is selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or an amino protecting group;
Q^{2a} is T^{2a}-R¹, Q^{2b} is T^{2b}-R², T^{2a}, T^{2b} are as defined in compound formula (I), R¹, R² are selected from: 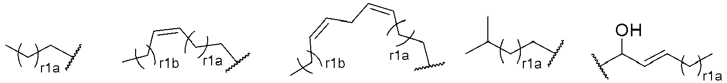 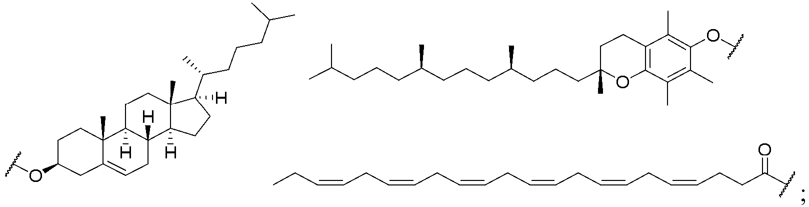
wherein, r1a, rlb are integers from 1 to 30.

In some embodiments, X⁰ is O.

In some embodiments, X¹ is O.

In some embodiments, X² is O.

In some embodiments, M⁰ is -T⁰-(L-D)ₙ, preferably -L-D, n is preferably 1.

In some embodiments, T⁰ is absent or present, when present, T⁰ is preferably selected from -NHCH₂- and 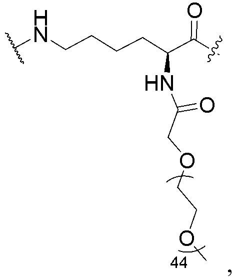 wherein the C end is preferably connected to L.

In some embodiments, when L is 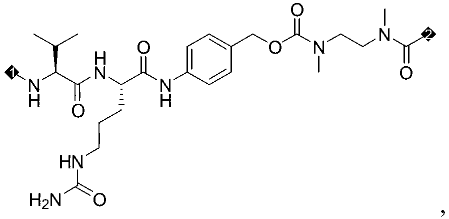 T⁰ is 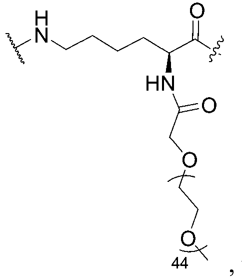 wherein the C end is connected to L.

In some embodiments, M² is absent. In some embodiments, L¹ is 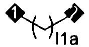 or 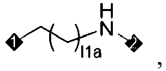 preferably 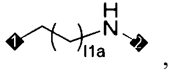 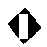 is connected to X¹, 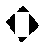 is connected to P¹, l1a is an integer from 0 to 10, preferably an integer from 0 to 5, for example 1 or 2.

In some embodiments, L² is 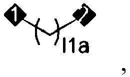 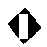 is connected to X¹, 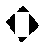 is connected to P¹; l1a is an integer from 0 to 10, preferably an integer from 0 to 5, for example 0 or 2.

In some embodiments, K¹ is P¹, P¹ is a pharmacokinetic modulating moiety unit, selected from a polyethylene glycol derivative residue, a polysarcosine derivative residue, a quaternary ammonium salt derivative residue, a zwitterionic derivative or zwitterionic polymer, and a polysaccharide polymer; preferably selected from a polyethylene glycol derivative residue, a polysarcosine derivative residue, a quaternary ammonium salt derivative residue, and a polysaccharide polymer; more preferably a polyethylene glycol derivative residue.

In some embodiments, the polyethylene glycol derivative residue is preferably

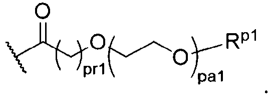

In some embodiments, pa1 is an integer from 5 to 250; preferably an integer from 5 to 150, for example 22, 45, or 113; more preferably an integer from 10 to 60, for example 45.

In some embodiments, pr1 is an integer from 0 to 8, preferably an integer from 0 to 2, for example 0.

In some embodiments, R^{p1} is hydrogen, C1-C10 alkyl, C1-C10 heteroalkyl, C3-C10 cycloalkyl, C3-C10 alkenyl, C3-C10 alkynyl, or a hydroxyl protecting group, preferably hydrogen, C1-C10 alkyl, or a hydroxyl protecting group, more preferably C1-C10 alkyl.

In some embodiments, the polysarcosine derivative residue is preferably

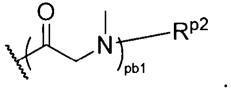

In some embodiments, pb1 is an integer from 5 to 250, preferably an integer from 5 to 150, more preferably an integer from 10 to 50, for example 28.

In some embodiments, R^{p2} is selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, and an amino protecting group, preferably an amino protecting group, for example -C(O)CH₃.

In some embodiments, the quaternary ammonium salt derivative residue is preferably

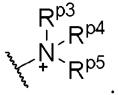

In some embodiments, R^{p3}, R^{p4}, R^{p5} are each independently selected from C1-C10 alkyl, C1-C10 alkoxy, C3-C8 cycloalkyl, or C2-C8 heterocycloalkyl, preferably C1-C10 alkyl, for example methyl.

In some embodiments, the polysaccharide polymer is preferably selected from: 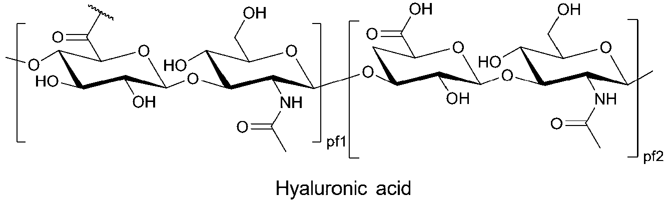 and 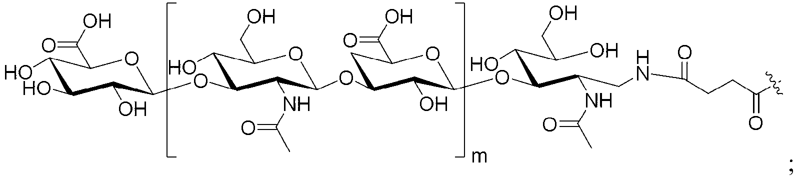 wherein, pf1, pf2, and m are each independently integers from 1 to 250.

In some embodiments, Y² is a branching center, selected from 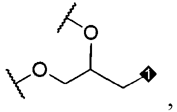 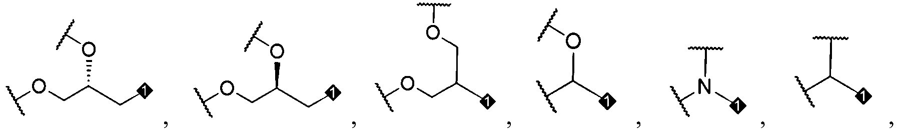 and 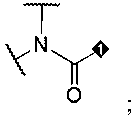 preferably selected from 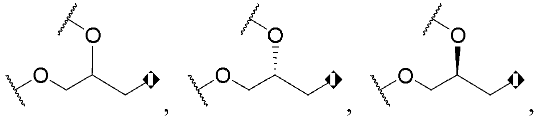 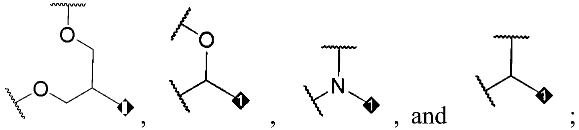 more preferably 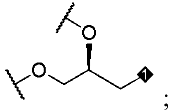 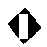 is the connection point to L².

In some embodiments, T^{2a}, T^{2b} are absent or present, when present, T^{2a}, T^{2b} are each independently selected from -C(O)-.

In some embodiments, R¹ and R² are each independently selected from 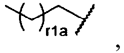 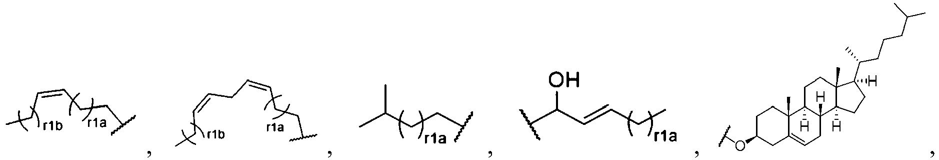 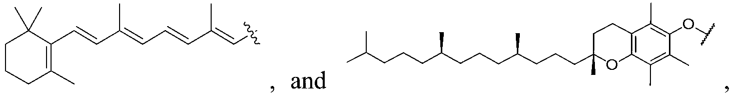 preferably selected from 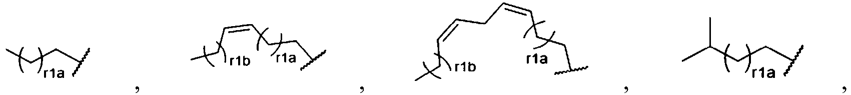 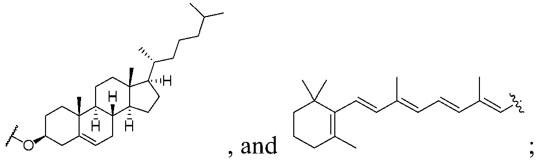 wherein, r1a, r1b are integers from 1 to 30, the 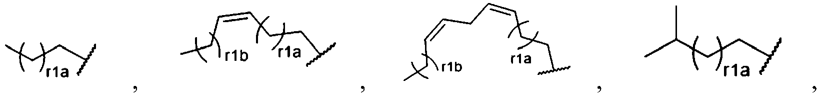 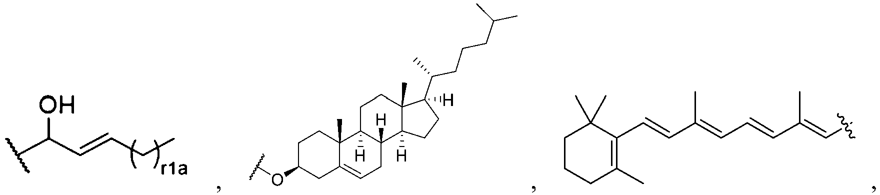 and 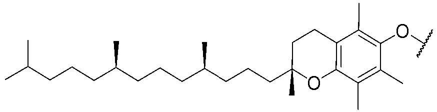 are optionally substituted by one or more substituents selected from hydroxyl, halogen, C₁₋₁₀ alkyl, C₁₋₁₀ alkoxy, C₃₋₁₂ cycloalkyl, and 3-12 membered heterocycloalkyl, the C₁₋₁₀ alkyl, C₁₋₁₀ alkoxy, C₃₋₁₂ cycloalkyl, and 3-12 membered heterocycloalkyl are optionally substituted by one or more substituents selected from hydroxyl, halogen, C₁₋₃ alkyl, C₁₋₃ alkoxy, and C₃₋₆ cycloalkyl.

In some embodiments, R¹ and R² are each independently preferably selected from 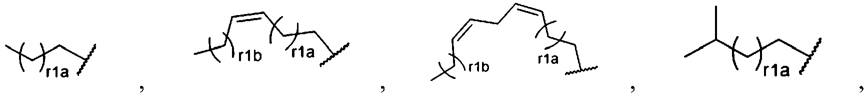 and 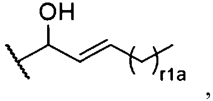 more preferably selected from 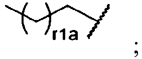 wherein, r1a, rlb are integers from 1 to 30, the 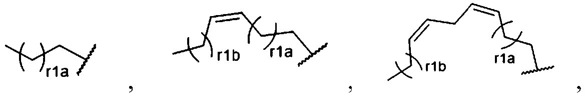 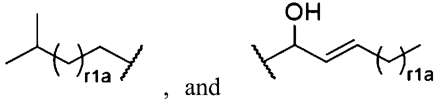 are optionally substituted by one or more substituents selected from hydroxyl, halogen, C₁₋₁₀ alkyl, C₁₋₁₀ alkoxy, C₃₋₁₂ cycloalkyl, and 3-12 membered heterocycloalkyl, the C₁₋₁₀ alkyl, C₁₋₁₀ alkoxy, C₃₋₁₂ cycloalkyl, and 3-12 membered heterocycloalkyl are optionally substituted by one or more substituents selected from hydroxyl, halogen, C₁₋₃ alkyl, C₁₋₃ alkoxy, and C₃₋₆ cycloalkyl.

In some embodiments, R¹ and R² are each independently selected from 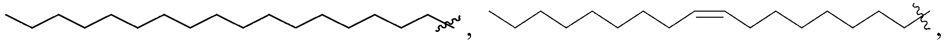 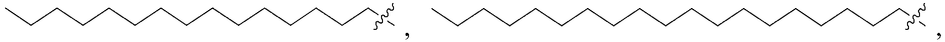 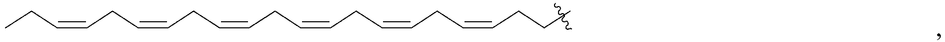 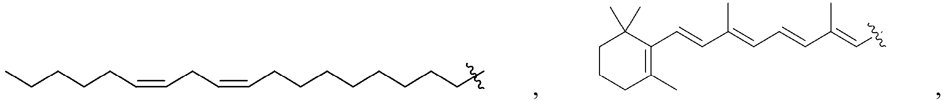 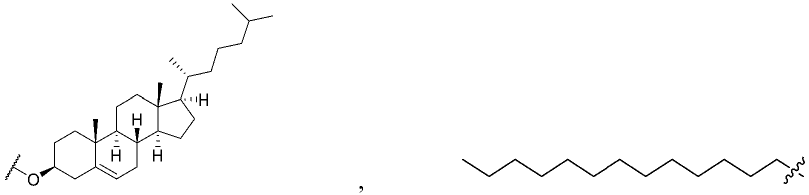 and 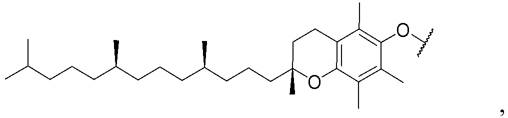 preferably selected from 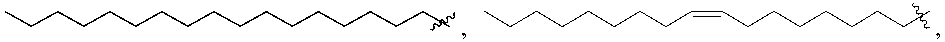 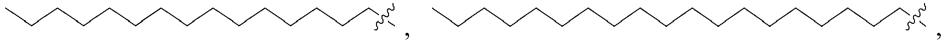 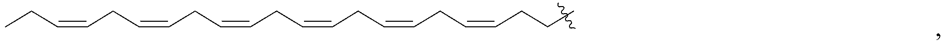 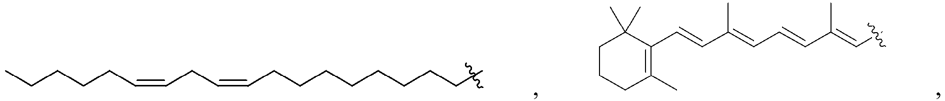 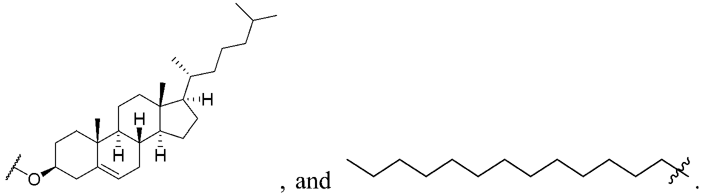 In some embodiments, R¹ and R² are each independently preferably selected from 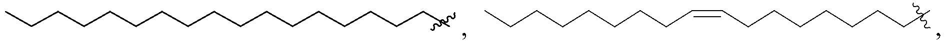 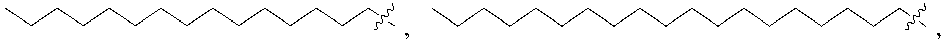 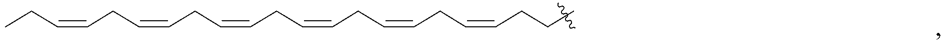 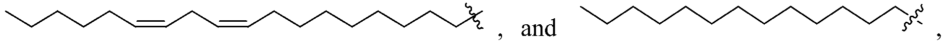 more preferably

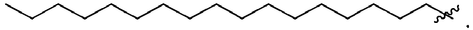

In some embodiments, Q^{2a} and Q^{2b} are each independently selected from 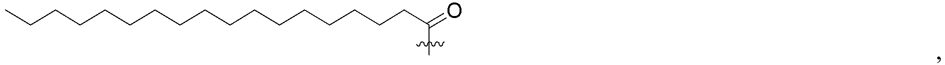 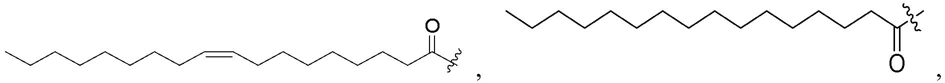 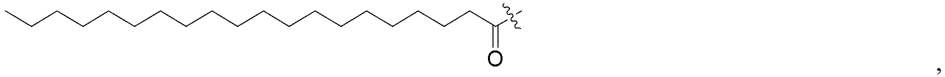 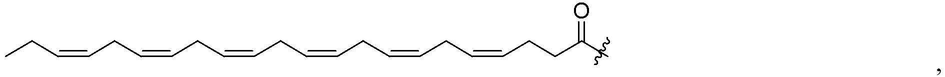 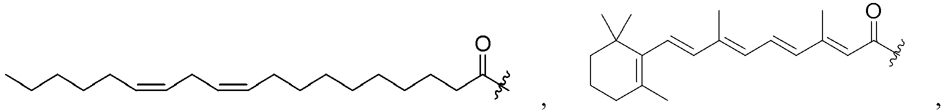 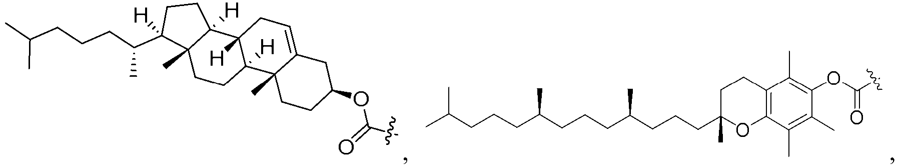 and 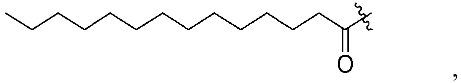 preferably selected from

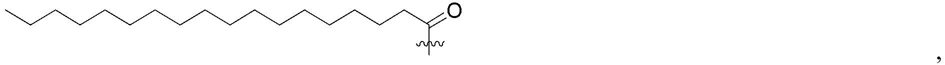

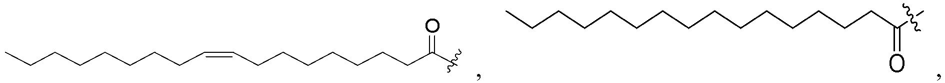

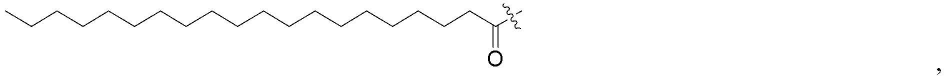

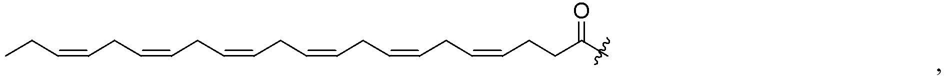

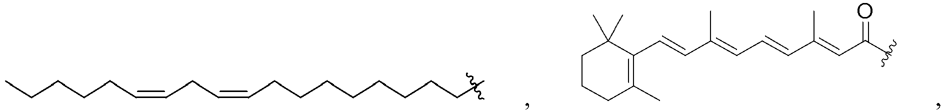

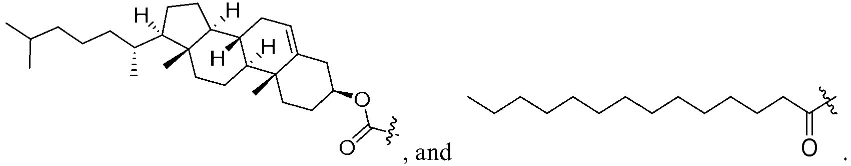

In some embodiments, Q^{2a} and Q^{2b} are each independently preferably selected from 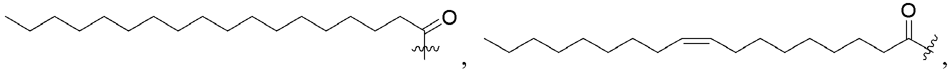 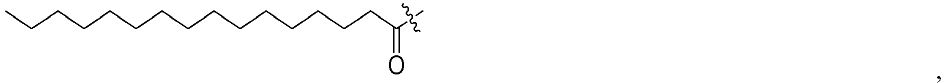 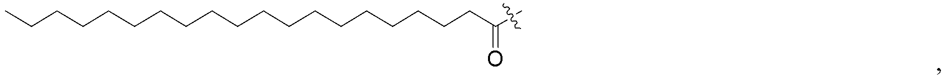 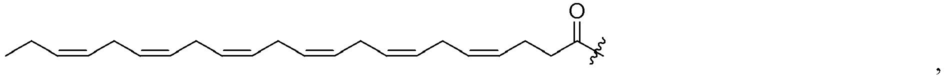 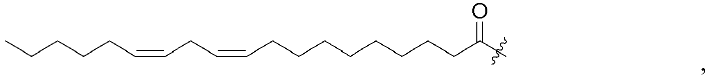 and 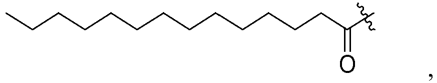 more preferably selected from

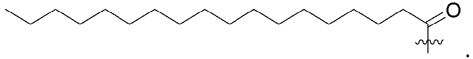

In some embodiments, the phospholipid drug conjugate having nano-size and comprising a stimuli-responsive linker, or a stereoisomer, prodrug, pharmaceutically acceptable salt, or pharmaceutically acceptable solvate thereof provided by the present invention is characterized in that the bioactive molecule fragment D is selected from:
(1) Cytotoxic drugs:
   Topoisomerase inhibitor camptothecins as shown in formula (III): 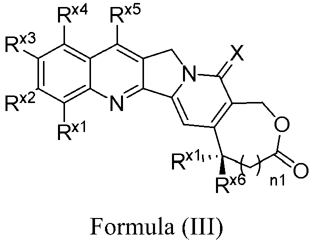
   wherein, **R^{x1}** is selected from C1-C4 alkyl, C1-C4 haloalkyl, C1-C4 cycloalkyl, C1-C4 deuterated alkyl, C2-C4 alkynyl, or C2-C4 alkenyl;
   **R^{x2}**, **R^{x3}**, **R^{x4}**, and **R^{x5}** are each independently selected from hydrogen atom, deuterium atom, halogen, cyano, hydroxyl, mercapto, sulfonyl, sulfinyl, substituted or unsubstituted amino, nitro, alkynyl, alkenyl, alkyl, haloalkyl, cycloalkyl, alkoxy, alkylthio, heterocycloalkyl, deuterated alkyl, aryl, or heteroaryl;
   or, **R^{x1}** and **R^{x2}**, **R^{x2}** and **R^{x3}**, **R^{x3}** and **R^{x4}**, **R^{x4}** and **R^{x5}** together with the carbon atom to which they are commonly attached form a 5-7 membered cycloalkyl or heterocycloalkyl;
   X is selected from O or S;
   **Z** is selected from OH or F;
   **n1** is selected from 0 or 1;
   Preferably, selected from the following structures: 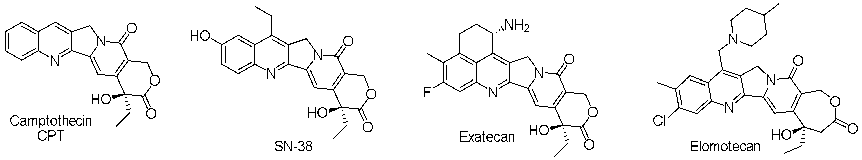 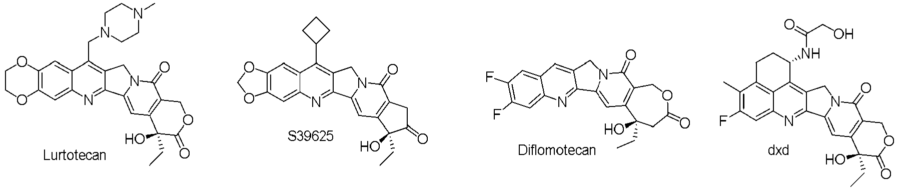 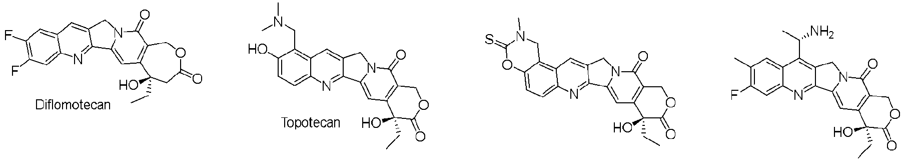 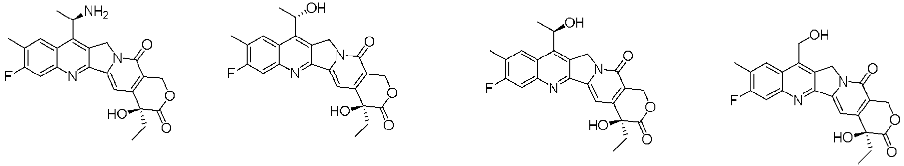 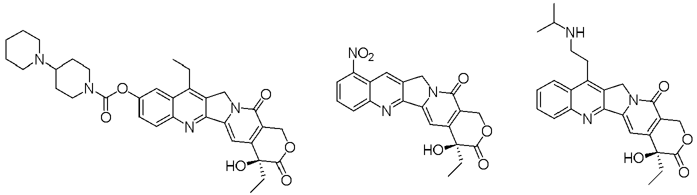 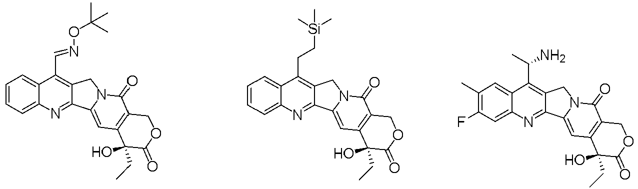
   tubulin inhibitors, preferably, selected from: 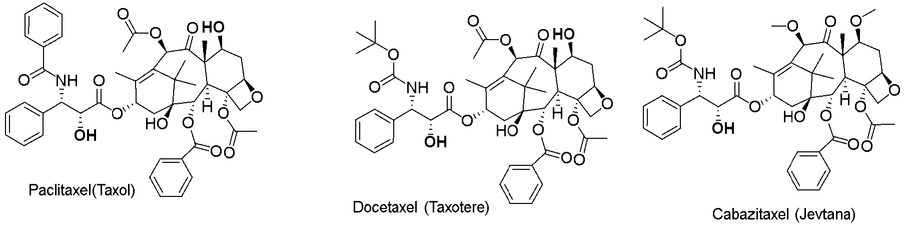 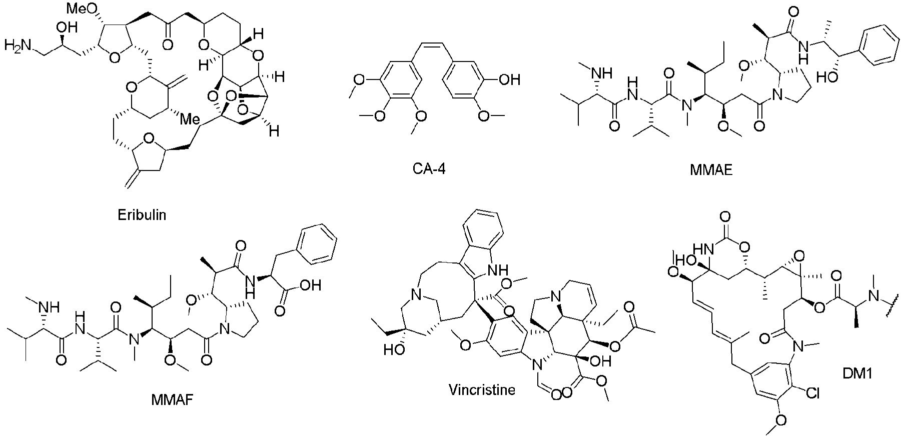
   nucleic acid transcription inhibitors, preferably, selected from: 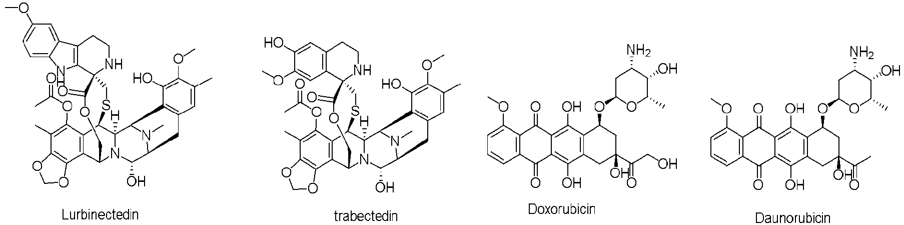 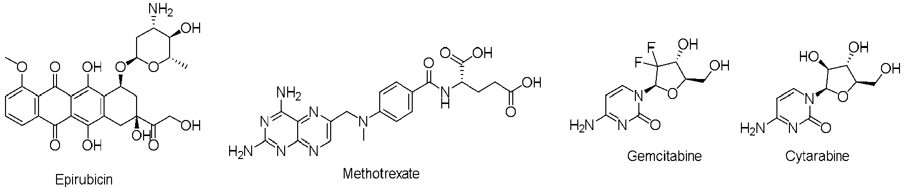 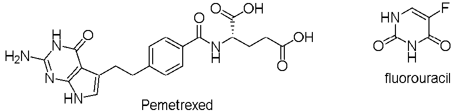
   drugs acting on DNA structure, preferably, selected from: 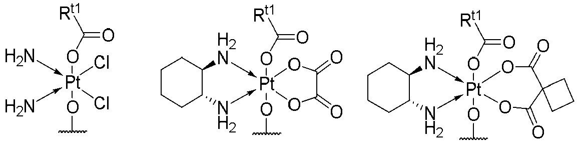 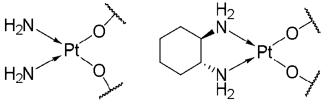 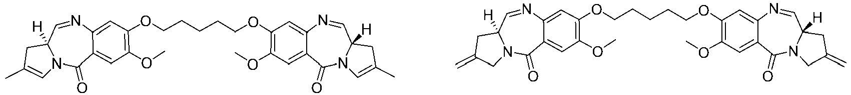 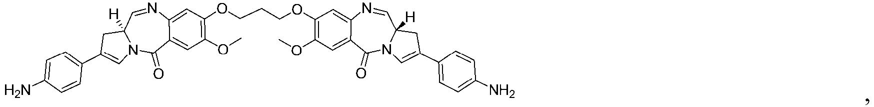
   wherein, Rt1 is selected from hydrogen, alkyl, alkoxy, heteroalkyl, aryl, or heteroaryl;
(2) small molecule targeted drugs:
   EGFR inhibitors, for example: gefitinib, erlotinib, icotinib, afatinib, or osimertinib;
   HER2 inhibitors, for example: lapatinib, pyrotinib, neratinib, or tucatinib;
   ALK inhibitors, for example: crizotinib, alectinib, ceritinib, lorlatinib, or brigatinib;
   MEK inhibitors, for example: trametinib, cabozantinib, or binimetinib;
   MET inhibitors, for example: savolitinib, volitinib, or capmatinib;
   BRAF inhibitors, for example: sorafenib, dabrafenib, vemurafenib, or encorafenib; CDK4/6 inhibitors, for example: palbociclib, ribociclib, abemaciclib, or 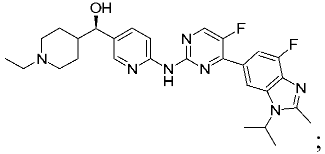
   PARP inhibitors, for example: olaparib, niraparib, rucaparib, or talazoparib;
   BTK inhibitors, for example: acalabrutinib, ibrutinib, tirabrutinib, or zanubrutinib;
   Protease inhibitors, for example: bortezomib, carfilzomib, or ixazomib;
   mTOR inhibitors, for example: temsirolimus or everolimus;
   JAK inhibitors, for example: ruxolitinib, tofacitinib, upadacitinib, baricitinib, deucravacitinib, peficitinib, filgotinib, abrocitinib, pacritinib, or deuterated decernotinib;
   PI3K inhibitors, for example: idelalisib, copanlisib, duvelisib, or umbralisib;
   VEGFR inhibitors, for example: sorafenib, axitinib, apatinib, sunitinib, regorafenib, vandetanib, pazopanib, lenvatinib, cabozantinib, ponatinib, aflibercept, or fruquintinib;
   PDGFR, c-Kit inhibitors, for example: imatinib, nilotinib, or avapritinib;
   BCL inhibitors, for example: venetoclax;
   HDAC inhibitors, for example: vorinostat, romidepsin, belinostat, panobinostat, or chidamide;
   FLT3 inhibitors, for example: midostaurin, gilteritinib, or quizartinib;
   RET inhibitors, for example: selpercatinib or pralsetinib;
   KRAS inhibitors, for example: sotorasib or adagrasib;
   IDH inhibitors, for example: enasidenib, ivosidenib, or olutasidenib;
   BCR-ABL inhibitors, for example: imatinib, dasatinib, nilotinib, ponatinib, or olverembatinib;
(3) hormonal molecules, for example fulvestrant, abiraterone, prednisone, dexamethasone, bicalutamide, enzalutamide, tamoxifen, toremifene, letrozole, exemestane, goserelin, or leuprorelin;
(4) immunotherapeutic molecules, for example small molecule PD-L1 inhibitors, TLR agonists, stimulator of interferon genes (STING) agonists, or indoleamine-2,3-dioxygenase (IDO) inhibitors;
(5) other small molecules and polypeptides, for example lenalidomide, thalidomide, pomalidomide, iberdomide, octreotide, lanreotide, or pasireotide.

In some embodiments, the drugs acting on DNA structure are selected from: 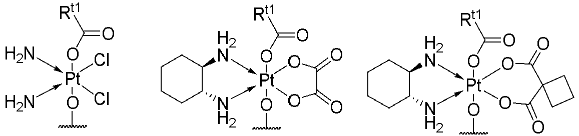 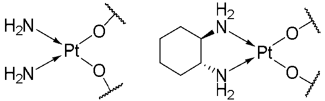 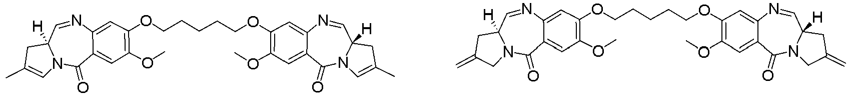 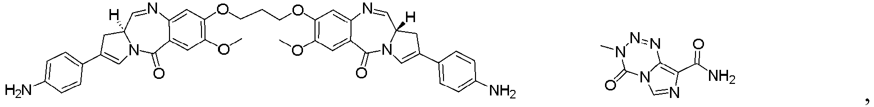 wherein, Rt1 is selected from hydrogen, alkyl, alkoxy, heteroalkyl, aryl, or heteroaryl.

In some embodiments, the bioactive molecule fragment D is preferably selected from cytotoxic drugs, small molecule targeted drugs, hormonal molecules, and other small molecules and polypeptides.

In some embodiments, the cytotoxic drugs are preferably selected from topoisomerase inhibitor camptothecins as shown in formula (III), tubulin inhibitors, and drugs acting on DNA structure, more preferably selected from topoisomerase inhibitor camptothecins as shown in formula (III) and tubulin inhibitors;

The topoisomerase inhibitor camptothecins as shown in formula (III) are preferably selected from:

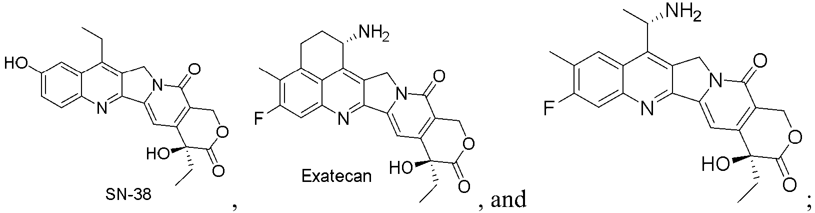

The tubulin inhibitors are preferably

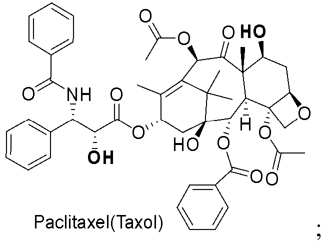

The drugs acting on DNA structure are preferably 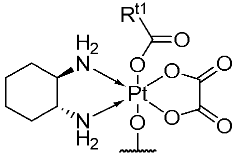 and 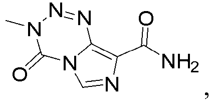 wherein, Rt1 is selected from hydrogen, alkyl, alkoxy, heteroalkyl, aryl, and heteroaryl.

In some embodiments, the small molecule targeted drugs are preferably selected from ALK inhibitors, CDK4/6 inhibitors, and PARP inhibitors; the ALK inhibitors are preferably crizotinib; the CDK4/6 inhibitors are preferably selected from palbociclib and 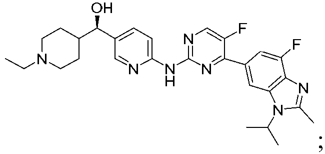 the PARP inhibitors are preferably niraparib.

In some embodiments, the hormonal molecules are preferably fulvestrant.

In some embodiments, the other small molecules and polypeptides are preferably octreotide.

In some embodiments, D is selected from the following structures: 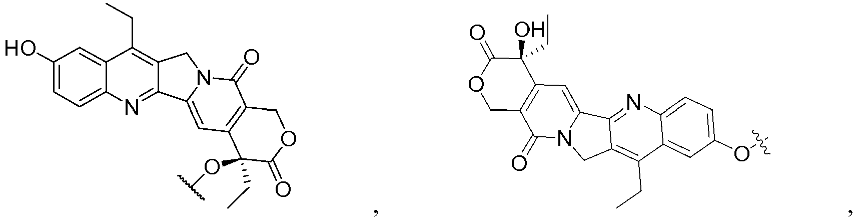 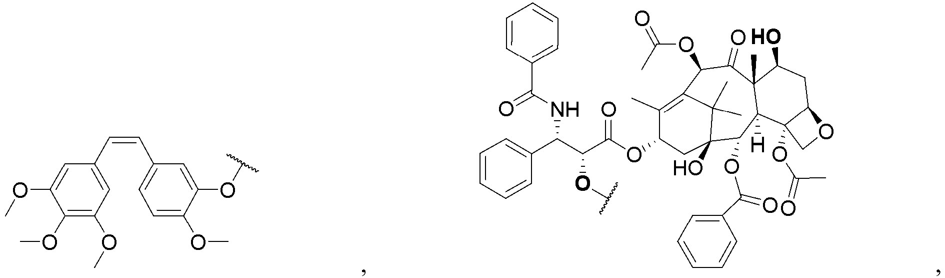 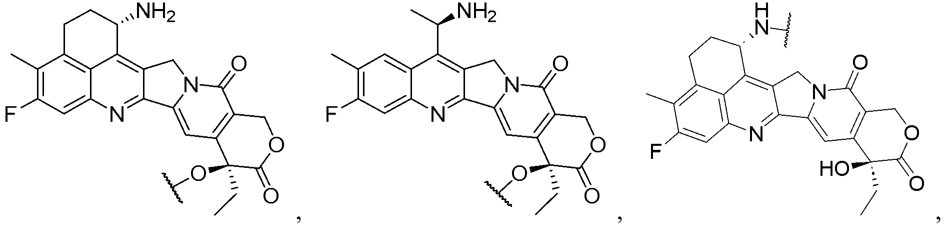 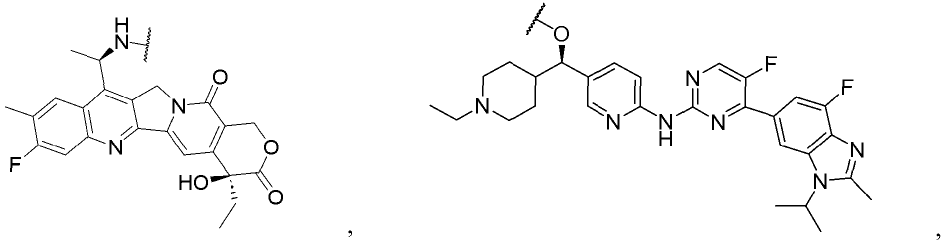 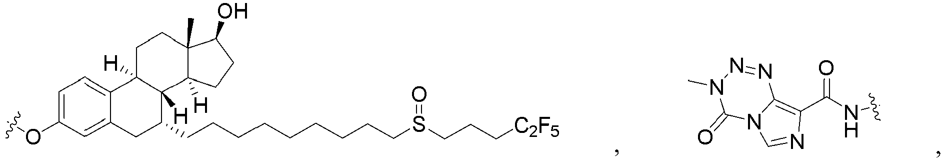 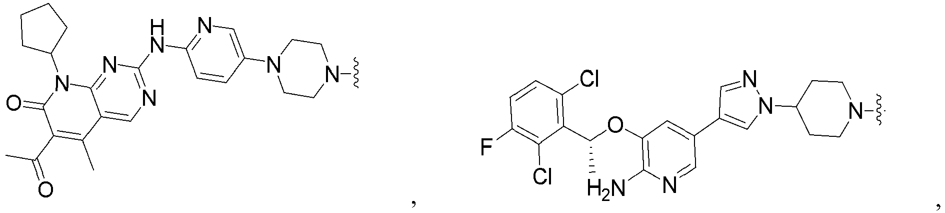 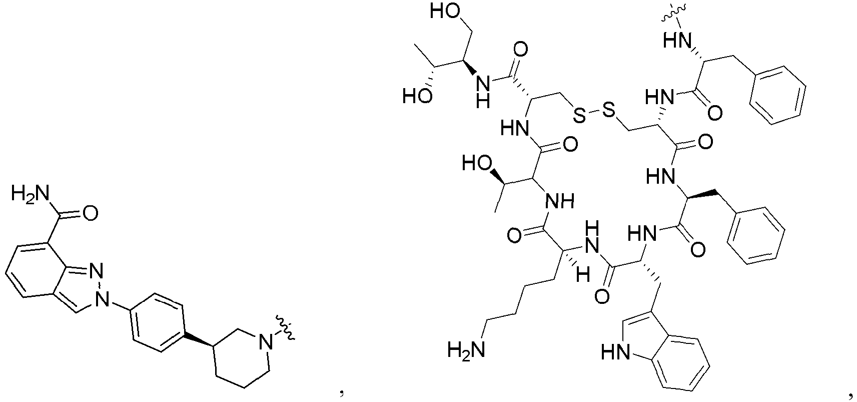 and 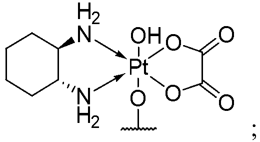 preferably selected from 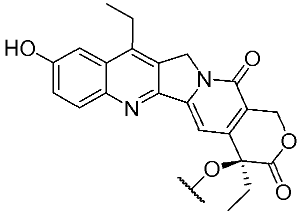 and

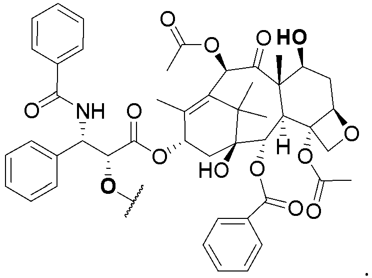

In some embodiments, L is selected from the following structures (the connection site to D is indicated by 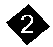 ): 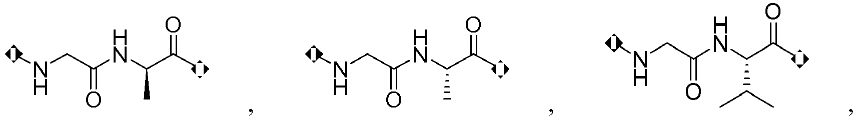 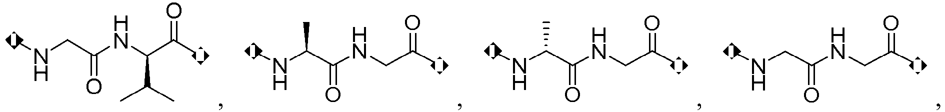 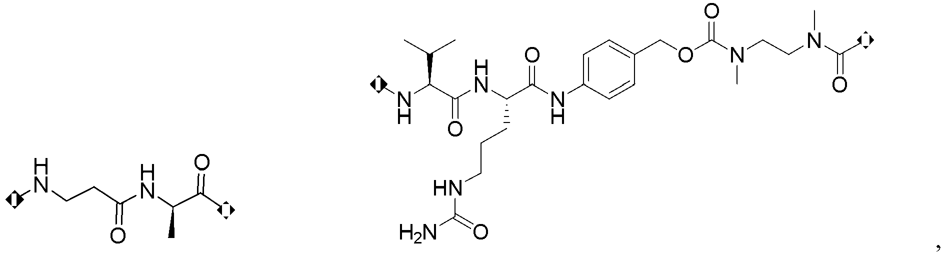 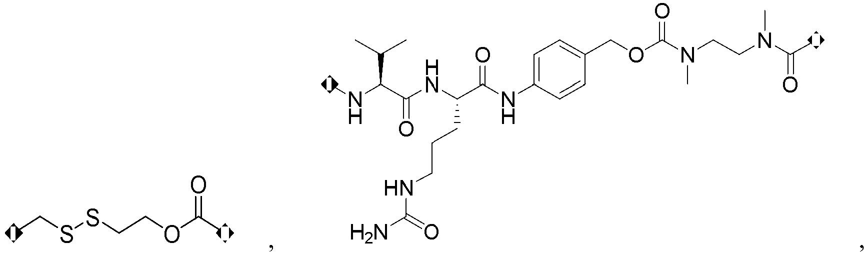 and 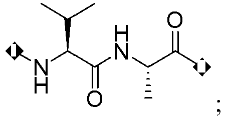 preferably selected from 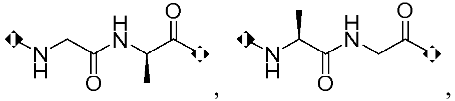 and

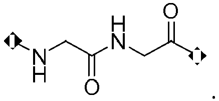

In some embodiments, when L is 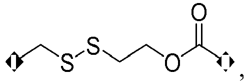 T⁰ is -NHCH₂-, wherein the C-terminus is connected to L.

In some embodiments, when L is 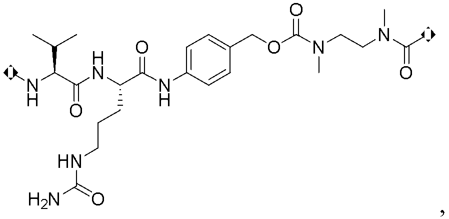 T⁰ is 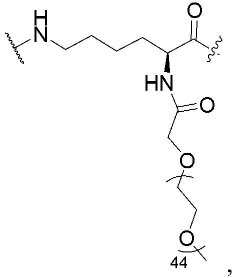 wherein the C-terminus is connected to L.

In some embodiments, the phospholipid drug conjugate having nano-size and comprising a stimuli-responsive linker, or a stereoisomer, prodrug, pharmaceutically acceptable salt, or pharmaceutically acceptable solvate thereof provided by the present invention is characterized in that L-D is selected from any one of the following conditions:
(1) When the drug molecule D is the following structure, wherein the connection site to L is indicated by 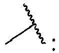 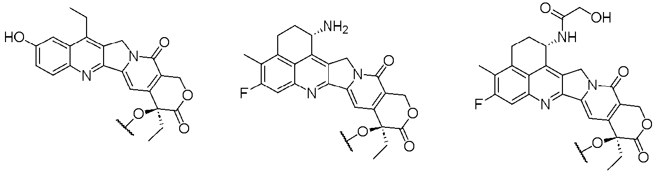 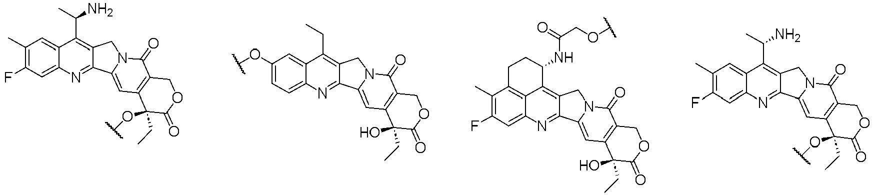 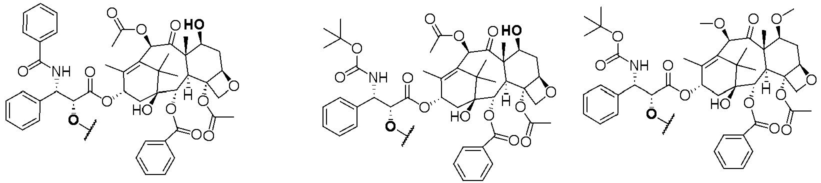 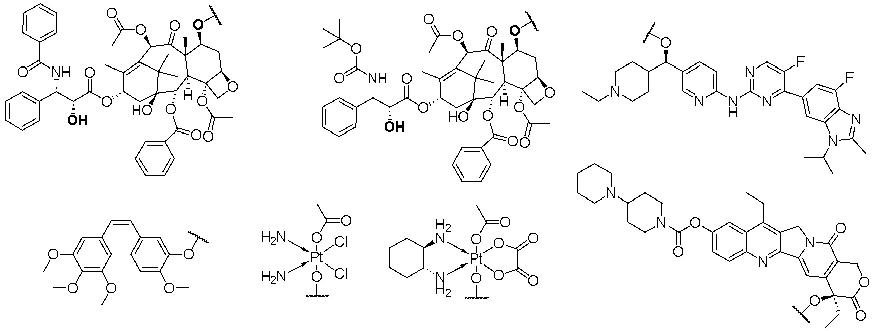 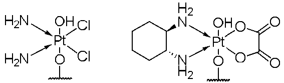 L is selected from the following structures (the connection site to drug D is indicated by 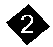 ): 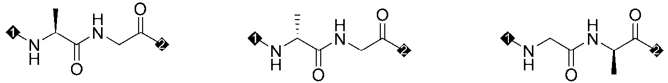 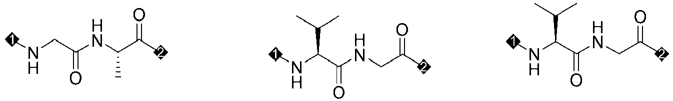 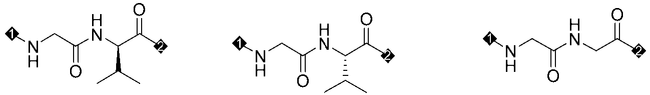 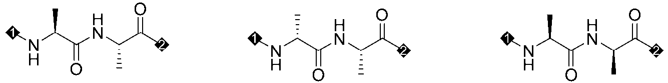 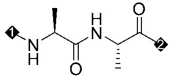 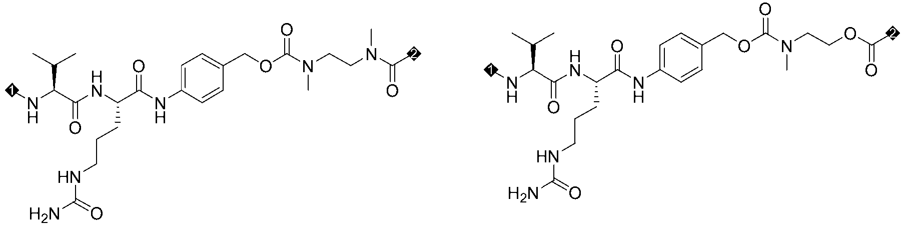 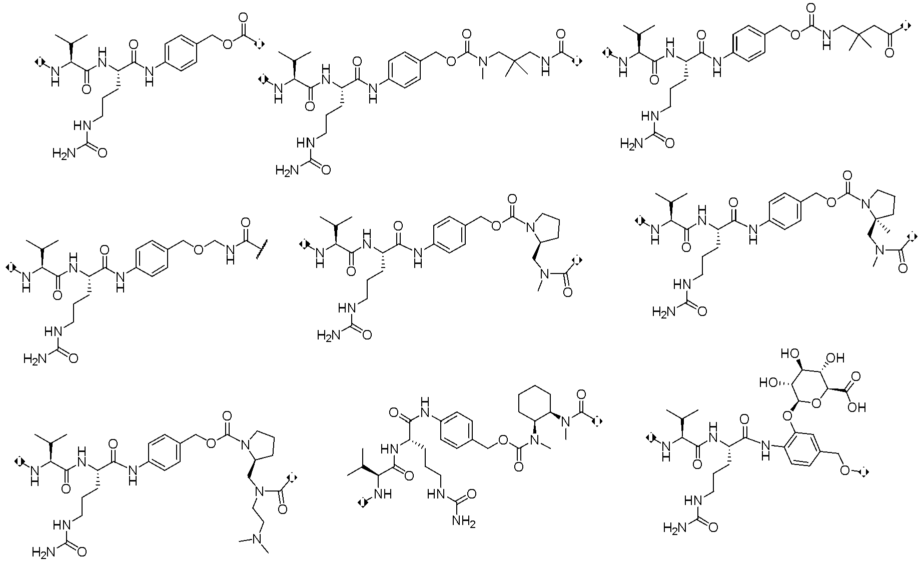 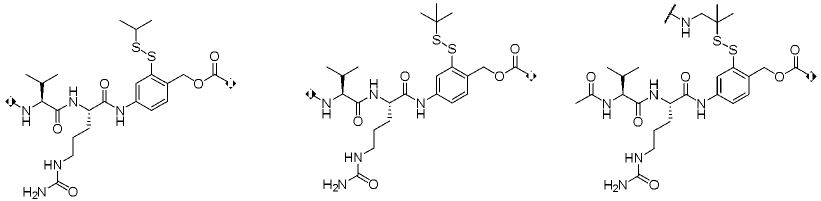 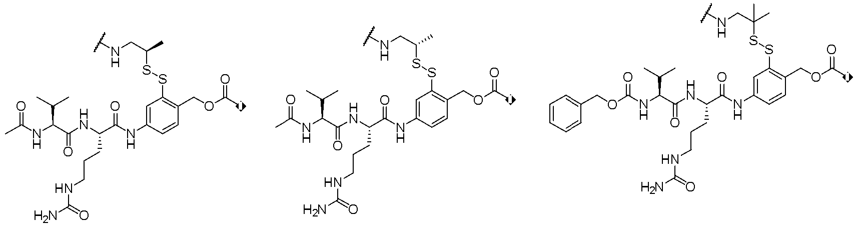 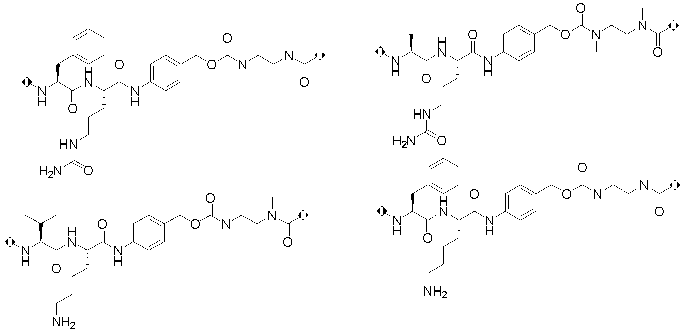 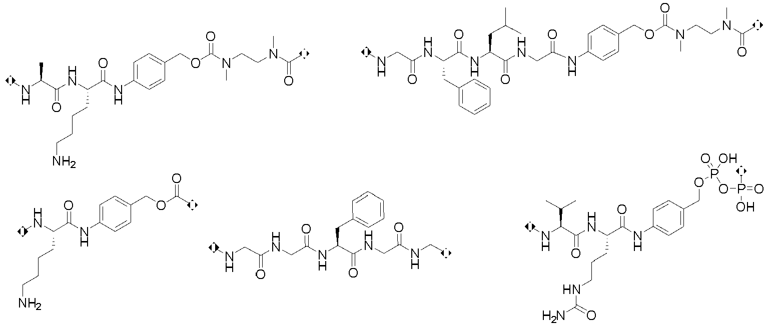 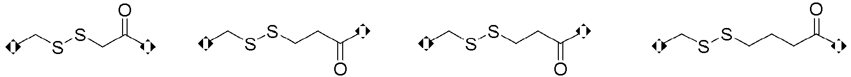 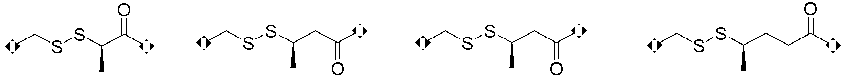 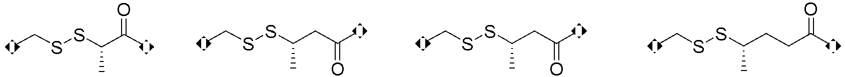 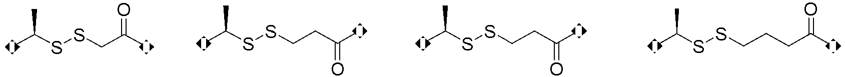 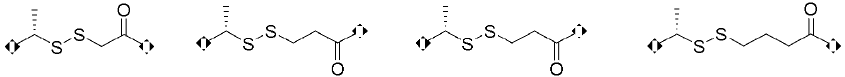 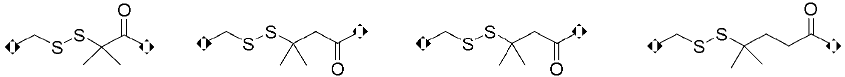 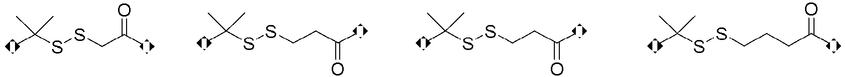 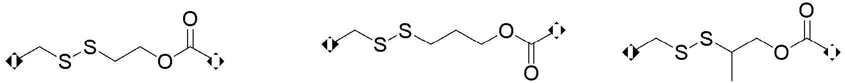 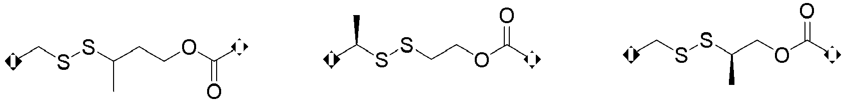 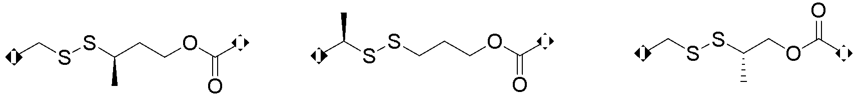 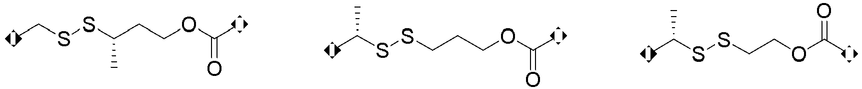 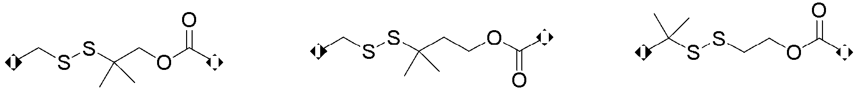 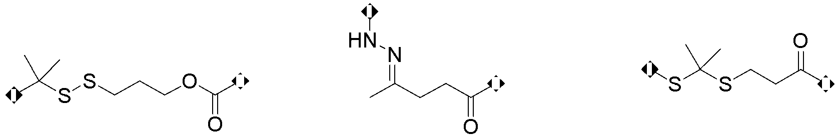 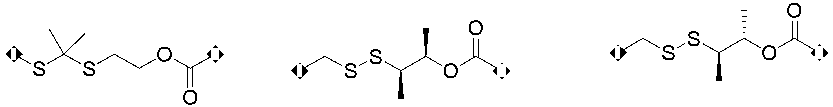 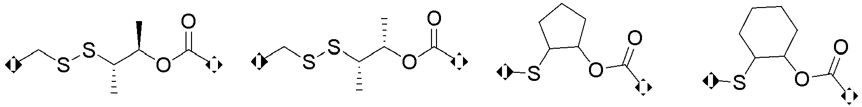 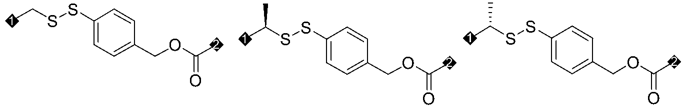 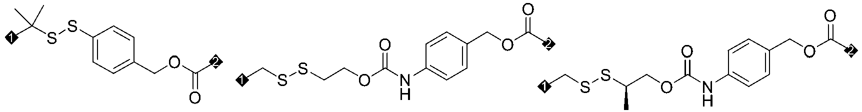 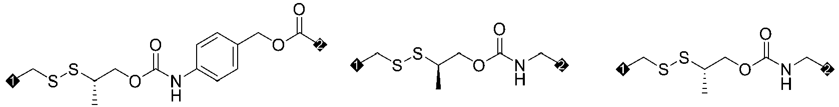 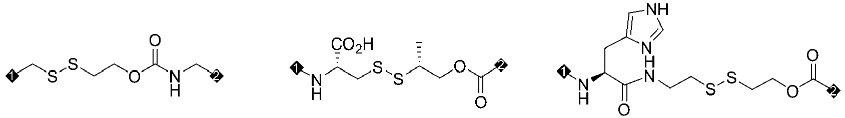
(2) When the drug molecule D is the following structure, wherein the connection site to L is indicated by 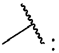 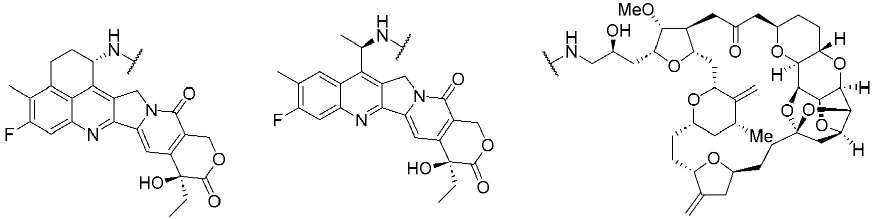 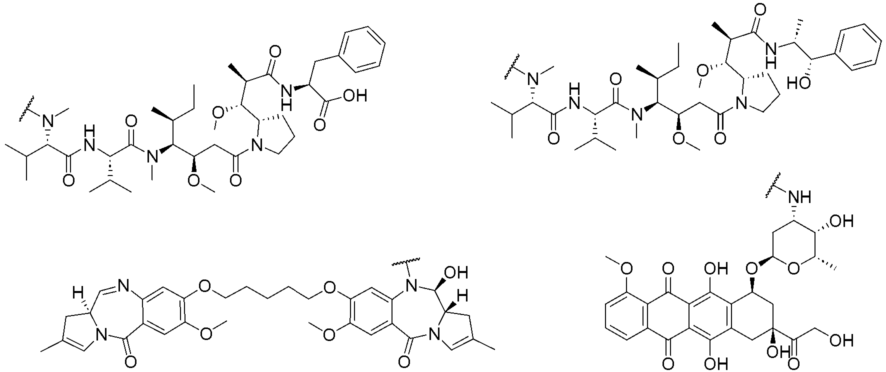 L is selected from the following structures (the connection site to drug D is indicated by 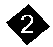 ):

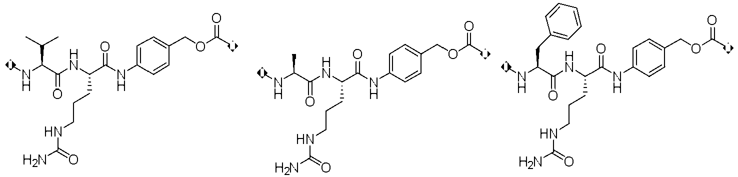

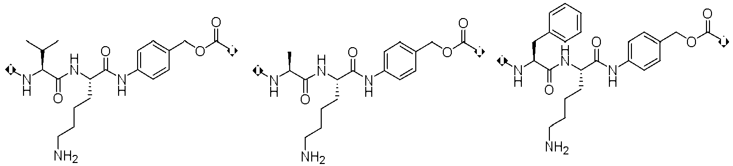

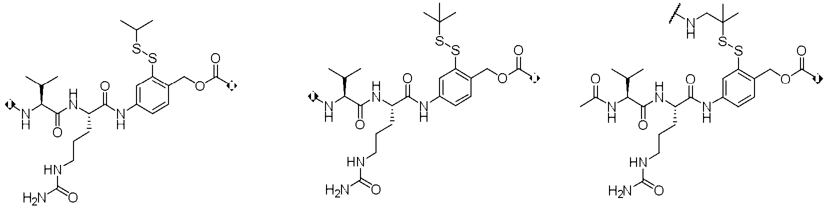

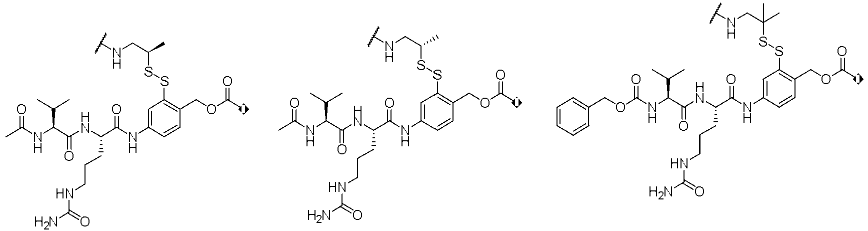

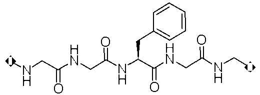

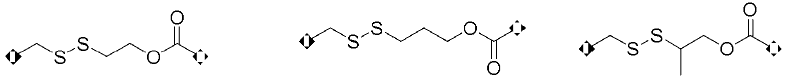

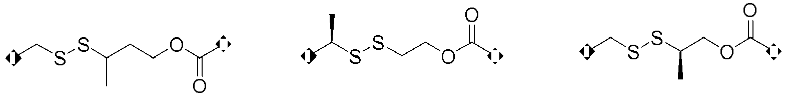

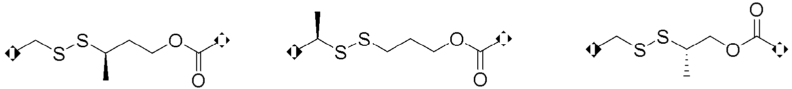

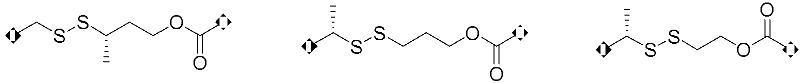

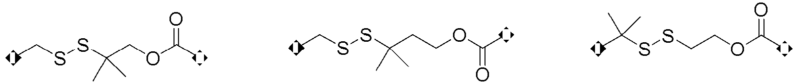

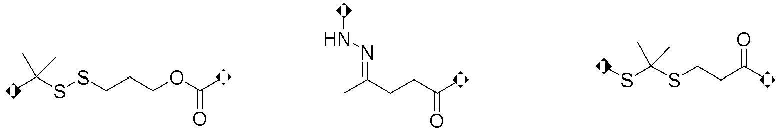

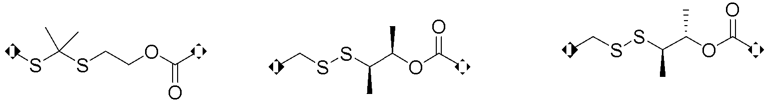

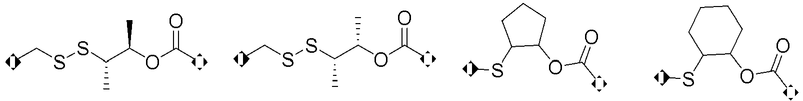

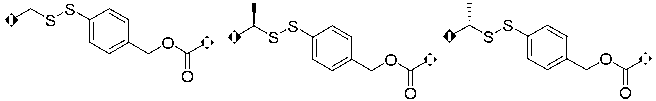

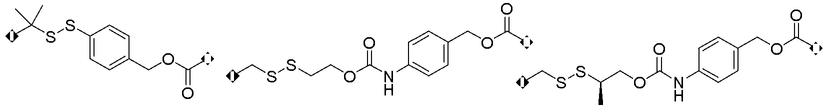

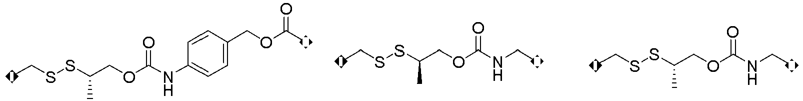

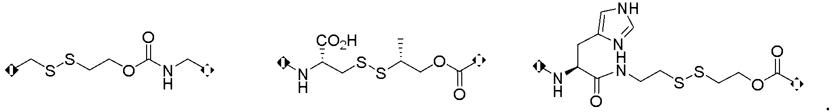

In some embodiments, the phospholipid drug conjugate having nano-size and comprising a stimuli-responsive linker, or a stereoisomer, prodrug, pharmaceutically acceptable salt, or pharmaceutically acceptable solvate thereof provided by the present invention has the structure shown in formula (IV): 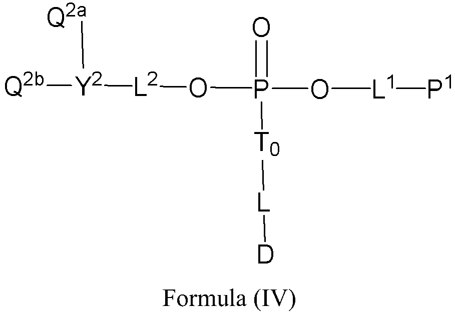 wherein,
(1) 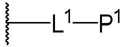 is selected from the following structures: 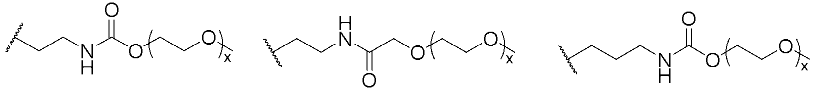 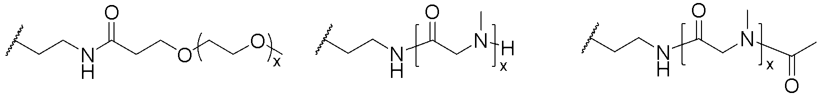 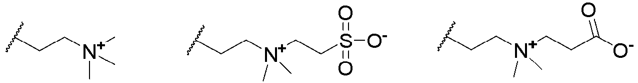 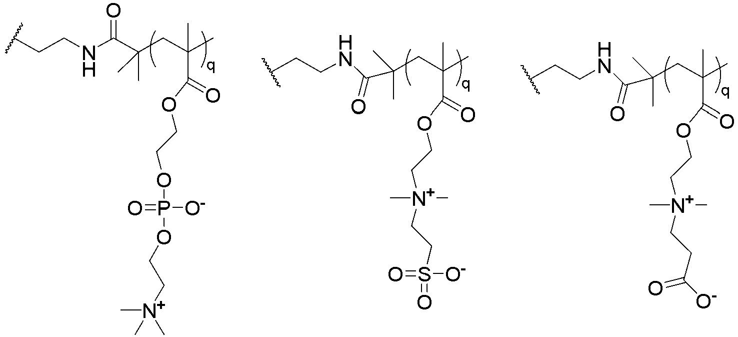 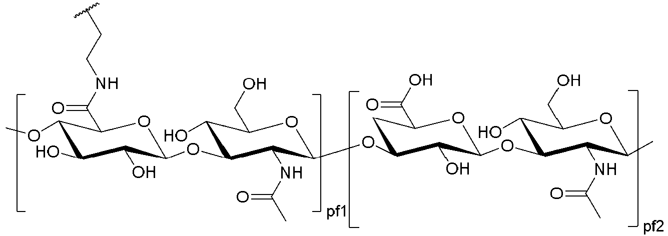 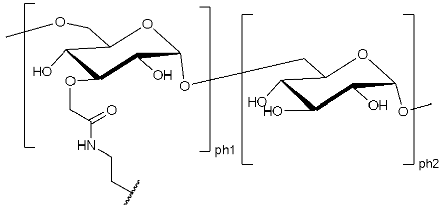 wherein, x, q are integers from 10 to 60; pf1, pf2, ph1, ph2 are integers from 1 to 100;
(2) 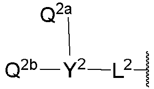 is selected from the following structures: 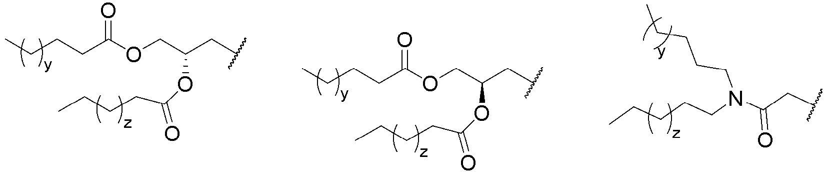 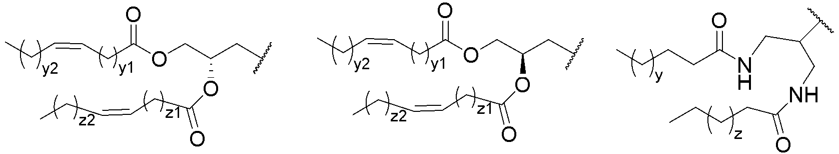 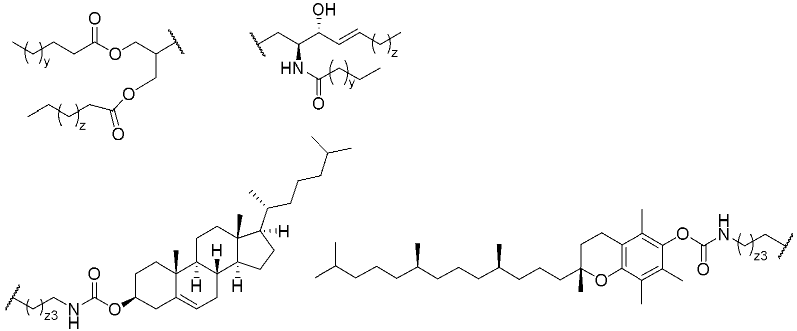 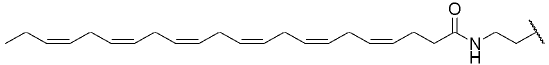 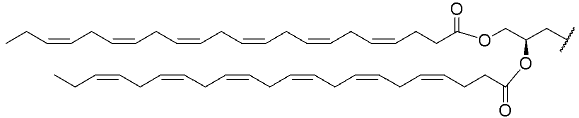 wherein, y, z, y1, y2, z1, z2, z3 are integers from 1 to 20;
(3) 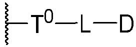 is selected from the following structures:

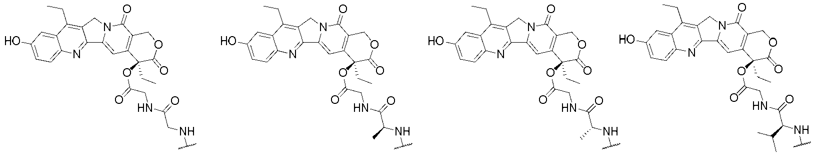

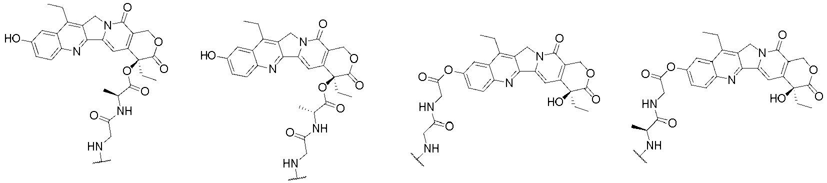

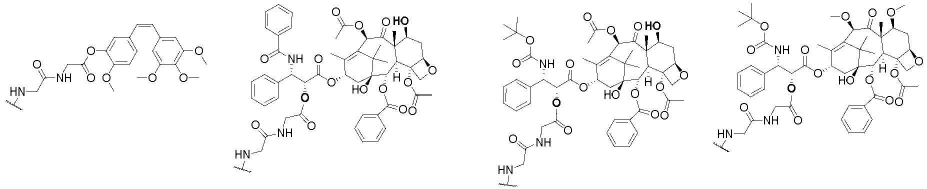

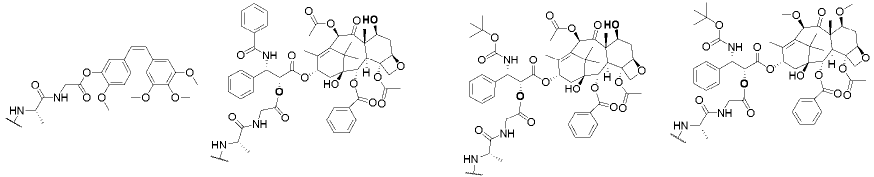

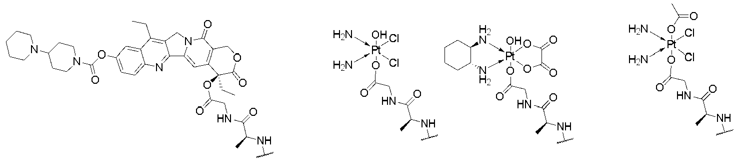

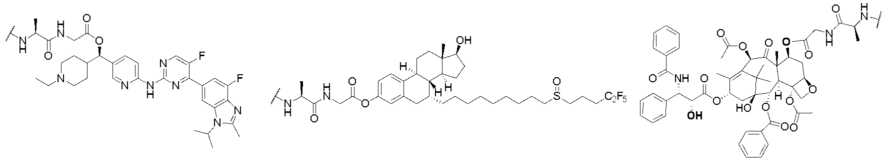

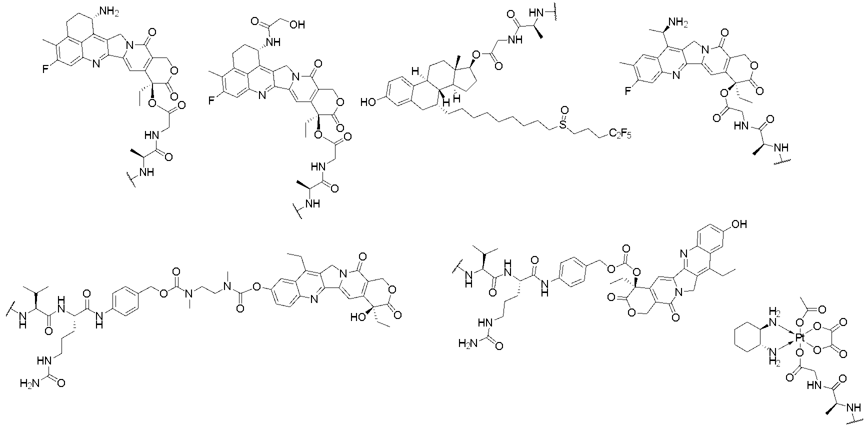

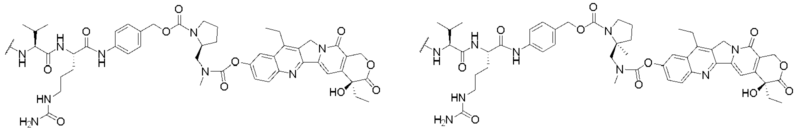

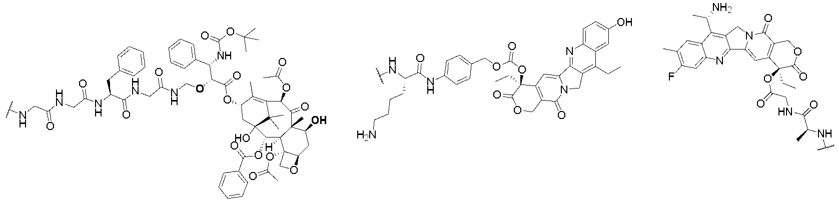

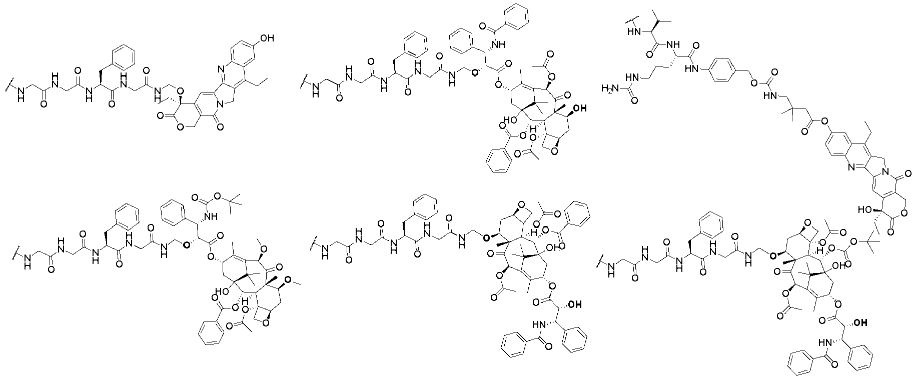

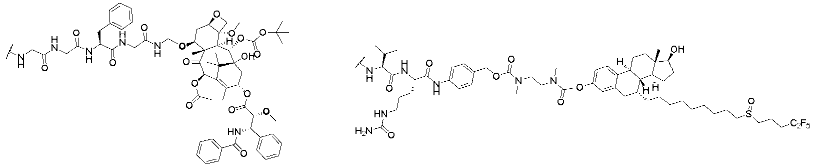

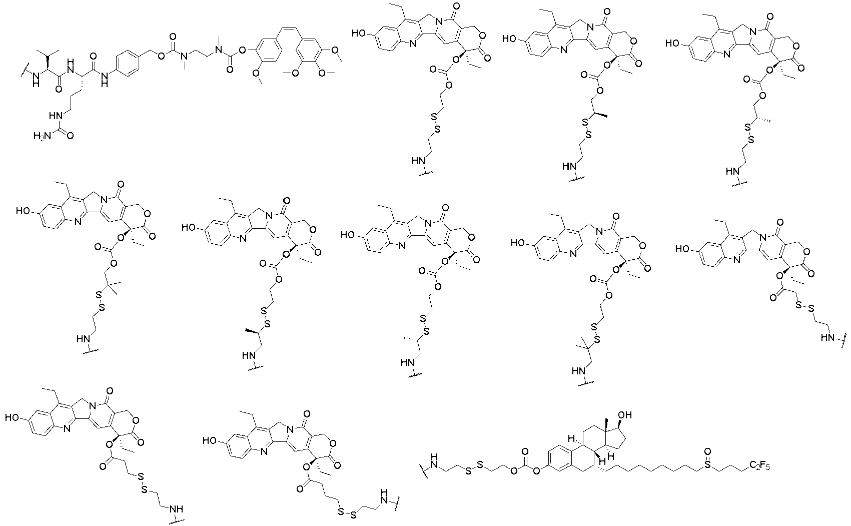

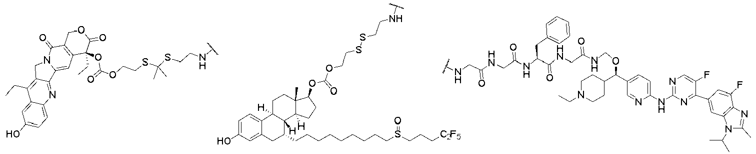

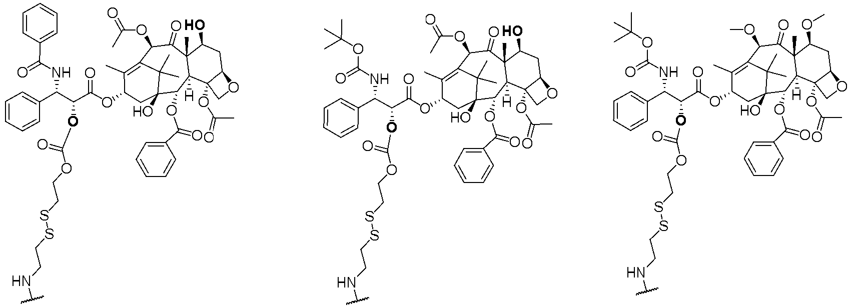

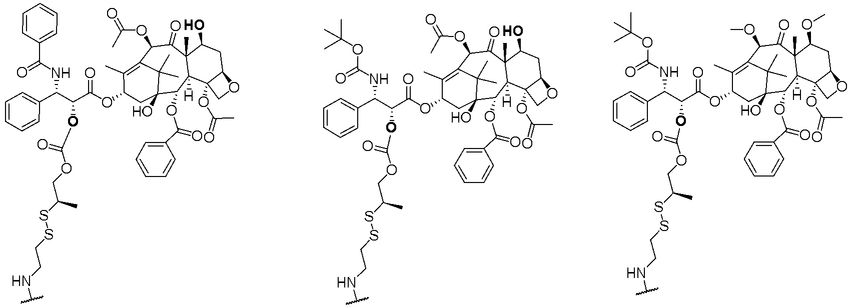

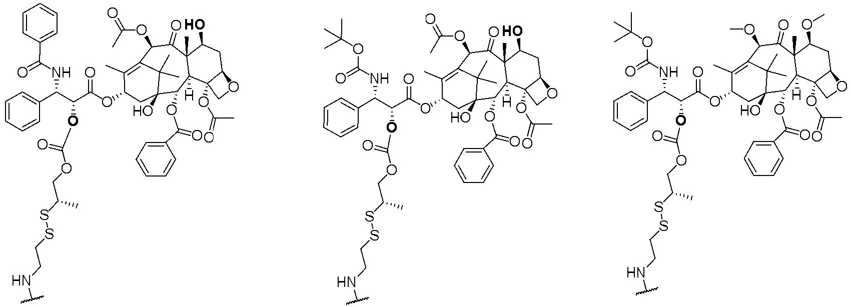

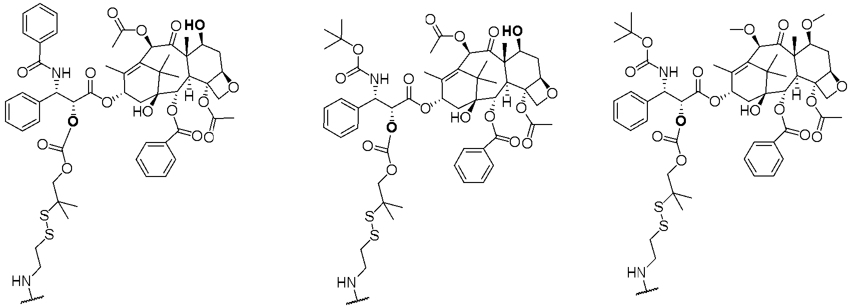

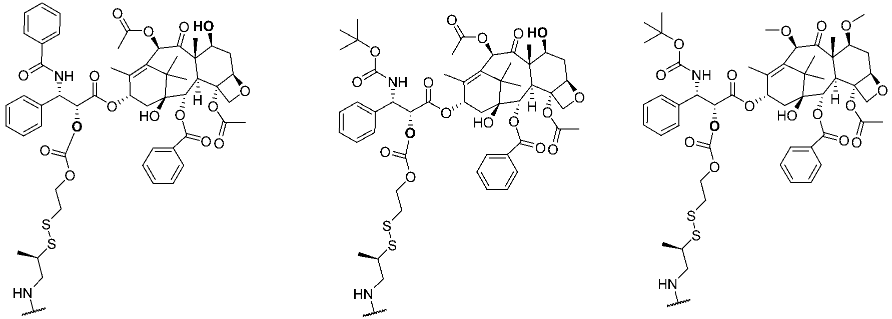

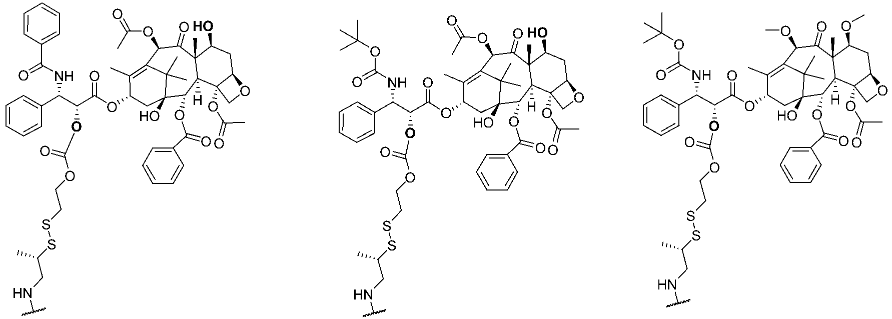

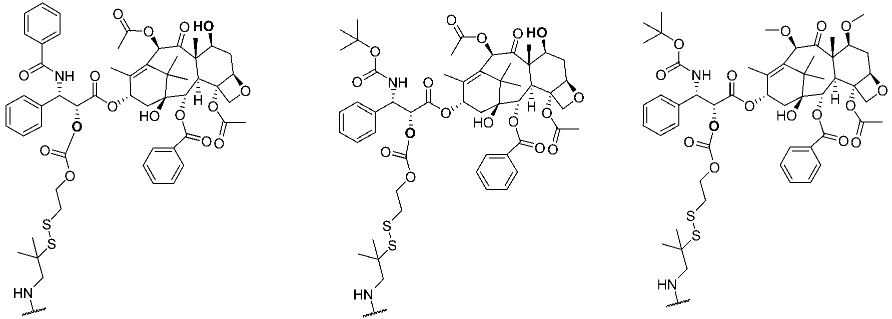

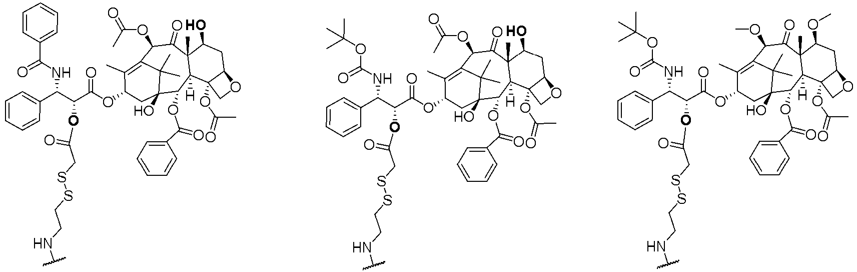

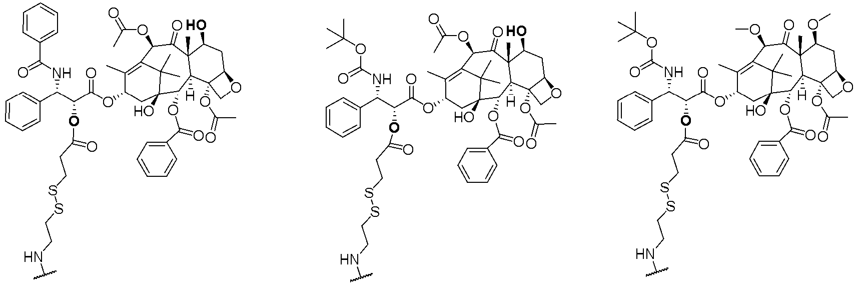

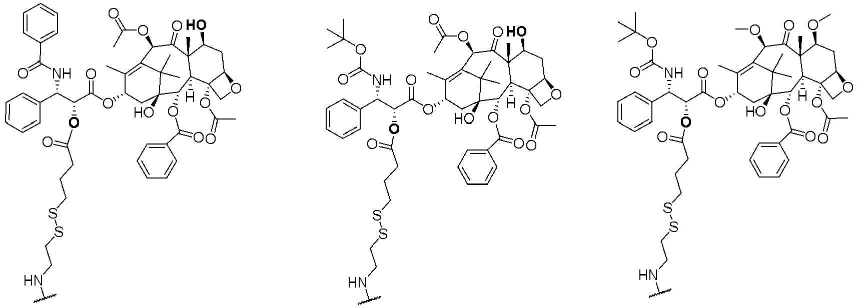

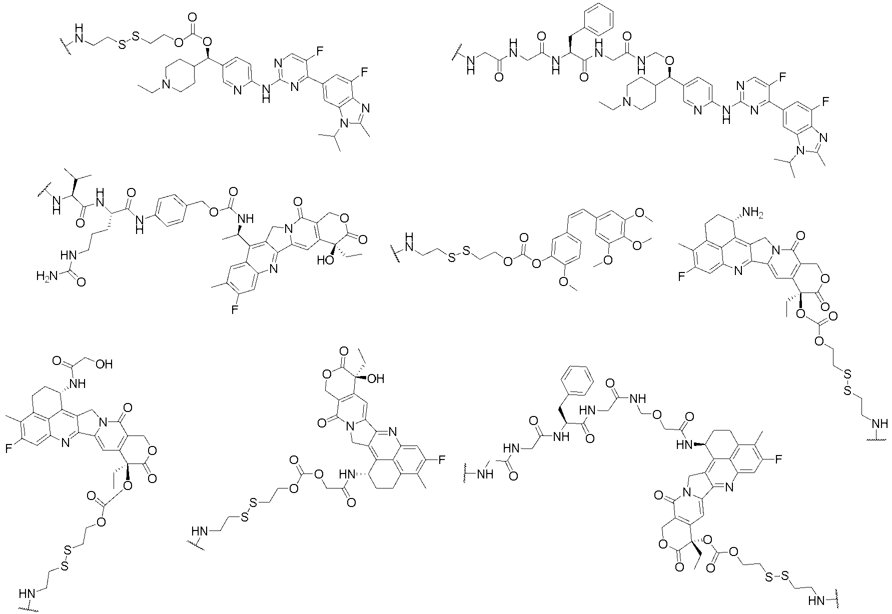

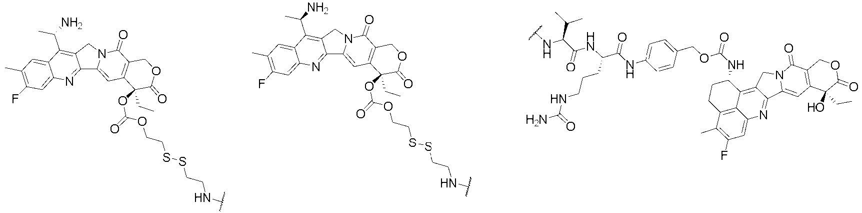

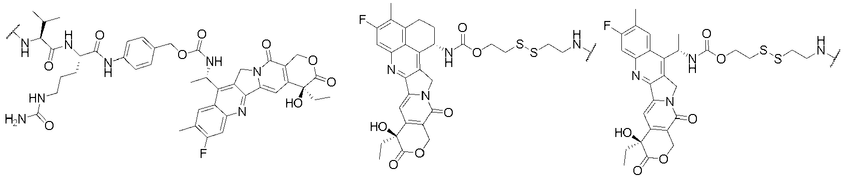

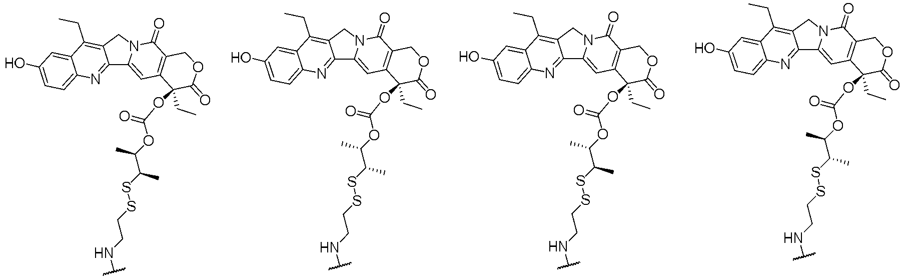

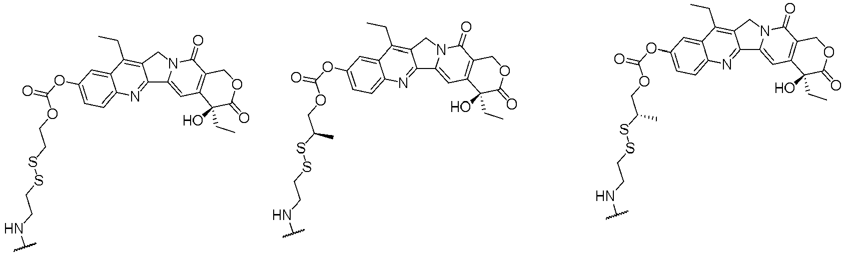

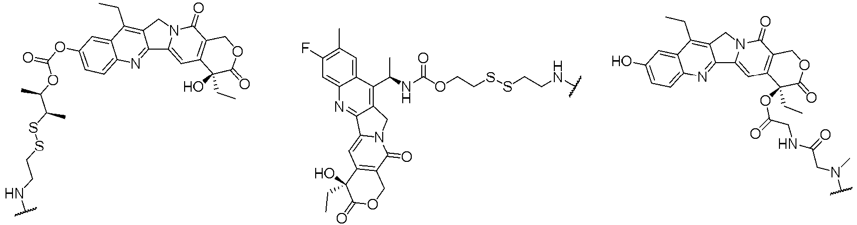

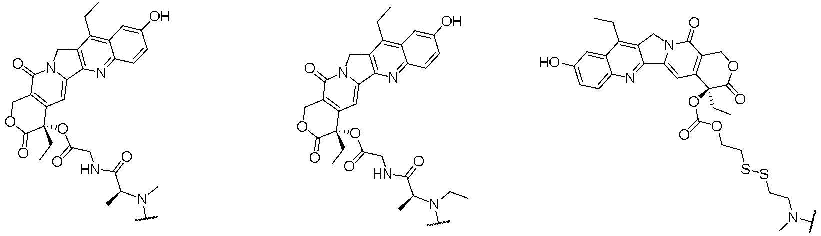

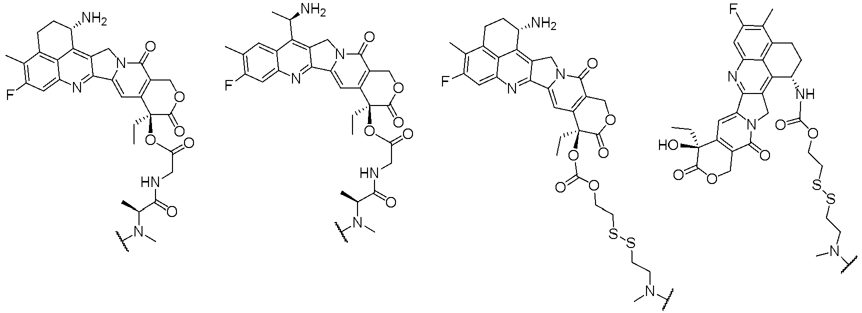

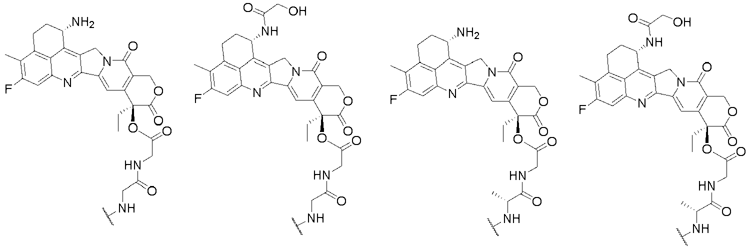

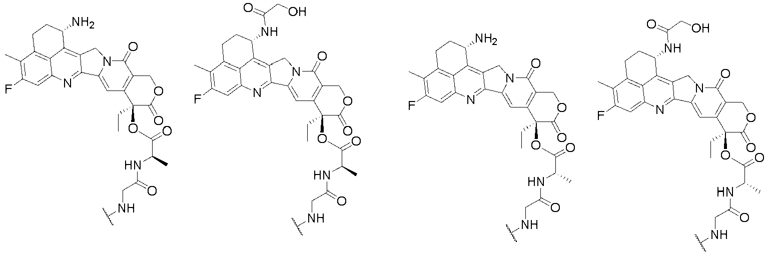

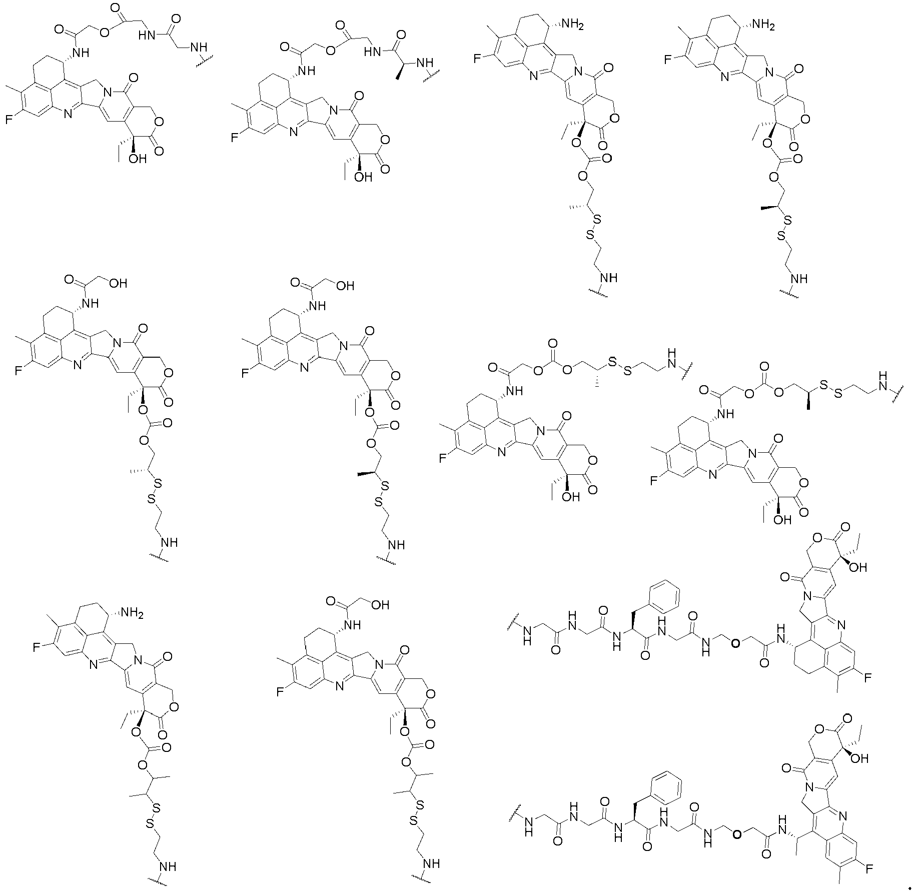

In some embodiments, 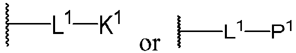 is selected from the following structures: 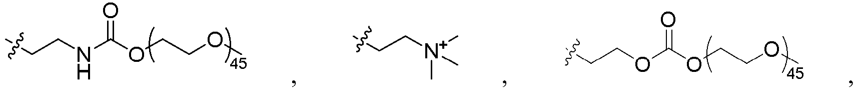 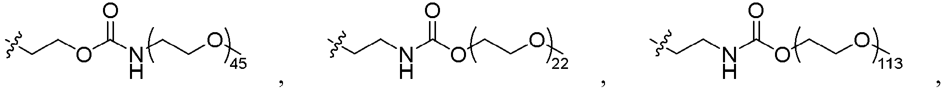 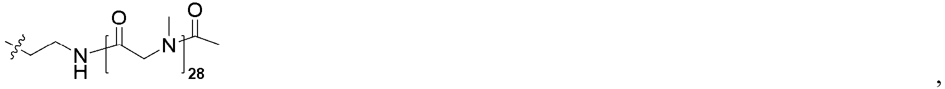 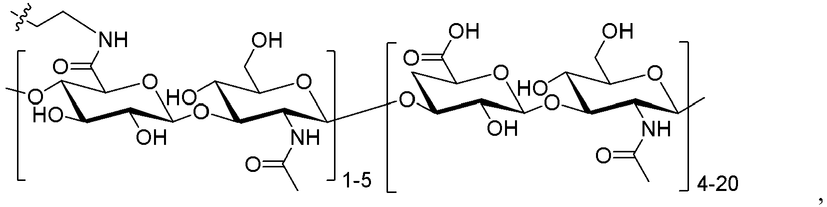 and

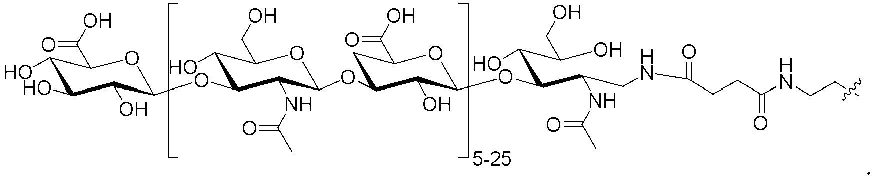

In some embodiments, 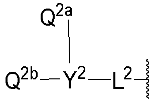 is selected from the following structures:

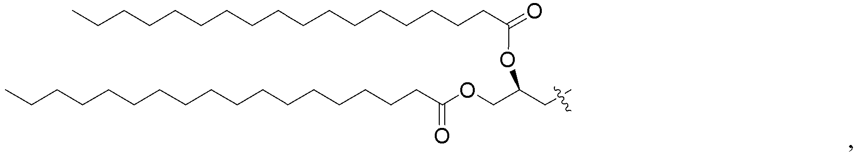

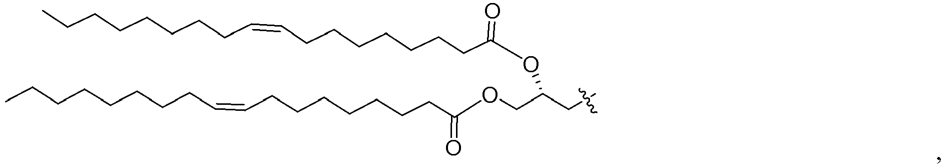

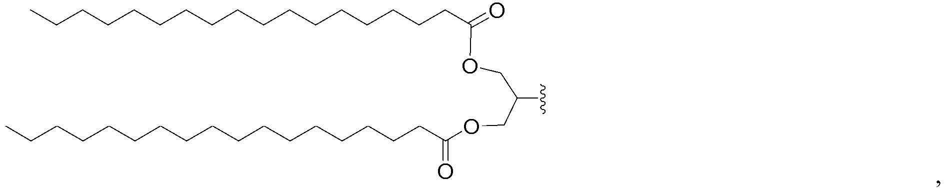

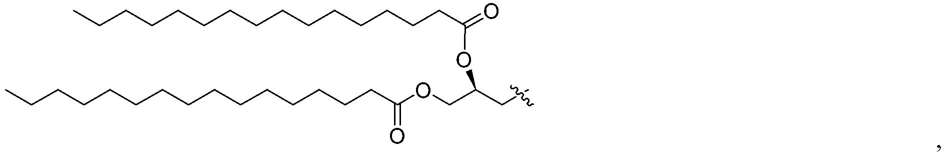

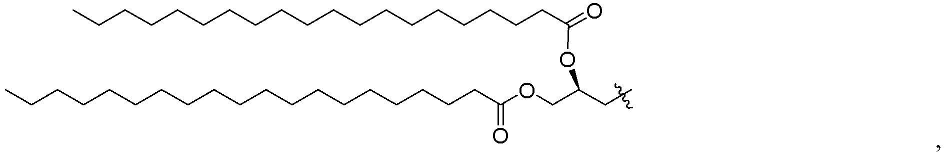

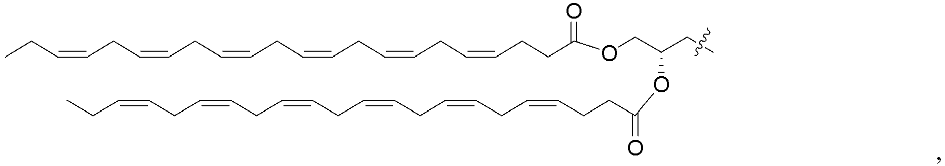

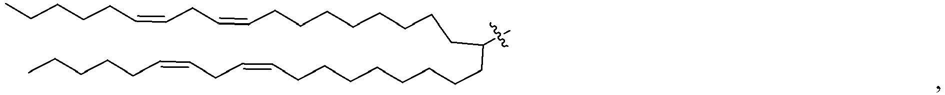

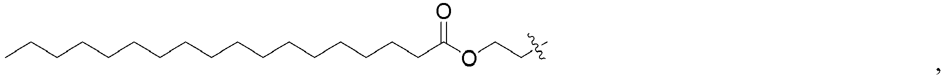

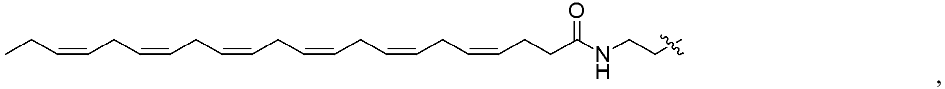

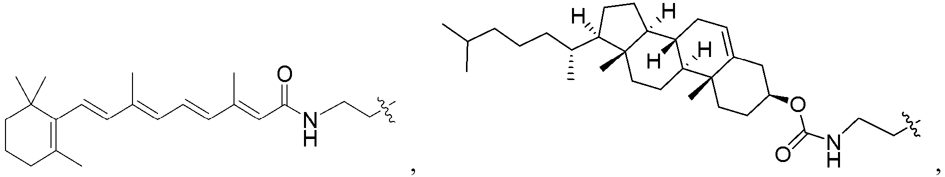

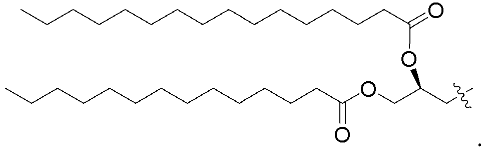

In some embodiments, 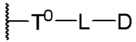 is selected from the following structures:

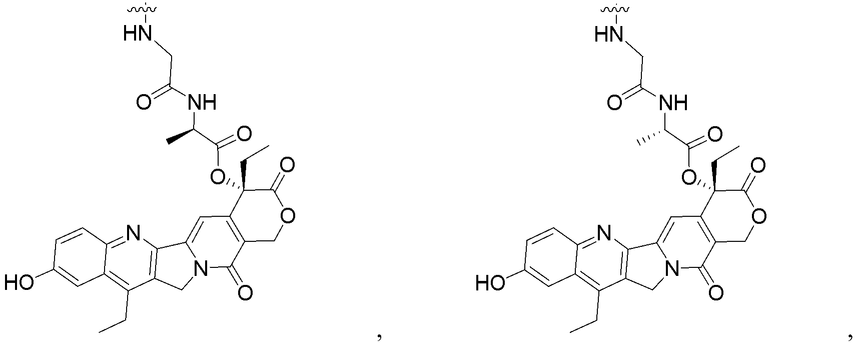

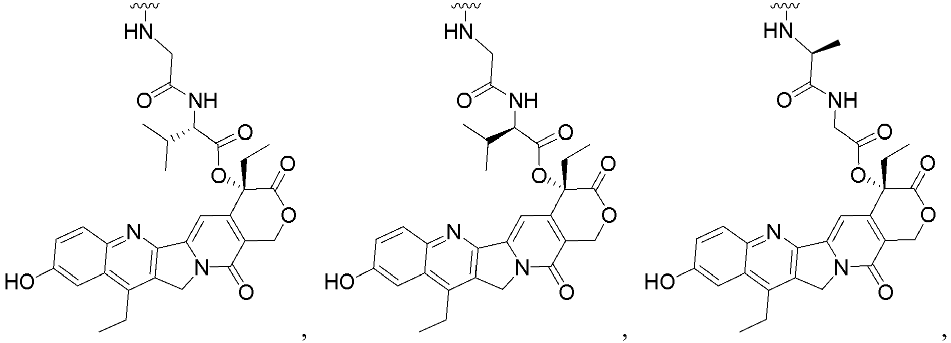

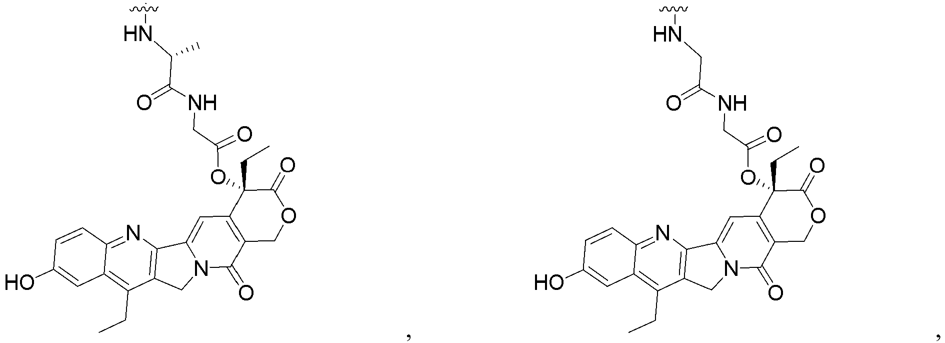

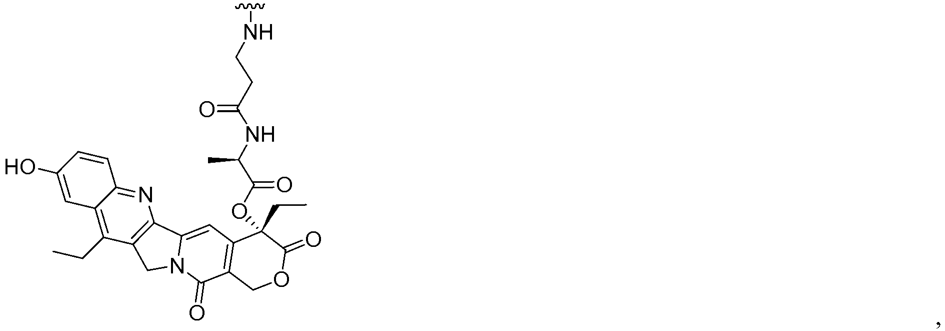

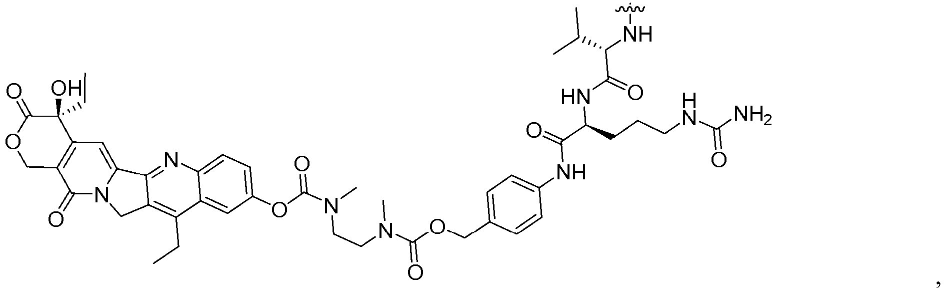

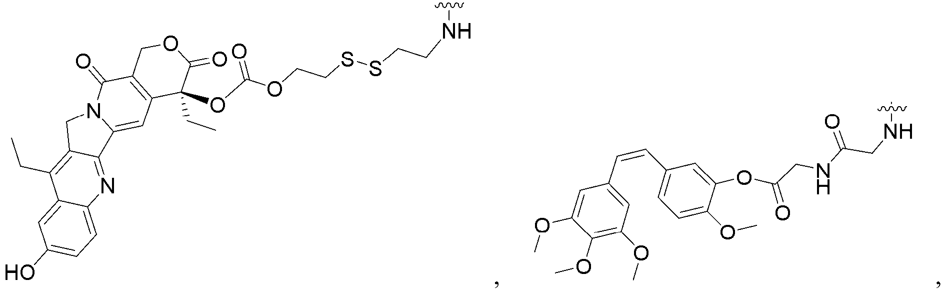

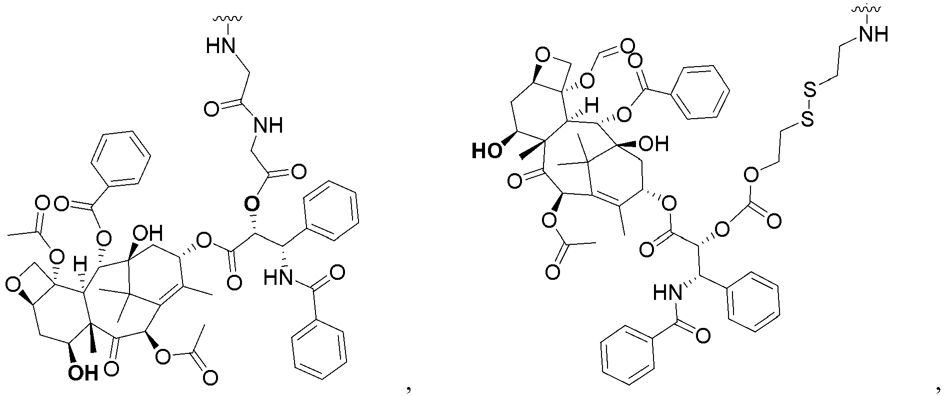

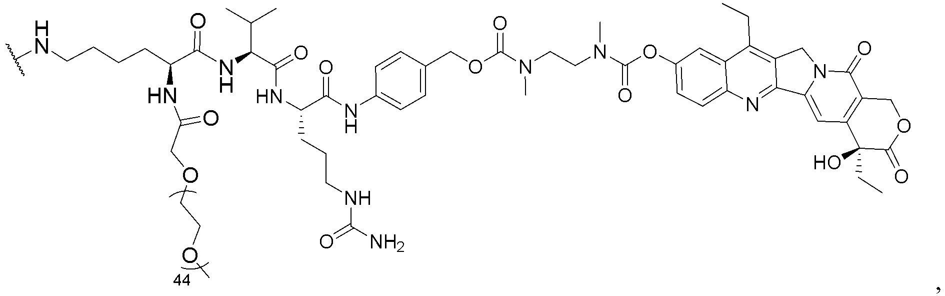

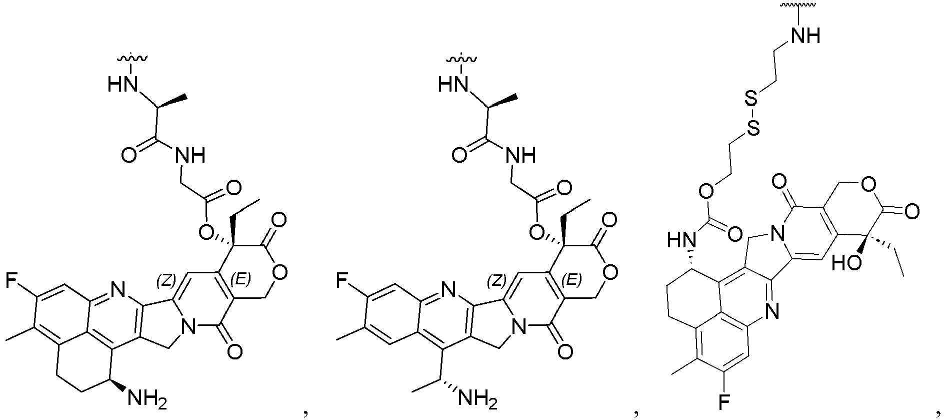

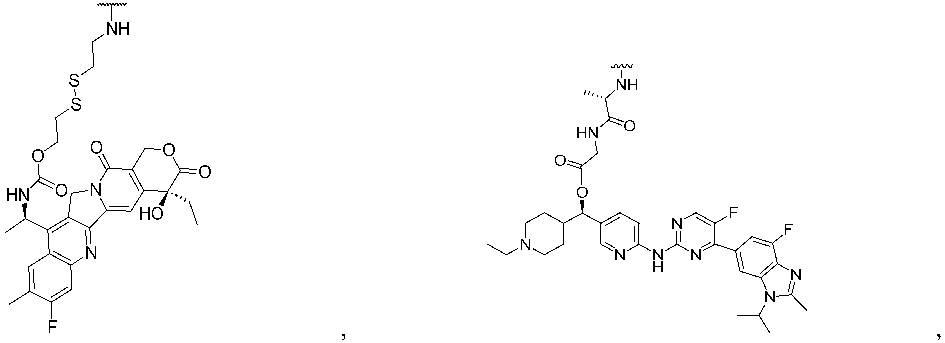

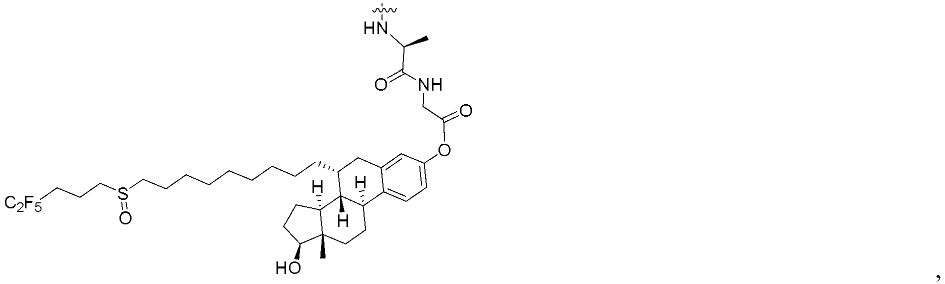

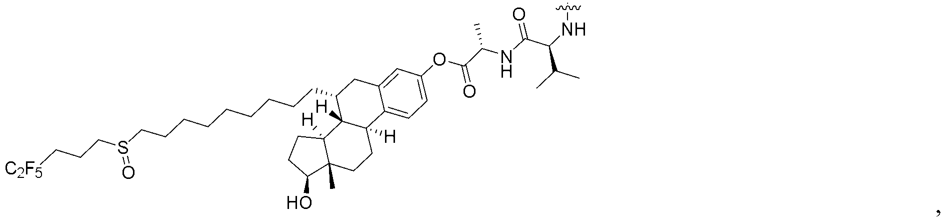

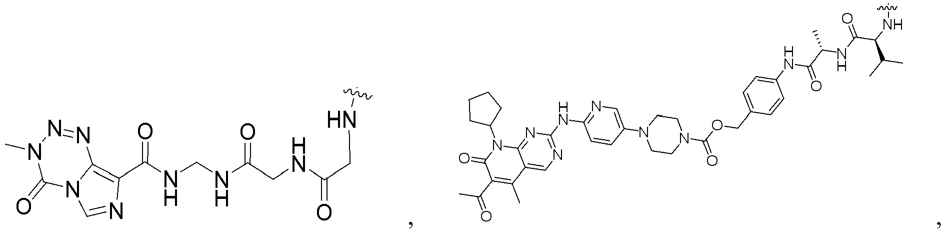

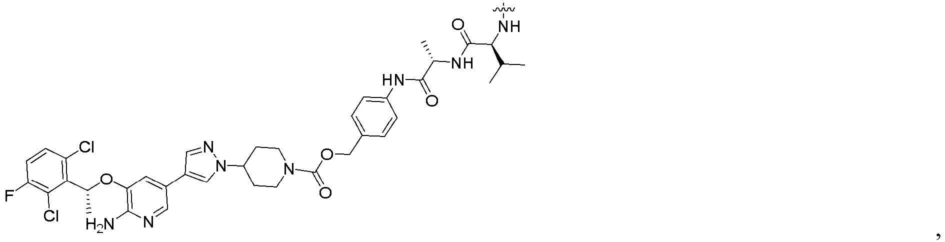

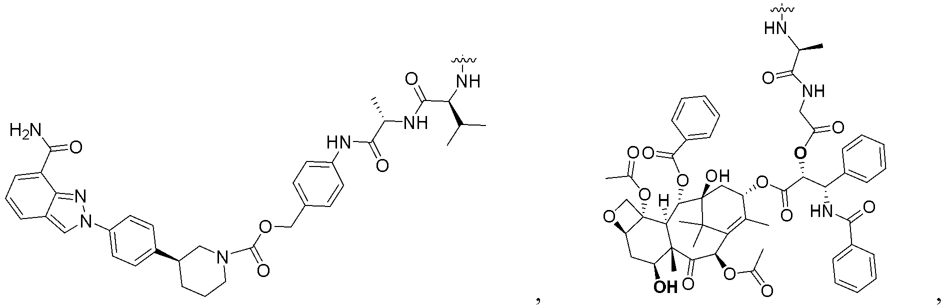

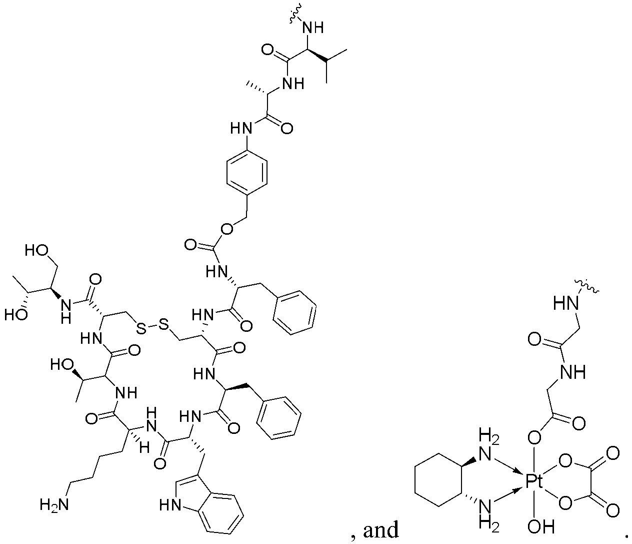

In some embodiments, the phospholipid drug conjugate having nano-size and comprising a stimuli-responsive linker, or a stereoisomer, prodrug, pharmaceutically acceptable salt, or pharmaceutically acceptable solvate thereof provided by the present invention is selected from the following structures:

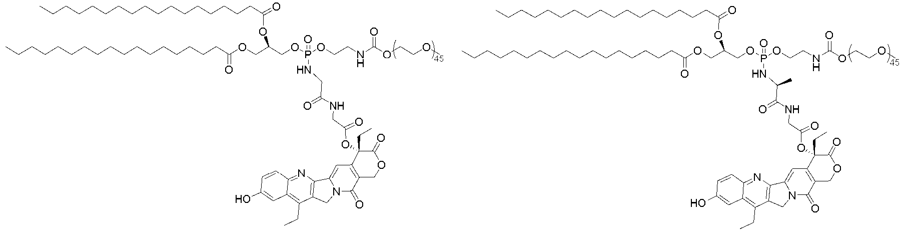

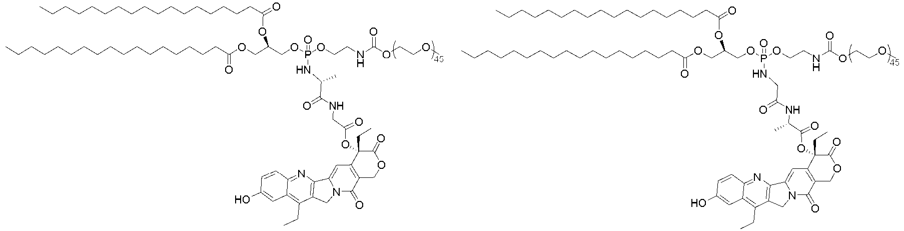

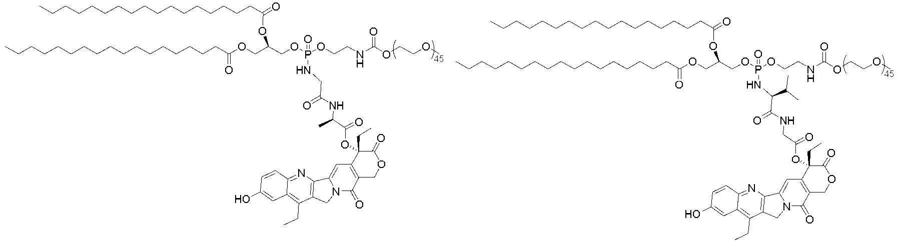

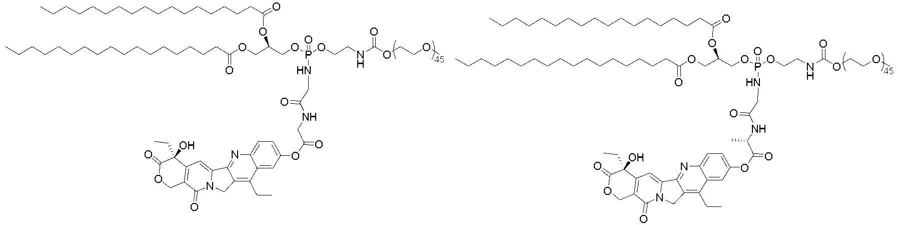

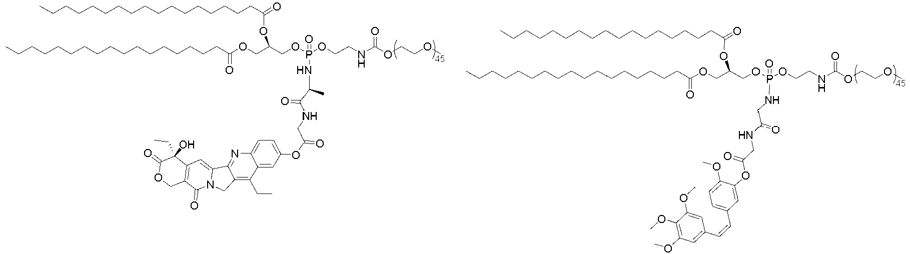

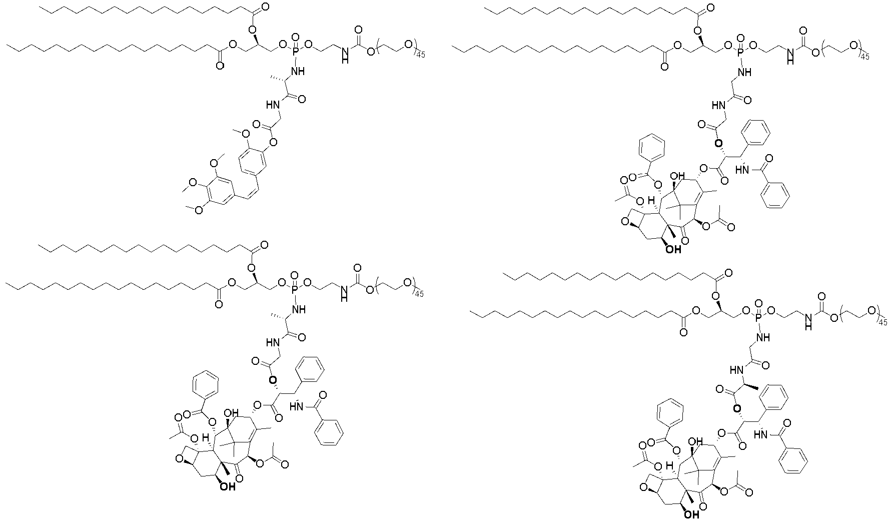

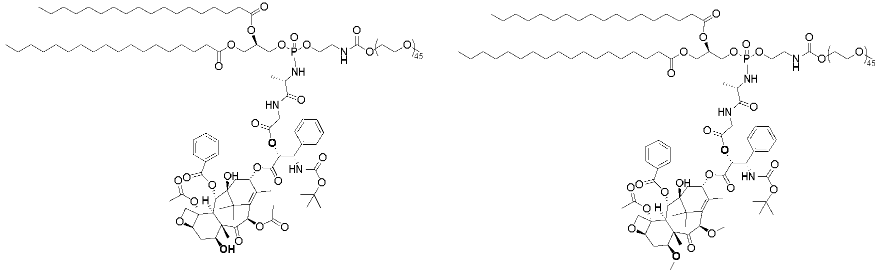

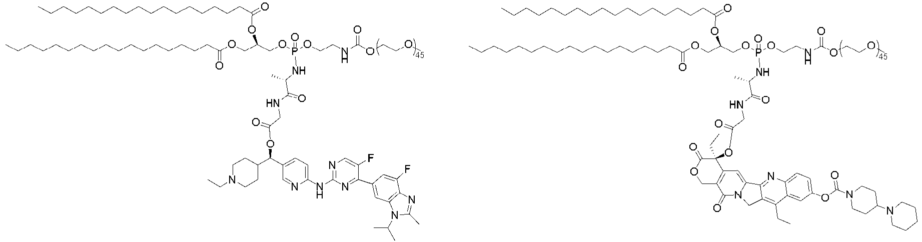

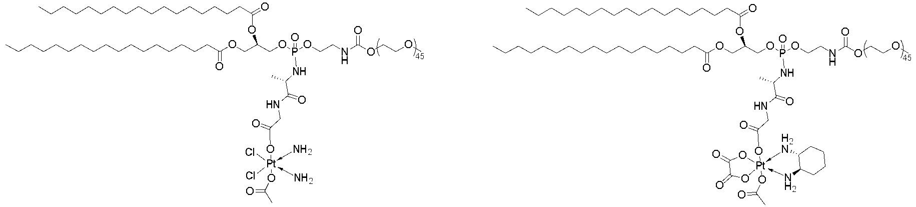

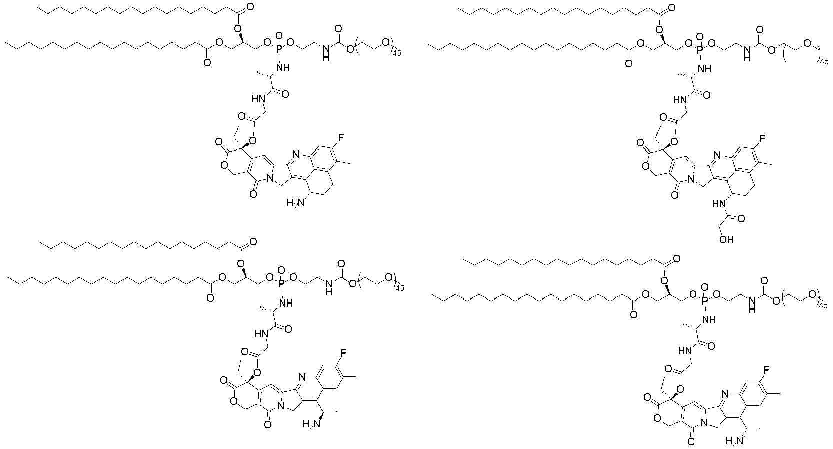

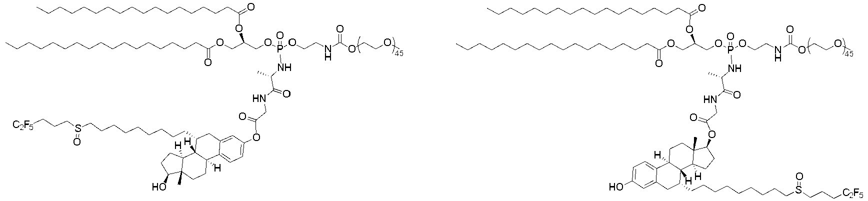

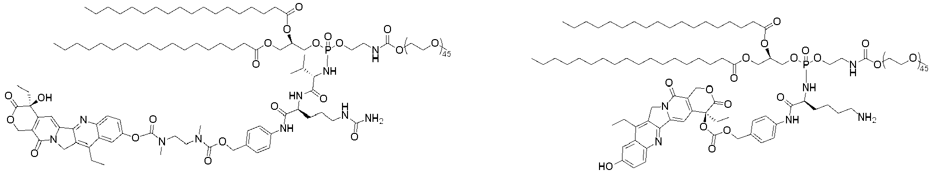

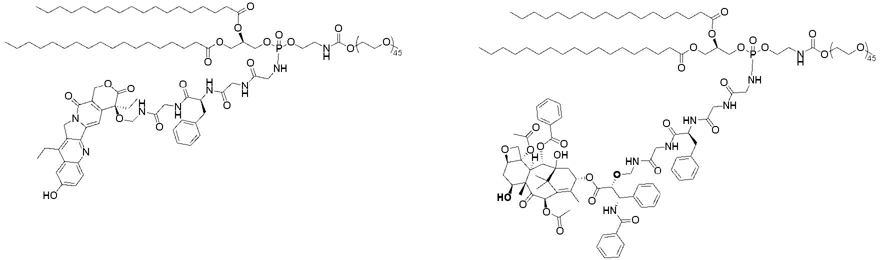

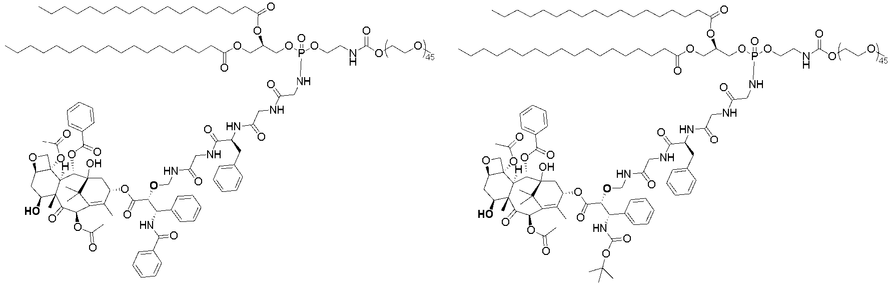

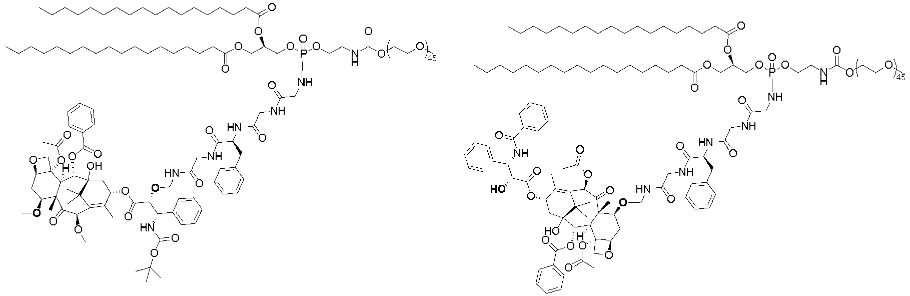

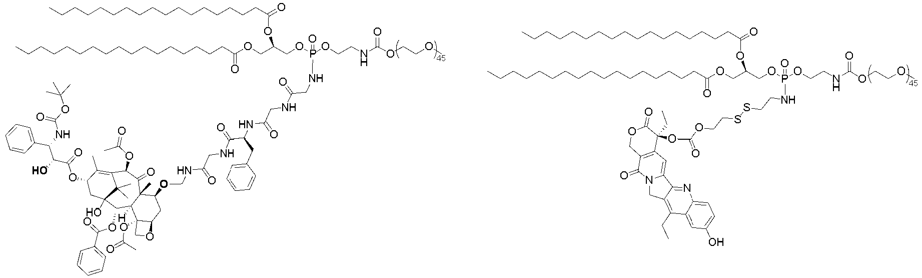

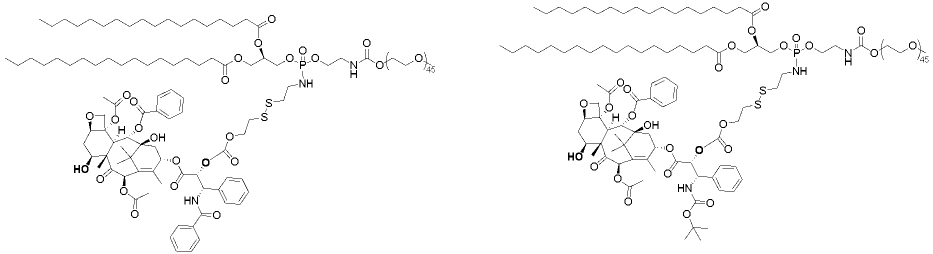

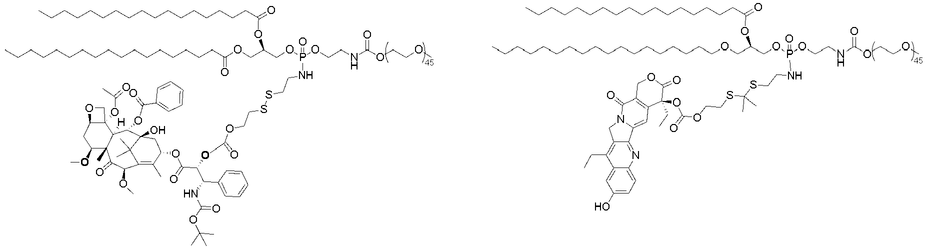

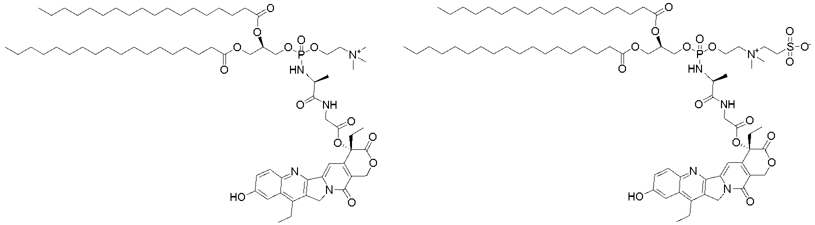

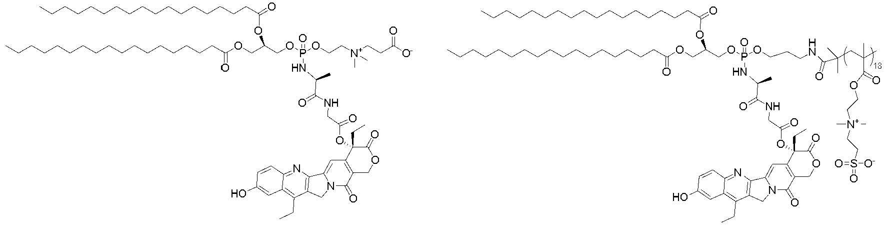

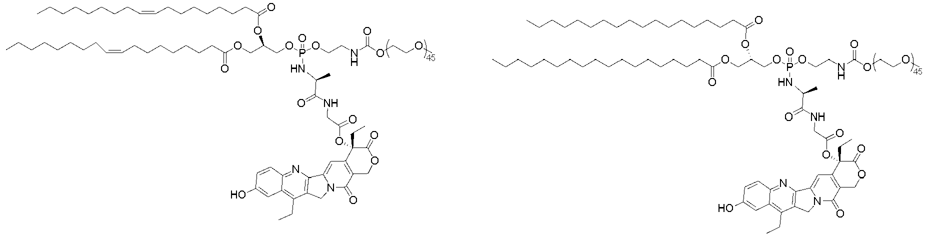

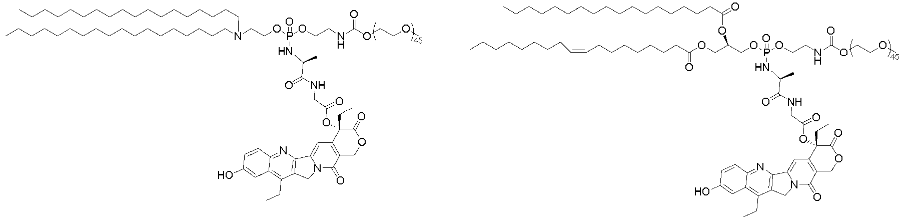

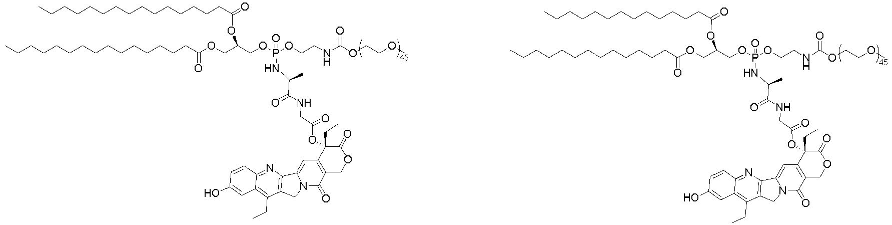

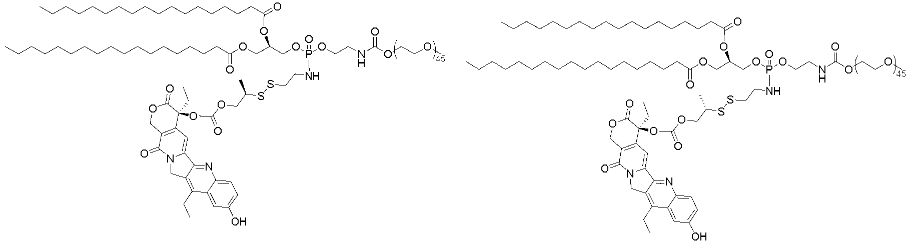

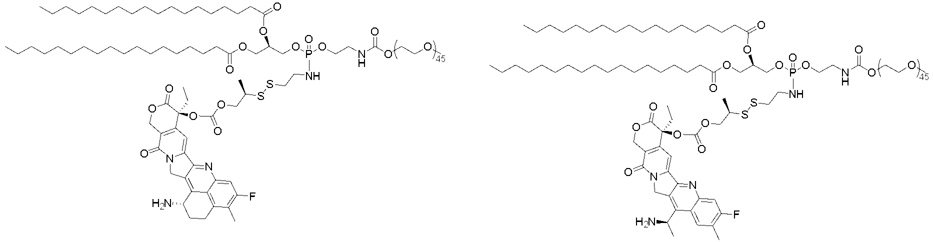

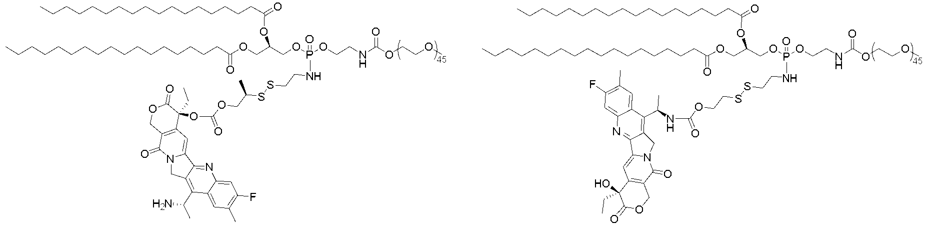

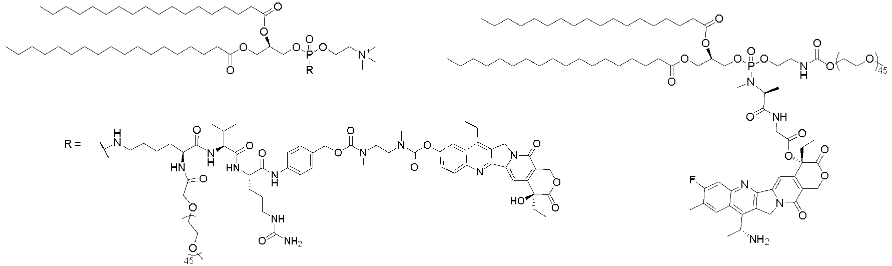

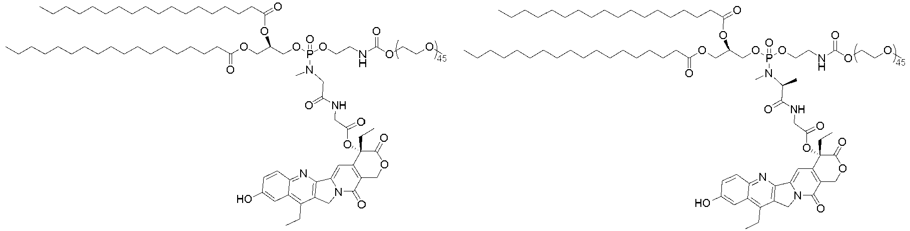

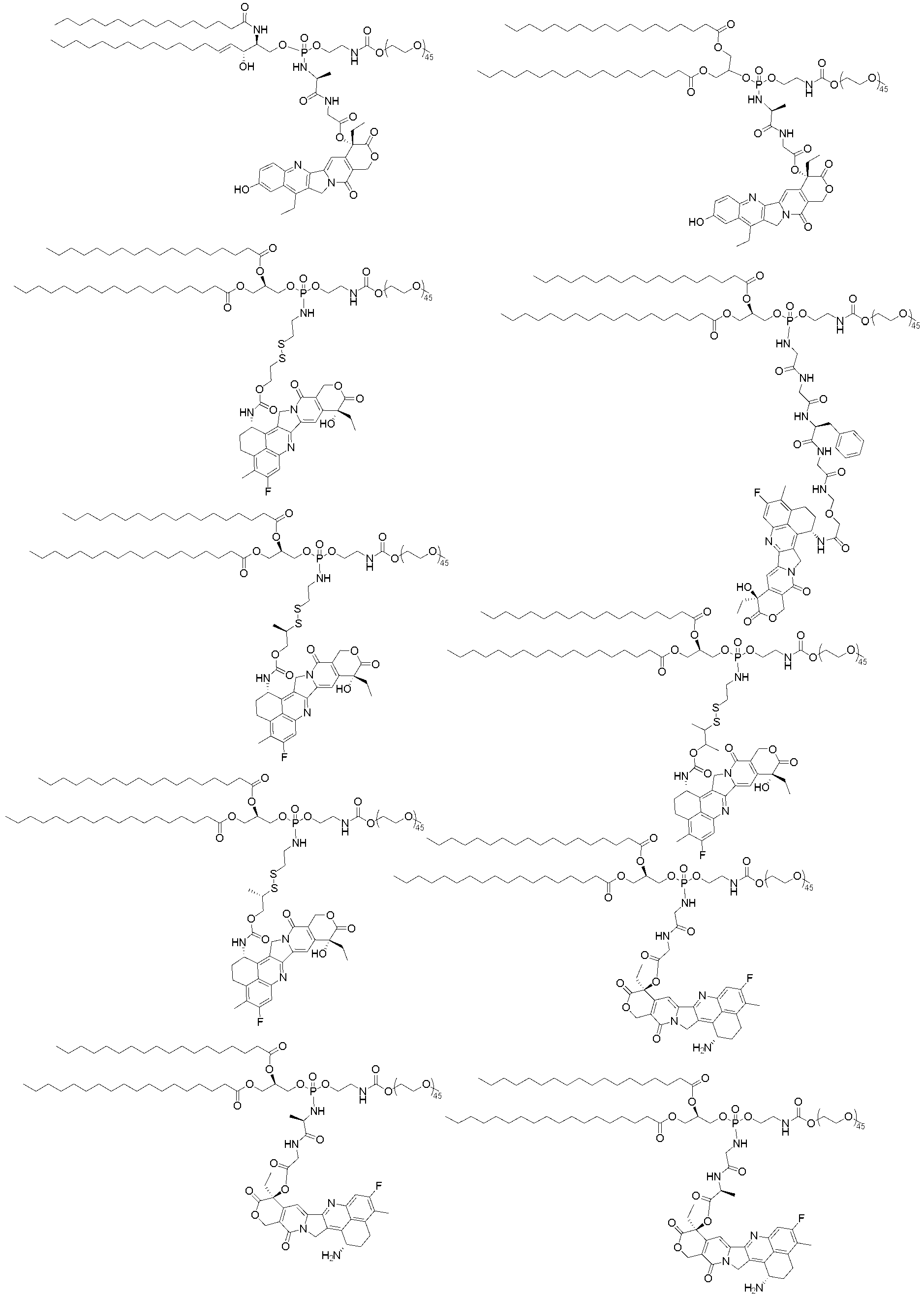

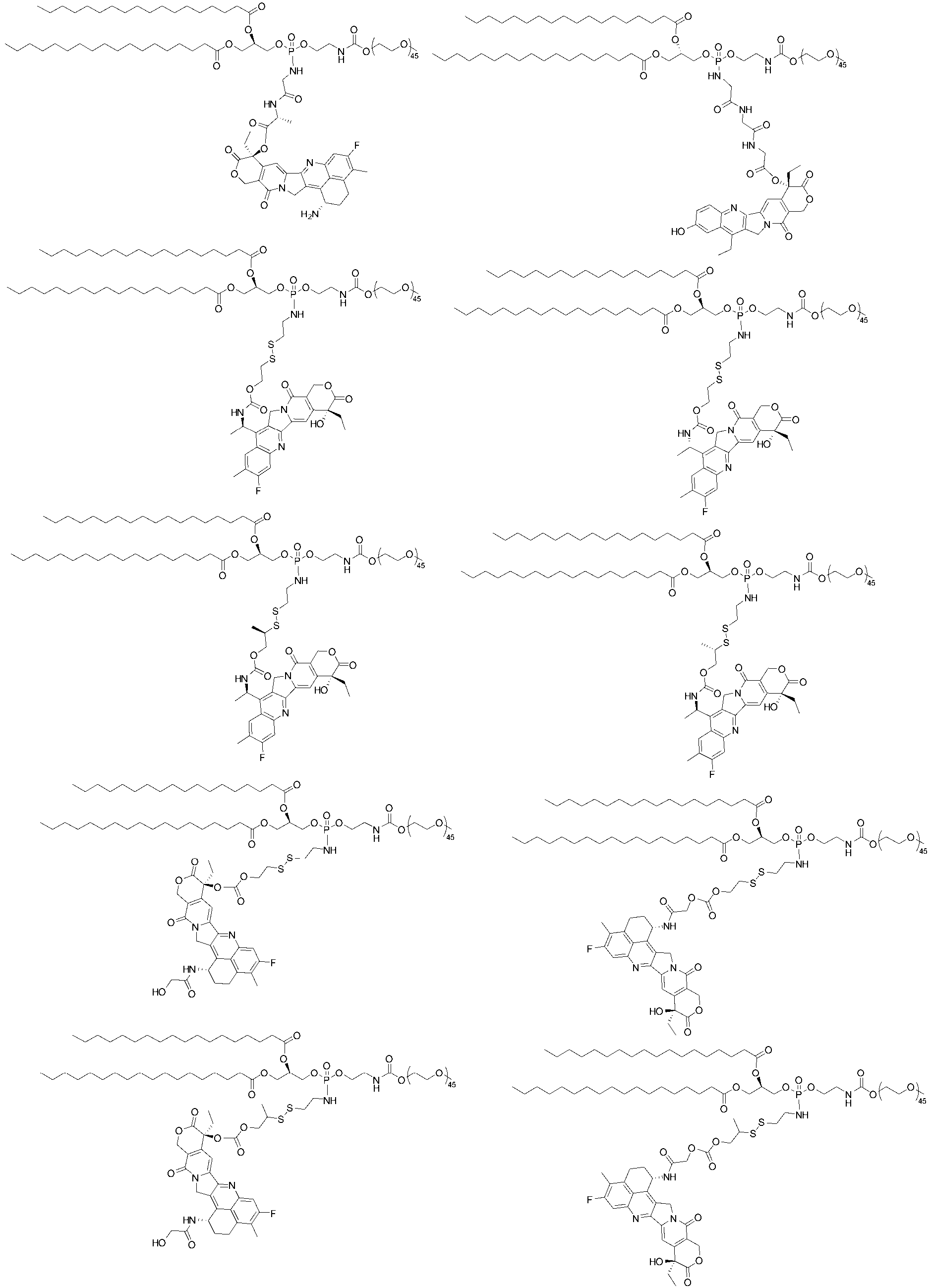

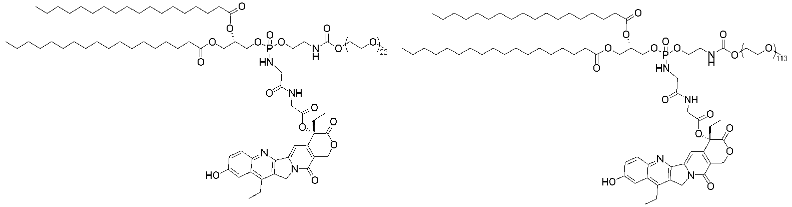

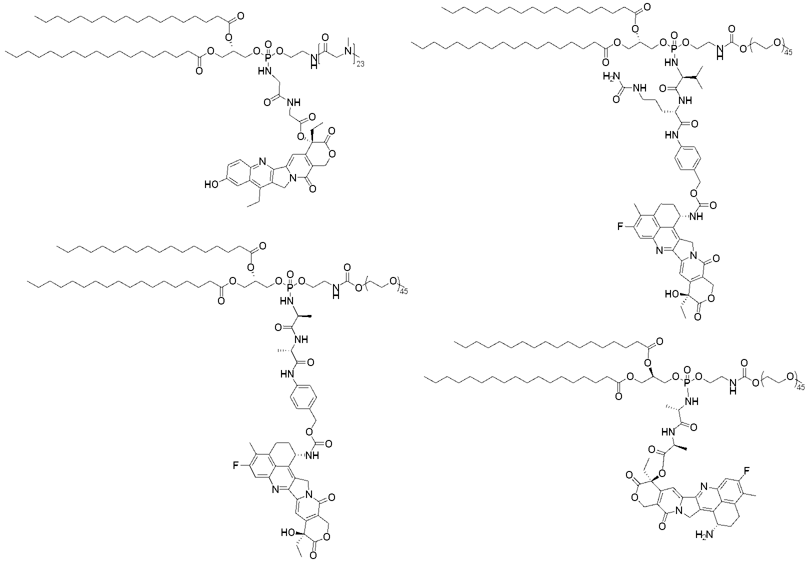

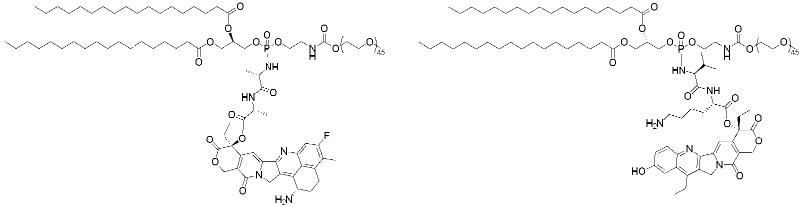

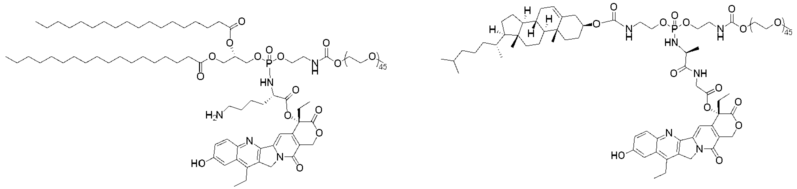

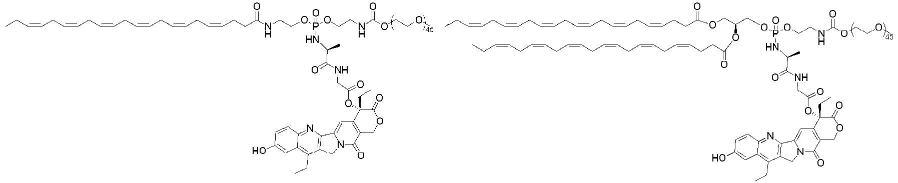

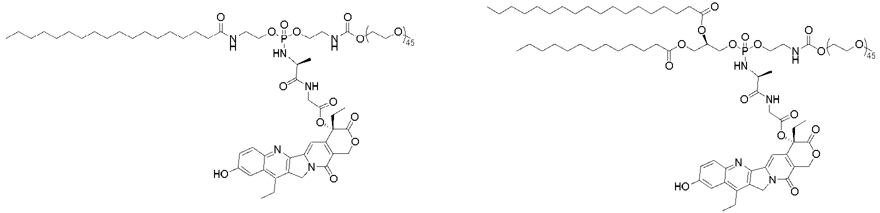

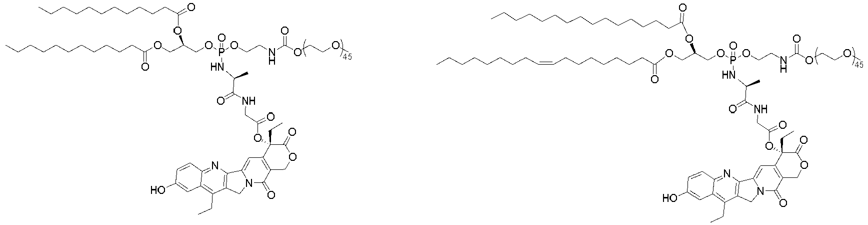

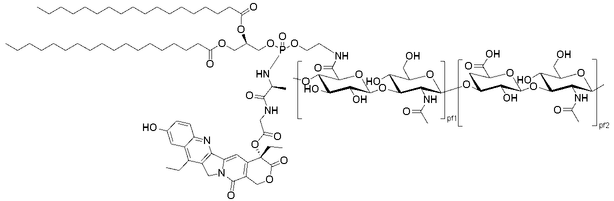

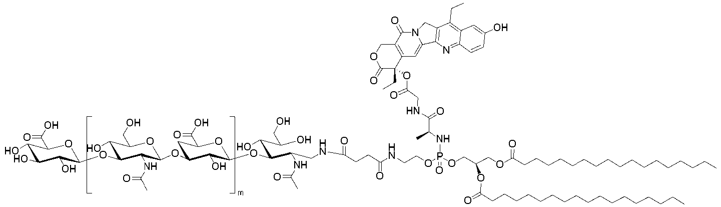

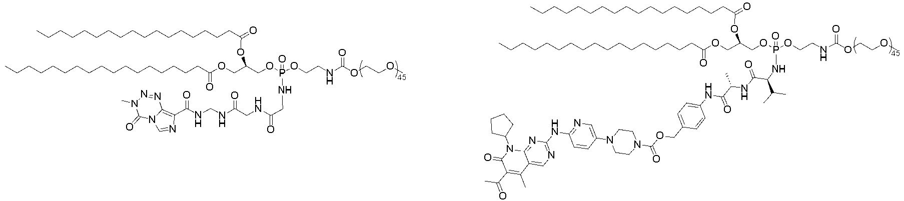

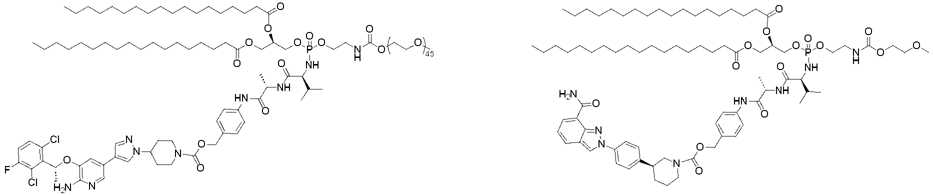

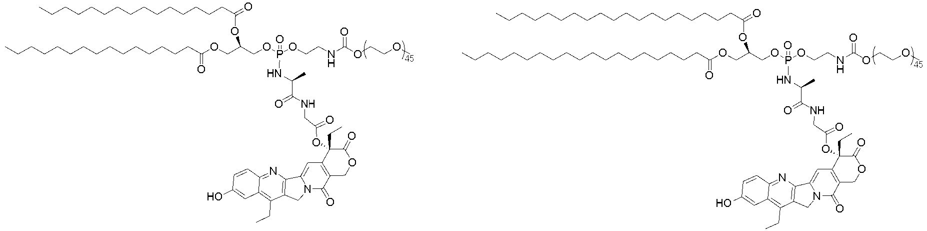

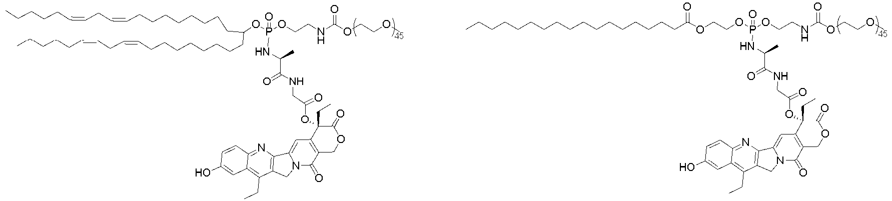

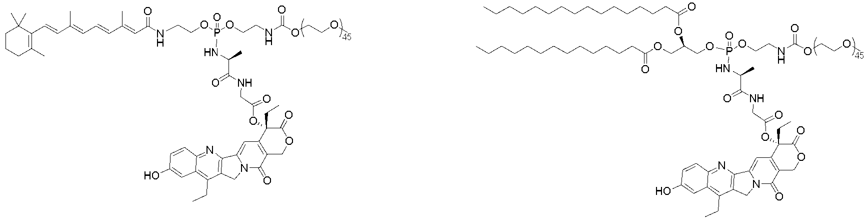

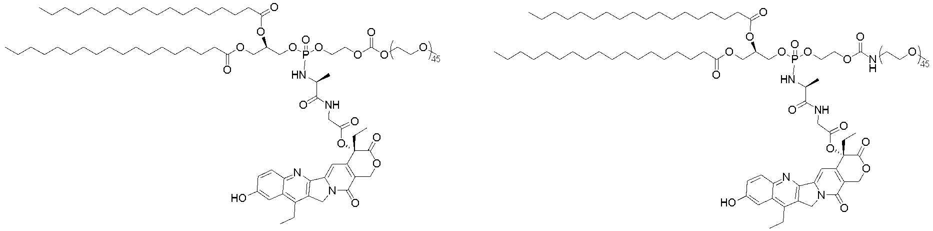

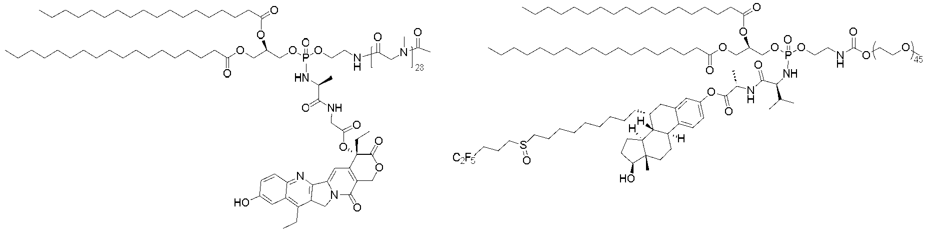

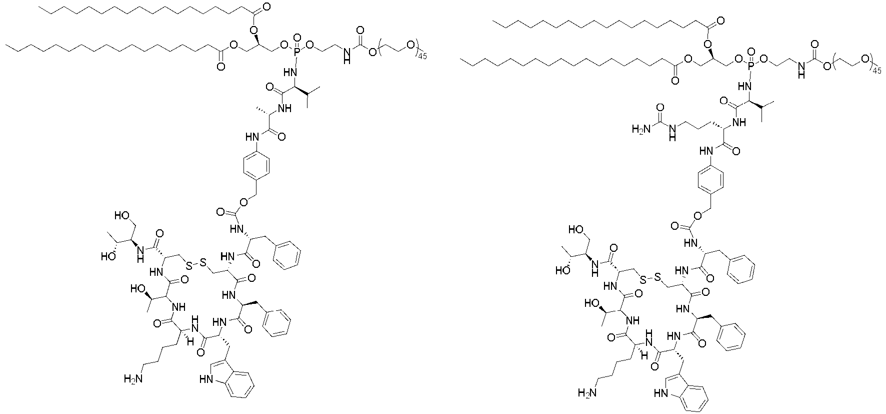

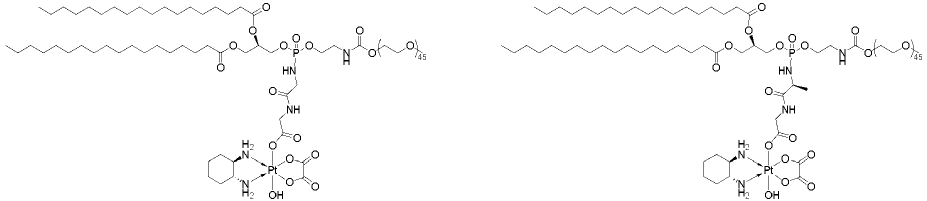

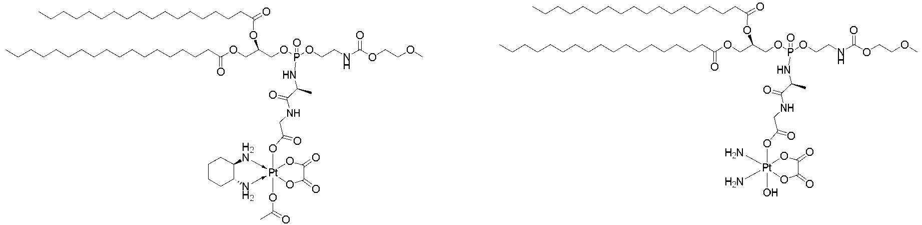

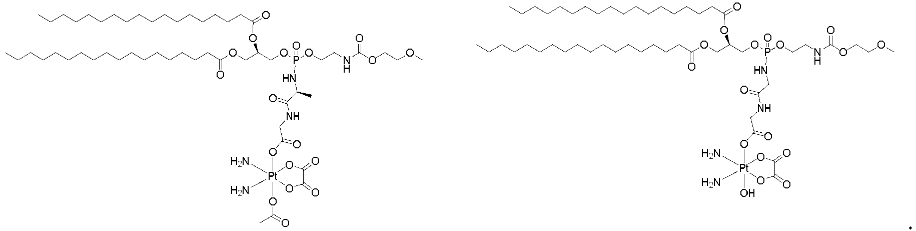

In some embodiments, use of the phospholipid drug conjugate having nano-size and comprising a stimuli-responsive linker, or a stereoisomer, prodrug, pharmaceutically acceptable salt, or pharmaceutically acceptable solvate thereof, or the pharmaceutical composition thereof, in a medicament for preventing, alleviating and/or treating abnormal cell proliferation, infection (e.g., viral infection, such as HIV, HBV), inflammatory diseases (e.g., rheumatoid arthritis), autoimmune diseases (e.g., psoriasis, lupus erythematosus, diabetes), cardiovascular diseases (e.g., stroke, myocardial infarction, atherosclerosis), or neurodegenerative diseases (e.g., Alzheimer's disease, Parkinson's disease), wherein the abnormal cell proliferative disease can be cancer.

In some embodiments, use of the phospholipid drug conjugate having nano-size and comprising a stimuli-responsive linker, or a stereoisomer, prodrug, pharmaceutically acceptable salt, or pharmaceutically acceptable solvate thereof, or the pharmaceutical composition thereof, in a medicament for preventing, alleviating and/or treating abnormal cell proliferation, infection (e.g., viral infection, such as HIV, HBV), inflammatory diseases (e.g., rheumatoid arthritis), autoimmune diseases (e.g., psoriasis, lupus erythematosus, diabetes), cardiovascular diseases (e.g., stroke, myocardial infarction, atherosclerosis), ocular diseases, or neurodegenerative diseases (e.g., Alzheimer's disease, Parkinson's disease), wherein the abnormal cell proliferative disease can be cancer.

In some embodiments, use of the phospholipid drug conjugate having nano-size and comprising a stimuli-responsive linker, or a stereoisomer, prodrug, pharmaceutically acceptable salt, or pharmaceutically acceptable solvate thereof, or the pharmaceutical composition thereof, in a medicament for treating cancer, wherein the cancer includes breast cancer, ovarian cancer, brain cancer, prostate cancer, melanoma, nasopharyngeal carcinoma, esophageal cancer, gastric cancer, liver cancer, pancreatic cancer, colorectal cancer (e.g., colon cancer, rectal cancer), lung cancer (e.g., small cell lung cancer, non-small cell lung cancer, squamous cell carcinoma, undifferentiated carcinoma), kidney cancer, skin cancer, neuroblastoma, sarcoma, liposarcoma, osteochondroma, bone cancer, osteosarcoma, seminoma, testicular tumor, uterine tumor (e.g., cervical cancer, endometrial cancer), head and neck tumor (e.g., laryngeal cancer, pharyngeal cancer, tongue cancer), multiple myeloma, malignant lymphoma (e.g., reticulum cell sarcoma, lymphosarcoma, Hodgkin's lymphoma, mantle cell lymphoma), polycythemia vera, leukemia (e.g., acute myelogenous leukemia, chronic myelogenous leukemia, acute lymphoblastic leukemia, chronic lymphocytic leukemia), thyroid tumor, ureteral tumor, bladder tumor, gallbladder cancer, bile duct cancer, choriocarcinoma, or pediatric tumor (e.g., neuroblastoma, embryonal carcinoma of testis, retinoblastoma).

The terms used in the present invention, unless otherwise stated, have the following meanings:
The amino protecting groups, hydroxyl protecting groups, and carboxyl protecting groups used in the present invention are appropriate protecting groups for amino, hydroxyl, and carboxyl groups known in the art, referring to various protecting groups in the literature ("Protective Groups in Organic Synthesis", 5^{th} Ed. T. W. Greene & P. G. M. Wuts).

The term **"alkyl"** refers to a saturated aliphatic hydrocarbon group, including a straight or branched chain group having 1 to 30 carbon atoms. Preferably, an alkyl having 1 to 10 carbon atoms, more preferably 1 to 8 carbon atoms, non-limiting examples include but are not limited to: methyl, ethyl, n-propyl, n-butyl, isobutyl, tert-butyl, sec-butyl, n-pentyl, 2-methylbutyl, 3-methylbutyl, 1,1-dimethylpropyl, 1,2-dimethylpropyl, 2,2-dimethylpropyl, 1-ethylpropyl, n-hexyl, 2-methylpentyl, 3-methylpentyl, 4-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1,1,2-trimethylpropyl, 1-ethyl-2-methylpropyl, n-heptyl, 2-methylhexyl, 3-methylhexyl, 4-methylhexyl, 5-methylhexyl, 2,2-dimethylpentyl, 2,3-dimethylpentyl, 2,4-dimethylpentyl, 3,3-dimethylpentyl, 3,4-dimethylpentyl, 2-ethylpentyl, 3-ethylpentyl, octyl, nonyl, decyl, undecyl, dodecyl, and their various isomers, etc. The "alkyl" may be substituted or unsubstituted.

The term **"cycloalkyl"** refers to a saturated or partially unsaturated monocyclic or polycyclic cyclic substituent, including 3 to 20 carbon atoms, preferably 3 to 12 carbon atoms, more preferably 3 to 10 carbon atoms, most preferably including 3 to 6 carbon atoms. Non-limiting examples of monocyclic cycloalkyl include but are not limited to: cyclopropyl, cyclobutyl, cyclopentyl, cyclopentenyl, cyclohexyl, cyclohexenyl, cyclohexadienyl, cycloheptyl, cyclooctyl, etc. Non-limiting examples of polycyclic cycloalkyl include but are not limited to spiro, fused, and bridged cycloalkyls. The "cycloalkyl" may be substituted or unsubstituted.

The term **"alkenyl"** refers to an alkyl as defined in the present invention comprising at least two carbon atoms and at least one carbon-carbon double bond, preferably C2-C10 alkenyl, more preferably C2-C6 alkenyl, for example, vinyl, propenyl, 1-propenyl, etc. The "alkenyl" may be substituted or unsubstituted.

The term **"alkynyl"** refers to an alkyl as defined in the present invention comprising at least two carbon atoms and at least one carbon-carbon triple bond, preferably C2-C10 alkynyl, more preferably C2-C6 alkynyl, for example, ethynyl, 1-propynyl, 2-propynyl, etc. The "alkynyl" may be substituted or unsubstituted.

The term **"heterocycloalkyl"** refers to a saturated or partially unsaturated monocyclic or polycyclic cyclic hydrocarbon substituent, including 3 to 20 ring atoms, wherein one or more ring atoms are heteroatoms selected from N, O, Si, B, S(O)ₘ, P(O)ₘ (where m is an integer from 0 to 2), but excluding ring portions of -O-O-, -O-S- or -S-S-, and the remaining ring atoms are carbon. Preferably 3 to 12 ring atoms, containing 1 to 4 heteroatoms. Non-limiting examples of monocyclic heterocycloalkyl include pyrrolyl, piperidinyl, piperazinyl, morpholinyl, tetrahydrofuranyl, pyranyl, etc. Polycyclic heterocycloalkyl includes spiro, fused, and bridged heterocycloalkyls. The "heterocycloalkyl" may be substituted or unsubstituted.

The term **"alkoxy"** refers to -O-(alkyl) and -O-(cycloalkyl), wherein alkyl and cycloalkyl are as defined in this description. Non-limiting examples include but are not limited to methoxy, ethoxy, propoxy, butoxy, cyclopropoxy, cyclobutoxy, cyclopentoxy, cyclohexoxy, etc. The "alkoxy" may be substituted or unsubstituted.

The term **"alkylthio"** refers to -S-(alkyl) and -S-(cycloalkyl), wherein alkyl and cycloalkyl are as defined in this description. Non-limiting examples include but are not limited to methylthio, ethylthio, propylthio, butylthio, cyclopropylthio, cyclobutylthio, cyclopentylthio, cyclohexylthio, etc. The "alkylthio" may be substituted or unsubstituted.

The term **"aryl"** refers to any stable conjugated hydrocarbon ring system group having 6 to 18 carbon atoms, preferably 6 to 10 carbon atoms, which may be a monocyclic, bicyclic, tricyclic, or more polycyclic aromatic group, such as phenyl, naphthyl, anthracene. The aryl may include aryl fused with heterocycloalkyl or cycloalkyl rings. The "aryl" may be substituted or unsubstituted.

The term **"heteroaryl"** refers to an aromatic ring system formed by replacing at least one carbon atom on a ring with a heteroatom selected from N, O, or S, preferably a 5- to 7-membered monocyclic structure or a 7- to 12-membered bicyclic structure, more preferably a 5- to 6-membered heteroaryl, for example, pyrrolyl, imidazolyl, pyridinyl, pyrimidinyl, thiazolyl, thienyl, pyrazinyl, triazolyl, tetrazolyl, oxazolyl, indazolyl. The heteroaryl may include heteroaryl fused with heteroaryl, heterocycloalkyl, or cycloalkyl rings. The "heteroaryl" may be substituted or unsubstituted.

The term "hydroxy" refers to -OH.

The term "halogen" refers to fluorine, chlorine, bromine, or iodine.

The term "nitro" refers to -NO2.

The term "amino" refers to -NH2.

The term "cyano" refers to -CN.

The term "carboxylic acid" refers to -C(O)OH.

The term "mercapto" refers to -SH.

The term **"substituted"** means that one or more hydrogen atoms in a group are independently substituted by a corresponding number of deuterium atoms or substituents.

The term **"substituent"** refers to a molecular group that replaces hydrogen in a compound, which may include but is not limited to halogen, alkyl, alkoxy, nitro, cyano, hydroxy, sulfonyl, sulfinyl, cycloalkyl, heterocycloalkyl, amino, substituted amino, azido, oxo, carboxyl, carboxylate, alkenyl, alkynyl, mercapto, alkylthio, aromatic group, heterocyclic aryl, or groups formed by their respective combinations.

The term **"sulfonyl"** refers to -S(O)₂-alkyl, cycloalkyl, aryl, or heteroaryl, wherein alkyl, cycloalkyl, aryl, and heteroaryl are as defined above.

The term **"sulfinyl"** refers to -SO-alkyl, cycloalkyl, aryl, or heteroaryl, wherein alkyl, cycloalkyl, aryl, and heteroaryl are as defined above.

The term **"carboxylate"** refers to -C(O)O-alkyl, aryl, or cycloalkyl, wherein alkyl, aryl, and cycloalkyl are as defined above.

The term **"amino acid"** includes those selected from naturally occurring α-*L*-amino acids, such as alanine (Ala or A), cysteine (Cys or C), aspartic acid (Asp or D), glutamic acid (Glu or E), phenylalanine (Phe or F), glycine (Gly or G), histidine (His or H), isoleucine (Ile or I), lysine (Lys or K), leucine (Leu or L), methionine (Met or M), asparagine (Asn or N), proline (Pro or P), glutamine (Gln or Q), arginine (Arg or R), serine (Ser or S), threonine (Thr or T), valine (Val or V), tryptophan (Trp or W), tyrosine (Tyr or Y) and citrulline (Cit), as well as D-amino acids, synthetic α, β, or γ amino acids, which may be substituted or non-substituted.

The term **"peptide"** or **"polypeptide"** refers to natural and synthetic peptides, which may contain natural amino acids, non-natural amino acids, or a combination of natural and non-natural amino acids.

**"Pharmaceutically acceptable salt"** refers to a salt that retains the biological effectiveness of the free base without other toxic side effects. It can be an acidic group, a basic group, or an amphoteric group. Non-limiting examples include but are not limited to: acid salts including hydrochloride, hydrobromide, sulfate, pyrosulfate, bisulfate, sulfite, bisulfite, phosphate, monohydrogen phosphate, dihydrogen phosphate, metaphosphate, pyrophosphate, nitrate, acetate, propionate, decanoate, octanoate, formate, acrylate, isobutyrate, hexanoate, heptanoate, oxalate, malonate, succinate, suberate, benzoate, methylbenzoate, phthalate, maleate, methanesulfonate, p-toluenesulfonate, benzenesulfonate, (D, L)-tartrate, citrate, maleate, (*D, L*)-malate, fumarate, stearate, oleate, cinnamate, laurate, glutamate, aspartate, trifluoromethanesulfonate, mandelate, ascorbate, salicylate, etc. When the compound of the present invention contains an acidic group, its pharmaceutically acceptable salts may also include: alkali metal salts (e.g., sodium or potassium salts), alkaline earth metal salts (e.g., calcium or magnesium salts), organic base salts (e.g., alkyl aromatic amines, amino acids).

**"Solvate"** refers to an aggregate (or association) formed by one or more solvent molecules with the compound of the present invention. Solvents forming the solvate include, but are not limited to: water, dimethyl sulfoxide, methanol, ethanol, isopropanol, acetic acid, etc.

**"Pharmaceutical composition"** refers to a mixture comprising one or more compounds described herein, or physiologically acceptable salts or prodrugs thereof, with other chemical components, as well as other components such as physiologically acceptable carriers and excipients. The purpose of the pharmaceutical composition is to facilitate administration to an organism and promote the absorption of the active ingredient to exert biological activity.

**"Prodrug"** refers to a compound that can be converted into a bioactive compound of the present invention under physiological conditions (e.g., in vivo) or by solvolysis, and can be understood as a pharmaceutically acceptable metabolic precursor. A prodrug may be an inactive substance or have less activity than the active parent compound, but can be rapidly converted in vivo to produce the parent compound of the present invention. It can improve its solubility in animals and certain metabolic properties. Prodrugs include, for example, amino protecting groups, carboxyl protecting groups, phospholipids

**"Isomer"** refers to a stereoisomer, including: enantiomers and diastereomers. Cis-trans isomers are a type of diastereomer. The isomers of the compound of the present invention may be its enantiomers, diastereomers, and any mixture thereof, existing in free or salt form.

The term "effective amount" or "therapeutically effective amount" refers to a sufficient amount of a drug or agent that is non-toxic but achieves the desired effect. The determination of an effective amount varies from person to person, depending on the age and general condition of the recipient, as well as the specific active substance. A suitable effective amount in individual cases can be determined by those skilled in the art through routine experimentation.

Any abbreviations for protecting groups, amino acids, and other compounds used in the present invention, unless otherwise specified, follow their commonly used, recognized abbreviations, or refer to the IUPAC-IUBC Commission on Biochemical Nomenclature (see Biochem. 1972, 11, 942-944).

### BRIEF DESCRIPTION OF THE DRAWINGS

FIG. 1 Nanoparticle size distribution diagram of the examples of the present invention
FIG. 2 Efficacy evaluation of Example **1** of the present invention in a human hepatocellular carcinoma HepG2 xenograft mouse tumor model
FIG. 3 Efficacy evaluation of Examples **5, 7, 15,** and **16** of the present invention in a human hepatocellular carcinoma HepG2 xenograft mouse tumor model
FIG. 4 Efficacy evaluation of Examples **1, 5,** and **7** of the present invention in a human gastric cancer NCI-N87 xenograft mouse tumor model
FIG. 5 Body weight changes during the efficacy evaluation of Examples **1, 5,** and **7** of the present invention in a human gastric cancer NCI-N87 xenograft mouse tumor model
FIG. 6 Efficacy evaluation of Example **5** and **T-Dxd** of the present invention in a human colon cancer cell HT-29 xenograft mouse model
FIG. 7 Efficacy evaluation of Example **5** in a human fibrosarcoma cell HT-1080 xenograft mouse model
FIG. 8 Pharmacodynamic study of Example **5** and **46** in a U87-MG glioblastoma orthotopic mouse model

### DETAILED DESCRIPTION OF THE PREFERRED EMBODIMENT

The present invention is further illustrated by the following examples, but it is not thereby limited to the scope of the described examples. For experimental methods in the following examples where specific conditions are not indicated, conventional methods and conditions are followed, or selection is made according to commercial instructions.

Abbreviations used in the present invention are shown in the following table:

| Abbreviation | Full Name |
|---|---|
| DMF | *N, N*-Dimethylformamide |
| DCM or CH₂Cl₂ | Dichloromethane |
| DIPEA | *N, N*-Diisopropylethylamine |
| THF | Tetrahydrofuran |
| EA | Ethyl acetate |
| TFA | Trifluoroacetic acid |
| Et₃N | Triethylamine |
| DMAP | 4-Dimethylaminopyridine |
| Boc | tert-Butoxycarbonyl |
| Cbz | Benzyloxycarbonyl |
| DCC | Dicyclohexylcarbodiimide |
| EDCI | 1-(3-Dimethylaminopropyl)-3-ethylcarbodiimide hydrochloride |
| Fmoc | Fluorenylmethyloxycarbonyl |
| DIPEA | *N, N*-Diisopropylethylamine |
| ESI | Electrospray ionization mass spectrometry |
| MS | Mass spectrometry |
| PBS | Phosphate buffered saline |
| FmocOSU | 9-Fluorenylmethyl-*N*-succinimidyl carbonate |
| TCFH | *N, N, N'*, *N-*Tetramethylchloroformamidinium hexafluorophosphate |
| SN-38 | 10-Hydroxycamptothecin |
| TBDPSCl | tert-Butyldiphenylchlorosilane |
| DSPE-mPEG2000 | Distearoylphosphatidylethanolamine-polyethylene glycol 2000 |
| TBAF | Tetrabutylammonium fluoride |
| AcOH | Acetic acid |
| H₂O | Water |
| MeOH | Methanol |
| NaCl | Sodium chloride |
| Na₂SO₄ | Sodium sulfate |
| PE | Petroleum ether |
| DCC | Dicyclohexylcarbodiimide |
| DMAP | 4-Dimethylaminopyridine |
| TBAF | Tetra-n-butylammonium fluoride |
| CH₃CN | Acetonitrile |
| AcOH | Acetic acid |
| HCl/dioxane | Hydrogen chloride dioxane |
| NaHCO₃ | Sodium bicarbonate |
| HATU | 1-[Bis(dimethylamino)methylene]-1H-1,2,3-triazolo[4,5-b]pyridinium 3-oxide |
| | hexafluorophosphate |
| NH₄Cl | Ammonium chloride |
| TLC | Thin layer chromatography |
| Bn | Benzyl |
| *p*-TsOH | p-Toluenesulfonic acid |
| Pd/C | Palladium on carbon |
| H₂ | Hydrogen |
| N₂ | Nitrogen |

Since the present invention has been described in terms of its specific embodiments, certain modifications and equivalent variations will be obvious to those of ordinary skill in the art and are included within the scope of the present invention.

### HPLC Analysis Method for Drug Molecule Content:

Instrument: Agilent 1260 HPLC;
Column: Two-way valve, 1 m damping tube, column temperature 40 °C;
Phase A: Methanol, flow rate 0.3 mL/min, detection wavelength 254 nm, isocratic: 0.0-5.0 min, 100%, Phase A.
General HPLC Analysis Method for Compound Purity Content:
Chromatographic Conditions:

| | |
|---|---|
| Instrument | Agilent High Performance Liquid Chromatograph Agilent 1260 |
| Column | Welch XB-C8, 3*100mm 300A, 3 µm |
| DAD detector (wavelength 200-400 nm) | Wavelength: 210 nm and 254 nm |
| Flow Rate | 0.7 mL/min |
| Column Temperature | 40°C |
| Sample Tray Temperature | Room Temperature |
| Injection Volume | 5 µL |
| Run Time | 19 min |

Solvent Gradient Conditions:

| Time (min) | Mobile Phase A (%) | Mobile Phase B (%) |
|---|---|---|
| 0 | 90 | 10 |
| 2 | 90 | 10 |
| 10 | 10 | 90 |
| 14 | 10 | 90 |
| 14.1 | 90 | 10 |
| 19 | 90 | 10 |

The structures of all compounds of the present invention can be determined by nuclear magnetic resonance (NMR) or mass spectrometry (MS). NMR shifts (δ) are recorded in units of 10⁻⁶ (ppm). NMR measurements were performed on a Bruker AVANCE-400 spectrometer. Deuterated solvents used for testing were deuterated chloroform (CDCl₃), deuterated methanol (MeOD), deuterated dimethyl sulfoxide (DMSO-D₆), with tetramethylsilane (TMS) as the internal standard.

Low-resolution mass spectrometry (MS) was determined by an Agilent 6120 quadruple LCMS mass spectrometer.

### Example 1: Synthesis of Compound 1

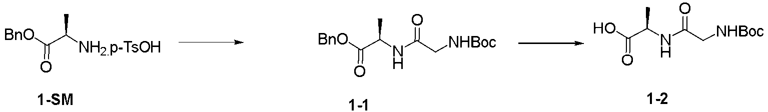

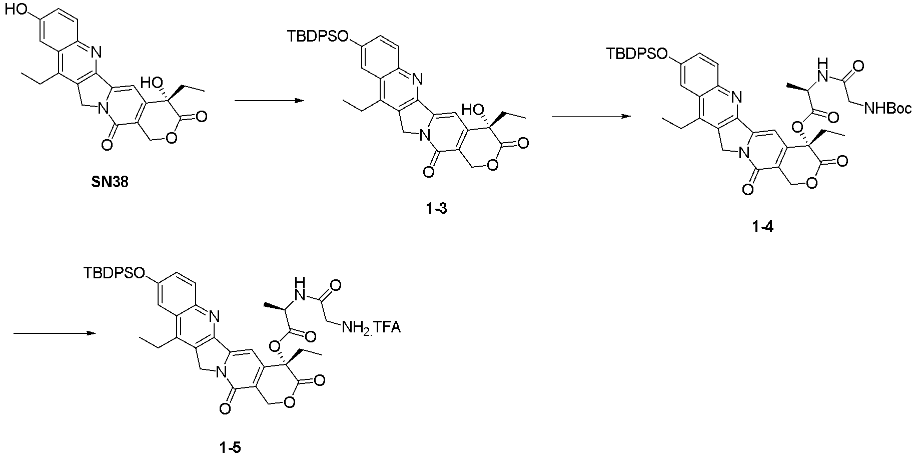

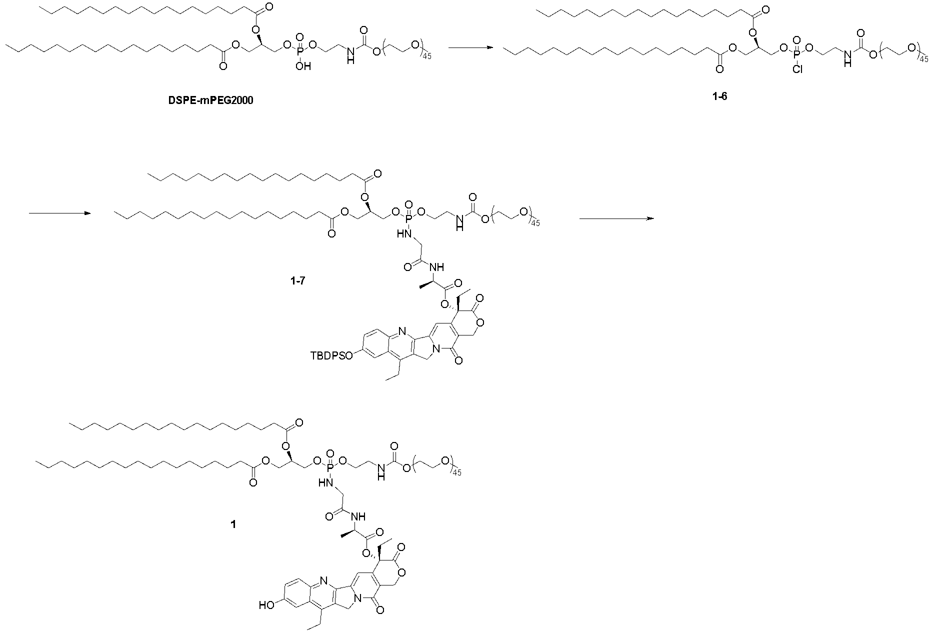

### Synthesis of Compound 1-1

At 0°C, to a solution of D-alanine benzyl ester p-toluenesulfonate (10 g, 28.5 mmol, 1.2 eq) in acetonitrile (160 mL) were successively added *N*-Boc glycine (4.2 g, 23.7 mmol, 1.0 eq), DIPEA (3.67 g, 28.5 mmol, 1.2 eq), TCFH (7.99 g, 28.5 mmol, 1.2 eq), and *N-*methylimidazole (4.9 g, 60.0 mmol, 2.1 eq). After the addition, the mixture was warmed to room temperature and stirred for 2 h. Upon completion of the reaction, the mixture was concentrated under reduced pressure at low temperature. The residue was purified by silica gel column chromatography (PE/EA = 1:1) to give a crude product. The crude product was further purified by C18 column chromatography (CH₃CN:H₂O = 5-50%, 0.05% TFA) to afford 8.2 g of pure white solid.

MS (ESI), m/z, 281.0 [M-55]⁺.

### Synthesis of Compound 1-2

At room temperature 25 °C, to a solution of **1-1** (8.2 g, 24.4 mmol, 1.0 eq) in methanol (200 mL) was added 10% Pd/C (800 mg). The atmosphere was replaced with H₂ (balloon), and the mixture was stirred under H₂ atmosphere at room temperature for 2 h. After the reaction was complete, MeOH (200 mL) was added, and the mixture was filtered through Celite. The filtrate was concentrated under reduced pressure and dried under vacuum to give 6.1 g of pure white solid.

MS (ESI), m/z, 191.0 [M-55]⁺.

### Synthesis of Compound 1-3

At room temperature 25 °C, to a solution of **SN38** (3.0 g, 7.65 mmol, 1.0 eq) in DMF (120 mL) were successively added dropwise TBDPSCl (7.4 g, 26.9 mmol, 3.5 eq) and Et₃N (3.6 g, 35.6 mmol, 4.5 eq). The mixture was stirred at room temperature for 3 h. After the reaction was complete, the reaction mixture was poured into ice water (400 mL), precipitating a large amount of solid. The solid was collected by filtration, washed with water, and pressed dry. The filter cake was dissolved in DCM (200 mL), washed twice with saturated NaCl (150 mL x 2, to remove residual triethylamine acetate), dried over anhydrous Na₂SO₄, concentrated under reduced pressure, and dried under vacuum to give 4.3 g of yellow solid.

MS (ESI), m/z, 631.20 [M+H]⁺.

### Synthesis of Compound 1-4

At room temperature 25 °C, to a solution of **1-3** (3.0 g, 4.76 mmol, 1.0 eq) in CH₂Cl₂ (80 mL) were successively added **1-2** (2.9 g, 11.9 mmol, 2.5 eq), DCC (2.5 g, 11.9 mmol, 2.5 eq), and DMAP (581 mg, 4.76 mmol, 1.0 eq). The mixture was stirred at room temperature for 2 h. Upon completion of the reaction, the mixture was filtered, and the filtrate was concentrated under reduced pressure. Purification by silica gel column chromatography (eluent: PE/EA = 1:1) afforded 2.9 g of pure product.

¹H NMR (400 MHz, Chloroform-d) δ 8.02 (d, *J* = 9.2 Hz, 1H), 7.76 (ddd, *J* = 8.0, 3.1, 1.4 Hz, 4H), 7.50 - 7.35 (m, 7H), 7.12 - 7.04 (m, 2H), 6.68 (d, *J* = 7.5 Hz, 1H), 5.65 (d, *J =* 17.2 Hz, 1H), 5.38 (d, *J* = 17.2 Hz, 1H), 5.10 (s, 2H), 4.79 - 4.64 (m, 1H), 3.94 (d, *J* = 15.6 Hz, 1H), 3.71 (dd, *J=* 16.7, 5.8 Hz, 1H), 3.21 (s, 2H), 2.64 (q, *J* = 7.6 Hz, 2H), 2.38 - 2.08 (m, 2H), *1.52 (d, J=* 8.0 Hz, 3H), 1.19 (s, 9H), 1.17 (s, 9H), 0.96 (t, *J =* 7.5 Hz, 3H), 0.89 (t, *J =* 8.1 Hz, 3H).

### Synthesis of Compound 1-5

At room temperature 25 °C, to a solution of **1-4** (2.2 g, 2.60 mmol, 1.0 eq) in CH₂Cl₂ (6 mL) was added dropwise TFA (6 mL). The mixture was stirred at room temperature for 2 h. Upon completion of the reaction, the mixture was concentrated, and the residue was thoroughly concentrated using an oil pump. CH₃CN (500 mL) was added and the mixture was concentrated under reduced pressure again to give 2.1 g of yellow solid.

MS (ESI), m/z, 759.20 [M+H]⁺.

### Synthesis of Compound 1-6

At room temperature 25 °C under N₂ protection, to a solution of **DSPE-mPEG2000** (2.07 g, 0.742 mmol, 1.0 eq) in CH₂Cl₂ (20 mL) was added DMF (200 µL, 3.71 mmol, 5.0 eq). The mixture was cooled to 0 °C, and oxalyl chloride (600 µL, 6.68 mmol, 9.0 eq) was added dropwise. After the dropwise addition, the mixture was stirred at room temperature for 3 h. Upon completion of the reaction, under N₂ protection, the mixture was concentrated under reduced pressure to give 2.0 g of crude white solid, which was used directly in the next step.

### Synthesis of Compound 1-7

At room temperature 25 °C under N₂ protection, the crude product **1-6** (2.0 g, 0.713 mmol, 1.0 eq) was dissolved in CH₂Cl₂ (15 mL). The mixture was cooled to 0 °C, and to the reaction mixture were successively added dropwise a solution of **1-5** (3.05 g, 3.56 mmol, 5.0 eq) in DCM (5 mL) and triethylamine (1.5 mL, 10.7 mmol, 15.0 eq). After the dropwise addition, the ice-water bath was removed, and the mixture was stirred at room temperature for 2 h. Upon completion of the reaction, the mixture was concentrated under reduced pressure, dissolved in DCM (50 mL), and washed three times with saturated NaCl (200 mL × 3). The organic phases were combined, dried over anhydrous Na₂SO₄, and concentrated under reduced pressure. The crude product was first purified by normal phase column chromatography (first using PE/EA = 1:1 - 0:1, then DCM/MeOH = 20:1 - 10:1 - 6:1), followed by C18 reverse phase column chromatography (THF: H₂O = 5-75%, t = 28 min, product peak gradient: 60%). The collected fractions were concentrated under reduced pressure at low temperature (<30 °C) to give 1.6 g of light yellow pure product.

### Synthesis of Compound 1

At 0 °C, to a solution of **1-7** (1.67 g, 0.49 mmol, 1.0 eq) in THF (24 mL) were added TBAF (980 µL, 0.98 mmol, 2.0 eq) and AcOH (143 mg, 2.45 mmol, 5.0 eq). The mixture was warmed to room temperature and stirred for 2 h. The reaction mixture was concentrated under reduced pressure, dissolved in DCM (100 mL), and washed twice with saturated NaCl (150 mL × 2). The organic phases were combined, dried over anhydrous Na₂SO₄, and concentrated under reduced pressure. The crude product was first purified by silica gel column chromatography (DCM/MeOH = 9:1), then further purified by neutral alumina column chromatography to give compound **1,** 770 mg, as a white solid.

MS (ESI), m/z, 606.9 [DAG-OH]⁺. 693.3 [M-DAG+4Na]⁴⁺. DAG (Diglyceride) is diacylglycerol.

¹H NMR(400MHz,CDCl₃, ppm): δ (ppm) 8.15-8.04 (m, 1H), 7.51-7.43 (m, 1H), 7.43-7.29 (m, 2H), 7.12 (s, 1H), 5.80-5.53 (m, 2H), 5.46-5.34 (m, 1H), 5.26-5.15 (m, 1H), 4.81-4.69 (m, 1H), 4.38-4.27 (m, 1H), 4.26-3.98 (m, 8H), 3.86-3.78 (m, 1H), 3.75-3.53 (m, 203H), 3.47-3.44 (m, 1H), 3.43-3.35 (m, 5H), 3.13-3.03 (m, 2H), 2.36-2.25 (m, 6H), 2.21-2.10 (m, 2H), 2.05-2.00 (m, 2H), 1.64-1.52 (m, 8H), 1.38-1.20 (m, 72H), 0.99 (t, J=8.0 Hz, 3H), 0.88 (t, J=8.0 Hz, 6H)

### Example 2: Synthesis of Compound 2

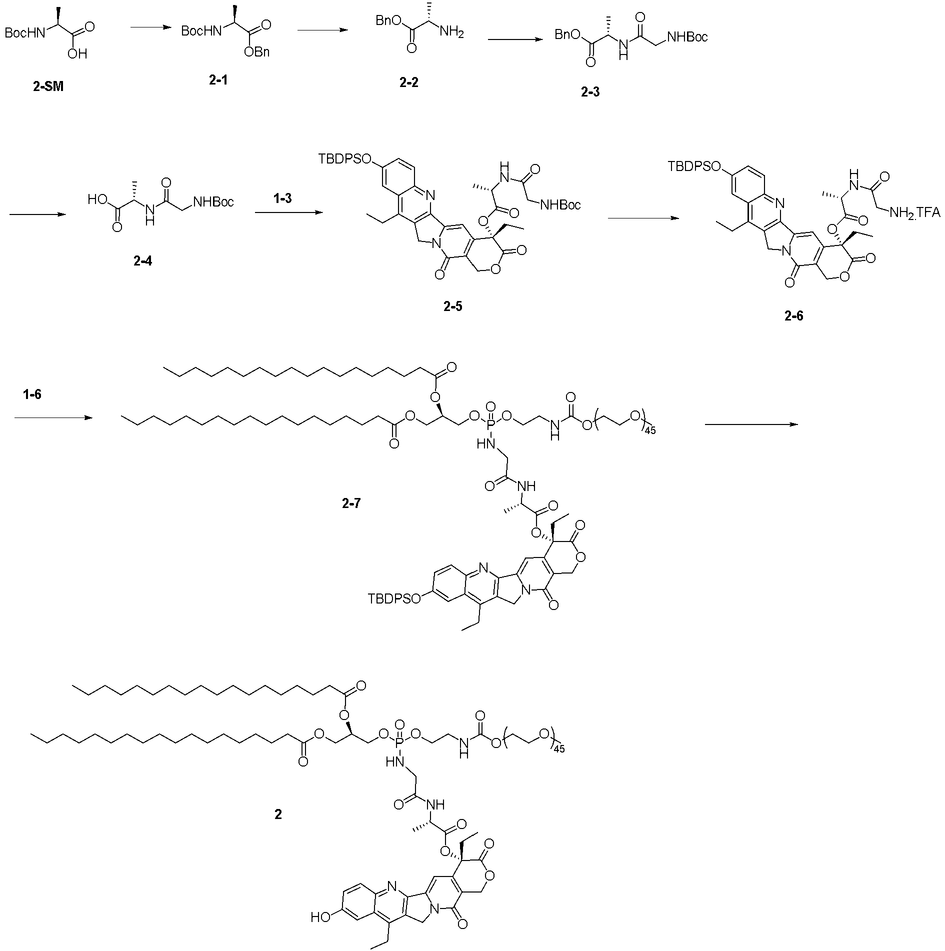

### Synthesis of Compound 2-1

At 0 °C, to a solution of *N*-tert-butoxycarbonyl-L-alanine (2.5 g, 13.2 mmol, 1.0 eq) in DMF (25 mL) was added cesium carbonate (8.6 g, 26.4 mmol, 2.0 eq). The mixture was stirred at room temperature for 30 min. Benzyl bromide (2.37 g, 13.86 mmol, 1.05 eq) was added dropwise. After the addition, the mixture was stirred at room temperature for 16 h. Upon completion of the reaction, the mixture was poured into water (150 mL), extracted with EA (100 mL × 2), washed with saturated brine (100 mL), dried over anhydrous sodium sulfate, and concentrated under reduced pressure. The residue was purified by reverse phase column chromatography (CH₃CN/H₂O = 5-70%, 0.05% TFA) to give 4.1 g of pure white solid.

### Synthesis of Compound 2-2

At 25 °C, **2-1** (4.1 g, 14.5 mmol, 1.0 eq) was dissolved in 4 M HCl/dioxane (20 mL) and stirred at room temperature for 3 h. Upon completion of the reaction, the mixture was concentrated under reduced pressure at low temperature. Thorough drying under vacuum afforded 3.6 g of pure white solid.

MS (ESI), m/z, 179.80 [M+H]⁺.

### Synthesis of Compound 2-3

At 0 °C, to a solution of **2-2** (3.18 g, 14.76 mmol, 1.2 eq) in acetonitrile (50 mL) were sequentially added N-Boc-glycine (2.15 g, 12.30 mmol, 1.0 eq), DIPEA (1.9 g, 14.76 mmol, 1.2 eq), TCFH (4.14 g, 14.76 mmol, 1.2 eq), and N-methylimidazole (3.54 g, 43.1 mmol, 3.5 eq). After the addition, the mixture was warmed to room temperature and stirred for 2 h. Upon completion of the reaction, the mixture was concentrated under reduced pressure at low temperature and purified by silica gel column chromatography (PE/EA = 2:1 - 1:1) to give 3.5 g of pure white solid.

MS (ESI), m/z, 280.80 [M-55]⁺.

### Synthesis of Compound 2-4

At room temperature (25 °C), to a solution of **2-3** (3.5 g, 10.4 mmol, 1.0 eq) in methanol (100 mL) was added 10% Pd/C (350 mg). The atmosphere was replaced with H₂ (balloon), and the mixture was stirred at room temperature under H₂ atmosphere for 4 h. Upon completion of the reaction, MeOH (200 mL) was added, filtered through Celite, and the filtrate was concentrated under reduced pressure. Purification by reverse phase column chromatography (CH₃CN:H₂O = 5-60%, 0.05% TFA) afforded pure white solid, 2.85 g.

MS (ESI), m/z, 190.80 [M-55]⁺.

### Synthesis of Compound 2-5

At room temperature (25 °C), to a solution of **1-3** (2.66 g, 4.22 mmol, 1.0 eq) in CH₂Cl₂ (20 mL) were sequentially added **2-4** (2.603 g, 10.54 mmol, 2.5 eq), EDCI (2.42 g, 12.67 mmol, 3.0 eq), and DMAP (1.03 g, 8.44 mmol, 2.0 eq). The mixture was stirred at room temperature for 2 h. Upon completion of the reaction, the mixture was concentrated under reduced pressure and purified by silica gel column chromatography (using PE/EA = 2:1 - 1:1 as eluent) to give 2.25 g of pure product.

### Synthesis of Compound 2-6

At room temperature (25 °C), TFA (20 mL) was added dropwise to a solution of **2-5** (2.4 g, 2.79 mmol, 1.0 eq) in CH₂Cl₂ (20 mL). The mixture was stirred at room temperature for 2 h. Upon completion of the reaction, the mixture was concentrated under reduced pressure to give the product as a yellow solid, 2.6 g.

MS (ESI), m/z, 759.20 [M-55]⁺.

### Synthesis of Compound 2-7

At room temperature (25 °C) under N₂ protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **2-6** (1.22 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was first purified by normal phase column chromatography (first using EA/MeOH = 1:0 - 9:1, then switching to the elution system DCM/MeOH = 95:5 - 90:10) to give 745 mg of crude product. The crude product was further purified by reverse phase column chromatography (THF/H₂O = 50:50). The obtained crude product was then purified by neutral alumina column chromatography to give 210 mg of pure product.

### Synthesis of Compound 2

At 0 °C, to a solution of **2-7** (200 mg, 0.057 mmol, 1.0 eq) in THF (15 mL) was added pyridine hydrofluoride solution (70%, 80 mg, 0.57 mmol, 10 eq). The mixture was stirred at room temperature for 2 h. A small amount of saturated aqueous NaHCO₃ solution was added to neutralize the pH to neutral. The mixture was concentrated under reduced pressure, and the crude product was purified by silica gel column chromatography (DCM/MeOH = 15:1 - 13:1 - 11:1) to give 110 mg of pure product.

MS (ESI), m/z, 607.0 [DAG-OH]⁺. 693.5 [M-DAG+4Na]⁴⁺. DAG (Diglyceride) is diacylglycerol.

### Example 3: Synthesis of Compound 3

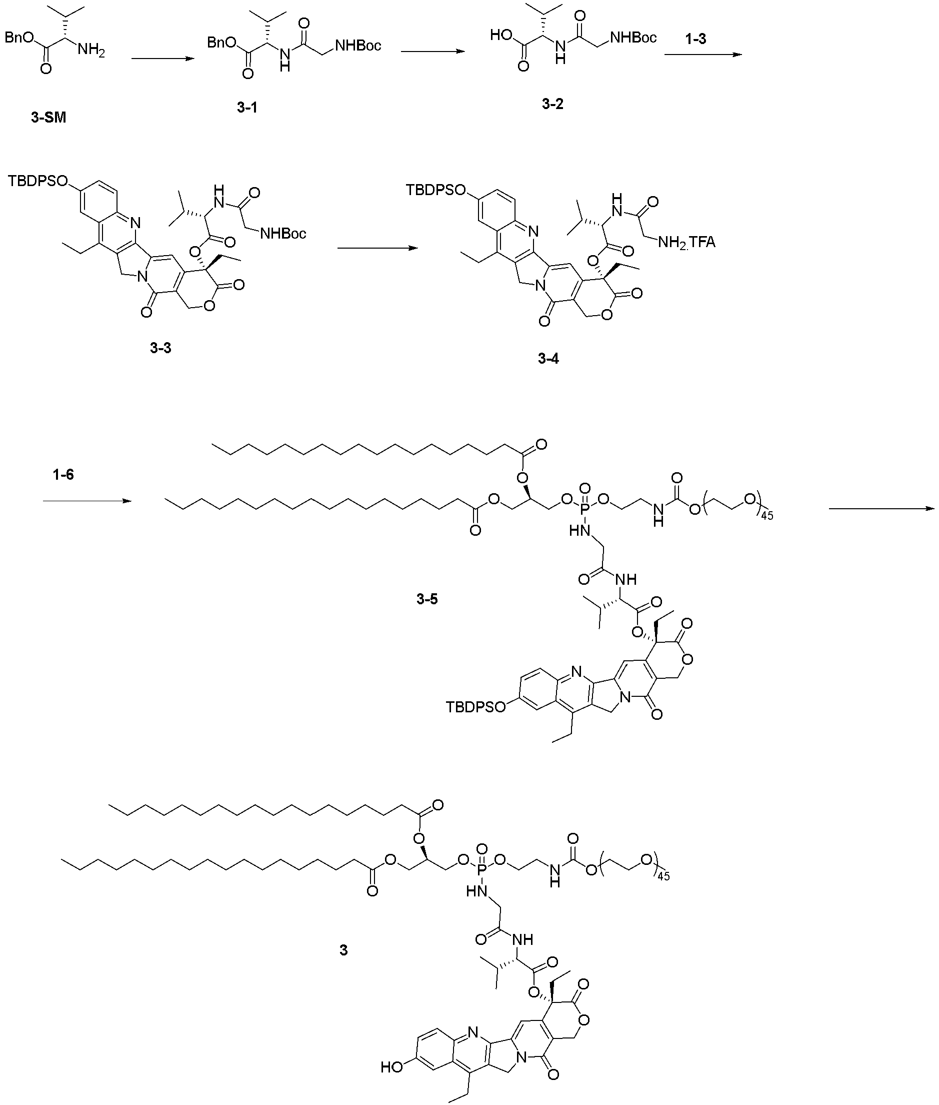

### Synthesis of Compound 3-1

At 0 °C, to a solution of *N*-Boc-glycine (3.3 g, 22.5 mmol, 1.1 eq) in DMF (20 mL) were sequentially added *L*-valine benzyl ester (4.2 g, 23.7 mmol, 1.0 eq) and HATU (9.6 g, 31.3 mmol, 1.5 eq). DIPEA (4.4 g, 41.0 mmol, 2.0 eq) was added dropwise slowly. After the addition, the mixture was stirred at room temperature for 2 h. Upon completion of the reaction, the mixture was poured into water (150 mL), extracted with EA (100 mL × 2), washed with saturated brine (100 mL), dried over anhydrous sodium sulfate, and concentrated under reduced pressure. The residue was purified by silica gel column chromatography (PE/EA = 1:1) to give 4.7 g of pure white solid.

MS (ESI), m/z, 364.80 [M+H]⁺.

### Synthesis of Compound 3-2

At room temperature (25 °C), to a solution of 3-1 (6.1 g, 15.8 mmol, 1.0 eq) in methanol (300 mL) was added 10% Pd/C (600 mg). The atmosphere was replaced with H₂ (balloon), and the mixture was stirred at room temperature under H₂ atmosphere for 4 h. Upon completion of the reaction, MeOH (200 mL) was added, filtered through Celite, and the filtrate was concentrated under reduced pressure. Purification by reverse phase column chromatography (CH₃CN:H₂O = 5-60%) afforded pure white solid, 2.9 g.

MS (ESI), m/z, 218.80 [M-55]⁺.

### Synthesis of Compound 3-3

At room temperature (25 °C), to a solution of **1-3** (1.7 g, 2.68 mmol, 1.0 eq) in CH₂Cl₂ (25 mL) were sequentially added **3-2** (2.2 g, 8.03 mmol, 3.0 eq), DCC (1.7 g, 8.03 mmol, 3.0 eq), and DMAP (327 mg, 2.68 mmol, 1.0 eq). The mixture was stirred at room temperature for 16 h. Upon completion of the reaction, the mixture was filtered, and the filtrate was concentrated under reduced pressure. Purification by silica gel column chromatography (using PE/EA = 1:1 as eluent) gave 2.6 g of yellow solid.

MS (ESI), m/z, 886.60 [M+H]⁺.

### Synthesis of Compound 3-4

At room temperature (25 °C), TFA (15 mL) was added dropwise to a solution of **3-3** (2.6 g, 2.93 mmol, 1.0 eq) in CH₂Cl₂ (15 mL). The mixture was stirred at room temperature for 2 h. Upon completion of the reaction, the mixture was concentrated under reduced pressure to give the product as a yellow solid, 2.7 g.

MS (ESI), m/z, 786.60 [M+H]⁺.

### Synthesis of Compound 3-5

At room temperature (25 °C) under N₂ protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **3-4** (1.26 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using the elution system EA/MeOH = 1:0 - 10:1, then switching to the elution system DCM/MeOH = 10:1 - 5:1, to give 589 mg of crude product. The crude product was further purified by neutral alumina column chromatography (DCM/MeOH = 98:2 - 97:3) to give 277 mg of light yellow solid.

### Synthesis of Compound 3

At 0 °C, to a solution of **3-5** (200 mg, 0.056 mmol, 1.0 eq) in THF (10 mL) was added pyridine hydrofluoride solution (70%, 100 mg, 0.707 mmol, 12.6 eq). The mixture was stirred at room temperature for 2 h. A small amount of saturated aqueous NaHCO₃ solution was added to neutralize the pH to neutral. The mixture was concentrated under reduced pressure, and the crude product was purified by silica gel column chromatography (DCM/MeOH = 15:1 - 13:1 - 11:1) to give 130 mg of pure product.

MS (ESI), m/z, 607.1 [DAG-OH]⁺. 700.6 [M-DAG+4Na]⁴⁺. DAG (Diglyceride) is diacylglycerol.

### Example 4: Synthesis of Compound 4

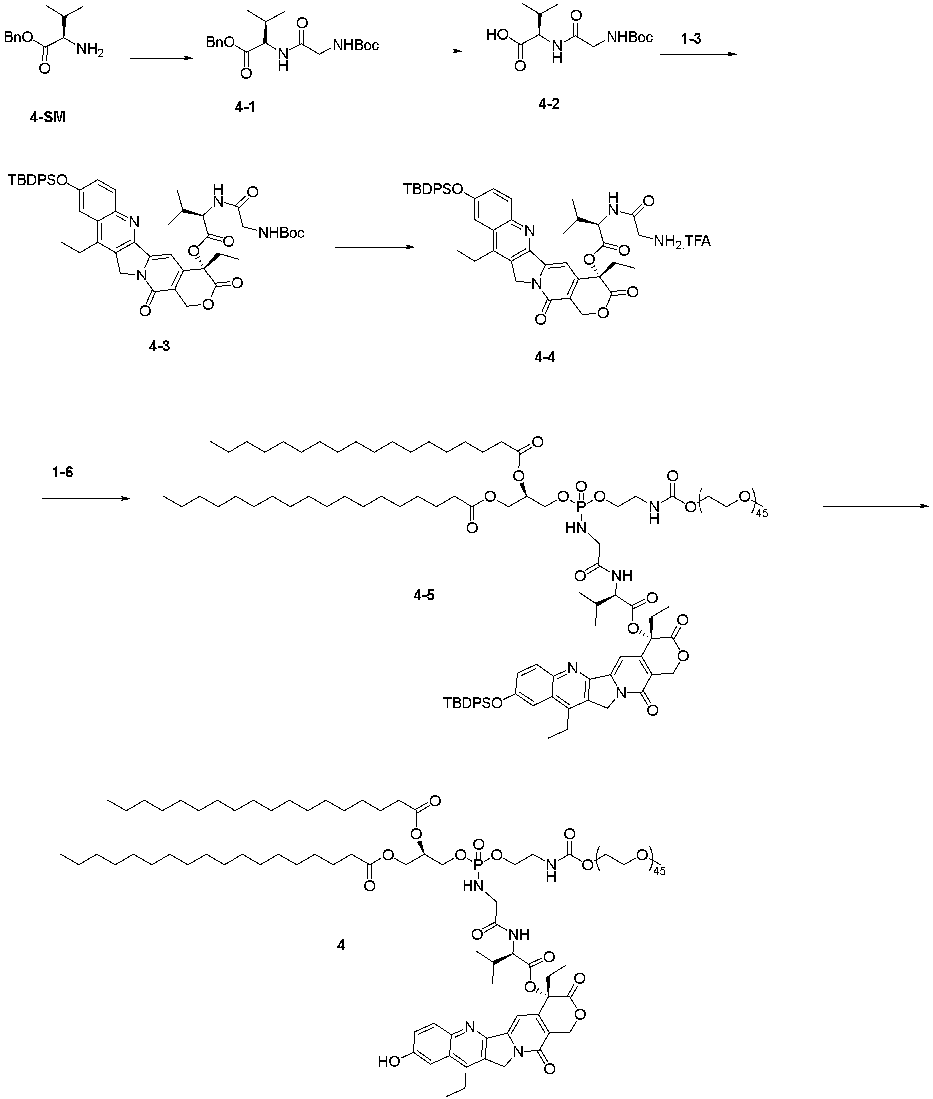

### Synthesis of Compound 4-1

At 0 °C, to a solution of D-valine benzyl ester (3.21 g, 13.2 mmol, 1.2 eq) in acetonitrile (50 mL) were sequentially added N-Boc-glycine (1.93 g, 11.0 mmol, 1.0 eq), DIPEA (1.7 g, 13.2 mmol, 1.2 eq), TCFH (3.7 g, 13.2 mmol, 1.2 eq), and N-methylimidazole (3.16 g, 38.5 mmol, 3.5 eq). After the addition, the mixture was warmed to room temperature and stirred for 2 h. Upon completion of the reaction, the acetonitrile was concentrated under reduced pressure at low temperature. The residue was purified by silica gel column chromatography (PE/EA = 1:1) to give crude product. The crude product was further purified by reverse phase C18 column chromatography (CH₃CN:H₂O = 5-50%) to give 3.5 g of pure white solid.

MS (ESI), m/z, 308.80 [M-55]⁺.

### Synthesis of Compound 4-2

At room temperature (25 °C), to a solution of **4-1** (2.6 g, 7.13 mmol, 1.0 eq) in methanol (100 mL) was added 10% Pd/C (260 mg). The atmosphere was replaced with H₂ (balloon), and the mixture was stirred at room temperature under H₂ atmosphere for 4 h. Upon completion of the reaction, MeOH (200 mL) was added, filtered through Celite, and the filtrate was concentrated under reduced pressure. Purification by reverse phase column chromatography (CH₃CN:H₂O = 5-60%) afforded pure white solid, 2.6 g.

MS (ESI), m/z, 218.80 [M-55]⁺.

### Synthesis of Compound 4-3

At room temperature (25 °C), to a solution of **1-3** (1.9 g, 2.94 mmol, 1.0 eq) in CH₂Cl₂ (20 mL) were sequentially added **4-2** (2.01 g, 7.34 mmol, 2.5 eq), EDCI (1.7 g, 8.82 mmol, 3.0 eq), and DMAP (717 mg, 5.88 mmol, 2.0 eq). The mixture was stirred at room temperature for 2 h. Upon completion of the reaction, the mixture was concentrated under reduced pressure and purified by silica gel column chromatography (using PE/EA = 2:1 - 1:1 as eluent) to give 2.25 g of pure product.

MS (ESI), m/z, 886.60 [M+H]⁺.

### Synthesis of Compound 4-4

At room temperature (25 °C), TFA (15 mL) was added dropwise to a solution of **4-3** (2.2 g, 2.60 mmol, 1.0 eq) in CH₂Cl₂ (15 mL). The mixture was stirred at room temperature for 2 h. Upon completion of the reaction, the mixture was concentrated under reduced pressure to give the product as a yellow solid, 2.2 g.

MS (ESI), m/z, 786.60 [M+H]⁺.

### Synthesis of Compound 4-5

At room temperature (25 °C) under N₂ protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **4-4** (1.26 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure, and the crude product was purified by normal phase column chromatography, first using an elution system of EA/MeOH = 1:0 - 9:1, then switching to an elution system of DCM/MeOH = 95:5 - 90:10, to give 745 mg of crude product. The crude product was further purified by reverse phase column chromatography (THF/H₂O = 50:50), and finally purified by neutral alumina column chromatography to give 280 mg of pure product.

### Synthesis of Compound 4

At 0 °C, to a solution of **4-5** (210 mg, 0.059 mmol, 1.0 eq) in THF (15 mL) was added pyridine hydrofluoride solution (70%, 0.59 mmol, 10 eq). The mixture was stirred at room temperature for 2 h. A small amount of saturated sodium bicarbonate solution was added to neutralize the pH to neutral. The mixture was concentrated under reduced pressure, and the crude product was purified by silica gel column chromatography (DCM/MeOH = 15:1 - 13:1 - 11:1) to give 120 mg of pure product.

MS (ESI), m/z, 606.7 [DAG-OH]⁺. 700.8 [M-DAG+4Na]⁴⁺. DAG (Diglyceride) is diacylglycerol.

### Example 5: Synthesis of Compound 5

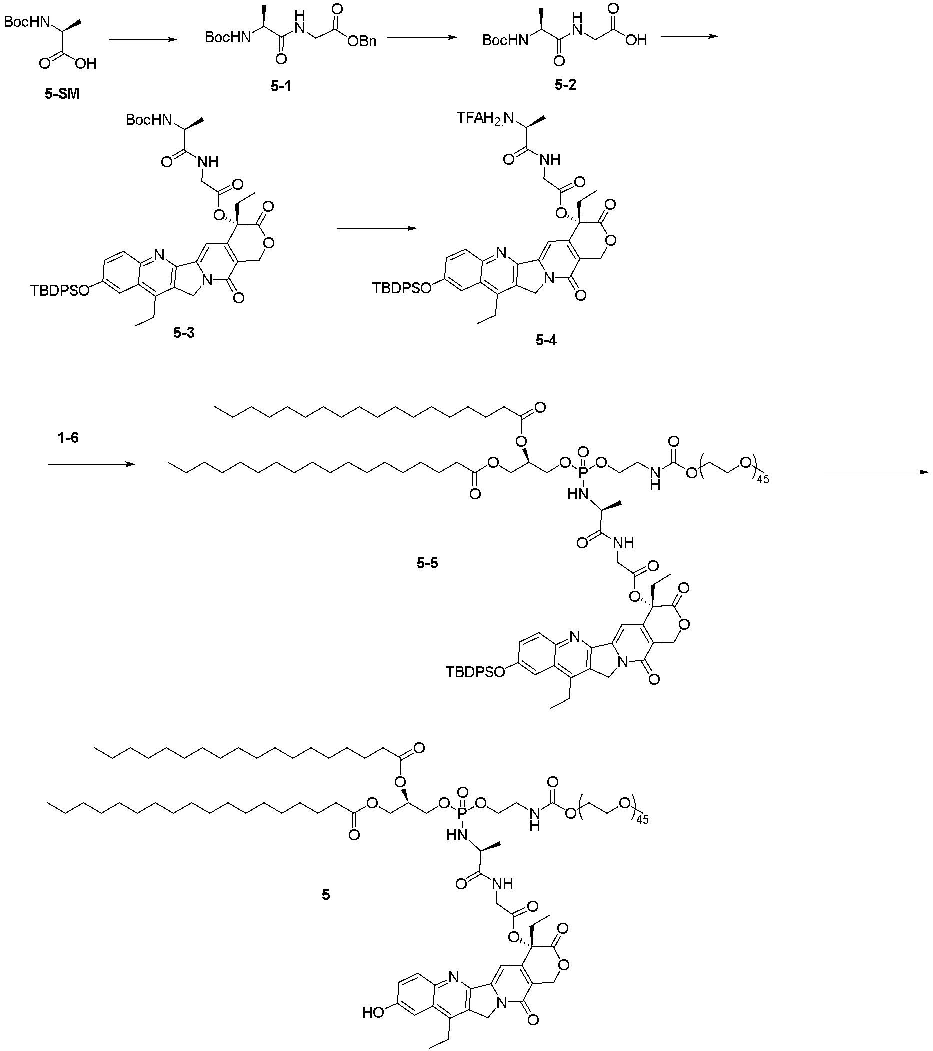

### Synthesis of Compound 5-1

At 0 °C, to a solution of glycine benzyl ester hydrochloride (6.7 g, 31.7 mmol, 1.2 eq) in acetonitrile (80 mL) were sequentially added N-Boc-L-alanine (5.0 g, 26.4 mmol, 1.0 eq), DIPEA (4.1 g, 31.7 mmol, 1.2 eq), TCFH (8.9 g, 31.7 mmol, 1.2 eq), and N-methylimidazole (7.6 g, 92.4 mmol, 3.5 eq). After the addition, the mixture was warmed to room temperature and stirred for 2 h. Upon completion of the reaction, the mixture was concentrated under reduced pressure at low temperature. The residue was purified by silica gel column chromatography (PE/EA = 1:1) to give 6.4 g of white solid.

MS (ESI), m/z, 280.80 [M-55]⁺.

### Synthesis of Compound 5-2

At room temperature (25 °C), to a solution of **5-1** (7.0 g, 20.8 mmol, 1.0 eq) in methanol (200 mL) was added 10% Pd/C (700 mg). The atmosphere was replaced with H₂ (balloon), and the mixture was stirred at room temperature under H₂ atmosphere for 2 h. Upon completion of the reaction, MeOH (200 mL) was added, filtered through Celite, and the filtrate was concentrated under reduced pressure and dried under vacuum to give a white solid, 4.1 g.

MS (ESI), m/z, 190.80 [M-55]⁺.

### Synthesis of Compound 5-3

At room temperature (25 °C), to a solution of **1-3** (2.1 g, 3.3 mmol, 1.0 eq) in CH₂Cl₂ (70 mL) were sequentially added **5-2** (2.01 g, 8.16 mmol, 2.5 eq), EDCI (1.9 g, 9.9 mmol, 3.0 eq), and DMAP (610 mg, 5.0 mmol, 1.5 eq). The mixture was stirred at room temperature for 2 h. Upon completion of the reaction, the mixture was concentrated under reduced pressure and purified by silica gel column chromatography (using PE/EA = 2:1 - 1:1 as eluent) to give 2.2 g of yellow-green solid.

MS (ESI), m/z, 859.80 [M+H]⁺.

### Synthesis of Compound 5-4

At room temperature (25 °C), TFA (15 mL) was added dropwise to a solution of **5-3** (2.5 g, 2.91 mmol, 1.0 eq) in CH₂Cl₂ (15 mL). The mixture was stirred at room temperature for 2 h. Upon completion of the reaction, the mixture was concentrated under reduced pressure to give the product as a yellow solid, 2.6 g.

MS (ESI), m/z, 759.80 [M+H]⁺.

### Synthesis of Compound 5-5

At room temperature (25 °C) under N₂ protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **5-4** (1.22 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure, and the crude product was purified by normal phase column chromatography, first using an elution system of EA/MeOH = 1:0 - 10:1, then switching to an elution system of DCM/MeOH = 15:1 - 10:1, to give 290 mg of the product.

### Synthesis of Compound 5

At 0 °C, to a solution of **5-5** (440 mg, 0.125 mmol, 1.0 eq) in THF (15 mL) were added TBAF (154 µL, 0.154 mmol, 1.23 eq) and acetic acid (38 mg, 0.633 mmol, 5.0 eq). The mixture was warmed to room temperature and stirred for 2 h. The reaction mixture was concentrated under reduced pressure, dissolved in DCM (100 mL), and washed with water three times (150 mL × 2). The organic phases were combined, dried over anhydrous Na₂SO₄, and concentrated under reduced pressure. The crude product was purified by silica gel column chromatography (DCM/MeOH = 15:1 - 13:1 - 11:1) to give 170 mg of pure product.

MS (ESI), m/z, 607.0 [DAG-OH]⁺. 693.4 [M-DAG+4Na]⁴⁺. DAG (Diglyceride) is diacylglycerol.

¹H NMR (400 MHz, Chloroform-d) δ (ppm) 8.63 (s, 1H), 8.06 (d, *J* = 9.2 Hz, 1H), 7.58 - 7.33 (m, 3H), 7.21 (d, *J* = 2.6 Hz, 1H), 7.10 (ddd, *J* = 18.3, 8.5, 2.6 Hz, 1H), 5.69 (dt, *J* = 17.0, 2.6 Hz, 1H), 5.57 (d, *J* = 16.1 Hz, 1H), 5.37 (d, *J=* 17.1 Hz, 1H), 5.26 - 5.05 (m, 3H), 4.40 (dd, *J=* 17.9, 6.0 Hz, 1H), 4.30 - 3.74 (m, 6H), 3.71-3.52 (s, 193H), 3.38 (s, 3H), 3.31 - 3.00 (m, 2H), 2.38 - 2.17 (m, 5H), 1.57 (q, *J* = 7.1 Hz, 5H), 1.46 - 1.14 (m, 116H), 1.00 (t, *J* = 7.4 Hz, 4H), 0.88 (t, *J* = 6.7 Hz, 9H).

### Example 6: Synthesis of Compound 6

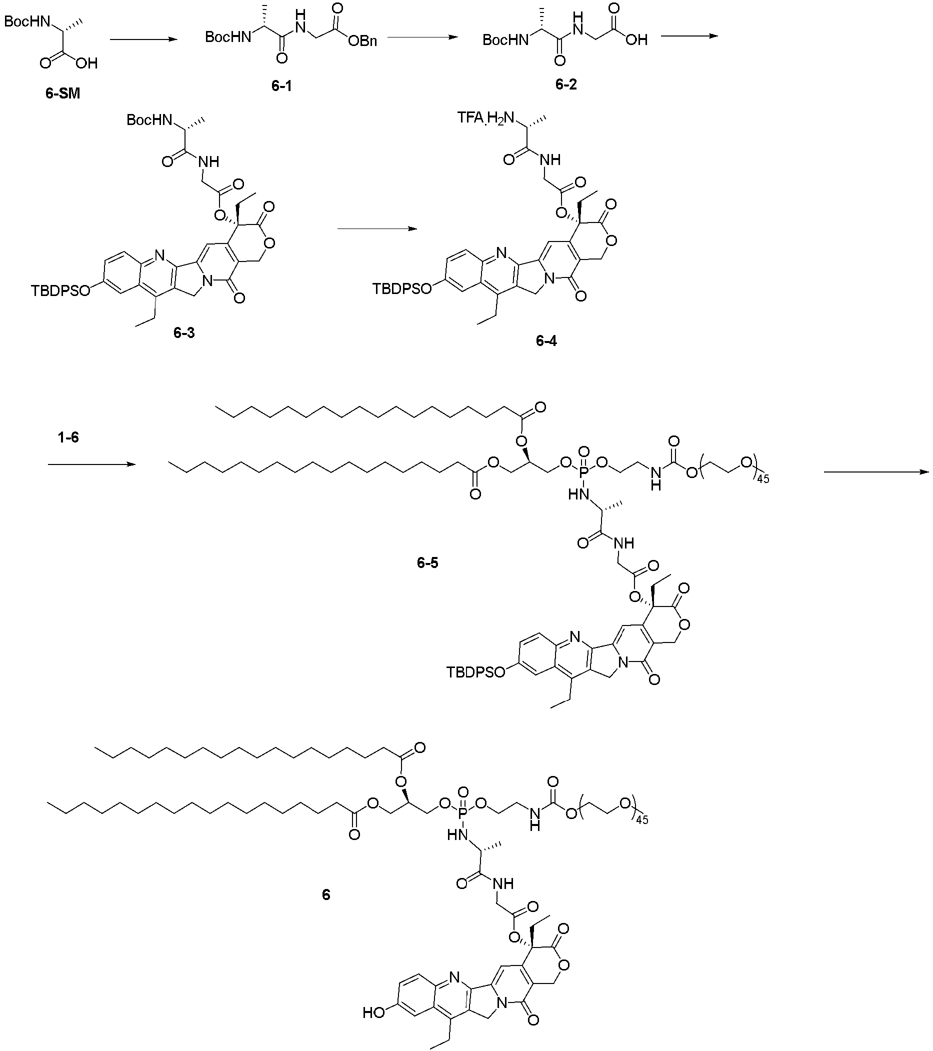

### Synthesis of Compound 6-1

At 0 °C, to a solution of glycine benzyl ester hydrochloride (6.7 g, 31.7 mmol, 1.2 eq) in acetonitrile (80 mL) were sequentially added N-Boc-D-alanine (5.0 g, 26.4 mmol, 1.0 eq), DIPEA (4.1 g, 31.7 mmol, 1.2 eq), TCFH (8.9 g, 31.7 mmol, 1.2 eq), and N-methylimidazole (7.6 g, 92.4 mmol, 3.5 eq). After the addition, the mixture was warmed to room temperature and stirred for 2 h. Upon completion of the reaction, the acetonitrile was concentrated under reduced pressure at low temperature. The residue was purified by silica gel column chromatography (PE/EA = 1:1) to give 6.4 g of white solid.

MS (ESI), m/z, 280.80 [M-55]⁺.

### Synthesis of Compound 6-2

At room temperature (25 °C), to a solution of **6-1** (7.0 g, 20.8 mmol, 1.0 eq) in methanol (200 mL) was added 10% Pd/C (700 mg). The atmosphere was replaced with H₂ (balloon), and the mixture was stirred at room temperature under H₂ atmosphere for 2 h. Upon completion of the reaction, MeOH (200 mL) was added, filtered through Celite, and the filtrate was concentrated under reduced pressure and dried under vacuum to give a white solid, 4.1 g.

MS (ESI), m/z, 190.80 [M-55]⁺.

### Synthesis of Compound 6-3

At room temperature (25 °C), to a solution of **1-3** (2.1 g, 3.3 mmol, 1.0 eq) in CH₂Cl₂ (70 mL) were sequentially added **6-2** (2.01 g, 8.16 mmol, 2.5 eq), EDCI (1.9 g, 9.9 mmol, 3.0 eq), and DMAP (610 mg, 5.0 mmol, 1.5 eq). The mixture was stirred at room temperature for 2 h. Upon completion of the reaction, the mixture was concentrated under reduced pressure and purified by silica gel column chromatography (using PE/EA = 2:1 - 1:1 as eluent) to give 2.1 g of yellow-green solid.

MS (ESI), m/z, 859.80 [M+H]⁺.

### Synthesis of Compound 6-4

At room temperature (25 °C), TFA (15 mL) was added dropwise to a solution of **6-3** (2.5 g, 2.91 mmol, 1.0 eq) in CH₂Cl₂ (15 mL). The mixture was stirred at room temperature for 2 h. Upon completion of the reaction, the mixture was concentrated under reduced pressure to give the product as a yellow solid, 2.9 g.

MS (ESI), m/z, 759.80 [M+H]⁺.

### Synthesis of Compound 6-5

At room temperature (25 °C) under N₂ protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **6-4** (1.22 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 10:1, to give 170 mg of the product.

### Synthesis of Compound 6

At 0 °C, to a solution of **6-5** (170 mg, 0.048 mmol, 1.0 eq) in THF (15 mL) were added TBAF (60 µL, 0.060 mmol, 1.25 eq) and acetic acid (15 mg, 0.25 mmol, 5.2 eq). The mixture was warmed to room temperature and stirred for 2 h. The reaction mixture was concentrated under reduced pressure, dissolved in DCM (100 mL), and washed with water three times (150 mL × 2). The organic phases were combined, dried over anhydrous Na₂SO₄, and concentrated under reduced pressure. The crude product was purified by silica gel column chromatography (DCM/MeOH = 15:1 - 13:1 - 11:1) to give 95 mg of pure product.

MS (ESI), m/z, 606.9 [DAG-OH]⁺. 693.3 [M-DAG+4Na]⁴⁺. DAG (Diglyceride) is diacylglycerol.

### Example 7: Synthesis of Compound 7

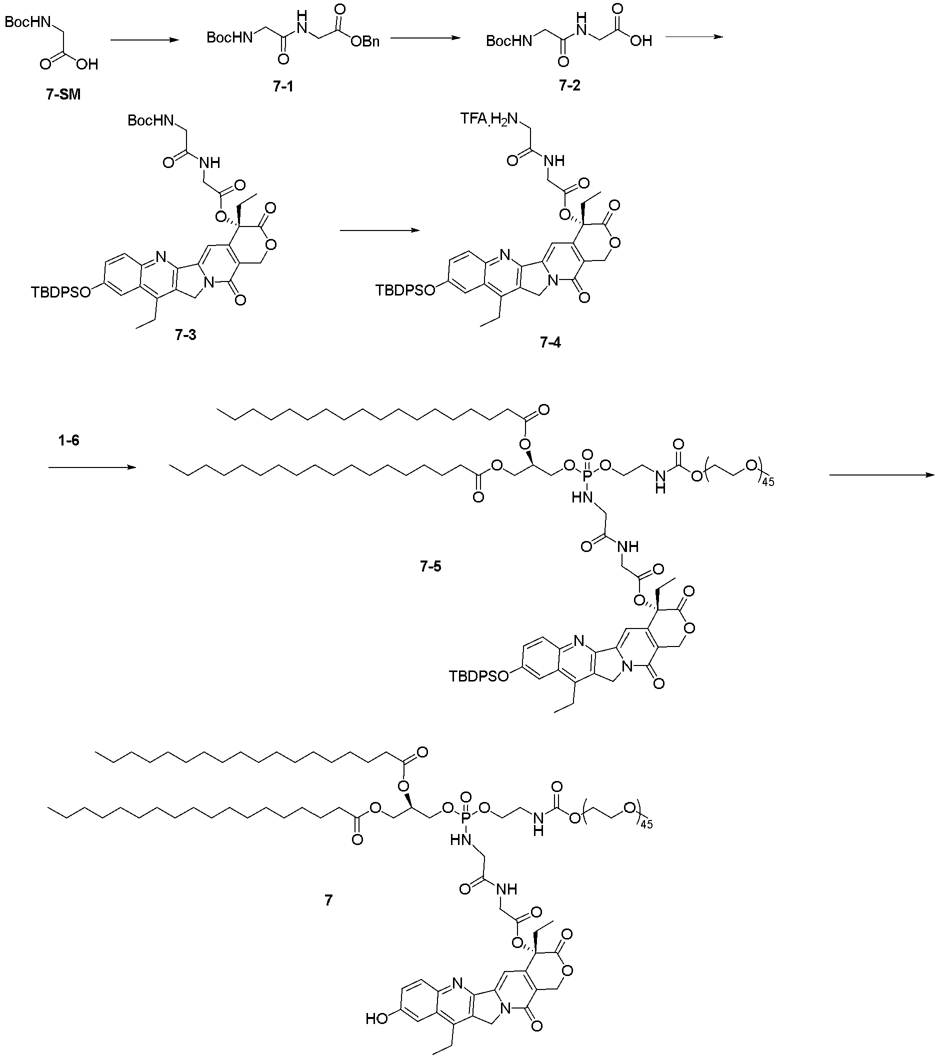

### Synthesis of Compound 7-1

At 0 °C, to a solution of glycine benzyl ester hydrochloride (4.2 g, 20 mmol, 1.0 eq) in DMF (20 mL) were sequentially added Boc-glycine (3.5 g, 20 mmol, 1.0 eq) and HATU (11.4 g, 30 mmol, 1.5 eq). DIPEA (7.74 g, 60 mmol, 3.0 eq) was added dropwise slowly. After the addition, the mixture was stirred at room temperature for 2 h. Upon completion of the reaction, the mixture was poured into water (150 mL), extracted with EA (100 mL × 2), washed with saturated brine (100 mL), dried over anhydrous sodium sulfate, concentrated under reduced pressure, and the residue was purified by silica gel column chromatography (PE/EA = 1:1) to give 4.8 g of pure white solid.

MS (ESI), m/z, 266.80 [M+H]⁺.

### Synthesis of Compound 7-2

At room temperature (25 °C), 10% Pd/C (500 mg) was added to a solution of **7-1** (5.0 g, 15.5 mmol, 1.0 eq) in methanol (100 mL). The atmosphere was replaced with H₂ (balloon), and the mixture was stirred at room temperature under H₂ atmosphere for 2 h. Upon completion of the reaction, MeOH (200 mL) was added, filtered through Celite, the filtrate was concentrated under reduced pressure, and purified by reverse phase column chromatography (CH₃CN:H₂O = 5-70%) to give a white solid, 3.0 g.

MS (ESI), m/z, 176.80 [M-55]⁺.

### Synthesis of Compound 7-3

At room temperature (25 °C), to a solution of **1-3** (3.4 g, 5.39 mmol, 1.0 eq) in CH₂Cl₂ (20 mL) were sequentially added **7-2** (2.5 g, 10.78 mmol, 2.0 eq), EDCI (3.1 g, 16.17 mmol, 3.0 eq), and DMAP (1.32 g, 10.78 mmol, 2.0 eq). The mixture was stirred at room temperature for 2 h. Upon completion of the reaction, the mixture was concentrated under reduced pressure and purified by silica gel column chromatography (using PE/EA = 2:1 - 1:1 as eluent) to give 2.0 g of yellow-green solid.

MS (ESI), m/z, 844.60 [M+H]⁺.

### Synthesis of Compound 7-4

At room temperature (25 °C), TFA (10 mL) was added dropwise to a solution of **7-3** (2.0 g, 2.36 mmol, 1.0 eq) in CH₂Cl₂ (10 mL). The mixture was stirred at room temperature for 2 h. Upon completion of the reaction, the mixture was concentrated under reduced pressure, and the residue was thoroughly dried under oil pump vacuum. Acetonitrile (500 mL × 3) was added and concentrated under reduced pressure to remove residual TFA, yielding 2.0 g of yellow solid.

MS (ESI), m/z, 744.60 [M+H]⁺.

### Synthesis of Compound 7-5

At room temperature (25 °C) under N₂ protection, crude product **1-6** (800 mg, 0.29 mmol, 1.0 eq) was dissolved in CH₂Cl₂ (20 mL). The temperature was lowered to 0 °C, and to the reaction mixture were sequentially added dropwise a solution of **7-4** (1.2 g, 1.43 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 10:1, to give a crude product, which was finally purified by neutral alumina column chromatography to give 290 mg of pure product.

### Synthesis of Compound 7

At 0 °C, to a solution of **7-5** (140 mg, 0.042 mmol, 1.0 eq) in THF (3 mL) was added pyridine hydrofluoride solution (70%, 60 mg, 0.42 mmol, 10 eq). The mixture was stirred at room temperature for 2 h. A small amount of saturated aqueous NaHCO₃ solution was added to neutralize the pH to neutral, concentrated under reduced pressure, and the crude product was purified by silica gel column chromatography (DCM/MeOH = 15:1 - 13:1 - 11:1) to give 90 mg of pure product.

MS (ESI), m/z, 606.8 [DAG-OH]⁺. 689.8 [M-DAG+4Na]⁴⁺. DAG (Diglyceride) is diacylglycerol.

¹H NMR (400 MHz, Chloroform-d) δ 8.50 (s, 1H), 8.12 - 8.00 (m, 1H), 7.93 (d, *J =* 8.4 Hz, 1H), 7.55 - 7.32 (m, 2H), 7.18 (d, *J=* 1.9 Hz, 1H), 7.10 (d, *J=* 8.6 Hz, 1H), 5.76 - 5.53 (m, 2H), 5.37 (d, *J=* 17.0 Hz, 1H), 5.26 - 5.04 (m, 3H), 4.48 - 3.75 (m, 12H), 3.72 - 3.41 (m, 165H), 3.37 (s, 3H), 3.19 (dq, *J* = 86.4, 7.7, 6.5 Hz, 3H), 2.39 - 2.09 (m, 6H), 1.58 (q, *J* = 9.2, 8.4 Hz, 5H), 1.45 - 1.15 (m, 91H), 0.99 (t, *J=* 7.4 Hz, 3H), 0.91 - 0.82 (m, 14H).

### Example 8: Synthesis of Compound 8

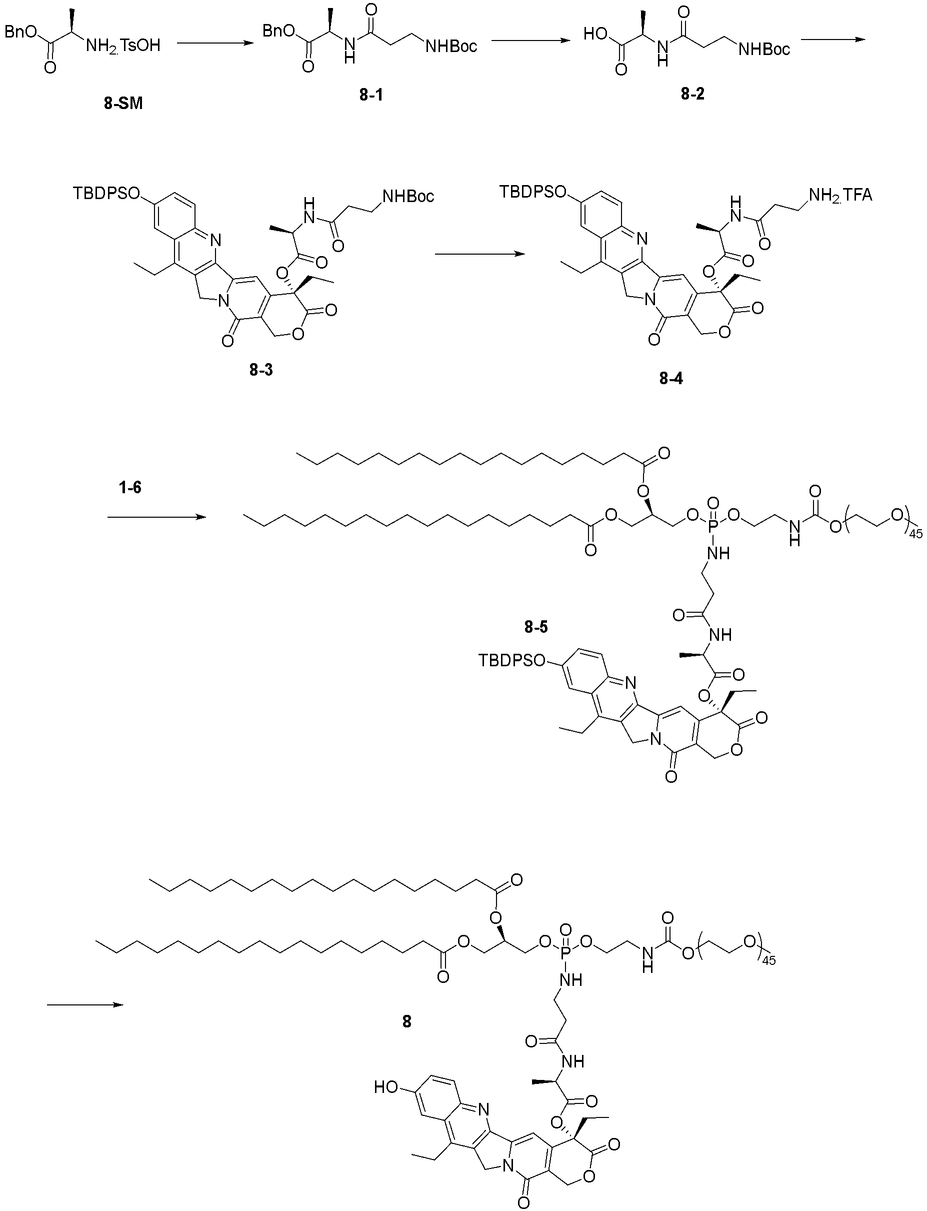

### Synthesis of Compound 8-1

At 0 °C, to a solution of *D*-alanine benzyl ester p-toluenesulfonate (10.2 g, 29.07 mmol, 1.1 eq) in acetonitrile (80 mL) were sequentially added *N*-Boc-*β*-alanine (5.0 g, 26.42 mmol, 1.0 eq), DIPEA (4.09 g, 31.7 mmol, 1.2 eq), TCFH (8.9 g, 31.7 mmol, 1.2 eq), and *N-*methylimidazole (8.3 g, 101.75 mmol, 3.5 eq). After the addition, the mixture was warmed to room temperature and stirred for 2 h. Upon completion of the reaction, the acetonitrile was concentrated under reduced pressure at low temperature. The residue was purified by silica gel column chromatography (PE/EA = 1:1) to give 8.8 g of white solid.

MS (ESI), m/z, 295.20 [M-55]⁺.

### Synthesis of Compound 8-2

At room temperature (25 °C), 10% Pd/C (880 mg) was added to a solution of **8-1** (8.8 g, 25.11 mmol, 1.0 eq) in methanol (100 mL). The atmosphere was replaced with H₂ (balloon), and the mixture was stirred at room temperature under H₂ atmosphere for 2 h. Upon completion of the reaction, MeOH (200 mL) was added, filtered through Celite, the filtrate was concentrated under reduced pressure and dried under vacuum to give a white solid, 6.0 g.

MS (ESI), m/z, 205.20 [M-55]⁺.

### Synthesis of Compound 8-3

At room temperature (25 °C), to a solution of **1-3** (4.0 g, 6.44 mmol, 1.0 eq) in CH₂Cl₂ (50 mL) were sequentially added **8-2** (5.0 g, 19.3 mmol, 3.0 eq), DCC (4.6 g, 22.54 mmol, 3.5 eq), and DMAP (1.2 g, 9.66 mmol, 1.5 eq). The mixture was stirred at room temperature for 2 h. Upon completion of the reaction, the mixture was filtered, the filtrate was concentrated under reduced pressure, and purified by silica gel column chromatography (using PE/EA = 1:1 as eluent) to give 6.0 g of pure product.

MS (ESI), m/z, 817.20 [M-55]⁺.

### Synthesis of Compound 8-4

At room temperature (25 °C), TFA (10 mL) was added dropwise to a solution of **8-3** (2.5 g, 2.86 mmol, 1.0 eq) in CH₂Cl₂ (10 mL). The mixture was stirred at room temperature for 2 h. Upon completion of the reaction, the mixture was concentrated under reduced pressure to give the product as a yellow solid, 2.5 g.

MS (ESI), m/z, 773.20 [M+H]⁺.

### Synthesis of Compound 8-5

At room temperature (25 °C) under nitrogen protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **8-4** (1.24 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 13:1 - 11:1, to give 730 mg of crude product, which was directly used in the next step.

### Synthesis of Compound 8

At 0 °C, to a solution of **8-5** (686 mg, 0.19 mmol, 1.0 eq) in THF (20 mL) was added pyridine hydrofluoride solution (70%, 315 mg, 2.22 mmol, 12 eq). The mixture was stirred at room temperature for 2 h. A small amount of saturated aqueous NaHCO₃ solution was added to neutralize the pH to neutral, concentrated under reduced pressure, and the crude product was purified by silica gel column chromatography (DCM/MeOH = 15:1 - 13:1 - 11:1) to give 300 mg of pure product.

MS (ESI), m/z, 607.0 [DAG-OH]⁺. 697.1 [M-DAG+4Na]⁴⁺. DAG (Diglyceride) is diacylglycerol.

¹H NMR (400 MHz, Chloroform-d) δ 7.96 (d, *J* = 9.0 Hz, 1H), 7.48 - 7.31 (m, 2H), 7.19 (d, *J* = 14.2 Hz, 1H), 7.03 (d, *J =* 7.3 Hz, 1H), 5.68 (dd, *J =* 17.1, 3.6 Hz, 1H), 5.40 (dd, *J* = 17.1, 2.7 Hz, 1H), 5.27 - 5.04 (m, 3H), 4.60-4.44 (m, 1H), 4.40 - 3.90 (m, 5H), 3.87-3.77 (m, 1H), 3.77-3.42 (m, 219H), 3.38 (s, 3H), 3.33-2.90 (m, 2H), 2.72-2.57 (m, 1H), 2.52 - 1.93 (m, 6H), 1.68-1.50 (m, 8H), 1.40-1.20 (m, 79H), 0.97 (td, *J* = 7.4, 5.3 Hz, 2H), 0.88 (t, *J* = 6.7 Hz, 6H).

### Example 9: Synthesis of Compound 9

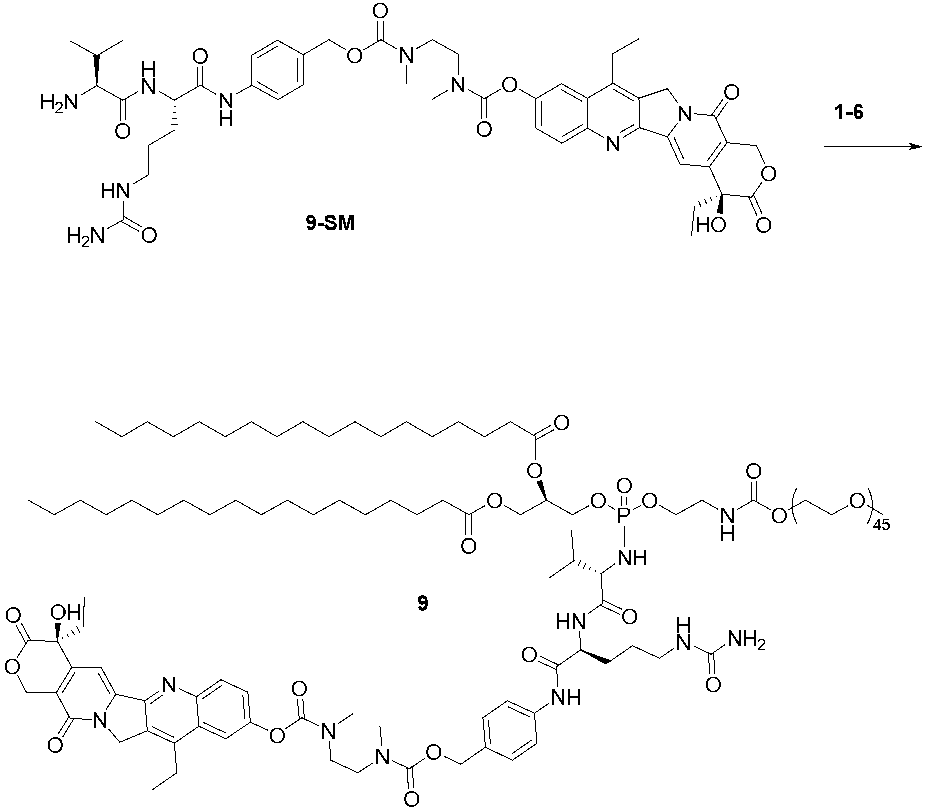

### Synthesis of Compound 9

At room temperature (25 °C) under N₂ protection, crude product **1-6** (800 mg, 0.29 mmol, 1.0 eq) was dissolved in CH₂Cl₂ (20 mL). The temperature was lowered to 0 °C, and to the reaction mixture were sequentially added dropwise a solution of **9-SM** (prepared according to the synthesis method described in Example 10114A of patent WO2023078464A1) (1.3 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 13:1 - 11:1, to give 660 mg of white solid.

MS (ESI), m/z, 607.0 [DAG-OH]⁺. 791.4 [M-DAG+4Na]⁴⁺. DAG (Diglyceride) is diacylglycerol.

### Example 10: Synthesis of Compound 10

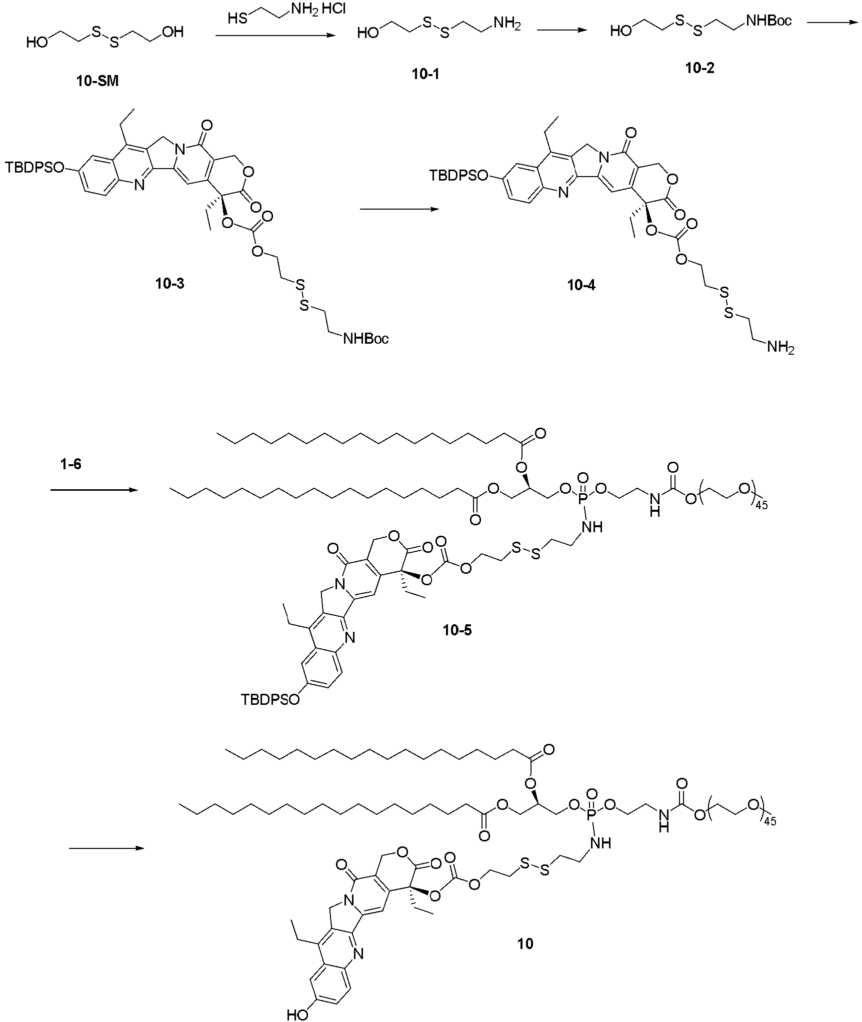

### Synthesis of Compounds 10-1 and 10-2

In an ice-water bath under nitrogen protection, to a solution of cysteamine hydrochloride (1.50 g, 13.2 mmol, 1.0 eq) in methanol (18 mL) were added triethylamine (1.85 mL, 26.4 mmol, 2.0 eq) and a solution of 2-hydroxyethyl disulfide (1.50 g, 13.2 mmol, 1.0 eq) in dichloromethane, and the mixture was stirred at room temperature for 16 h. The above solution of 10-1 was cooled to 0 °C, and di-tert-butyl dicarbonate (4.32 g, 19.8 mmol, 1.5 eq) was added. The mixture was stirred at room temperature for 4 h. LCMS monitoring indicated completion of the reaction. The reaction mixture was directly concentrated and purified by column chromatography (petroleum ether: ethyl acetate = 3:1) to give the compound as a colorless oil (0.9 g, with a yield of 26.9%).

MS (ESI), m/z, 154.0 [M-100]⁺.

### Synthesis of Compound 10-3

At 0 °C, to a solution of **10-2** (1.90 g, 7.5 mmol, 1.5 eq) in CH₂Cl₂ (25 mL) were sequentially added Et₃N (757 mg, 7.5 mmol, 1.5 eq) and bis(4-nitrophenyl) carbonate (2.28 g, 7.5 mmol, 1.5 eq), and the mixture was stirred at the same temperature for 2 h. To the above reaction mixture were added DMAP (610 mg, 5.00 mmol, 1.0 eq) and **1-3** (3.15 g, 5.00 mmol, 1.0 eq). The mixture was stirred at room temperature for 2 h. Upon completion of the reaction, the reaction mixture was poured into saturated aqueous ammonium chloride solution and thoroughly washed (150 mL × 3). The organic phases were combined, dried over anhydrous Na₂SO₄, and concentrated under reduced pressure. The crude product was purified by silica gel column chromatography using PE/EA = 1:1 as eluent to give 2.1 g of the product.

MS (ESI), m/z, 909.1 [M+H]⁺.

### Synthesis of Compound 10-4

At 0 °C, a solution of **10-3** (1.7 g, 1.80 mmol, 1.0 eq) in formic acid (20 mL) was stirred for 2 h. Upon completion of the reaction, the mixture was concentrated under reduced pressure and purified by C18 reverse phase column chromatography using CH₃CN /H₂O = 5-75% to give 1.2 g of yellow solid.

MS (ESI), m/z, 809.0 [M+H]⁺.

### Synthesis of Compound 10-5

At room temperature (25 °C) under N₂ protection, crude product **1-6** (800 mg, 0.285 mmol, 1.0 eq) was dissolved in CH₂Cl₂ (20 mL). The temperature was lowered to 0 °C, and to the reaction mixture were sequentially added dropwise a solution of **10-4** (1.1 g, 1.425 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 13:1 - 11:1, to give 960 mg of white solid.

### Synthesis of Compound 10

At 0 °C, to a solution of **10-5** (700 mg, 0.19 mmol, 1.0 eq) in THF (20 mL) was added pyridine hydrofluoride solution (70%, 320 mg, 2.22 mmol, 12.0 eq). The mixture was stirred at room temperature for 2 h. A small amount of saturated aqueous NaHCO₃ solution was added to neutralize the pH to neutral, concentrated under reduced pressure, and the crude product was purified by silica gel column chromatography (DCM/MeOH = 15:1 - 13:1 - 11:1) to give 370 mg of pure product.

MS (ESI), m/z, 606.7 [DAG-OH]⁺. 706.3 [M-DAG+4Na]⁴⁺. DAG (Diglyceride) is diacylglycerol.

### Example 11: Synthesis of Compound 11

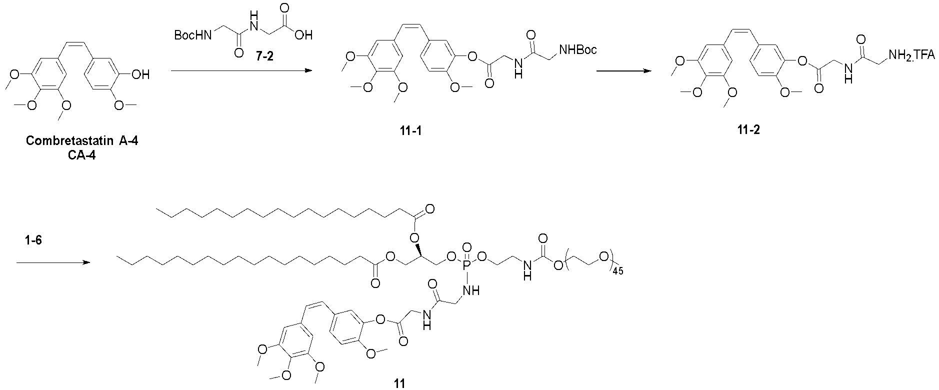

### Synthesis of Compound 11-1

At room temperature (25 °C), to a solution of **CA-4** (1.67 g, 5.27 mmol, 1.0 eq) in CH₂Cl₂ (40 mL) were sequentially added **7-2** (1.50 g, 6.46 mmol, 1.2 eq), DMAP (1.93 g, 15.7 mmol, 3.0 eq) and EDCI (3.0 g, 15.70 mmol, 3.0 eq). The mixture was stirred at room temperature for 2 h. Upon completion of the reaction, the reaction mixture was poured into saturated aqueous NH₄Cl solution and thoroughly washed (150 mL × 3). The organic phases were combined, dried over anhydrous Na₂SO₄, and concentrated under reduced pressure. The crude product was dried under vacuum, and purified by silica gel column chromatography using PE/EA = 1:1 as eluent to give 2.4 g of pure product.

MS (ESI), m/z, 431.0 [M-100]⁺.

### Synthesis of Compound 11-2

At room temperature (25 °C), TFA (4 mL) was added dropwise to a solution of 11-1 (2.1 g, 3.96 mmol, 1.0 eq) in CH₂Cl₂ (24 mL). The mixture was stirred at room temperature for 2 h. Upon completion of the reaction, the mixture was concentrated under reduced pressure to give the product as a white solid, 2.1 g.

MS (ESI), m/z, 431.0 [M+H]⁺.

### Synthesis of Compound 11

At room temperature (25 °C) under N₂ protection, crude product **1-6** (755 mg, 0.269 mmol, 1.0 eq) was dissolved in CH₂Cl₂ (10 mL). The temperature was lowered to 0 °C, and to the reaction mixture were sequentially added dropwise a solution of **11-2** (750 mg, 1.38 mmol, 5.0 eq) in DCM (2 mL) and triethylamine (271 mg, 2.69 mmol, 10 eq). After the dropwise addition, the ice-water bath was removed, and the mixture was stirred at room temperature for 2 h. TLC indicated completion of the reaction. The reaction mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 13:1 - 11:1, to give the compound as a white solid, 610 mg.

MS (ESI), m/z, 607.0 [DAG-OH]⁺. 671.0 [M-DAG+4Na]⁴⁺. DAG (Diglyceride) is diacylglycerol.

¹H NMR (400 MHz, Chloroform-d) δ 7.18 - 7.12 (m, 1H), 7.04 - 6.98 (m, 1H), 6.87 (d, *J* = 8.6 Hz, 1H), 6.49 (d, *J =* 5.4 Hz, 3H), 5.90 - 5.83 (m, 1H), 5.29 - 5.21(m, 1H), 4.37-4.32 (m, 3H), 4.23 - 4.04 (m, 8H), 3.84 (s, 3H), 3.81 (s, 3H), 3.71 (s, 6H), 3.68 - 3.65 (m, 193H), 3.58 - 3.48 (m, 3H), 3.51 - 3.42 (m, 4H), 3.40 (s, 3H), 2.36 - 2.30 (m, 4H), 1.64 - 1.59 (m, 5H), 1.32-1.27 (m, 70H), 0.90 (t, *J=* 6.7 Hz, 6H).

### Example 12: Synthesis of Compound 12

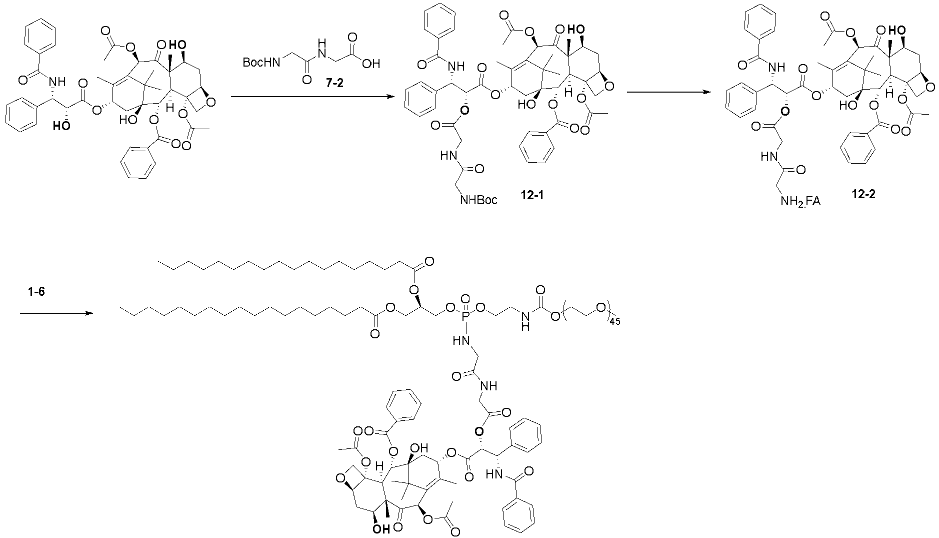

### Synthesis of Compound 12-1

At room temperature (25 °C), to a solution of paclitaxel (3.8 g, 4.45 mmol, 1.0 eq) in CH₂Cl₂ (60 mL) were sequentially added **7-2** (1.25 g, 5.35 mmol, 1.2 eq), DMAP (1.63 g, 13.35 mmol, 3.0 eq) and EDCI (2.56 g, 13.35 mmol, 3.0 eq). The mixture was stirred at room temperature for 2 h. Upon completion of the reaction, the reaction mixture was poured into saturated aqueous ammonium chloride solution and thoroughly washed (150 mL × 3). The organic phases were combined, dried over anhydrous Na₂SO₄, and concentrated under reduced pressure. The crude product was dried under vacuum, and purified by silica gel column chromatography using PE/EA = 1:2 - 1:3 as eluent to give 3.0 g of pure product.

MS (ESI), m/z, 967.6 [M-100]⁺ .

### Synthesis of Compound 12-2

At 0 °C, a solution of **12-1** (2.0 g, 1.87 mmol, 1.0 eq) in formic acid (10 mL) was stirred for 6 h. Upon completion of the reaction, the mixture was concentrated to 3 mL at 0 °C. Methyl tert-butyl ether (MTBE) ether (100 mL) was added dropwise for trituration, precipitating a large amount of white solid. The solid was collected by filtration, and the filter cake was thoroughly dried under vacuum to give 1.2 g of pure white solid.

MS (ESI), m/z, 967.6 [M+H]⁺ .

### Synthesis of Compound 12

At room temperature (25 °C) under N₂ protection, crude product **1-6** (755 mg, 0.27 mmol, 1.0 eq) was dissolved in CH₂Cl₂ (10 mL). The temperature was lowered to 0 °C, and to the reaction mixture were sequentially added dropwise a solution of **12-2** (1.49 g, 1.38 mmol, 5.0 eq) in DCM (2 mL) and triethylamine (271 mg, 2.69 mmol, 10 eq). After the dropwise addition, the ice-water bath was removed, and the mixture was stirred at room temperature for 2 h. TLC indicated completion of the reaction. The reaction mixture was concentrated under reduced pressure, dissolved in DCM (100 mL), and washed with saturated brine three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 95:5 - 93:7 - 92:8 - 89:11, to give 710 mg of the product.

MS (ESI), m/z, 606.8 [DAG-OH]⁺. 805.4 [M-DAG+4Na]⁴⁺. DAG (Diglyceride) is diacylglycerol.

### Example 13: Synthesis of Compound 13

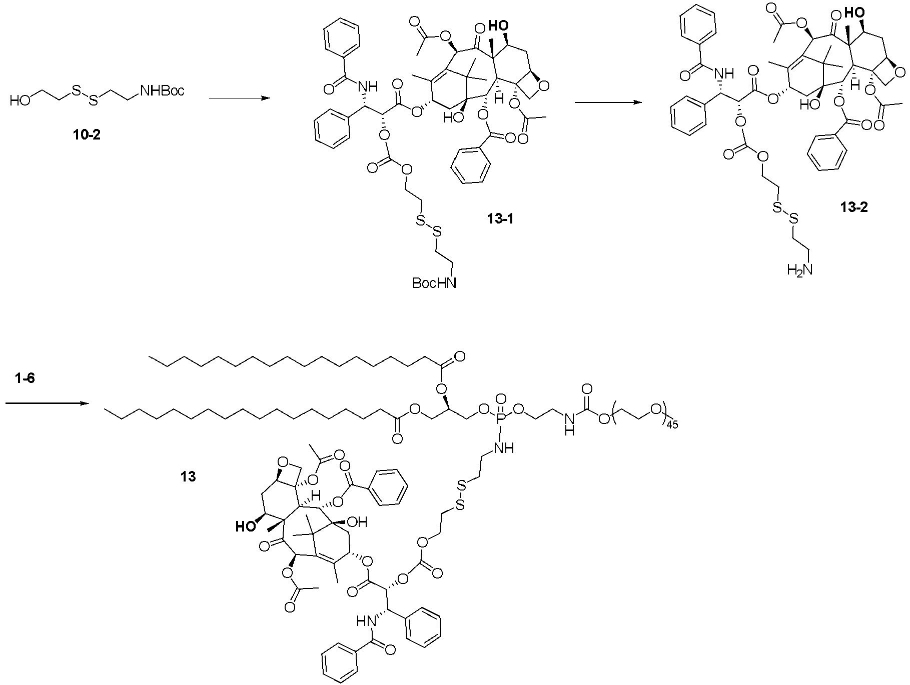

### Synthesis of Compound 13-1

At room temperature, **10-2** (300 mg, 1.18 mmol, 1.0 eq) was dissolved in dichloromethane (20 mL). Under nitrogen protection, triethylamine (120 mg, 1.18 mmol, 1.0 eq) and bis(4-nitrophenyl) carbonate (360 mg, 1.18 mmol, 1.0 eq) were added. The mixture was stirred at room temperature for 16 h. 4-Dimethylaminopyridine (144.6 mg, 1.18 mmol, 1.0 eq) and paclitaxel (1010 mg, 1.18 mmol, 1.0 eq) were added. The mixture was stirred at room temperature for 2 h. LCMS monitoring indicated completion of the reaction. The mixture was concentrated under reduced pressure and purified by reverse phase column chromatography (A = TFA (0.1% + H₂O), B = acetonitrile), followed by lyophilization to give **13-1** as a white solid (0.8 g, with a yield of 59.7%).

MS (ESI), m/z, 1133.0 [M+H]⁺.

### Synthesis of Compound 13-2

At room temperature, **13-1** (800 mg, 0.71 mmol, 1.0 eq) was dissolved in formic acid (10 mL). The mixture was stirred at room temperature under nitrogen protection for 2 h. LCMS monitoring was performed. The reaction was terminated. The mixture was concentrated under reduced pressure and purified by reverse phase chromatography (A = TFA (0.1% + H₂O), B = acetonitrile), followed by lyophilization to give **13-2** as a white solid (0.4 g, with a yield of 54.8%).

### Synthesis of Compound 13

At room temperature (25 °C) under N₂ protection, crude product **1-6** (800 mg, 0.29 mmol, 1.0 eq) was dissolved in CH₂Cl₂ (20 mL). The temperature was lowered to 0 °C, and to the reaction mixture were sequentially added dropwise a solution of **13-2** (1.47 g, 1.43 mmol, 5.0 eq) in DCM (5 mL) and triethylamine (288 mg, 2.85 mmol, 10 eq). After the dropwise addition, the ice-water bath was removed, and the mixture was stirred at room temperature for 1 h. Upon completion of the reaction, the mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 15:1 - 13:1 - 11:1, to yield 0.97 g of a white solid.

MS (ESI), m/z, 606.8 [DAG-OH]⁺. 821.6 [M-DAG+4Na]⁴⁺. DAG (Diglyceride) is diacylglycerol.

### Example 14: Synthesis of Compound 14

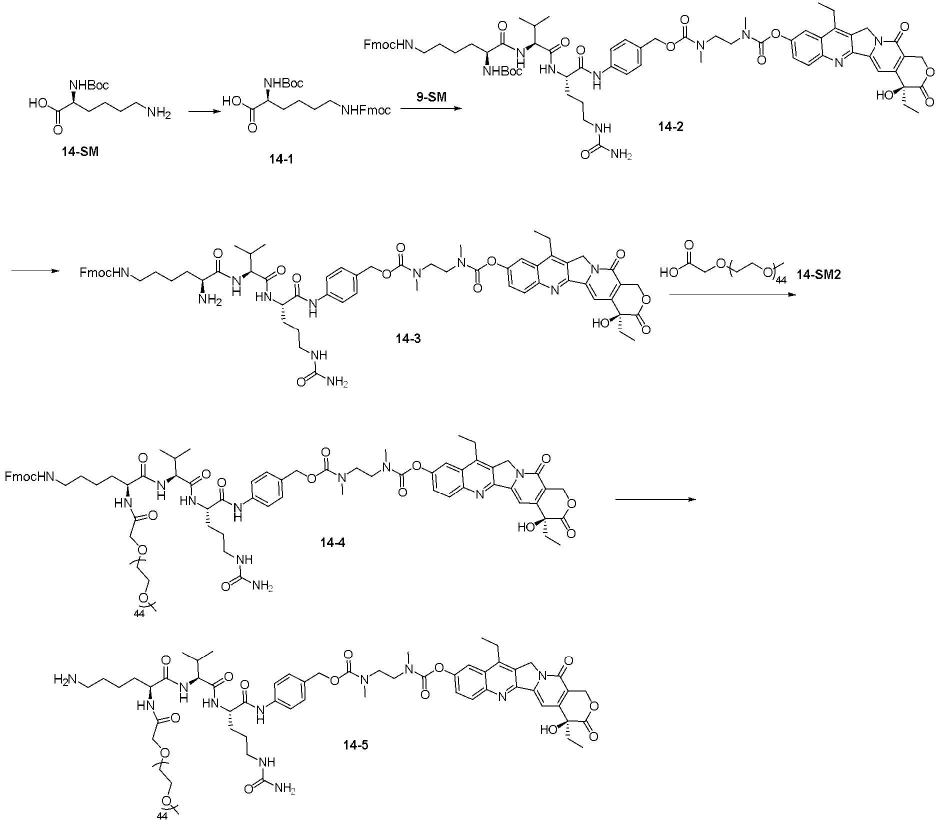

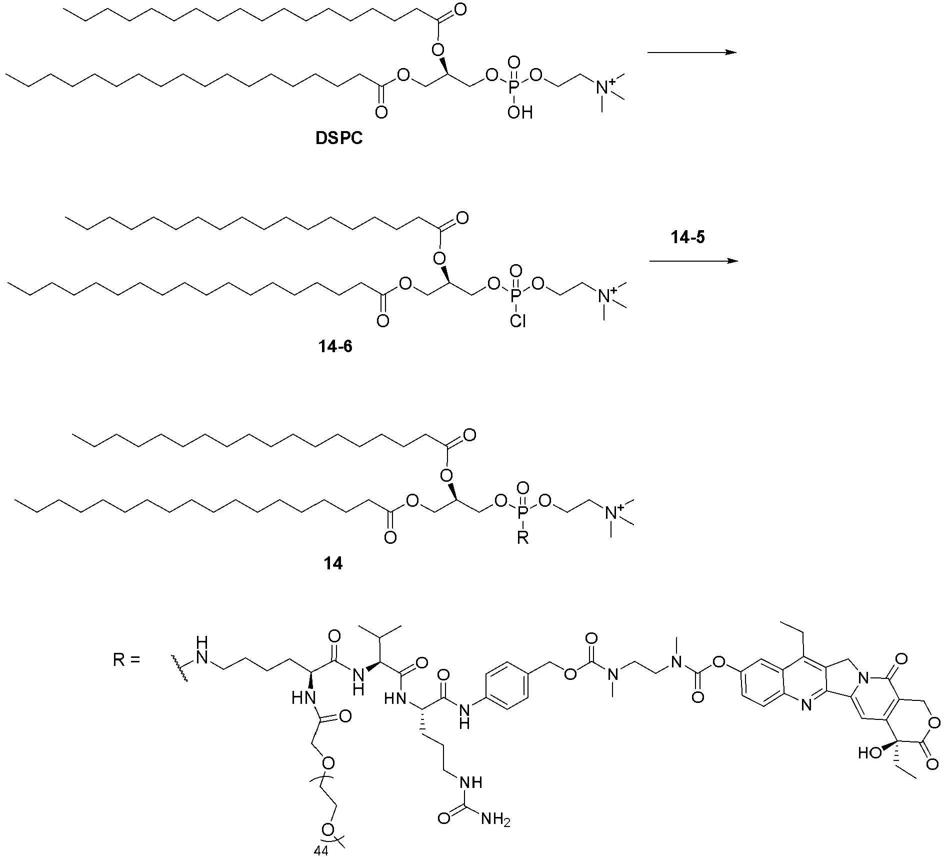

### Synthesis of Compound 14-1

At 25 °C, Fmoc-OSu (4.5 g, 13.4 mmol, 1.1 eq) and DIPEA (1.7 g, 13.4 mmol, 1.1 eq) were added to a solution of *N*-Boc-L-lysine (3.0 g, 12.2 mmol, 1.0 eq) in THF (60 mL). After the addition, the mixture was stirred at room temperature for 4 h. Upon completion of the reaction, the mixture was concentrated under reduced pressure. The crude product was purified by C18 column chromatography (CH₃CN:H₂O = 5-95%) to yield 4.0 g of a pure white solid.

MS (ESI), m/z, 369.0 [M-100]⁺.

### Synthesis of Compound 14-2

At room temperature (25 °C), **9-SM** (2.14 g, 2.35 mmol, 1.1 eq), HATU (1.22 g, 3.21 mmol, 1.5 eq), and DIPEA (550 mg, 4.28 mmol, 2.0 eq) were sequentially added to a solution of **14-1** (1.0 g, 2.14 mmol, 1.0 eq) in DMF (10 mL). After the addition, the mixture was stirred at room temperature for 2 h. Upon completion of the reaction, the DMF was directly concentrated under reduced pressure using an oil pump. The crude product was purified by C18 column chromatography (CH₃CN:H₂O = 5-95%) to yield 2.9 g of a pure white solid.

MS (ESI), m/z, 1361.6 [M-100]⁺.

### Synthesis of Compound 14-3

At room temperature (25 °C), TFA (5 mL) was added dropwise to a solution of **14-2** (1.0 g, 7.34 mmol, 1.0 eq) in CH₂Cl₂ (5 mL). The mixture was stirred at room temperature for 2 h. Upon completion of the reaction, the mixture was concentrated under reduced pressure to yield the compound as a white solid (1.0 g).

MS (ESI), m/z, 1361.60[M-100]⁺.

### Synthesis of Compound 14-4

At room temperature (25 °C), **9-SM2** (prepared according to the synthesis method of Example 10114-1K in patent WO2016046574A1, 1.22 g, 0.60 mmol, 1.2 eq), HATU (342 mg, 0.9 mmol, 1.8 eq), and DIPEA (155 mg, 1.2 mmol, 2.4 eq) were sequentially added to a solution of **14-4** (700 mg, 0.5 mmol, 1.0 eq) in DMF (4 mL). After the addition, the mixture was stirred at room temperature for 2 h. The reaction mixture was poured into water (100 mL) and extracted with EA (70 mL × 3). The organic phases were combined, dried over anhydrous Na₂SO₄, and concentrated under reduced pressure. The crude product was purified by normal phase column chromatography (DCM/MeOH = 10:1) to yield 1.2 g of pure product.

### Synthesis of Compound 14-5

At room temperature (25 °C), Et₃N (3 mL) was added dropwise to a solution of **14-4** (1.1 g, 0.34 mmol, 1.0 eq) in DMF (15 mL). The mixture was stirred at room temperature for 9 h. Upon completion of the reaction, the mixture was concentrated under reduced pressure, and the residue was thoroughly concentrated using an oil pump. The crude product was purified by normal phase column chromatography (DCM/MeOH/H₂O = 75:22:3 - DCM/MeOH/H₂O/Et₃N = 75:22:3:5) to yield 900 mg of pure product as a white solid.

### Synthesis of Compound 14

At room temperature (25 °C) under N₂ protection, DMF (11 mg, 0.148 mmol, 0.5 eq) was added to a solution of **DSPC** (CAS No. 816-94-4, 280 mg, 0.355 mmol, 1.2 eq) in CH₂Cl₂ (10 mL). The temperature was lowered to 0 °C, and oxalyl chloride (180 mg, 1.42 mmol, 4.8 eq) was added dropwise to the reaction mixture. After the dropwise addition, the mixture was stirred at room temperature for 2 h. Upon completion of the reaction, under N₂ protection, the mixture was concentrated to yield crude **14-6** as a white solid (290 mg), which was directly used in the next step.

At room temperature (25 °C) under N₂ protection, the above crude product was dissolved in CH₂Cl₂ (10 mL). The temperature was lowered to 0 °C, and a solution of **14-5** (900 mg, 0.296 mmol, 1.0 eq) in DCM (6 mL) and triethylamine (143 mg, 1.42 mmol, 4.8 eq) were sequentially added dropwise to the reaction mixture. After the dropwise addition, the ice-water bath was removed, and the mixture was stirred at room temperature for 2 h. Upon completion of the reaction, the mixture was concentrated under reduced pressure, dissolved in DCM (50 mL), and washed with saturated NaCl three times (200 mL × 3). The organic phases were combined, dried over anhydrous Na₂SO₄, and concentrated under reduced pressure. The crude product was purified by normal phase column chromatography (DCM/MeOH/H₂O = 75:22:3) to yield the compound (480 mg).

MS (ESI), m/z, 607.0 [DAG-OH]⁺. 826.6 [M-DAG+4Na]⁴⁺. DAG (Diglyceride) is diacylglycerol.

### Example 15: Synthesis of Compound 15

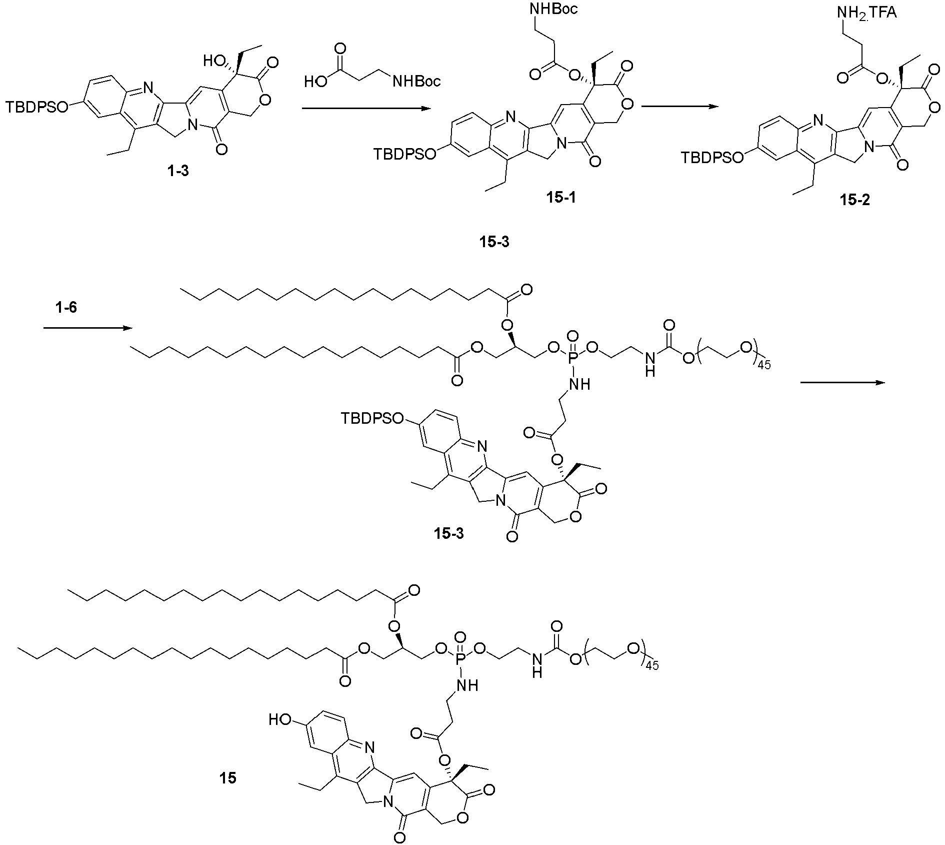

### Synthesis of Compound 15-1

At room temperature (25 °C), Boc-*β*-alanine (1.5 g, 7.93 mmol, 2.5 eq), EDCI (1.81 g, 9.50 mmol, 3.0 eq), and DMAP (581 mg, 4.76 mmol, 1.5 eq) were sequentially added to a solution of **SN38** (2.0 g, 3.17 mmol, 1.0 eq) in CH₂Cl₂ (70 mL). The mixture was stirred at room temperature for 2 h. Upon completion of the reaction, the mixture was concentrated under reduced pressure and purified by silica gel column chromatography (PE/EA = 2:1 - 1:1 as eluent) to yield 2.7 g of a yellow solid.

MS (ESI), m/z, 801.60 [M+H]⁺.

### Synthesis of Compound 15-2

At room temperature (25 °C), TFA (18 mL) was added dropwise to a solution of **15-**1 (2.3 g, 2.87 mmol, 1.0 eq) in CH₂Cl₂ (20 mL). The mixture was stirred at room temperature for 2 h. Upon completion of the reaction, the mixture was concentrated under reduced pressure to yield the compound as a yellow solid (2.8 g).

MS (ESI), m/z, 701.80 [M+H]⁺.

### Synthesis of Compound 15-3

At room temperature (25 °C) under N₂ protection, crude product **1-6** (800 mg, 0.285 mmol, 1.0 eq) was dissolved in CH₂Cl₂ (20 mL). The temperature was lowered to 0 °C, and a solution of **15-2** (1.14 g, 1.425 mmol, 5.0 eq) in DCM (5 mL) and triethylamine (288 mg, 2.85 mmol, 10 eq) were sequentially added dropwise to the reaction mixture. After the dropwise addition, the ice-water bath was removed, and the mixture was stirred at room temperature for 1 h. TLC monitoring indicated completion of the reaction. The mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal phase column chromatography, first using an elution system of EA/MeOH = 1:0 - 10:1, then switching to an elution system of DCM/MeOH = 10:0 - 10:1 - 5:1, to yield 745 mg of the product.

### Synthesis of Compound 15

At 0 °C, TBAF (100 µL, 0.102 mmol, 1.2 eq) and AcOH (25 mg, 0.424 mmol, 5.0 eq) were added to a solution of **15-3** (280 mg, 0.0848 mmol, 1.0 eq) in THF (4 mL). The mixture was warmed to room temperature and stirred for 2 h. The reaction mixture was concentrated under reduced pressure, dissolved in DCM (100 mL), and washed with water three times (150 mL × 2). The organic phases were combined, dried over anhydrous Na₂SO₄, and concentrated under reduced pressure. The crude product was purified by silica gel column chromatography (DCM/MeOH = 15:1 - 13:1 - 11:1) to yield 60 mg of pure product.

MS (ESI), m/z, 606.8 [DAG-OH]⁺. 679.3 [M-DAG+4Na]⁴⁺. DAG (Diglyceride) is diacylglycerol.

¹H NMR (400 MHz, Chloroform-d) δ (ppm) 9.20 (s, 1H), 8.04 (dd, *J* = 9.1, 1.4 Hz, 1H), 7.58 - 7.46 (m, 2H), 7.14 (s, 1H), 5.69 (dd, *J=* 17.0, 2.5 Hz, 1H), 5.59 (s, 1H), 5.39 (dd, *J =* 17.0, 4.2 Hz, 1H), 5.20 (d, *J* = 15.2 Hz, 3H), 4.31 (dt, *J =* 12.0, 4.2 Hz, 1H), 4.24 - 3.77 (m, 5H), 3.64 (d, *J =* 5.3 Hz, 171H), 3.38 (s, 7H), 3.10 (q, *J =* 7.7 Hz, 2H), 2.82 - 2.59 (m, 3H), 2.37 - 2.20 (m, 5H), 1.75 - 1.53 (m, 9H), 1.51 - 1.32 (m, 8H), 1.25 (d, *J=* 3.2 Hz, 91H), 1.00 (dd, *J =* 8.3, 6.2 Hz, 9H), 0.88 (t, *J* = 6.6 Hz, 9H).

### Example 16: Synthesis of Compound 16

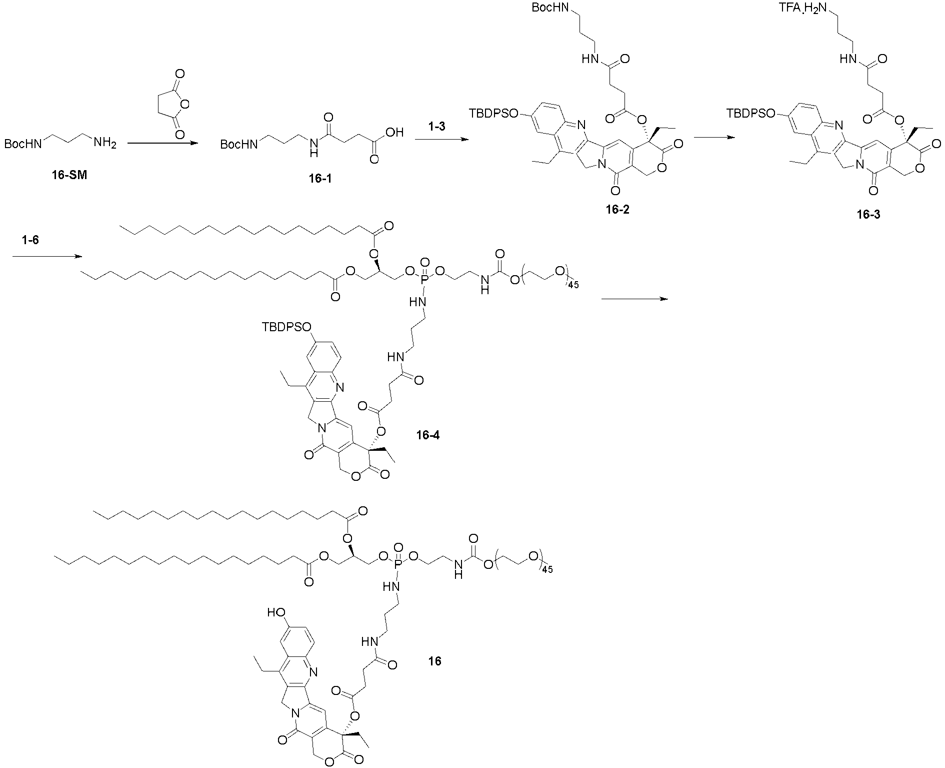

### Synthesis of Compound 16-1

At 25 °C, succinic anhydride (5.48 g, 54.7 mmol, 1.0 eq) and DIPEA (14.1 g, 109.4 mmol, 2.0 eq) were slowly added to a solution of *N*-tert-butoxycarbonyl-1,3-propanediamine (10 g, 57.4 mmol, 1.05 eq) in THF (100 mL). The reaction mixture was concentrated and thoroughly dried under vacuum to yield 17 g of a white solid.

MS (ESI), m/z, 274.80 [M+H]⁺.

### Synthesis of Compound 16-2

At room temperature (25 °C), **16-2** (2.2 g, 7.93 mmol, 2.5 eq), EDCI (1.81 g, 9.50 mmol, 3.0 eq), and DMAP (581 mg, 4.76 mmol, 1.5 eq) were sequentially added to a solution of **1-3** (2.0 g, 3.17 mmol, 1.0 eq) in CH₂Cl₂ (70 mL). The mixture was stirred at room temperature for 2 h. Upon completion of the reaction, the mixture was concentrated under reduced pressure and purified by silica gel column chromatography (PE/EA = 2:1 - 1:1 as eluent) to yield 1.6 g of a yellow-green solid.

MS (ESI), m/z, 886.60 [M+H]⁺.

### Synthesis of Compound 16-3

At room temperature (25 °C), TFA (18 mL) was added dropwise to a solution of **16-**2 (2.3 g, 2.60 mmol, 1.0 eq) in CH₂Cl₂ (20 mL). The mixture was stirred at room temperature for 2 h. Upon completion of the reaction, the mixture was concentrated under reduced pressure to yield the compound as a yellow solid (2.7 g).

MS (ESI), m/z, 786.60 [M+H]⁺.

### Synthesis of Compound 16-4

At room temperature (25 °C) under N₂ protection, crude product **1-6** (800 mg, 0.29 mmol, 1.0 eq) was dissolved in CH₂Cl₂ (20 mL). The temperature was lowered to 0 °C, and a solution of **16-3** (1.14 g, 1.43 mmol, 5.0 eq) in DCM (5 mL) and triethylamine (288 mg, 2.85 mmol, 10 eq) were sequentially added dropwise to the reaction mixture. After the dropwise addition, the ice-water bath was removed, and the mixture was stirred at room temperature for 1 h. TLC indicated completion of the reaction. The mixture was concentrated, dissolved in DCM (100 mL), and washed with water three times (150 mL × 3). The organic phases were combined, dried over anhydrous Na₂SO₄, and concentrated under reduced pressure. The crude product was purified by normal-phase column chromatography, first using an elution system of EA/MeOH = 1:0 - 10:1, then switching to DCM/MeOH = 10:0 - 10:1 - 5:1, to yield 710 mg of the product.

### Synthesis of Compound 16

At 0 °C, TBAF (175 µL, 0.175 mmol, 1.1 eq) and AcOH (95 mg, 1.59 mmol, 10 eq) were added to a solution of **16-4** (590 mg, 0.159 mmol, 1.0 eq) in THF (15 mL). The mixture was warmed to room temperature and stirred for 2 h. The reaction mixture was concentrated under reduced pressure, dissolved in DCM (100 mL), and washed with water three times (150 mL × 2). The organic phases were combined, dried over anhydrous Na₂SO₄, and concentrated under reduced pressure. The crude product was purified by silica gel column chromatography (DCM/MeOH = 15:1 - 13:1 - 11:1) to yield 710 mg of pure product.

MS (ESI), m/z, 606.8 [DAG-OH]⁺. 700.1 [M-DAG+4Na]⁴⁺. DAG (Diglyceride) is diacylglycerol.

¹H NMR (400 MHz, Chloroform-*d*) δ (ppm) 8.84 (s, 1H), 8.08 (dd, *J* = 9.2, 1.5 Hz, 1H), 7.47 (dd, *J* = 9.1, 2.6 Hz, 1H), 7.57 - 7.32 (m, 2H), 7.21 (d, *J* = 2.7 Hz, 1H), 6.57-6.44 (m, 1H), 5.90 - 5.56 (m, 2H), 5.37 (d, *J=* 17.1 Hz, 1H), 5.27-5.13 (m, 3H), 4.37-4.26 (m, 1H), 4.25 - 3.77 (m, 6H), 3.75 - 3.40 (m, 214H), 3.38-3.30 (m, 4H), 3.29 - 3.18 (m, 2H), 3.08 (d, *J* = 7.9 Hz, 2H), 3.00 - 2.75 (m, 4H), 2.60-2.45 (m, 2H), 2.347-2.26 (m, 5H), 1.67-1.45 (m, 9H), 1.39 - 1.21 (m, 104H), 1.13-1.04 (m, 1H), 0.99 (td, *J =* 7.5, 1.5 Hz, 3H), 0.88 (t, *J* = 6.7 Hz, 9H).

### Example 17: Synthesis of Compound 17

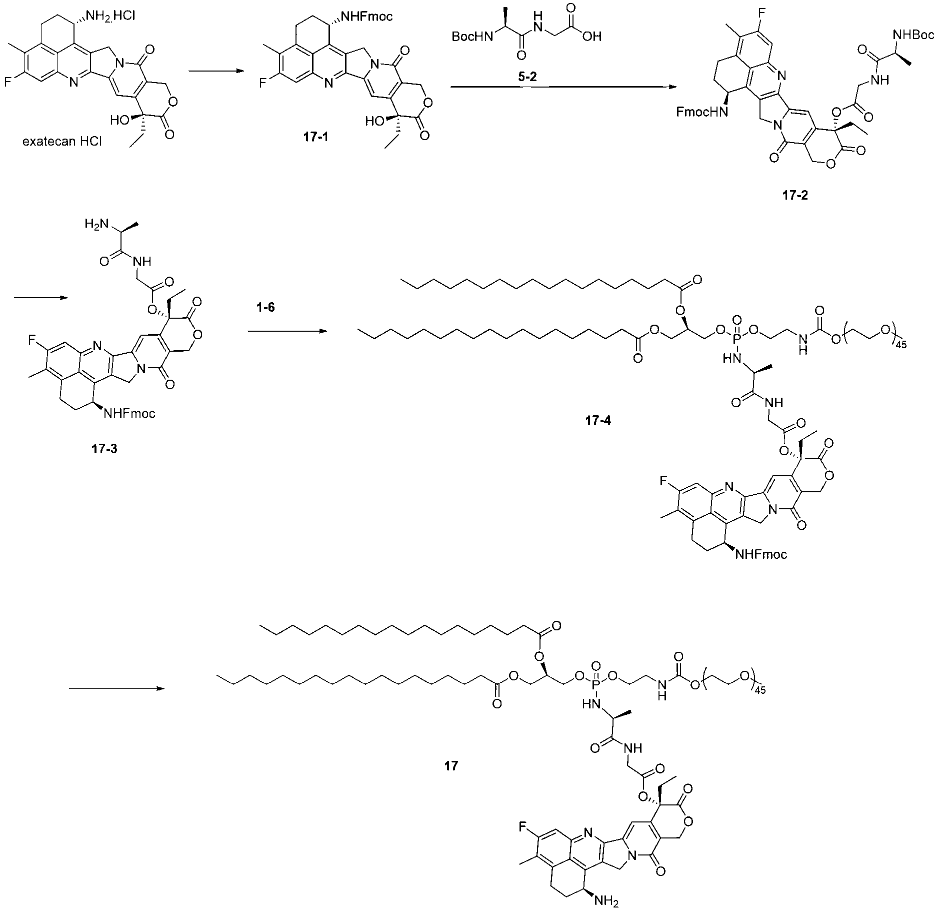

### Synthesis of Compound 17-1

At 25 °C, Fmoc-OSu (536 mg, 1.59 mmol, 1.5 eq) and Et₃N (160 mg, 1.59 mmol, 1.5 eq) were added to a solution of exatecan hydrochloride (500 mg, 1.06 mmol, 1.0 eq) in THF (30 mL). After the addition, the mixture was stirred at room temperature for 4 h. Upon completion of the reaction, the mixture was concentrated and purified by silica gel column chromatography (PE/EA = 1:2) to yield 560 mg of pure white solid.

MS (ESI), m/z, 657.8 [M+H]⁺.

### Synthesis of Compound 17-2

At room temperature (25 °C), **5-2** (630 mg, 2.56 mmol, 3.0 eq), EDCI (571 mg, 2.99 mmol, 3.5 eq), and DMAP (156 mg, 1.28 mmol, 1.5 eq) were sequentially added to a solution of **17-1** (560 mg, 0.85 mmol, 1.0 eq) in CH₂Cl₂ (10 mL). The mixture was stirred at room temperature for 3 h. Upon completion of the reaction, the mixture was washed with saturated ammonium chloride solution. The phases were separated, and the organic phase was dried over anhydrous Na₂SO₄ and concentrated under reduced pressure. Purification by silica gel column chromatography (PE/EA = 1:2) yielded 630 mg of solid.

MS (ESI), m/z, 885.8 [M+H]⁺.

### Synthesis of Compound 17-3

At room temperature (25 °C), 4 M HCl/dioxane (8 mL) was added dropwise to a solution of **17-2** (630 mg, 0.71 mmol, 1.0 eq) in CH₂Cl₂ (4 mL). The mixture was stirred at room temperature for 2 h. Upon completion of the reaction, the mixture was concentrated, dissolved in acetonitrile/methanol (1:1), and concentrated again to yield 630 mg of yellow solid.

MS (ESI), m/z, 785.6 [M+H]⁺.

### Synthesis of Compound 17-4

At room temperature (25 °C) under nitrogen protection, crude product **1-6** (755 mg, 0.27 mmol, 1.0 eq) was dissolved in dichloromethane (10 mL). The temperature was lowered to 0 °C, and a solution of **17-3** (663 mg, 0.81 mmol, 3.0 eq) in dichloromethane was added to the reaction mixture, followed by dropwise addition of triethylamine (271 mg, 2.69 mmol, 10 eq). After the dropwise addition, the ice-water bath was removed, and the mixture was stirred at room temperature for 2 h. TLC indicated completion of the reaction. The mixture was concentrated, dissolved in dichloromethane (100 mL), and washed with water three times (150 mL × 3). The organic phases were combined, dried over anhydrous Na₂SO₄, and concentrated under reduced pressure. The crude product was purified by normal-phase column chromatography (EA/MeOH = 1:0 - 10:1, then DCM/MeOH = 95:5 - 94:6 - 10:1) to yield 700 mg of crude product. The crude product was further purified by reverse-phase column chromatography ((THF/MeOH = 3:1):H₂O = 5-90%) to yield 440 mg of pure product.

### Synthesis of Compound 17

At room temperature (25 °C), Et₃N (2 mL) was added to a solution of **17-4** (370 mg, 0.10 mmol, 1.0 eq) in DMF (10 mL). The mixture was stirred at room temperature for 4 h. Upon completion of the reaction, the mixture was concentrated under reduced pressure at room temperature. The crude product was purified by normal-phase column chromatography (DCM/MeOH/H₂O = 10:1:0 - 75:22:3) to yield 118 mg of the product. HPLC purity: 95.5%.

MS (ESI), m/z, 606.8 [DAG-OH]⁺. 704.4 [M-DAG+4Na]⁴⁺. DAG (Diglyceride) is diacylglycerol.

¹H NMR (400 MHz, Chloroform-d) δ 7.73 (d, *J=* 10.5 Hz, 1H), 7.37-7.20 (m, 1H), 5.72-5.61 (m, 2H), 5.43-5.35 (m, 2H), 5.20-5.18 (m, 1H), 4.46-3.67 (m, 8H), 3.60-3.50 (m, 191H), 3.40 (s, 3H), 3.36 - 3.06 (m, 1H), 2.44 (s, 2H), 2.39 - 2.14 (m,8H), 1.67-1.60 (m, 6H), 1.40-1.27 (m, 77H), 1.01 (t, *J=* 7.4 Hz, 3H), 0.90 (t, *J=* 6.7 Hz, 6H).

### Example 18: Synthesis of Compound 18

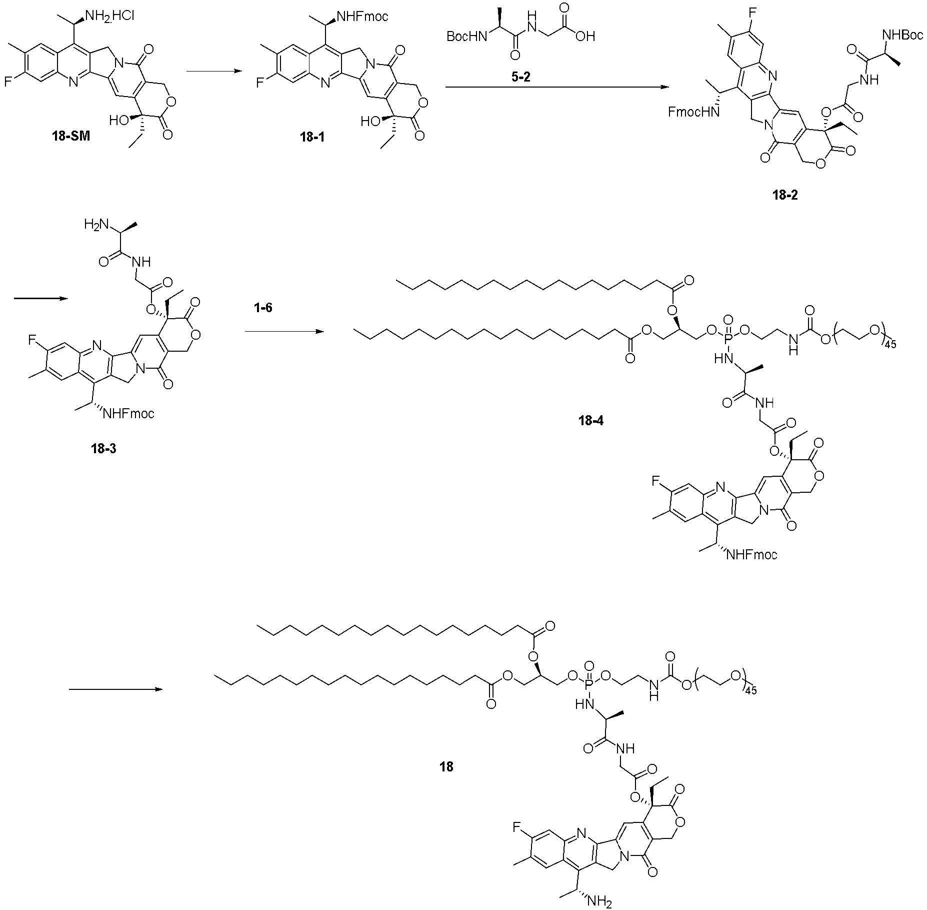

### Synthesis of Compound 18-1

At 25 °C, Fmoc-OSu (661 mg, 1.96 mmol, 1.5 eq) and Et₃N (198 mg, 1.96 mmol, 1.5 eq) were added to a solution of **18-SM** (prepared according to the synthesis method in Example 1 of patent WO2023030364A1, 600 mg, 1.30 mmol, 1.0 eq) in THF (30 mL). After the addition, the mixture was stirred at room temperature for 4 h. Upon completion of the reaction, purification by silica gel column chromatography (PE/EA = 2:1 - 1:2) yielded 810 mg of pure light yellow-green solid.

MS (ESI), m/z, 645.80 [M+H]⁺.

### Synthesis of Compound 18-2

At room temperature (25 °C), **5-2** (927 mg, 3.77 mmol, 3.0 eq), EDCI (856 mg, 4.48 mmol, 3.5 eq), and DMAP (234 mg, 1.92 mmol, 1.5 eq) were sequentially added to a solution of **18-1** (810 mg, 1.26 mmol, 1.0 eq) in CH₂Cl₂ (15 mL). The mixture was stirred at room temperature for 3 h. Upon completion of the reaction, the mixture was washed with saturated ammonium chloride. The phases were separated, and the organic phase was dried over anhydrous Na₂SO₄ and concentrated under reduced pressure. Purification by silica gel column chromatography (PE/EA = 1:2) yielded 1.2 g of solid.

MS (ESI), m/z, 873.8 [M+H]⁺.

### Synthesis of Compound 18-3

At room temperature (25 °C), TFA (1 mL) was added dropwise to a solution of **18-2** (1.1 g, 1.26 mmol, 1.0 eq) in CH₂Cl₂ (6 mL). The mixture was stirred at room temperature for 2 h. Upon completion of the reaction, the mixture was concentrated and thoroughly dried under vacuum to yield 1.1 g of yellow solid.

MS (ESI), m/z, 773.80 [M+H]⁺.

### Synthesis of Compound 18-4

At room temperature (25 °C) under N₂ protection, crude product **1-6** (1.51 g, 0.54 mmol, 1.0 eq) was dissolved in CH₂Cl₂ (20 mL). The temperature was lowered to 0 °C, and a solution of **18-3** (1.12 g, 1.34 mmol, 2.5 eq) in dichloromethane was added to the reaction mixture, followed by dropwise addition of triethylamine (543 mg, 5.38 mmol, 10 eq). After the dropwise addition, the ice-water bath was removed, and the mixture was stirred at room temperature for 2 h. TLC indicated completion of the reaction. The reaction mixture was concentrated, dissolved in dichloromethane (100 mL), and washed with water three times (150 mL × 3). The organic phases were combined, dried over anhydrous Na₂SO₄, and concentrated under reduced pressure. The crude product was purified by normal-phase column chromatography (DCM/MeOH = 9:1) to yield 1.37 g of crude product. The crude product was further purified by reverse-phase C8 column chromatography ((THF/MeOH = 3:1):H₂O = 5-90%) to yield 1.06 g of pure product.

### Synthesis of Compound 18

At room temperature (25 °C), Et₃N (2 mL) was added to a solution of **18-4** (1.05 g, 0.31 mmol, 1.0 eq) in DMF (10 mL). The mixture was stirred at room temperature for 4 h. Upon completion of the reaction, the mixture was concentrated under reduced pressure. The crude product was purified by normal-phase column chromatography (DCM/MeOH/H₂O = 10:1:0 - 75:22:3) to yield 118 mg of the product.

MS (ESI), m/z, 606.8 [DAG-OH]⁺. 701.3 [M-DAG+4Na]⁴⁺. DAG (Diglyceride) is diacylglycerol.

¹H NMR (400 MHz, Chloroform-d) δ 8.11 (d, *J =* 8.0 Hz, 1H), 7.84 (d, *J =* 10.3 Hz, 1H), 7.40-7.30 (m, 1H), 5.87 - 5.63 (m, 2H), 5.53 - 5.15 (m, 3H), 4.51 - 3.76 (m, 9H), 3.74-3.48 (m, 205H), , 3.40 (s, 3H), 2.56 (s, 2H), 2.34 - 2.24 (m, 5H), 1.68 - 1.54 (m, 8H), 1.46 - 1.19 (m, 72H), 1.01 (t, *J* = 7.4 Hz, 3H), 0.90 (t, *J* = 6.7 Hz, 6H).

### Example 19: Synthesis of Compound 19

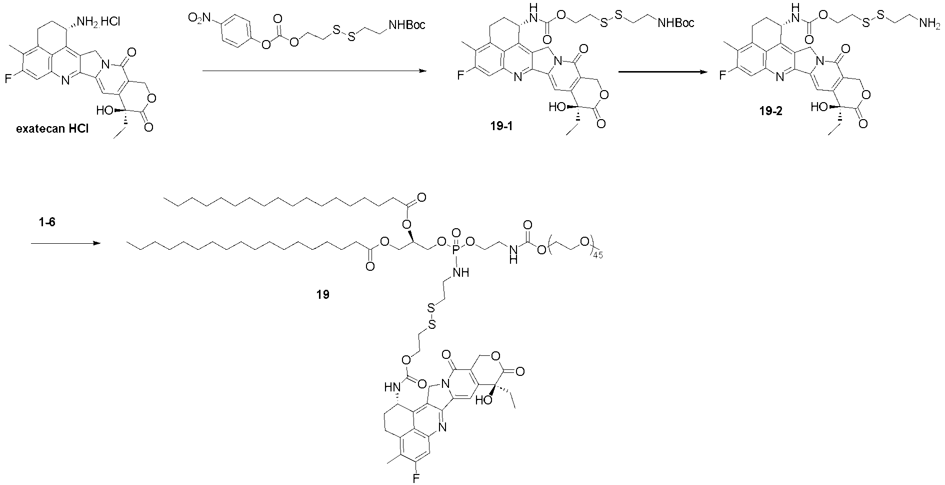

### Synthesis of Compound 19-1

At 0 °C, Et₃N (510 mg, 5.0 mmol, 1.5 eq) and bis(4-nitrophenyl) carbonate (1.52 g, 5.0 mmol, 1.5 eq) were sequentially added to a solution of 10-2 (1.3 g, 5.0 mmol, 1.5 eq) in CH₂Cl₂ (25 mL). The mixture was stirred at the same temperature for an additional 2 h. DIPEA (494 mg, 3.83 mmol, 3.0 eq) and a solution of exatecan hydrochloride (600 mg, 1.28 mmol, 1.0 eq) in DMF (15 mL) were then added. The mixture was stirred at room temperature for 2 h. Upon completion of the reaction, the mixture was poured into water (100 mL). Extraction was performed with EA (100 mL × 3). The organic phases were combined, dried over anhydrous Na₂SO₄, and concentrated under reduced pressure. The crude product was purified by normal-phase column chromatography using an elution system of PE/EA = 1:1 - 1:2 to yield 720 mg of light yellow-green solid.

MS (ESI), m/z, 715.8 [M+H]⁺.

### Synthesis of Compound 19-2

At 0 °C, a solution of **19-1** (700 mg, 0.98 mmol, 1.0 eq) in formic acid (10 mL) was stirred for 6 h. Upon completion of the reaction, the mixture was concentrated to 3 mL at 0 °C. MTBE (30 mL) was added dropwise for slurrying, precipitating a large amount of solid. The solid was collected by filtration, and the filter cake was thoroughly dried under vacuum to yield 720 mg of solid.

MS (ESI), m/z, 615.8 [M+H]⁺.

### Synthesis of Compound 19

At room temperature (25 °C) under nitrogen protection, the crude product of **1-6** (755 mg, 0.269 mmol, 1.0 eq) was dissolved in CH₂Cl₂ (15 mL). The temperature was lowered to 0 °C, and **19-2** (710 mg, 1.076 mmol, 4.0 eq) and triethylamine (271 mg, 2.69 mmol, 10 eq) were sequentially added to the reaction mixture. After the addition, the ice-water bath was removed, and the mixture was stirred at room temperature for 2 h. TLC indicated completion of the reaction. The reaction mixture was concentrated under pressure, dissolved in DCM (100 mL), and washed with water three times (150 mL × 3). The organic phases were combined, dried over anhydrous Na₂SO₄, and concentrated under reduced pressure. The crude product was purified by normal-phase column chromatography, first using an elution system of EA/MeOH = 1:0 - 10:1, then switching to an elution system of DCM/MeOH = 15:1 - 13:1 - 11:1, to yield 330 mg of the product.

MS (ESI), m/z, 606.8 [DAG-OH]⁺. 717.0 [M-DAG+4Na]⁴⁺. DAG (Diglyceride) is diacylglycerol.

¹H NMR (400 MHz, Chloroform-d) δ 7.67 (d, *J* = 10.5 Hz, 1H), 7.62 (s, 1H), 6.70-6.54 (m, 1H), 5.75-5.71 (m, 2H), 5.50 - 5.11 (m, 6H), 4.65 - 4.53 (m, 1H), 4.48 - 3.75 (m, 11H), 3.73-3.55 (m, 187H), 3.53-3.46 (m, 2H), 3.40 (s, 3H), 3.35-3.20 (m, 4H), 3.18-3.02 (m, 4H), 2.93-2.81 (m, 2H), 2.43 (s, 3H), 2.38 - 2.22 (m, 9H), 1.39-1.20 (m, 82H), 1.07 (t, *J* = 7.4 Hz, 3H), 0.90 (t, *J=* 6.7 Hz, 9H).

### Example 20: Synthesis of Compound 20

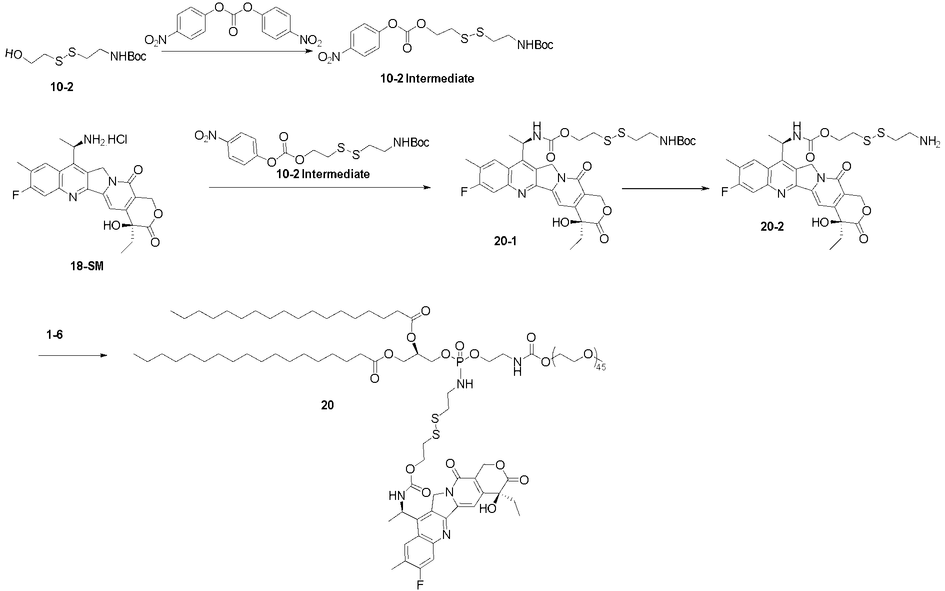

### Synthesis of Compound 20-1

At 0 °C, bis(4-nitrophenyl) carbonate (3.19 g, 10.5 mmol, 1.05 eq) and Et₃N (1.11 g, 11 mmol, 1.1 eq) were successively added to a solution of **10-2** (2.53 g, 10.0 mmol, 1.0 eq) in DCM (50 mL). After the addition, the mixture was warmed to room temperature and stirred for 2 h. Upon completion of the reaction, half of the reaction mixture was taken for the next step. MS (ESI), m/z, 318.8.

At room temperature (25 °C), the **10-2** intermediate reaction mixture from the previous step, DIPEA (503 mg, 3.9 mmol, 3.0 eq), DMAP (476 mg, 3.90 mmol, 3.0 eq), and pyridine (2 mL) were successively added to a solution of **18-SM** (600 mg, 1.30 mmol, 1.0 eq) in DMF (200 mL). The mixture was stirred at room temperature for 2 h. Upon completion of the reaction, the mixture was poured into water (800 mL) and extracted with ethyl acetate (100 mL x 3). The organic phases were combined, dried over anhydrous Na₂SO₄, and concentrated under reduced pressure. The crude product was purified by normal-phase column chromatography using an elution system of PE/EA = 1:1 - 1:2 to yield 1.0 g of crude product. The crude product was further purified by reverse-phase column chromatography (CH₃CN:H₂O = 5-90%) to obtain 640 mg of white solid.

MS (ESI), m/z, 702.80 [M+H]⁺.

### Synthesis of Compound 20-2

At 0 °C, a solution of **20-1** (620 mg, 0.88 mmol, 1.0 eq) in formic acid (10 mL) was stirred for 6 h. Upon completion of the reaction, the mixture was concentrated to 3 mL at 0 °C. MTBE (30 mL) was added dropwise for slurrying, precipitating a large amount of white solid. The solid was collected by filtration, and the filter cake was thoroughly dried under vacuum to yield 670 mg of solid.

MS (ESI), m/z, 602.8 [M+H]⁺.

### Synthesis of Compound 20

At room temperature (25 °C) under nitrogen protection, the crude product of **1-6** (755 mg, 0.27 mmol, 1.0 eq) was dissolved in CH₂Cl₂ (20 mL). The temperature was lowered to 0 °C, and **20-2** (696 mg, 1.08 mmol, 5.0 eq) and triethylamine (271 mg, 2.69 mmol, 10 eq) were sequentially added to the reaction mixture. After the dropwise addition, the ice-water bath was removed, and the mixture was stirred at room temperature for 1 h. TLC indicated completion of the reaction. The reaction mixture was concentrated under reduced pressure, dissolved in DCM (100 mL), and washed with water three times (150 mL × 3). The organic phases were combined, dried over anhydrous Na₂SO₄, and concentrated under reduced pressure. The crude product was purified by normal-phase column chromatography, first using an elution system of EA/MeOH = 1:0 - 10:1, then switching to an elution system of DCM/MeOH = 15:1 - 13:1 - 11:1, to yield 310 mg of the product.

MS (ESI), m/z, 607.0 [DAG-OH]⁺. 714.0 [M-DAG+4Na]⁴⁺. DAG (Diglyceride) is diacylglycerol.

¹H NMR (400 MHz, Chloroform-*d*) δ 8.15-8.05 (m, 1H), 7.82-7.74 (m, 1H), 7.64 (d, *J =* 11.1 Hz, 1H), 6.44-6.35 (m, 1H), 5.74-5.70 (m, 1H), 5.62 - 5.23 (m, 3H), 4.45 - 4.07 (m, 4H), 3.87 - 3.81 (m, 1H), 3.75-3.45 (m, 180H), 3.40 (s, 3H), 3.04 - 2.85 (m, 4H), 2.57 (s, 3H), 2.41-2.27 (m, 5H), 1.71 - 1.52 (m, 7H), 1.38 - 1.21 (m, 72H), 1.06 (t, *J* = 7.4 Hz, 3H), 0.90 (t, *J* = 6.7 Hz, 6H).

### Example 21: Synthesis of Compound 21

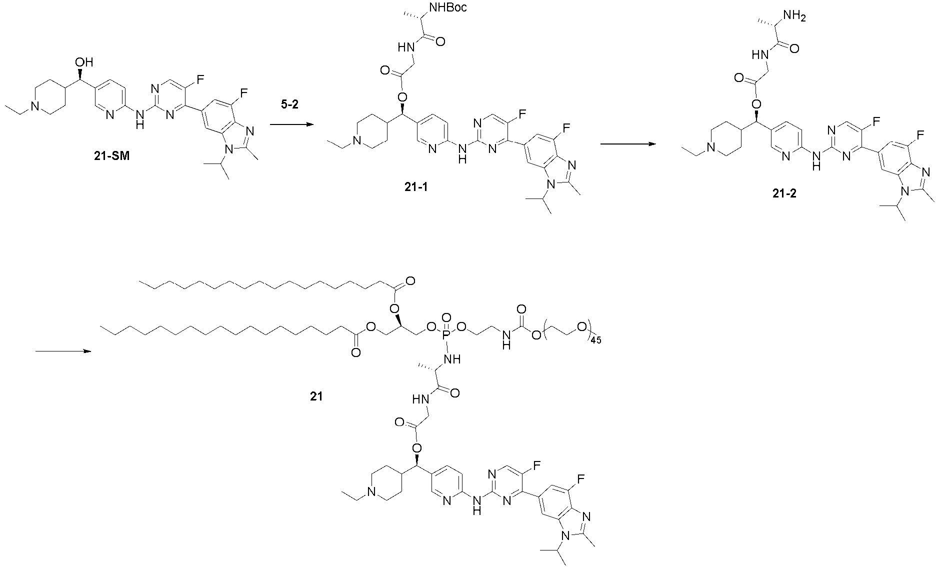

### Synthesis of Compound 21-1

At room temperature (25 °C), **5-2** (927 mg, 3.77 mmol, 3.0 eq), EDCI (856 mg, 4.48 mmol, 3.5 eq), and DMAP (234 mg, 1.92 mmol, 1.5 eq) were sequentially added to a solution of **21-SM** (prepared according to the synthesis method in Example 40 of patent WO2018202202A1, 680 mg, 1.26 mmol, 1.0 eq) in CH₂Cl₂ (15 mL). The mixture was stirred at room temperature for 3 h. Upon completion of the reaction, saturated ammonium chloride was added for washing. The layers were separated, and the organic phase was dried over anhydrous Na₂SO₄ and concentrated under reduced pressure. Purification by silica gel column chromatography (PE/EA = 1:2) yielded 1.0 g of solid.

MS (ESI), m/z, 750.7 [M+H]⁺.

### Synthesis of Compound 21-2

At room temperature (25 °C), TFA (1 mL) was added dropwise to a solution of **21-1** (0.8 g, 1.07 mmol, 1.0 eq) in CH₂Cl₂ (6 mL). The mixture was stirred at room temperature for 1 h. Upon completion of the reaction, the mixture was concentrated and thoroughly dried under vacuum to yield 0.7 g of yellow solid.

MS (ESI), m/z, 650.80 [M+H]⁺.

### Synthesis of Compound 21

At room temperature (25 °C) under N₂ protection, the crude product of **1-6** (1.02 g, 0.37 mmol, 1.0 eq) was dissolved in CH₂Cl₂ (15 mL). The temperature was lowered to 0 °C, and a solution of **21-2** (0.7 g, 0.91 mmol, 2.5 eq) in dichloromethane was added to the reaction mixture, followed by dropwise addition of triethylamine (370 mg, 3.64 mmol, 10 eq). After the dropwise addition, the ice-water bath was removed, and the mixture was stirred at room temperature for 2 h. TLC indicated completion of the reaction. The reaction mixture was concentrated, dissolved in dichloromethane (80 mL), and washed with water three times (100 mL x 3). The organic phases were combined, dried over anhydrous Na₂SO₄, and concentrated under reduced pressure. The crude product was purified by normal-phase column chromatography (DCM/MeOH = 9:1), followed by reverse-phase C8 column chromatography ((THF/MeOH = 3:1):H₂O = 5-90%) to yield 0.8 g of pure product.

MS (ESI), m/z, 606.8 [DAG-OH]⁺. 725.7 [M-DAG+4Na]⁴⁺. DAG (Diglyceride) is diacylglycerol.

### Example 22: Synthesis of Compound 22

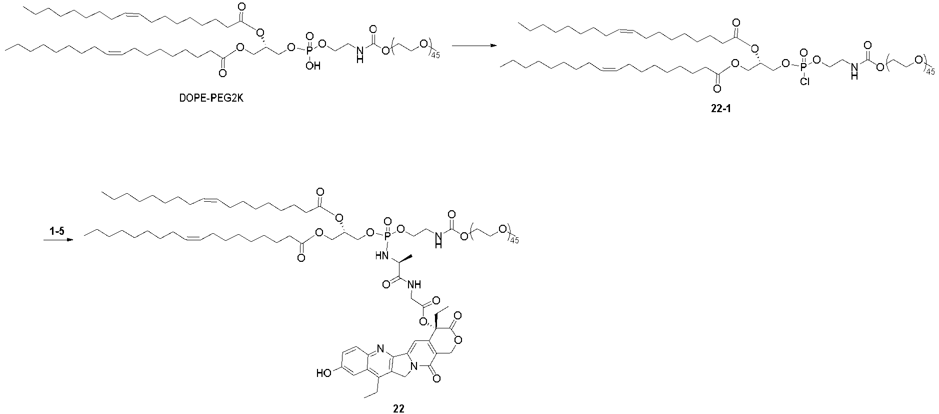

### Synthesis of Compound 22-1

In an ice-water bath at 5 °C under N₂ protection, DMF (0.1 mL) was added to a solution of **DOPE-PEG2K** (1.0 g, 0.36 mmol, 1.0 eq) in CH₂Cl₂ (20 mL), followed by dropwise addition of oxalyl chloride (0.5 g, 3.6 mmol, 10.0 eq). After the dropwise addition, the mixture was stirred at room temperature for 3 h. Upon completion of the reaction, under N₂ protection, the mixture was concentrated under reduced pressure to yield 1.0 g of white solid crude product, which was directly used in the next step.

### Synthesis of Compound 22

In an ice-water bath at 5 °C under N₂ protection, a solution of **1-5** (0.78 g, 0.89 mmol, 5.0 eq) in DCM (5 mL) and triethylamine (180 mg, 1.79 mmol, 15.0 eq) were added dropwise to a solution of the crude product **22-1** (0.5 g, 0.18 mmol, 1.0 eq) in CH₂Cl₂ (10 mL). After the dropwise addition, the reaction mixture was warmed to room temperature and stirred for 2 h. The reaction mixture was diluted with DCM (30 mL), washed with brine and water, separated, and the organic phase was dried over anhydrous sodium sulfate, filtered, and concentrated to yield 0.6 g of oily crude product. The crude product was dissolved in THF (10 mL), and TBAF (1.0 M in THF, 0.5 mL) and acetic acid (0.05 mL) were added. The mixture was stirred at room temperature for 2 h, then quenched with water and extracted with DCM (50 mL × 3). The organic phases were combined, washed with brine and water, dried over anhydrous sodium sulfate, filtered, and concentrated. The crude product was first purified by silica gel column chromatography (DCM/MeOH = 9:1), then further purified by neutral alumina column chromatography to yield compound **22,** 250 mg, as a white solid.

MS (ESI), m/z, 602.6 [DAG-OH]⁺. 693.4 [M-DAG+4Na]⁴⁺. DAG (Diglyceride) is diacylglycerol.

### General Synthesis Method A for Phospholipid PEG:

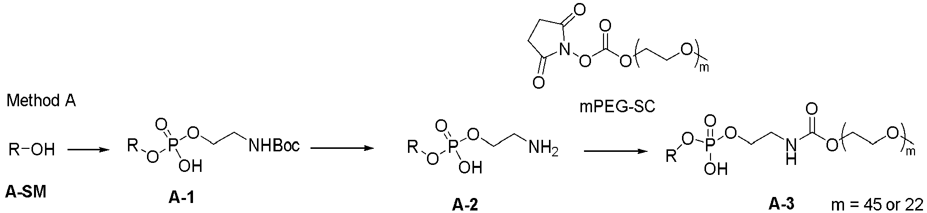

### General Synthesis Method for Compound A-1

In an ice-water bath under nitrogen protection, a solution of phosphorus oxychloride (1.0 eq) was added dropwise to a solution of alcohol R-OH and triethylamine (1.0 eq) in THF. The mixture was then warmed to room temperature and stirred for 4 h. The above reaction mixture was cooled in an ice-water bath, and a solution of *N*-(tert-butoxycarbonyl)ethanolamine (1.5 eq) and triethylamine (3.0 eq) in THF was added dropwise. The reaction mixture was stirred for an additional 2 hours, then stirred at room temperature for 12 hours. Water was added to quench the reaction, and the mixture was concentrated. The residue was acidified to pH=1 with 1% HCl. The aqueous phase was extracted with dichloromethane. The combined organic extracts were dried over anhydrous sodium sulfate, filtered, and concentrated. Purification by flash column chromatography (eluting with a MeOH/CH₂Cl₂ system) yielded compound **A-1.**

### General Synthesis Method for Compound A-2

**A-1** was dissolved in a mixture of dichloromethane and TFA (5/1, v/v). The reaction mixture was stirred at room temperature for 1-12 h, concentrated, and the residue was purified by column chromatography (eluting with a MeOH/CH₂Cl₂ system) to yield compound **A-2.**

### General Synthesis Method for Compound A-3

**A-2** (1.0 eq) was dissolved in dichloromethane and triethylamine (5.0 eq). A solution of mPEG-SC (1.0 eq) in dichloromethane was added dropwise. The reaction mixture was stirred at room temperature for 1-12 h, concentrated, and the residue was purified by column chromatography (eluting with a MeOH/CH₂Cl₂ system) to yield compound Compound

### Synthesis Method B for Phospholipid-Drug Conjugates:

### Method B

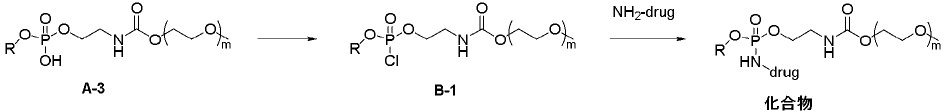

### General Synthesis Method for Compound B-1

In an ice-water bath at 5 °C under N₂ protection, DMF (5.0 eq) was added to a solution of **A-3** (1.0 eq) in CH₂Cl₂, followed by dropwise addition of oxalyl chloride (10.0 eq). After the dropwise addition, the mixture was stirred at room temperature for 1-3 h. Upon completion of the reaction, under N₂ protection, the mixture was concentrated under reduced pressure to yield crude product **B-1,** which was directly used in the next step.

### General Synthesis Method for Compound

In an ice-water bath at 5 °C under N₂ protection, a solution of the amine drug (3.0-5.0 eq) in CH₂Cl₂ and triethylamine (10-15.0 eq) were added dropwise to a solution of the crude product **B-1** (1.0 eq) in CH₂Cl₂. After the dropwise addition, the reaction mixture was stirred at room temperature for 2 h. The reaction mixture was quenched with water and extracted with DCM (50 mL × 3). The organic phases were combined, washed with brine and water, dried over anhydrous sodium sulfate, filtered, and concentrated. The residue was purified by column chromatography (eluting with a MeOH/CH₂Cl₂ system) to yield the compound. Reverse-phase column chromatography (eluting with a MeOH/0.05% TFA in water system) was performed for further purification if necessary.

### Example 23: Synthesis of Compound 23

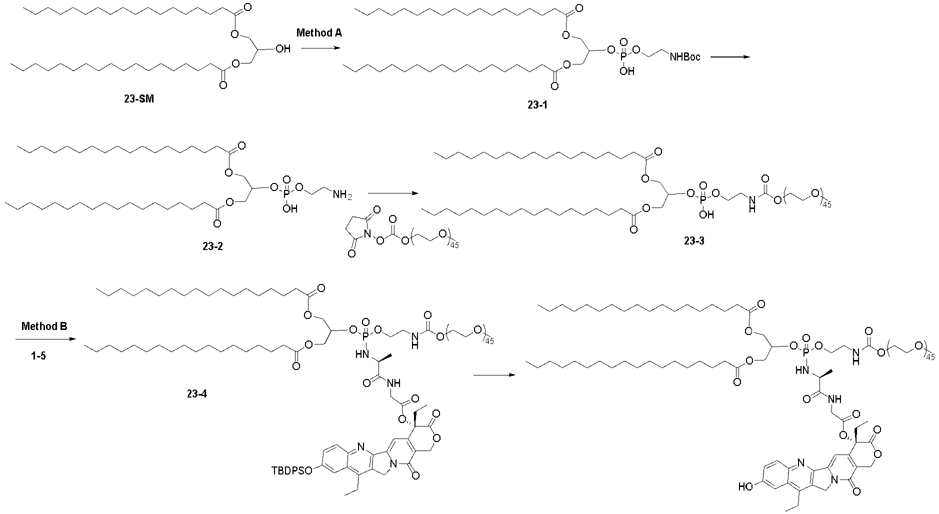

### Synthesis of Compound 23-4

Using starting materials 1,3-distearin and mPEG2000-SC, phospholipid compound **23-3** was synthesized according to General Synthesis Method A for Phospholipid PEG. Subsequently, compound **23-4** was synthesized by reacting it with intermediate **1-5** according to General Synthesis Method B for Drug Conjugates.

MS (ESI), m/z, 606.8 [DAG-OH]⁺. 753.1 [M-DAG+4Na]⁴⁺. DAG (Diglyceride) is diacylglycerol.

### Synthesis of Compound 23

Compound **23-4** (0.35 g) was dissolved in THF (10 mL), and TBAF (1.0 M in THF, 0.5 mL) and acetic acid (0.05 mL) were added. The mixture was stirred at room temperature for 5 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **23,** 160 mg, as a white solid, with an HPLC purity of 93.2%.

MS (ESI), m/z, 606.8 [DAG-OH]⁺. 693.4 [M-DAG+4Na]⁴⁺. DAG (Diglyceride) is diacylglycerol.

### Example 24: Synthesis of Compound 24

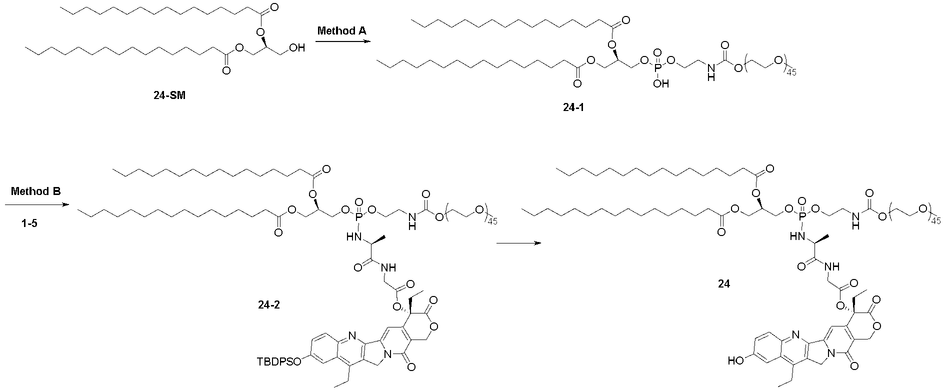

### Synthesis of Compound 24-2

Using starting materials 1,2-dipalmitin and mPEG2000-SC, phospholipid compound **24-1** was synthesized according to General Synthesis Method A for Phospholipid PEG. Subsequently, compound **24-2** was synthesized by reacting it with intermediate **1-5** according to General Synthesis Method B for Drug Conjugates.

### Synthesis of Compound 24

Compound **24-2** (0.75 g) was dissolved in THF (10 mL), and TBAF (1.0 M in THF, 0.5 mL) and acetic acid (0.05 mL) were added. The mixture was stirred at room temperature for 4 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **24,** 560 mg, as a white solid, with an HPLC purity of 98.6%.

MS (ESI), m/z, 550.8 [DAG-OH]⁺. 693.4 [M-DAG+4Na]⁴⁺. DAG (Diglyceride) is diacylglycerol.

### Example 25: Synthesis of Compound 25

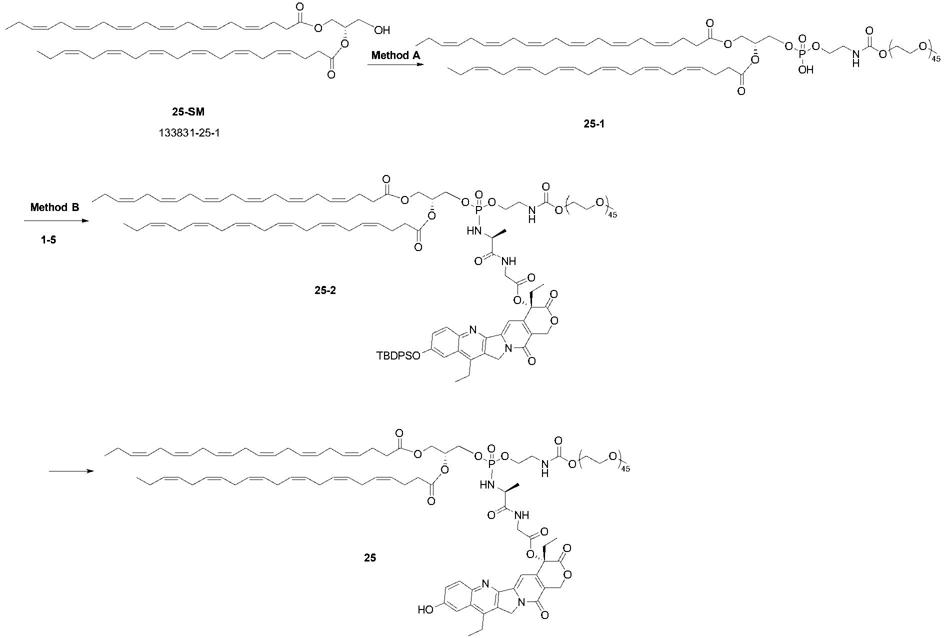

### Synthesis of Compound 25-2

Using starting material **25-SM** (CAS: 133831-25-1, commercially available) and mPEG2000-SC, phospholipid compound **25-1** was synthesized according to General Synthesis Method A for Phospholipid PEG. Subsequently, compound **25-2** was synthesized by reacting it with intermediate **1-5** according to General Synthesis Method B for Drug Conjugates.

### Synthesis of Compound 25

Compound **25-2** (0.6 g) was dissolved in THF (10 mL), and TBAF (1.0 M in THF, 1.0 mL) and acetic acid (0.05 mL) were added. The mixture was stirred at room temperature for 6 h, then quenched with water and extracted with DCM (50 mL × 3). The organic phases were combined, washed with brine and water, dried over anhydrous sodium sulfate, filtered, and concentrated. The crude product was first purified by silica gel column chromatography (eluting with a DCM/MeOH system) and then further purified by reverse-phase column chromatography (eluting with a MeOH/0.05% TFA in water system) to yield compound **25,** 320 mg, as a white solid, with an HPLC purity of 89.6%.

MS (ESI), m/z, 694.5 [DAG-OH]⁺. 693.4 [M-DAG+4Na]⁴⁺. DAG (Diglyceride) is a diacylglycerol fragment.

### Example 26: Synthesis of Compound 26

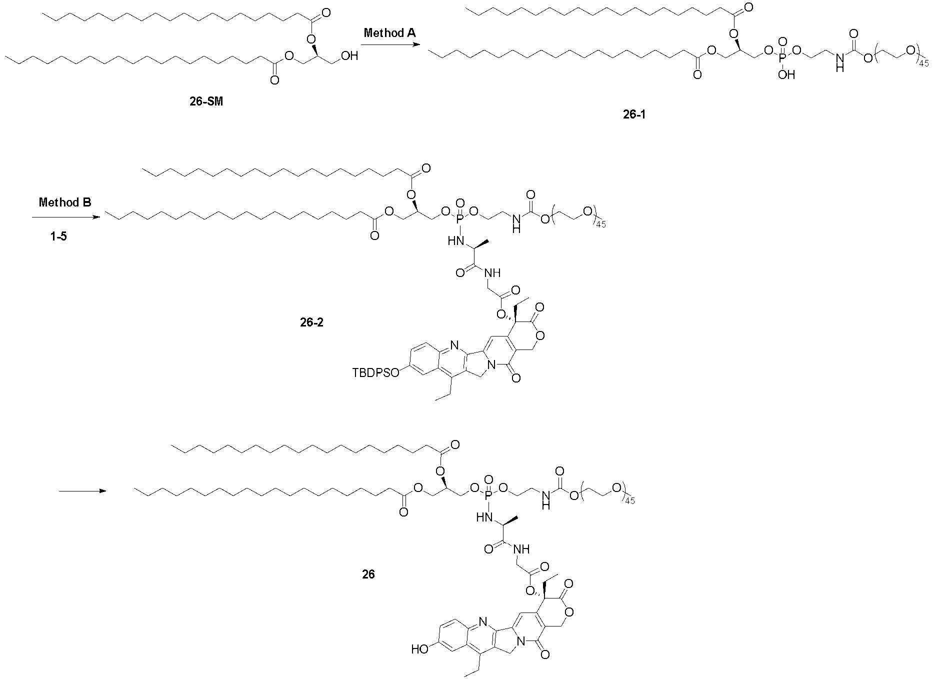

### Synthesis of Compound 26-2

Using starting material glycerol diarachidate and mPEG2000-SC, phospholipid compound **26-1** was synthesized according to General Synthesis Method A for Phospholipid PEG. Subsequently, compound **26-2** was synthesized by reacting it with intermediate **1-5** according to General Synthesis Method B for Drug Conjugates.

### Synthesis of Compound 26

Compound **26-2** (0.50 g) was dissolved in THF (10 mL), and TBAF (1.0 M in THF, 0.5 mL) and acetic acid (0.05 mL) were added. The mixture was stirred at room temperature for 2 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **26,** 230 mg, as a pale yellow solid, with an HPLC purity of 96.3%.

MS (ESI), m/z, 662.8 [DAG-OH]⁺. 693.4 [M-DAG+4Na]⁴⁺. DAG (Diglyceride) is a diacylglycerol fragment.

### Example 27: Synthesis of Compound 27

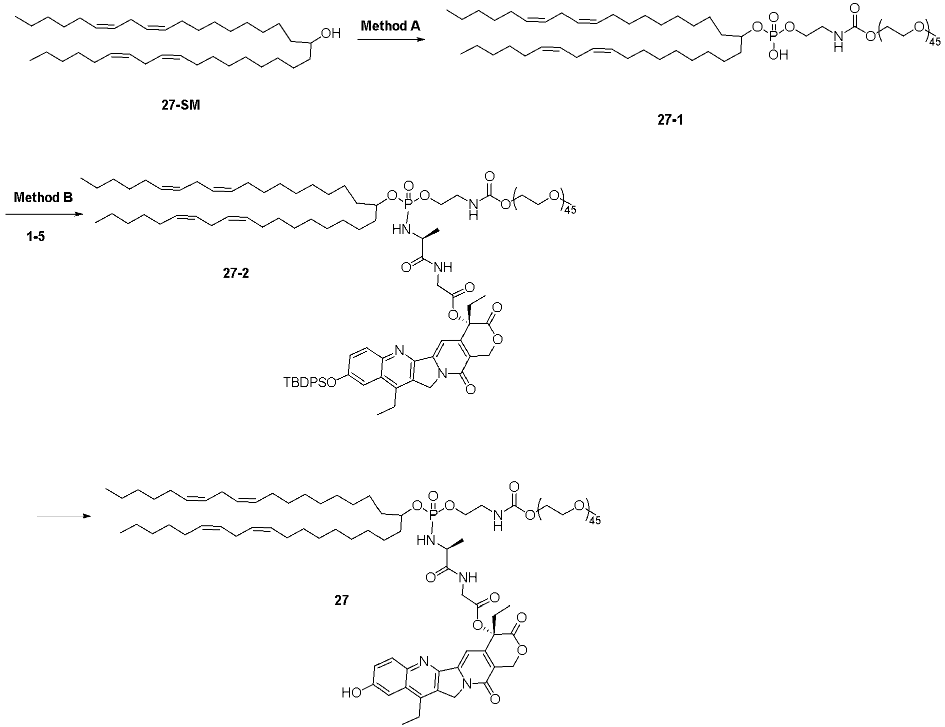

### Synthesis of Compound 27-2

Using starting material **27-SM** (CAS: 1169768-28-8, commercially available) and mPEG2000-SC, phospholipid compound **27-1** was synthesized according to General Synthesis Method A for Phospholipid PEG. Subsequently, compound **27-2** was synthesized by reacting it with intermediate **1-5** according to General Synthesis Method B for Drug Conjugates.

### Synthesis of Compound 27

Compound **27-2** (0.30 g) was dissolved in THF (5 mL), and TBAF (1.0 M in THF, 0.5 mL) and acetic acid (0.05 mL) were added. The mixture was stirred at room temperature for 3 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **27,** 120 mg, as a white solid, with an HPLC purity of 91.0%.

MS (ESI), m/z, 510.5 [ROH-OH]⁺. 693.4 [M-DAG+4Na]⁴⁺. ROH is an alkyl alcohol fragment.

### Example 28: Synthesis of Compound 28

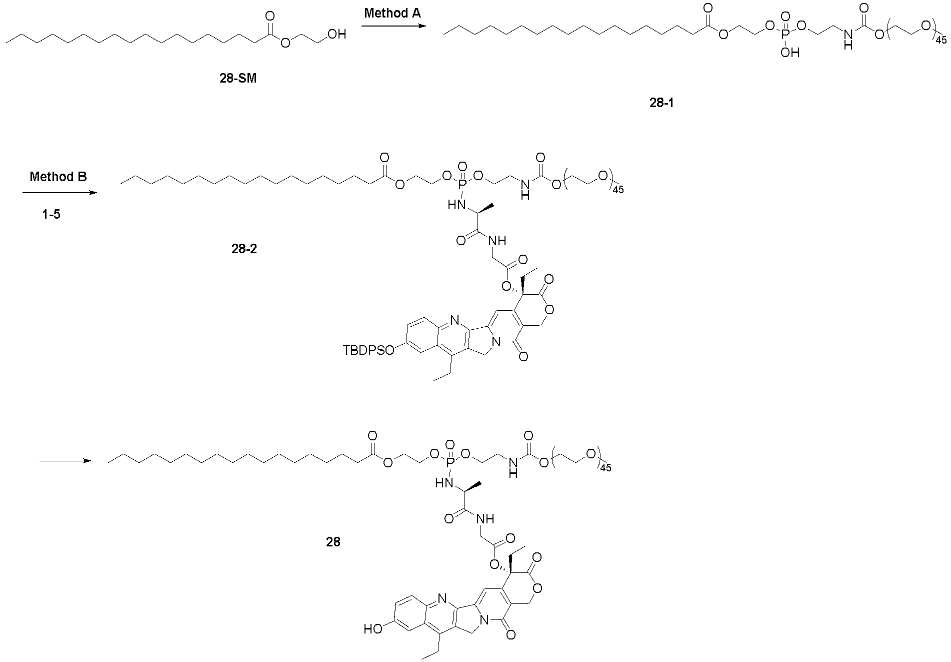

### Synthesis of Compound 28-2

Using starting material **28-SM** (ethylene glycol monostearate) and mPEG2000-SC, phospholipid compound **28-1** was synthesized according to General Synthesis Method A for Phospholipid PEG. Subsequently, compound **28-2** was synthesized by reacting it with intermediate **1-5** according to General Synthesis Method B for Drug Conjugates.

### Synthesis of Compound 28

Compound **28-2** (0.8 g) was dissolved in THF (10 mL), and TBAF (1.0 M in THF, 1.0 mL) and acetic acid (0.05 mL) were added. The mixture was stirred at room temperature for 5 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **28,** 630 mg, as a white solid, with an HPLC purity of 97.2%.

MS (ESI), m/z, 310.2 [ROH-OH]⁺. 693.4 [M-DAG+4Na]⁴⁺. ROH is a stearate alcohol fragment.

### Example 29: Synthesis of Compound 29

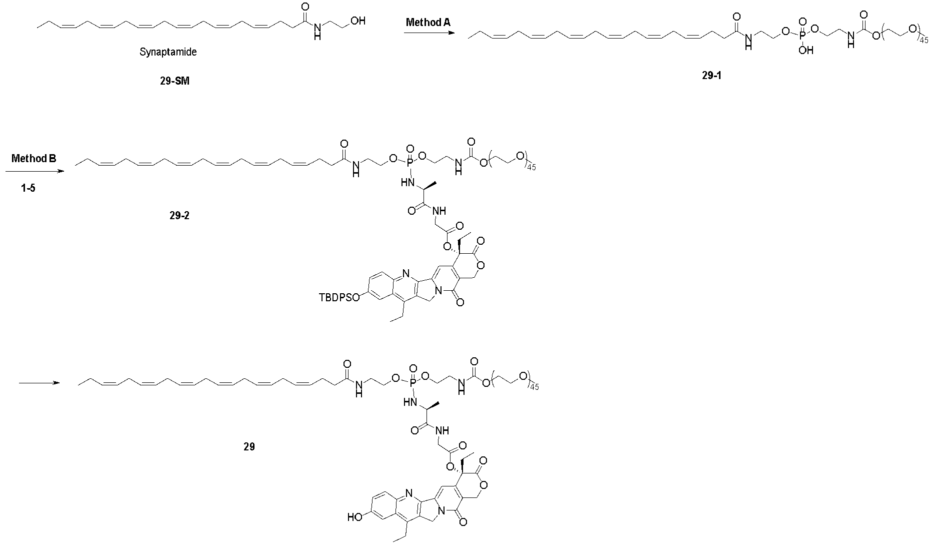

### Synthesis of Compound 29-2

Using starting material **29-SM** (Synaptamide, commercially available) and mPEG2000-SC, phospholipid compound **29-1** was synthesized according to General Synthesis Method A for Phospholipid PEG. Subsequently, compound **29-2** was synthesized by reacting it with intermediate **1-5** according to General Synthesis Method B for Drug Conjugates.

### Synthesis of Compound 29

Compound **29-2** (0.53 g) was dissolved in THF (10 mL), and TBAF (1.0 M in THF, 1.0 mL) and acetic acid (0.1 mL) were added. The mixture was stirred at room temperature for 5 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **29,** 363 mg, as a white solid, with an HPLC purity of 89.8%.

MS (ESI), m/z, 353.2 [ROH-OH]⁺. 693.4 [M-DAG+4Na]⁴⁺. ROH is an alkylamide alcohol fragment.

### Example 30: Synthesis of Compound 30

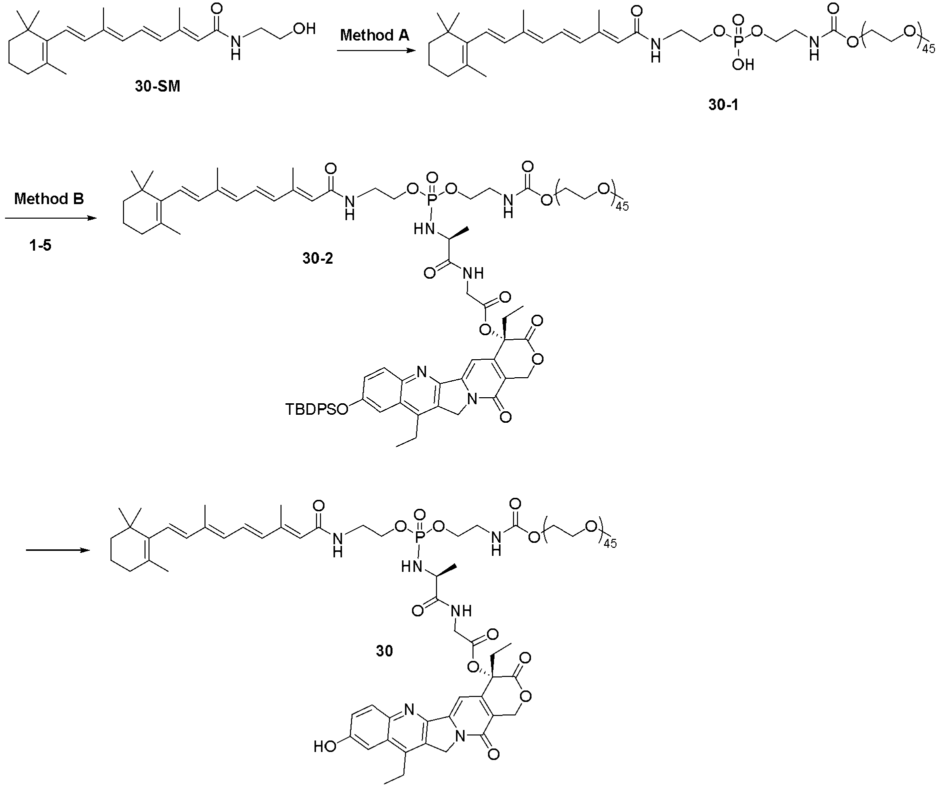

### Synthesis of Compound 30-2

Using starting material **30-SM** (synthesized according to the method for compound 48 in the literature J. Med. Chem. 2011, 54, 13, 4378-4387) and mPEG2000-SC, phospholipid compound **30-1** was synthesized according to General Synthesis Method A for Phospholipid PEG. Subsequently, compound **30-2** was synthesized by reacting it with intermediate **1-5** according to General Synthesis Method B for Drug Conjugates.

### Synthesis of Compound 30

Compound **30-2** (0.45 g) was dissolved in THF (10 mL), and TBAF (1.0 M in THF, 1.0 mL) and acetic acid (0.05 mL) were added. The mixture was stirred at room temperature for 6 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **30,** 260 mg, as a pale yellow solid, with an HPLC purity of 93.6%.

MS (ESI), m/z, 325.2 [ROH-OH]⁺. 693.4 [M-DAG+4Na]⁴⁺. ROH is an amide alcohol fragment.

### Example 31: Synthesis of Compound 31

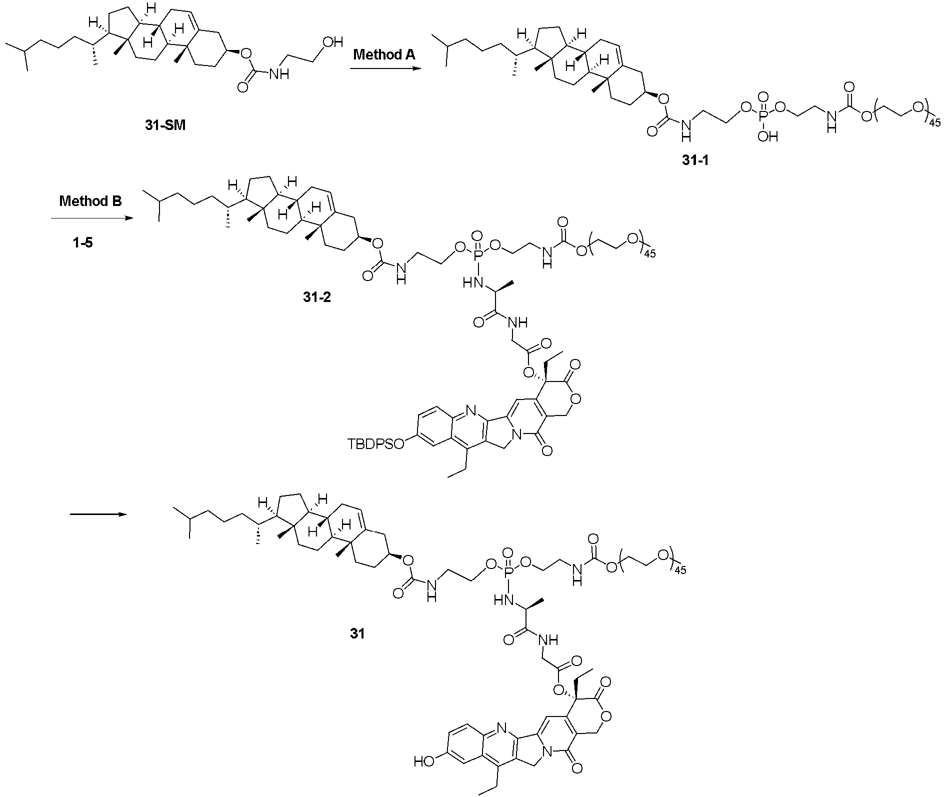

### Synthesis of Compound 30-2

Using starting material **31-SM** (synthesized according to the method for compound 4a in the literature Chemistry-A European Journal, 4(1), 137-151.) and mPEG2000-SC, phospholipid compound **31-1** was synthesized according to General Synthesis Method A for Phospholipid PEG. Subsequently, compound **31-2** was synthesized by reacting it with intermediate 1-5 according to General Synthesis Method B for Drug Conjugates.

### Synthesis of Compound 31

Compound **31-2** (1.0 g) was dissolved in THF (15 mL), and TBAF (1.0 M in THF, 1.2 mL) and acetic acid (0.1 mL) were added. The mixture was stirred at room temperature for 6 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **31,** 870 mg, as a pale yellow solid, with an HPLC purity of 99.0%.

MS (ESI), m/z, 455.2 [ROH-OH]⁺. 693.4 [M-DAG+4Na]⁴⁺. ROH is a cholesterol amide alcohol fragment.

### Example 32: Synthesis of Compound 32

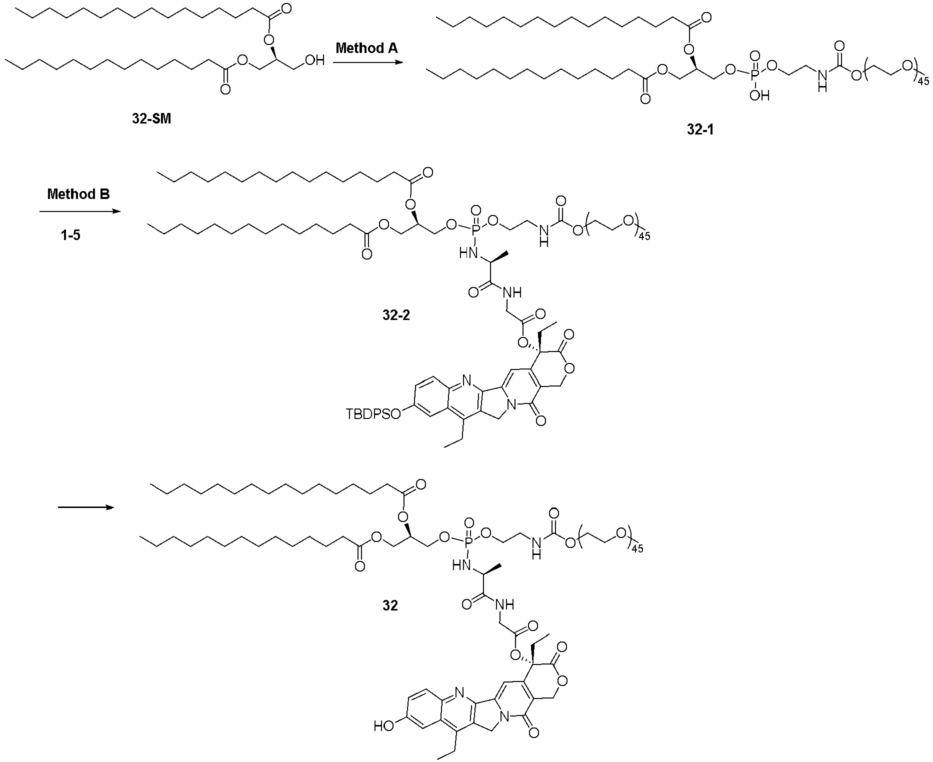

### Synthesis of Compound 32-2

Using starting material **32-SM** (synthesized according to the method for intermediate (S)-25 in the literature Tetrahedron, 2020, 76(2): 130813) and mPEG2000-SC, phospholipid compound **32-1** was synthesized according to General Synthesis Method A for Phospholipid PEG. Subsequently, compound **32-2** was synthesized by reacting it with intermediate 1-5 according to General Synthesis Method B for Drug Conjugates.

### Synthesis of Compound 32

Compound **32-2** (0.8 g) was dissolved in THF (10 mL), and TBAF (1.0 M in THF, 1.0 mL) and acetic acid (0.1 mL) were added. The mixture was stirred at room temperature for 12 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **32,** 654 mg, as a white solid, with an HPLC purity of 97.7%.

MS (ESI), m/z, 522.5 [DAG-OH]⁺. 693.4 [M-DAG+4Na]⁴⁺. DAG (Diglyceride) is a diglyceride fragment.

### Example 33: Synthesis of Compound 33

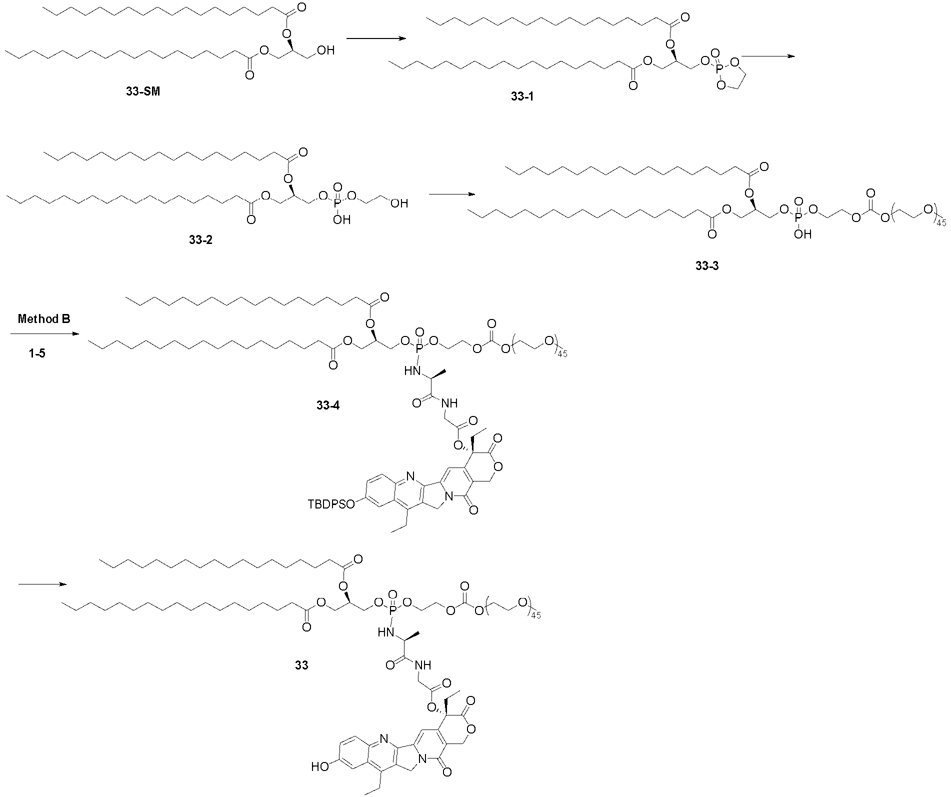

### Synthesis of Compound 33-2

Compound **33-2** was synthesized according to the method for compound 16 in the literature J. Org. Chem. 1999, 64, 21, 7727-7737.

### Synthesis of Compound 33-3

**33-2** (0.35 g, 0.46 mmol, 1.0 eq) was dissolved in dichloromethane and triethylamine (0.24 g, 2.32 mmol, 5.0 eq). A dichloromethane solution of mPEG-SC (1.0 g, 0.46 mmol, 1.0 eq) was added dropwise. The reaction mixture was stirred at room temperature for 12 h, then concentrated directly. The residue was purified by column chromatography (eluting with a MeOH/CH₂Cl₂ system) to yield compound **33-3,** 850 mg, with a yield of 66%.

### Synthesis of Compound 33-4

Compound **33-4** was synthesized according to General Synthesis Method B for Drug Conjugates.

### Synthesis of Compound 33

Compound **33-4** (0.64 g) was dissolved in THF (10 mL), and TBAF (1.0 M in THF, 1.0 mL) and acetic acid (0.05 mL) were added. The mixture was stirred at room temperature for 6 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **33,** 520 mg, as a white solid, with an HPLC purity of 88.3%.

MS (ESI), m/z, 606.5 [DAG-OH]⁺. 693.8 [M-DAG+4Na]⁴⁺. DAG (Diglyceride) is a diglyceride fragment.

### Example 34: Synthesis of Compound 34

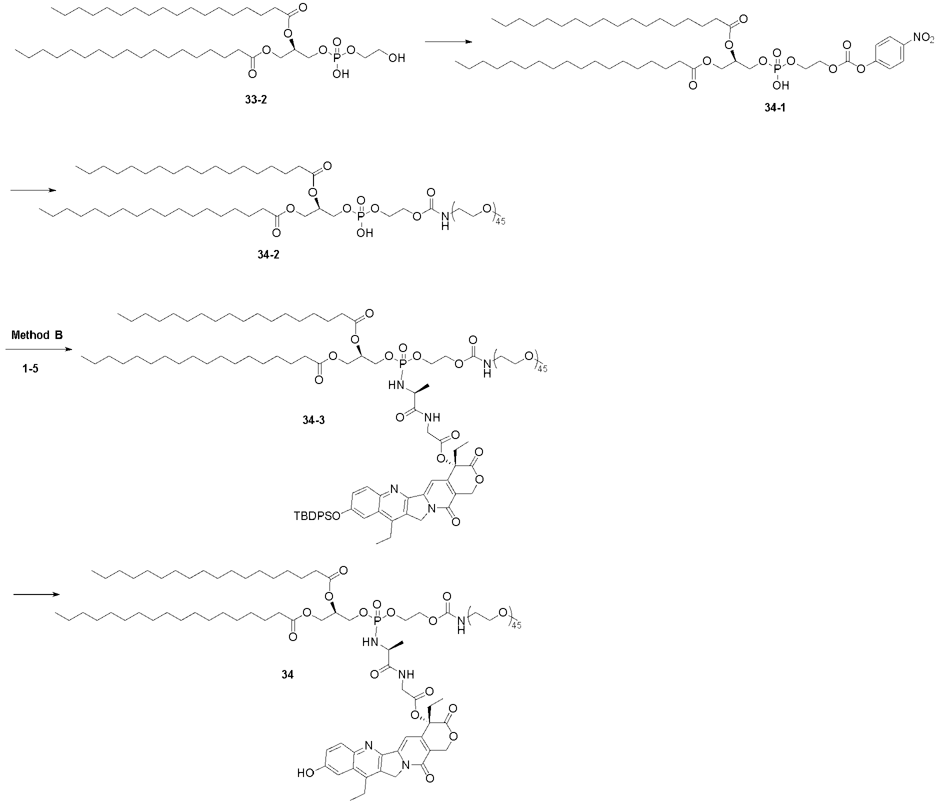

### Synthesis of Compound 34-1

Under an ice-water bath and nitrogen protection, p-nitrophenyl chloroformate (0.53 g, 2.67 mmol, 2.0 eq) and DMAP (0.33 g, 2.67 mmol, 2.0 eq) were added to a dichloromethane solution (30 mL) of compound **33-2** (1.0 g, 1.34 mmol, 1.0 eq). The reaction mixture was allowed to warm to room temperature and stirred overnight. The reaction was quenched with water and extracted with DCM (50 mL × 3). The organic phases were combined, washed with brine and water, dried over anhydrous sodium sulfate, filtered, and concentrated. The crude product was purified by column chromatography (eluting with a DCM/MeOH system) to yield compound **34-1,** 0.6 g, as a pale yellow solid.

### Synthesis of Compound 34-2

Under an ice-water bath and nitrogen protection, mPEG2000-NH₂ (1.1 g, 0.55 mmol, 1.0 eq) and DMAP (67 mg, 0.55 mmol, 1.0 eq) were added to a dichloromethane solution (20 mL) of compound **34-1** (0.5 g, 0.55 mmol, 1.0 eq). The reaction mixture was allowed to warm to room temperature and stirred for 6 h. The reaction was quenched with water and extracted with DCM (50 mL × 3). The organic phases were combined, washed with brine and water, dried over anhydrous sodium sulfate, filtered, and concentrated. The crude product was purified by column chromatography (eluting with a DCM/MeOH system) to yield compound **34-2,** 0.9 g, as a waxy solid, with a yield of 60%.

### Synthesis of Compound 34-3

Compound **34-2** and intermediate **1-5** were reacted according to General Synthesis Method B for Drug Conjugates to synthesize compound **34-3.**

### Synthesis of Compound 34

Compound **34-3** (0.5 g) was dissolved in THF (10 mL), and TBAF (1.0 M in THF, 1.0 mL) and acetic acid (0.1 mL) were added. The mixture was stirred at room temperature for 3 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **34,** 240 mg, as a white solid, with an HPLC purity of 99.2%.

MS (ESI), m/z, 607.0 [DAG-OH]⁺. 693.3 [M-DAG+4Na]⁴⁺. DAG (Diglyceride) is a diglyceride fragment.

### Example 35: Synthesis of Compound 35

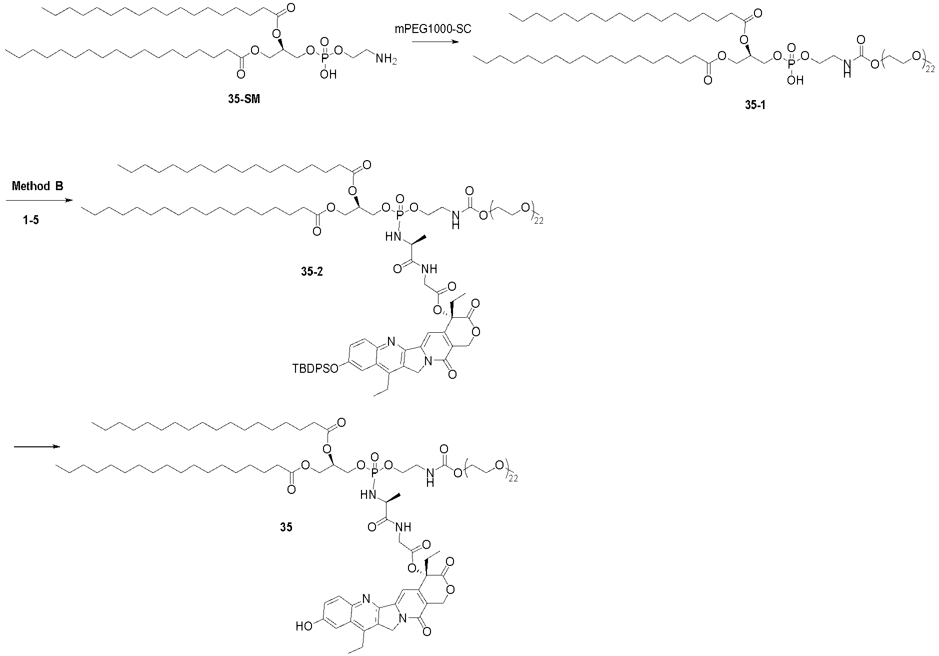

### Synthesis of Compound 35-1

1,2-Distearoyl-sn-glycero-3-phosphoethanolamine (1.0 g, 1.34 mmol, 1.0 eq) was dissolved in dichloromethane (20 mL) and triethylamine (0.68 g, 6.68 mmol, 5.0 eq). A dichloromethane solution (10 mL) of mPEG1000-SC (1.53 g, 1.34 mmol, 1.0 eq) was added dropwise. The reaction mixture was stirred at room temperature for 12 h, then concentrated directly. The residue was purified by column chromatography (eluting with a MeOH/CH₂Cl₂ system) to yield compound **35-1,** 2.0 g, as a waxy solid, with a yield of 84%.

### Synthesis of Compound 35-2

Compound **35-1** and intermediate **1-5** were reacted according to General Synthesis Method B for Drug Conjugates to synthesize compound **35-2.**

### Synthesis of Compound 35

Compound **35-2** (0.76 g) was dissolved in THF (10 mL), and TBAF (1.0 M in THF, 1.0 mL) and acetic acid (0.1 mL) were added. The mixture was stirred at room temperature for 12 h, then quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound 35, 530 mg, as a white solid, with an HPLC purity of 91.2%.

MS (ESI), m/z, 607.0 [DAG-OH]⁺. 440.4 [M-DAG+4Na]⁴⁺. DAG (Diglyceride) is a diglyceride fragment.

### Example 36: Synthesis of Compound 36

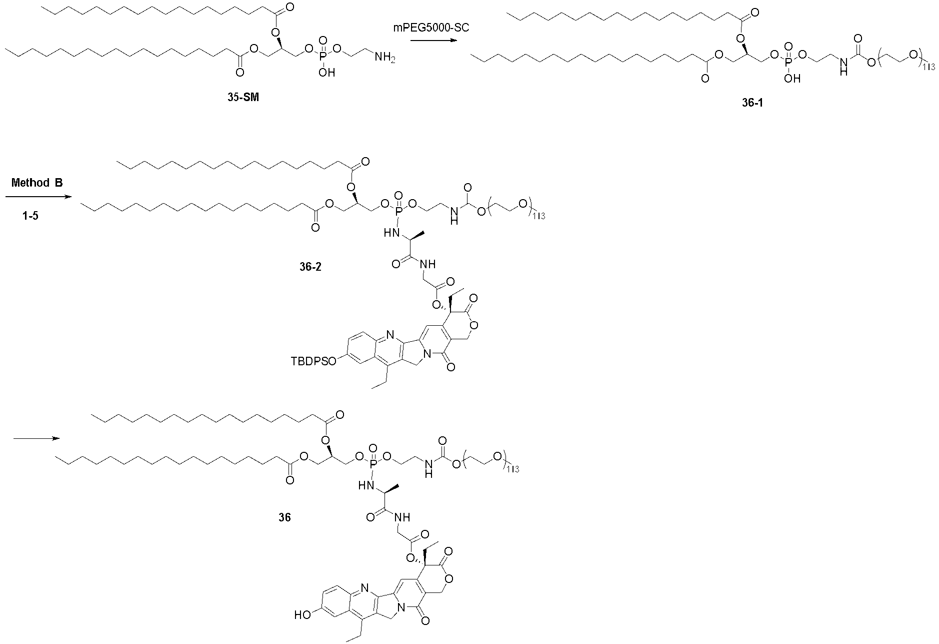

### Synthesis of Compound 36

Using starting material compound **35-SM,** mPEG5000-SC, and intermediate **1-5,** synthesis was performed according to the method for compound **35,** ultimately yielding compound **36,** 870 mg, as a white solid, with an HPLC purity of 95.5%.

MS (ESI), m/z, 607.0 [DAG-OH]⁺. 1442.0 [M-DAG+4Na]⁴⁺. DAG (Diglyceride) is a diglyceride fragment.

### Example 37: Synthesis of Compound 37

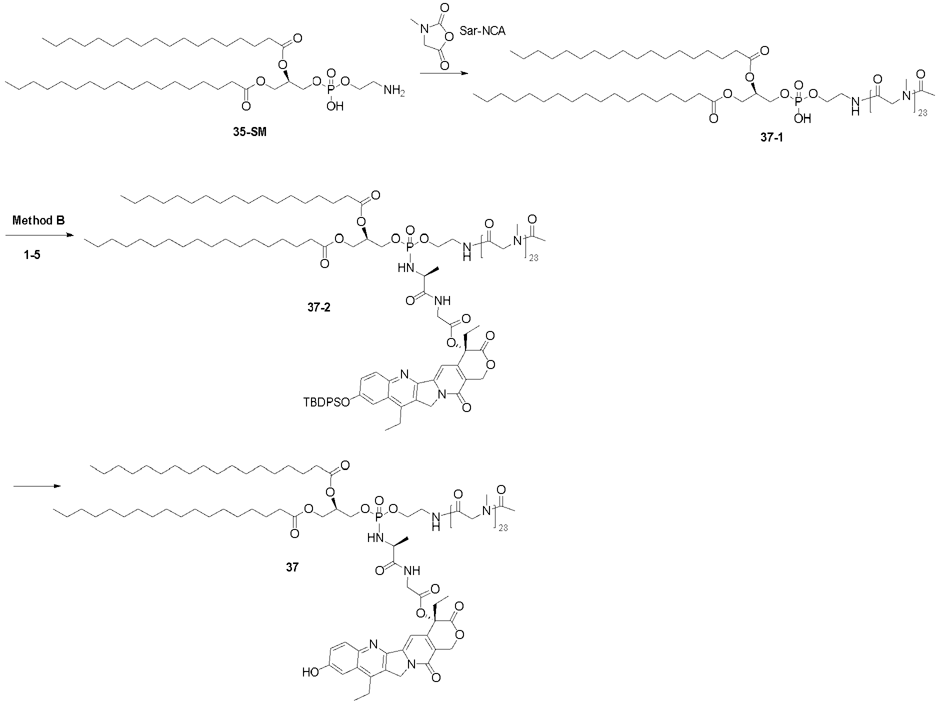

### Synthesis of Compound 37-1

At room temperature, **35-SM** (200 mg, 0.27 mmol, 1.0 eq), benzoic acid (163.1 mg, 1.34 mmol, 5.0 eq), DIPEA (34 mg, 0.27 mmol, 1.0 eq), 3-methyloxazolidine-2,5-dione **(Sar-NCA)** (0.89 g, 7.8 mmol, 29.0 eq), and DCM (50 mL) were added sequentially. The reaction was stirred at room temperature for 1 hour. Subsequently, DIPEA (172.5 mg, 1.34 mmol, 5.0 eq) and acetic anhydride (68 mg, 0.67 mmol, 2.5 eq) were added sequentially. The reaction was stirred at room temperature for 12 hours. After completion, the reaction mixture was dripped into MTBE, precipitating a white solid. The mixture was centrifuged, and the supernatant was removed. The solid was dried under vacuum to yield a white solid product, 1.1 g. ¹H NMR indicated a degree of polymerization of approximately 28.

### Synthesis of Compound 37

Compound **37-1** was then reacted with intermediate **1-5** according to General Synthesis Method B for Drug Conjugates to synthesize compound **37-2.** Compound **37-2** (0.89 g) was dissolved in THF (10 mL), and TBAF (1.0 M in THF, 1.0 mL) and acetic acid (0.05 mL) were added. The mixture was stirred at room temperature for 12 h, then quenched with water and extracted with DCM (50 mL × 3). The combined organic phases were washed with brine and water, dried over anhydrous sodium sulfate, filtered, and concentrated. The crude product was purified by silica gel column chromatography (eluting with a DCM/MeOH system) to yield compound **37,** 720 mg, as a white solid, with an HPLC purity of 98.6%.

MS (ESI), m/z, 607.0 [DAG-OH]⁺. 691.2 [M-DAG+4Na]⁴⁺. DAG (Diglyceride) denotes a diglyceride fragment.

### Example 38: Synthesis of Compound 38

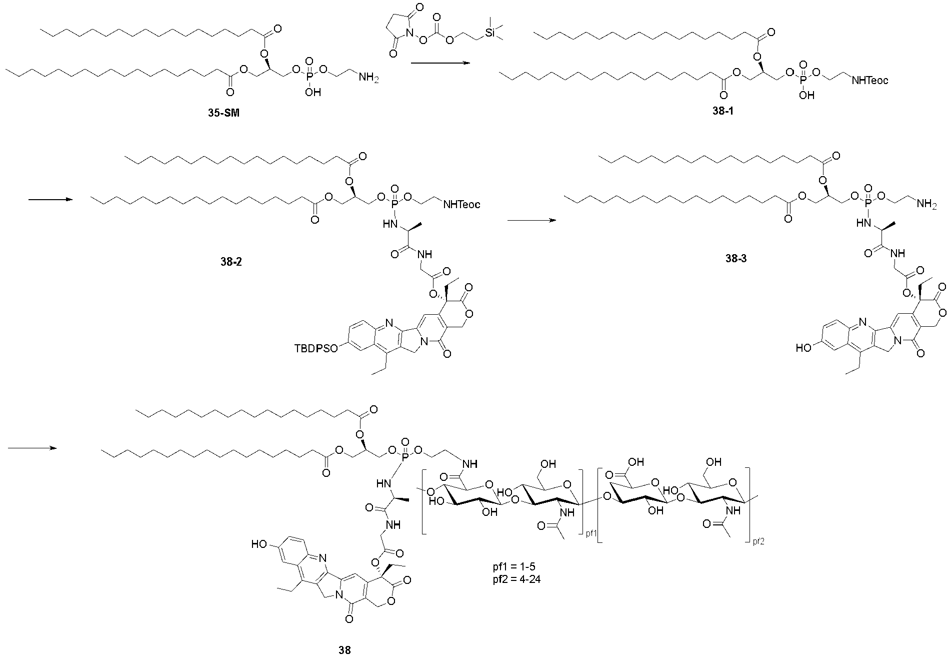

### Synthesis of Compound 38-1

Under an ice-water bath and nitrogen protection, to a solution of **35-SM** (1.0 g, 1.34 mmol, 1.0 eq) in dichloromethane (20 mL) were added *N-*[2-(trimethylsilyl)ethoxycarbonyloxy]succinimide (0.69 g, 2.67 mmol, 2.0 eq) and DIPEA (0.52 g, 4.0 mmol, 3.0 eq). The reaction mixture was then allowed to warm to room temperature and stirred for 12 h. The reaction mixture was quenched with water and extracted with dichloromethane (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **38-1,** 910 mg, as a white solid, with a yield of 77%.

### Synthesis of Compound 38-2

Using starting material compound **38-1** and intermediate **1-5,** synthesis was performed according to General Synthesis Method B for Drug Conjugates.

### Synthesis of Compound 38-3

Compound **38-2** (1.1 g) was dissolved in THF (20 mL), and TBAF (1.0 M in THF, 2.0 mL) and acetic acid (0.2 mL) were added. The mixture was stirred at room temperature for 14 h, then quenched with water and extracted with DCM (100 mL × 3). The organic phases were combined, washed with brine and water, dried over anhydrous sodium sulfate, filtered, and concentrated. The crude product was purified by silica gel column chromatography (eluting with a DCM/MeOH system) to yield compound **38-3,** 830 mg, as a white solid.

### Synthesis of Compound 38

Oligomeric hyaluronic acid (2K-10K Da, 98.5%, 500 mg), EDC (140 mg), and NHS (85 mg) were dissolved in phosphate buffer (0.1 M, pH 6.2, 5 mL), and the mixture was stirred at room temperature for 2 h. The mixture was centrifugally ultrafiltered (MWCO 3000) to remove excess EDC and NHS and concentrated to a 2 mL aqueous solution. A DMSO solution (1 mL) of compound **38-3** (180 mg) was added, and the mixture was stirred at room temperature overnight. TBAF (1.0 M in THF, 2.0 mL) in 95% ethanol and acetic acid (0.2 mL) were added. The mixture was stirred at room temperature for 14 h, then directly centrifugally ultrafiltered (MWCO 3000) and lyophilized to yield compound **38,** 450 mg, as a white solid, with a GPC purity of 98.5%.

MS (ESI), m/z, 607.0 [DAG-OH]⁺. 1004.3 [M-DAG+4Na]⁴⁺. DAG (Diglyceride) is a diglyceride fragment.

### Example 39: Synthesis of Compound 39

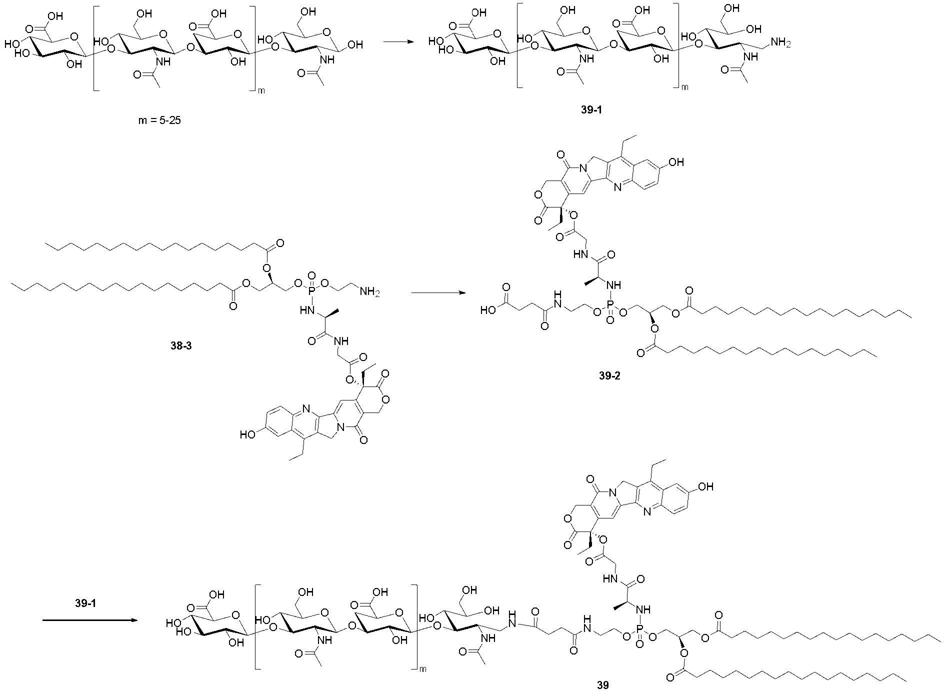

### Synthesis of Compound 39-1

Oligomeric hyaluronic acid (2K - 10K Da, 98.5%, 100 mg) was dissolved in water (10 mL), and NH₄OAc (200 mg) and sodium cyanoborohydride (20 mg × 10, added in portions) were added. The solution was heated to 40 °C and reacted for 24 h. The reaction mixture was directly centrifugally ultrafiltered (MWCO 3000) and lyophilized to yield 50 mg of hyaluronic acid amine **39-1.**

### Synthesis of Compound 39-2

To a THF solution (10 mL) of compound **38-3** (0.5 g, 0.4 mmol, 1.0 eq) were added succinic anhydride (200 mg, 2.0 mmol, 5.0 eq) and DIPEA (260 mg, 2.0 mmol, 5.0 eq). The reaction mixture was stirred at room temperature for 12 h. The mixture was concentrated, and the crude product was purified by silica gel column chromatography (eluting with a DCM/MeOH system) to yield compound **39-2,** 430 mg, as a white solid.

### Synthesis of Compound 39

To a DMSO solution of compound **39-2** (40 mg) were added EDC (30 mg), NHS (20 mg), and DIPEA (20 mg). The mixture was stirred at room temperature for 2 h, then a phosphate buffer (0.1 M, pH 6.2, 2 mL) solution of compound **39-1** was added, and the mixture was stirred at room temperature for 12 h. The reaction mixture was directly purified by centrifugal ultrafiltration (MWCO 3000) and lyophilized to yield compound **39,** 36 mg, as a white solid, with a GPC purity of 95.6%.

MS (ESI), m/z, 607.0 [DAG-OH]⁺. 1030.0 [M-DAG+4Na]⁴⁺. DAG (Diglyceride) is a diglyceride fragment.

### Example 40: Synthesis of Compound 40

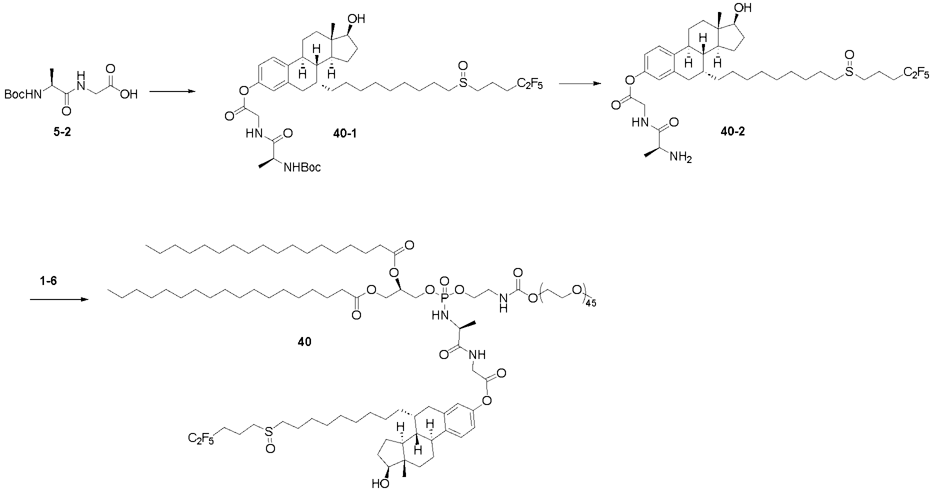

### Synthesis of Compound 40-1

At room temperature under nitrogen protection, to a solution of fulvestrant (0.5 g, 0.83 mmol, 1.0 eq) in CH₂Cl₂ (10 mL) were sequentially added **5-2** (0.31 g, 1.24 mmol, 1.5 eq), EDCI (0.24 g, 1.24 mmol, 1.5 eq), and DMAP (0.15 mg, 1.24 mmol, 1.5 eq). The mixture was stirred at room temperature for 2 h. Upon completion of the reaction, the mixture was directly concentrated and purified by silica gel column chromatography (eluting with PE/EA) to yield 0.6 g of solid, with a yield of 87%.

MS (ESI), m/z, 835.4 [M+H]⁺.

### Synthesis of Compound 40-2

To a solution of **40-1** (0.6 g) in CH₂Cl₂ (10 mL) was added dropwise TFA (2 mL). The mixture was stirred at room temperature for 2 h. Upon completion of the reaction, the reaction mixture was concentrated under reduced pressure to yield the product as a yellow solid, 0.55 g (as TFA salt).

MS (ESI), m/z, 735.4 [M+H]⁺.

### Synthesis of Compound 40

At room temperature (25 °C) under N₂ protection, crude product **1-6** (200 mg, 0.23 mmol, 1.0 eq) was dissolved in CH₂Cl₂ (20 mL). The temperature was lowered to 0 °C, and a DCM solution (5 mL) of **40-2** (0.55 g, 0.69 mmol, 3.0 eq) and triethylamine (120 mg, 1.15 mmol, 5.0 eq) were added dropwise sequentially to the reaction mixture. After the dropwise addition, the ice-water bath was removed, and the mixture was stirred at room temperature for 2 h. Upon completion of the reaction, the mixture was concentrated under reduced pressure, dissolved in DCM (50 mL), and washed with water three times (50 mL × 3). The organic phase was dried over anhydrous Na₂SO₄, filtered, and concentrated. The crude product was purified by column chromatography (eluting with a DCM and MeOH system) to yield 320 mg of the product.

MS (ESI), m/z, 607.0 [DAG-OH]⁺. 747.1 [M-DAG+4Na]⁴⁺. DAG (Diglyceride) is a diglyceride fragment.

### Example 41: Synthesis of Compound 41

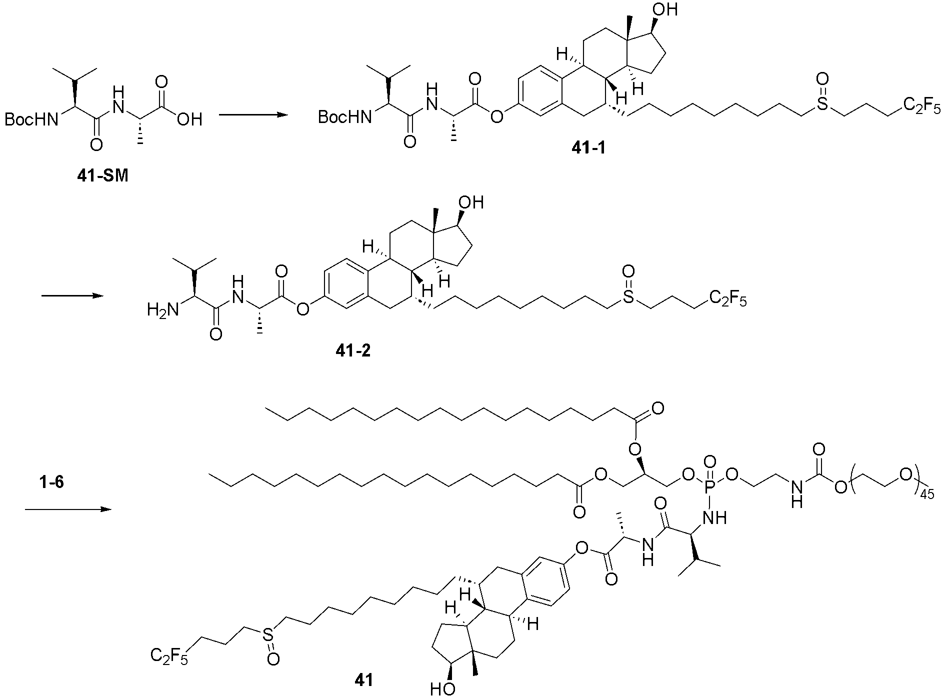

### Synthesis of Compound 41-1

At room temperature under nitrogen protection, to a solution of fulvestrant (0.5 g, 0.83 mmol, 1.0 eq) in CH₂Cl₂ (10 mL) were sequentially added (tert-butoxycarbonyl)-*L*-valyl-*L-*alanine (0.36 g, 1.24 mmol, 1.5 eq), EDCI (0.24 g, 1.24 mmol, 1.5 eq), and DMAP (0.15 mg, 1.24 mmol, 1.5 eq). The mixture was stirred at room temperature for 2 h. Upon completion of the reaction, the mixture was directly concentrated and purified by silica gel column chromatography (eluting with PE/EA) to yield 0.58 g of solid, with a yield of 80%.

MS (ESI), m/z, 877.5 [M+H]⁺.

### Synthesis of Compound 41-2

To a solution of **41-1** (0.5 g) in CH₂Cl₂ (10 mL) was added dropwise TFA (2 mL). The mixture was stirred at room temperature for 2 h. Upon completion of the reaction, the reaction mixture was concentrated under reduced pressure to yield the product as a yellow solid, 0.5 g (as TFA salt).

MS (ESI), m/z, 735.4 [M+H]⁺.

### Synthesis of Compound 41

At room temperature (25 °C) under N₂ protection, crude product 1-6 (200 mg, 0.23 mmol, 1.0 eq) was dissolved in CH₂Cl₂ (20 mL). The temperature was lowered to 0 °C, and a DCM solution (5 mL) of **41-2** (0.53 g, 0.69 mmol, 3.0 eq) and triethylamine (120 mg, 1.15 mmol, 5.0 eq) were added dropwise sequentially to the reaction mixture. After the dropwise addition, the ice-water bath was removed, and the mixture was stirred at room temperature for 2 h. Upon completion of the reaction, the mixture was concentrated under reduced pressure, dissolved in DCM (50 mL), and washed with water three times (50 mL × 3). The organic phase was dried over anhydrous Na₂SO₄, filtered, and concentrated. The crude product was purified by column chromatography (eluting with a DCM and MeOH system) to yield 260 mg of the product, with an HPLC purity of 99.2%.

MS (ESI), m/z, 607.0 [DAG-OH]⁺. 757.6 [M-DAG+4Na]⁴⁺. DAG (Diglyceride) is a diglyceride fragment.

### Example 42: Synthesis of Compound 42

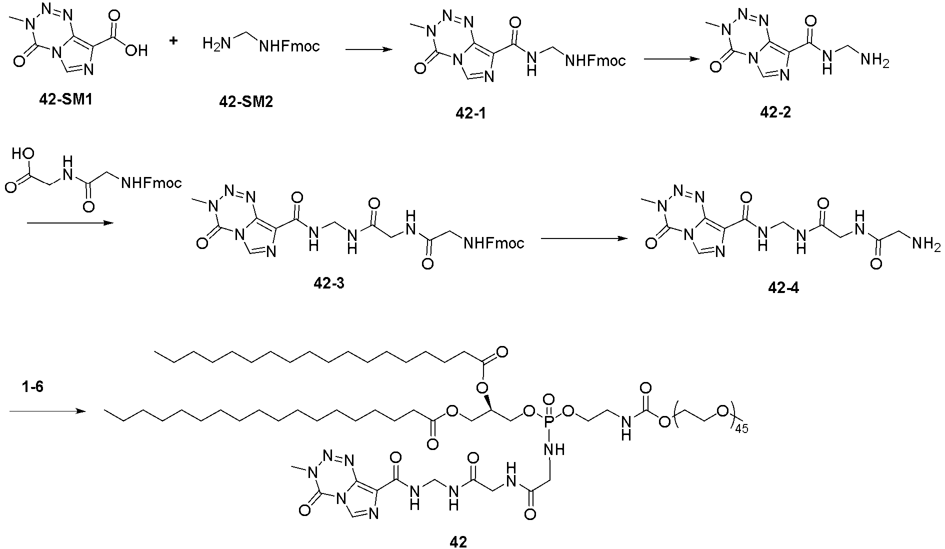

### Synthesis of Compound 42-1

At room temperature under nitrogen protection, to a solution of temozolomide acid**42-SM1** (0.5 g, 2.6 mmol, 1.0 eq) in CH₂Cl₂ (20 mL) were added **42-SM1** (prepared according to the method for intermediate 10 in patent WO2015104684A1) (1.0 g, 3.8 mmol, 1.5 eq), HATU (1.5 g, 3.8 mmol, 1.5 eq), and DIPEA (1.0 g, 7.7 mmol, 3.0 eq). The mixture was stirred at room temperature for 2 h. Upon completion of the reaction, the reaction mixture was quenched with water and extracted with dichloromethane (50 mL × 3). The organic phases were combined, washed with brine and water, dried over anhydrous sodium sulfate, filtered, and concentrated. The residue was purified by column chromatography (eluting with DCM and methanol) to yield 0.8 g of solid, with a yield of 70%.

MS (ESI), m/z, 446.1 [M+H]⁺.

### Synthesis of Compound 42-2

To a solution of **42-1** (0.8 g) in CH₂Cl₂ (5 mL) was added diethylamine (2 mL). The mixture was stirred at room temperature for 1 h. Upon completion of the reaction, the mixture was concentrated, and the residue was purified by reverse-phase column chromatography (eluting with water and acetonitrile) and lyophilized to yield product **42-2,** 0.3 g, with a yield of 75%.

MS (ESI), m/z, 224.1 [M+H]⁺.

### Synthesis of Compound 42-3

At room temperature (25 °C) under N₂ protection, to a solution of **42-2** (300 mg, 1.35 mmol, 1.0 eq) in CH₂Cl₂ (10 mL) were added N-Fmoc-glycyl-glycine (0.7 g, 2.0 mmol, 1.5 eq), EDCI (0.5 g, 2.69 mmol, 2.0 eq), and DIPEA (0.52 g, 4.0 mmol, 3.0 eq), and the mixture was stirred for 2 h. The reaction mixture was quenched with water and extracted with DCM (50 mL × 3). The organic phases were combined, washed with brine and water, dried over anhydrous sodium sulfate, filtered, and concentrated. The crude product was purified by silica gel column chromatography (eluting with a DCM/MeOH system) to yield compound **42-3,** 650 mg, with a yield of 86%.

MS (ESI), m/z, 560.2 [M+H]⁺.

### Synthesis of Compound 42-4

To a solution of **42-3** (0.6 g) in CH₂Cl₂ (5 mL) was added diethylamine (2 mL). The mixture was stirred at room temperature for 1 h. Upon completion of the reaction, the mixture was concentrated, and the residue was purified by reverse-phase column chromatography (eluting with water and acetonitrile) and lyophilized to yield product **42-4,** 0.3 g, with a yield of 83%.

MS (ESI), m/z, 338.1 [M+H]⁺.

### Synthesis of Compound 42

At room temperature (25 °C) under N₂ protection, crude product **1-6** (200 mg, 0.23 mmol, 1.0 eq) was dissolved in CH₂Cl₂ (20 mL). The temperature was lowered to 0 °C, and a DCM solution (5 mL) of **42-4** (0.23 g, 0.69 mmol, 3.0 eq) and triethylamine (120 mg, 1.15 mmol, 5.0 eq) were added dropwise sequentially to the reaction mixture. After the dropwise addition, the ice-water bath was removed, and the mixture was stirred at room temperature for 2 h. Upon completion of the reaction, the mixture was concentrated under reduced pressure, dissolved in DCM (50 mL), and washed with water three times (50 mL × 3). The organic phase was dried over anhydrous Na₂SO₄, filtered, and concentrated. The crude product was purified by column chromatography (eluting with a DCM and MeOH system) to yield 180 mg of the product, with an HPLC purity of 93.6%.

MS (ESI), m/z, 607.0 [DAG-OH]⁺. 647.8 [M-DAG+4Na]⁴⁺. DAG (Diglyceride) is a diglyceride fragment.

### Example 43: Synthesis of Compound 43

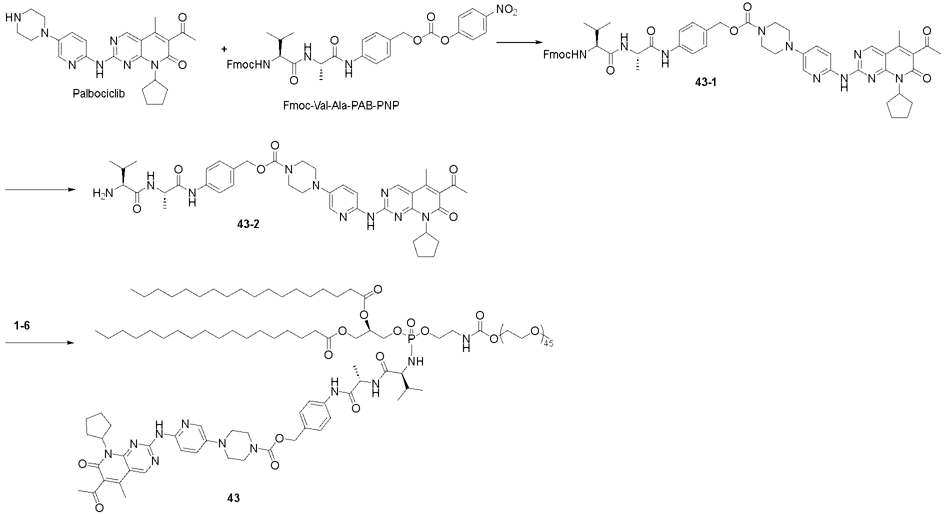

### Synthesis of Compound 43-1

To a solution of Palbociclib (0.5 g, 1.1 mmol, 1.0 eq) in DMF (10 mL) were added Fmoc-Val-Ala-PAB-PNP (0.92 g, 1.34 mmol, 1.2 eq) and DIPEA (0.43 g, 3.36 mmol, 3.0 eq). The mixture was stirred at room temperature for 12 h. The reaction mixture was quenched with water and extracted with dichloromethane (50 mL × 3). The organic phases were combined, washed with brine and water, dried over anhydrous sodium sulfate, filtered, and concentrated. The residue was purified by column chromatography (eluting with DCM and methanol) to yield 0.95 g of solid, with a yield of 86%.

MS (ESI), m/z, 989.5 [M+H]⁺.

### Synthesis of Compound 43-2

To a solution of **43-1** (0.9 g) in DMF (10 mL) was added diethylamine (3 mL). The mixture was stirred at room temperature for 2 h. The reaction mixture was concentrated directly, and the residue was purified by reverse-phase column chromatography (eluting with 0.05% TFA in water and methanol) and lyophilized to yield product **43-2,** 0.6 g, with a yield of 86%.

MS (ESI), m/z, 767.4 [M+H]⁺.

### Synthesis of Compound 43

At room temperature (25 °C) under N₂ protection, crude product **1-6** (200 mg, 0.23 mmol, 1.0 eq) was dissolved in CH₂Cl₂ (20 mL). The temperature was lowered to 0 °C, and a DCM solution (5 mL) of **43-2** (0.53 g, 0.69 mmol, 3.0 eq) and triethylamine (120 mg, 1.15 mmol, 5.0 eq) were added dropwise sequentially to the reaction mixture. After the dropwise addition, the ice-water bath was removed, and the mixture was stirred at room temperature for 2 h. Upon completion of the reaction, the mixture was concentrated under reduced pressure, dissolved in DCM (100 mL), and washed with water three times (50 mL × 3). The organic phase was dried over anhydrous Na₂SO₄, filtered, and concentrated. The crude product was purified by column chromatography (eluting with a DCM and MeOH system) to yield 310 mg of the product, with an HPLC purity of 97.2%.

MS (ESI), m/z, 607.0 [DAG-OH]⁺. 755.1 [M-DAG+4Na]⁴⁺. DAG (Diglyceride) is a diglyceride fragment.

### Example 44: Synthesis of Compound 44

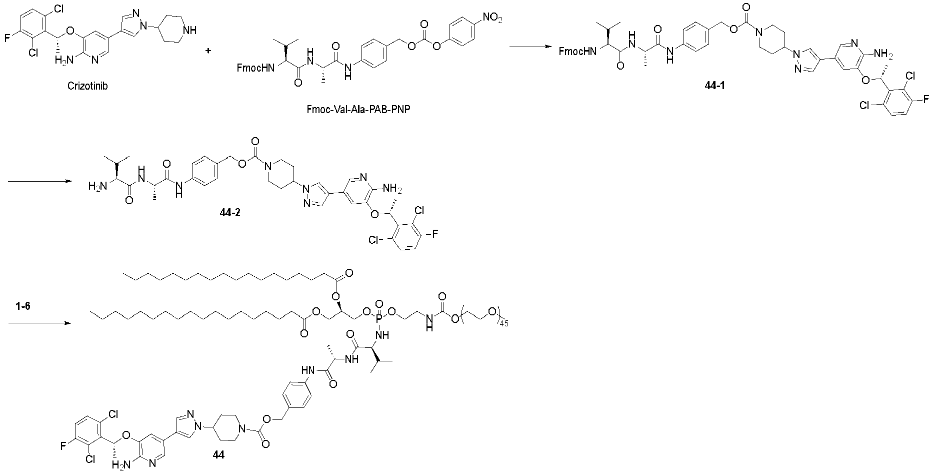

### Synthesis of Compound 44-1

To a solution of Crizotinib (0.50 g, 1.1 mmol, 1.0 eq) in DMF (10 mL) were added Fmoc-Val-Ala-PAB-PNP (0.92 g, 1.34 mmol, 1.2 eq) and DIPEA (0.43 g, 3.36 mmol, 3.0 eq). The mixture was stirred at room temperature for 6 h. The reaction mixture was quenched with water and extracted with dichloromethane (50 mL × 3). The organic phases were combined, washed with brine and water, dried over anhydrous sodium sulfate, filtered, and concentrated. The residue was purified by column chromatography (eluting with DCM and methanol) to yield 1.0 g of solid, with a yield of 90%.

MS (ESI), m/z, 991.3 [M+H]⁺.

### Synthesis of Compound 44-2

To a solution of **44-1** (1.0 g) in DMF (10 mL) was added diethylamine (5 mL). The mixture was stirred at room temperature for 2 h. The reaction mixture was concentrated directly, and the residue was purified by reverse-phase column chromatography (eluting with 0.05% TFA in water and methanol) and lyophilized to yield product **44-2,** 0.7 g, with a yield of 90%.

MS (ESI), m/z, 769.2 [M+H]⁺.

### Synthesis of Compound 44

At room temperature (25 °C) under N₂ protection, crude product **1-6** (200 mg, 0.23 mmol, 1.0 eq) was dissolved in CH₂Cl₂ (20 mL). The temperature was lowered to 0 °C, and a DCM solution (5 mL) of **44-2** (0.54 g, 0.70 mmol, 3.0 eq) and triethylamine (120 mg, 1.15 mmol, 5.0 eq) were added dropwise sequentially to the reaction mixture. After the dropwise addition, the ice-water bath was removed, and the mixture was stirred at room temperature for 5 h. Upon completion of the reaction, the mixture was concentrated under reduced pressure, dissolved in DCM (100 mL), and washed with water three times (50 mL × 3). The organic phase was dried over anhydrous Na₂SO₄, filtered, and concentrated. The crude product was purified by column chromatography (eluting with a DCM and MeOH system) to yield 296 mg of the product, with an HPLC purity of 88.6%.

MS (ESI), m/z, 607.0 [DAG-OH]⁺. 755.6 [M-DAG+4Na]⁴⁺. DAG (Diglyceride) is a diglyceride fragment.

### Example 45: Synthesis of Compound 45

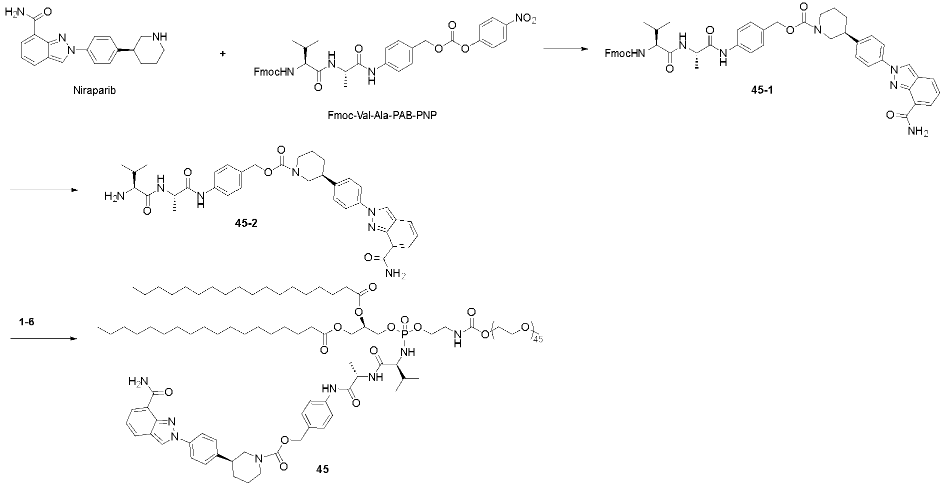

### Synthesis of Compound 45-1

To a solution of Niraparib (0.35 g, 1.1 mmol, 1.0 eq) in DMF (10 mL) were added Fmoc-Val-Ala-PAB-PNP (0.92 g, 1.34 mmol, 1.2 eq) and DIPEA (0.43 g, 3.36 mmol, 3.0 eq). The mixture was stirred at room temperature for 6 h. The reaction mixture was quenched with water and extracted with dichloromethane (50 mL × 3). The organic phases were combined, washed with brine and water, dried over anhydrous sodium sulfate, filtered, and concentrated. The residue was purified by column chromatography (eluting with DCM and methanol) to yield 0.75 g of solid, with a yield of 80%.

MS (ESI), m/z, 862.4 [M+H]⁺.

### Synthesis of Compound 45-2

To a solution of **45-1** (0.75 g) in DMF (5 mL) was added diethylamine (2 mL). The mixture was stirred at room temperature for 4 h. The reaction mixture was concentrated directly, and the residue was purified by reverse-phase column chromatography (eluting with 0.05% TFA in water and methanol) and lyophilized to yield product **45-2,** 0.5 g, with a yield of 90%.

MS (ESI), m/z, 640.3 [M+H]⁺.

### Synthesis of Compound 45

At room temperature (25 °C) under N₂ protection, crude product **1-6** (200 mg, 0.23 mmol, 1.0 eq) was dissolved in CH₂Cl₂ (20 mL). The temperature was lowered to 0 °C, and a DCM solution (5 mL) of **45-2** (0.45 g, 0.70 mmol, 3.0 eq) and triethylamine (120 mg, 1.15 mmol, 5.0 eq) were added dropwise sequentially to the reaction mixture. After the dropwise addition, the ice-water bath was removed, and the mixture was stirred at room temperature for 5 h. Upon completion of the reaction, the mixture was concentrated under reduced pressure, dissolved in DCM (80 mL), and washed with water three times (50 mL × 3). The organic phase was dried over anhydrous Na₂SO₄, filtered, and concentrated. The crude product was purified by column chromatography (eluting with a DCM and MeOH system) to yield 160 mg of the product, with an HPLC purity of 99.2%.

MS (ESI), m/z, 607.0 [DAG-OH]⁺. 723.3 [M-DAG+4Na]⁴⁺. DAG (Diglyceride) is a diglyceride fragment.

### Example 46: Synthesis of Compound 46

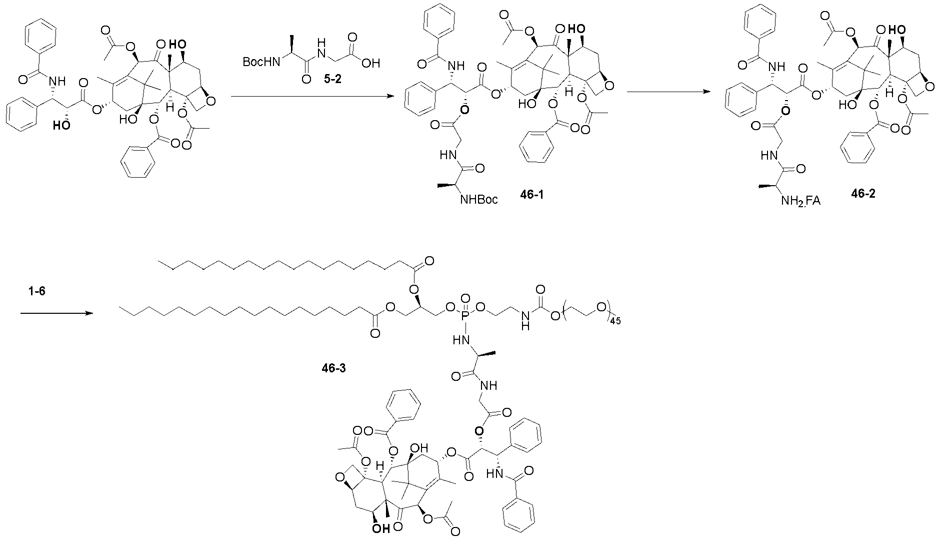

### Synthesis of Compound 46-1

At room temperature (25 °C), to a solution of Paclitaxel (2.0 g, 2.34 mmol, 1.0 eq) in CH₂Cl₂ (60 mL) were added sequentially **5-2** (0.69 g, 2.81 mmol, 1.2 eq), DMAP (0.86 g, 7.03 mmol, 3.0 eq), and EDCI (1.34 g, 7.03 mmol, 3.0 eq). The mixture was stirred at room temperature for 2 h. Upon completion of the reaction, the reaction mixture was poured into a saturated aqueous ammonium chloride solution and washed thoroughly (150 mL × 3). The organic phases were combined, dried over anhydrous Na₂SO₄, concentrated under reduced pressure. The crude product was dried under vacuum, and purified by silica gel column chromatography (eluting with PE/EA = 1:2 to 1:3) to yield 2.0 g of pure product, with a yield of 79%.

MS (ESI), m/z, 982.4 [M-100]⁺.

### Synthesis of Compound 46-2

At 0 °C, a solution of **46-1** (2.0 g, 1.85 mmol, 1.0 eq) in formic acid (10 mL) was stirred for 6 h. After completion, the reaction mixture was concentrated to 3 mL at 0 °C, and MTBE ether (100 mL) was added dropwise, precipitating a large amount of white solid. The solid was collected by suction filtration, and the filter cake was thoroughly dried under vacuum to yield 1.5 g of pure white solid.

MS (ESI), m/z, 982.4 [M+H]⁺.

### Synthesis of Compound 46

At room temperature (25 °C) under N₂ protection, crude product **1-6** (755 mg, 0.27 mmol, 1.0 eq) was dissolved in CH₂Cl₂ (10 mL). The temperature was lowered to 0 °C, and a DCM solution (2 mL) of **46-2** (1.36 g, 1.38 mmol, 5.0 eq) and triethylamine (271 mg, 2.69 mmol, 10 eq) were added dropwise sequentially to the reaction mixture. After the dropwise addition, the ice-water bath was removed, and the mixture was stirred at room temperature for 2 h. TLC indicated completion of the reaction. The reaction mixture was concentrated under reduced pressure, dissolved in DCM (100 mL), and washed with saturated brine three times (150 mL × 3). The organic phase was dried over anhydrous Na₂SO₄, concentrated under reduced pressure, and the crude product was purified by normal-phase column chromatography, first eluting with an EA/MeOH = 1:0 to 10:1 system, then switching to a DCM/MeOH = 95:5 to 93:7 to 92:8 to 89:11 elution system, to yield 650 mg of the product.

MS (ESI), m/z, 607.0 [DAG-OH]⁺. 808.8 [M-DAG+4Na]⁴⁺. DAG (Diglyceride) is a diglyceride fragment.

### Example 47: Synthesis of Compound 47

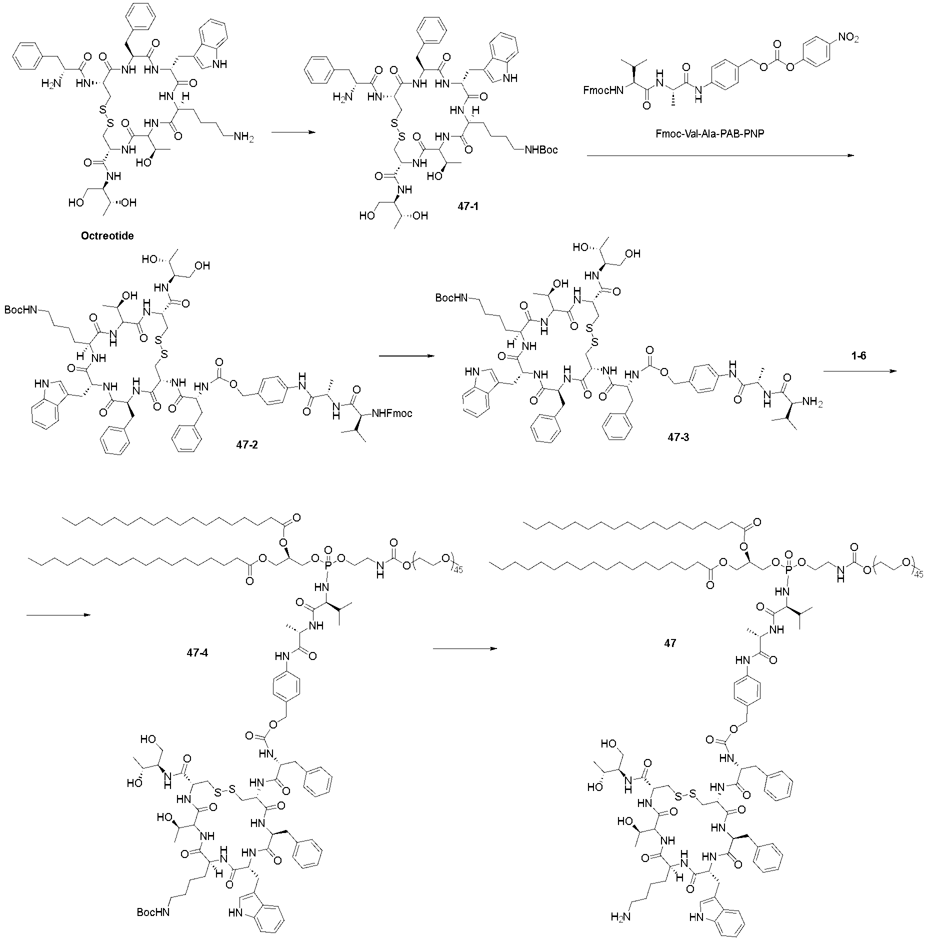

### Synthesis of Compound 47-1

At room temperature (25 °C) under nitrogen protection, DIPEA (1.14 g, 8.82 mmol, 3.0 eq) was added to a solution of Octreotide (3.0 g, 2.94 mmol, 1.0 eq) in DMF (60 mL). The reaction mixture was cooled to -40 °C, and a solution of *N*-succinimidyl tert-butyl carbonate (633 mg, 2.94 mmol, 1.0 eq) in DMF (10 mL) was added dropwise slowly. The reaction mixture was stirred at -40 °C for 0.5 hours, then allowed to warm naturally to room temperature and stirred for an additional 2 hours. LCMS indicated completion of the reaction. The mixture was concentrated and purified by silica gel column chromatography (eluting with a DCM/MeOH system) to yield product **47-1** as a white solid, 2.8 g, with a yield of 85%.

MS (ESI), m/z, 1118.40 [M+H]⁺.

### Synthesis of Compound 47-2

To a solution of **47-1** (1.5 g, 1.34 mmol, 1.0 eq) in DMF (10 mL) were added Fmoc-Val-Ala-PAB-PNP (0.9 g, 1.34 mmol, 1.0 eq) and DIPEA (0.43 g, 3.36 mmol, 3.0 eq). The mixture was stirred at room temperature for 4 h. The reaction mixture was quenched with water, concentrated, and the residue was directly purified by reverse-phase column chromatography (eluting with a system of 0.05% TFA H₂O and methanol). The product peak was collected, concentrated, and lyophilized to yield solid product **47-2,** 1.3 g, with a yield of 58%.

MS (ESI), m/z, 1660.5 [M+H]⁺.

### Synthesis of Compound 47-3

Diethylamine (3 mL) was added to a solution of **47-2** (1.2 g) in DMF (10 mL). The mixture was stirred at room temperature for 2 h. The reaction mixture was concentrated directly, and the residue was purified by reverse-phase column chromatography (eluting with a system of 0.05% TFA in water and methanol). The product peak was collected, concentrated, and lyophilized to yield solid product **47-3,** 0.84 g, with a yield of 81%.

MS (ESI), m/z, 1438.6 [M+H]⁺.

### Synthesis of Compound 47-4

At room temperature (25 °C) under N₂ protection, crude product **1-6** (300 mg, 0.11 mmol, 1.0 eq) was dissolved in CH₂Cl₂ (10 mL). The temperature was lowered to 0 °C, and a DCM/DMF solution (2/0.5 mL) of **47-3** (0.77 g, 0.54 mmol, 5.0 eq) and triethylamine (108 mg, 1.07 mmol, 10.0 eq) were added dropwise sequentially to the reaction mixture. After the dropwise addition, the ice-water bath was removed, and the mixture was stirred at room temperature for 12 h. The reaction mixture was concentrated directly. The crude product was first purified by normal-phase column chromatography (eluting with a DCM/MeOH system), then by reverse-phase column chromatography (eluting with a system of 0.05% TFA in water and methanol). The product peak was collected, concentrated, and lyophilized to yield solid product **47-4,** 180 mg.

MS (ESI), m/z, 607.0 [DAG-OH]⁺. 923.0 [M-DAG+4Na]⁴⁺. DAG (Diglyceride) is a diglyceride fragment.

### Synthesis of Compound 47

In an ice-water bath at 5 °C under N₂ protection, trifluoroacetic acid (1 mL) was added to a solution of **47-4** (100 mg) in CH₂Cl₂ (5 mL). The reaction mixture was allowed to warm to room temperature and stirred for 1 h. HPLC monitoring indicated complete reaction. The mixture was concentrated at low temperature (5 °C), dissolved in DMF (1 mL), and purified by Prep-HPLC (eluting with a system of 0.05% TFA in water and acetonitrile). The product peak was collected and lyophilized to yield solid product **47,** 54 mg.

MS (ESI), m/z, 607.0 [DAG-OH]⁺. 897.8 [M-DAG+4Na]⁴⁺. DAG (Diglyceride) is a diglyceride fragment.

### Example 48: Synthesis of Compound 48

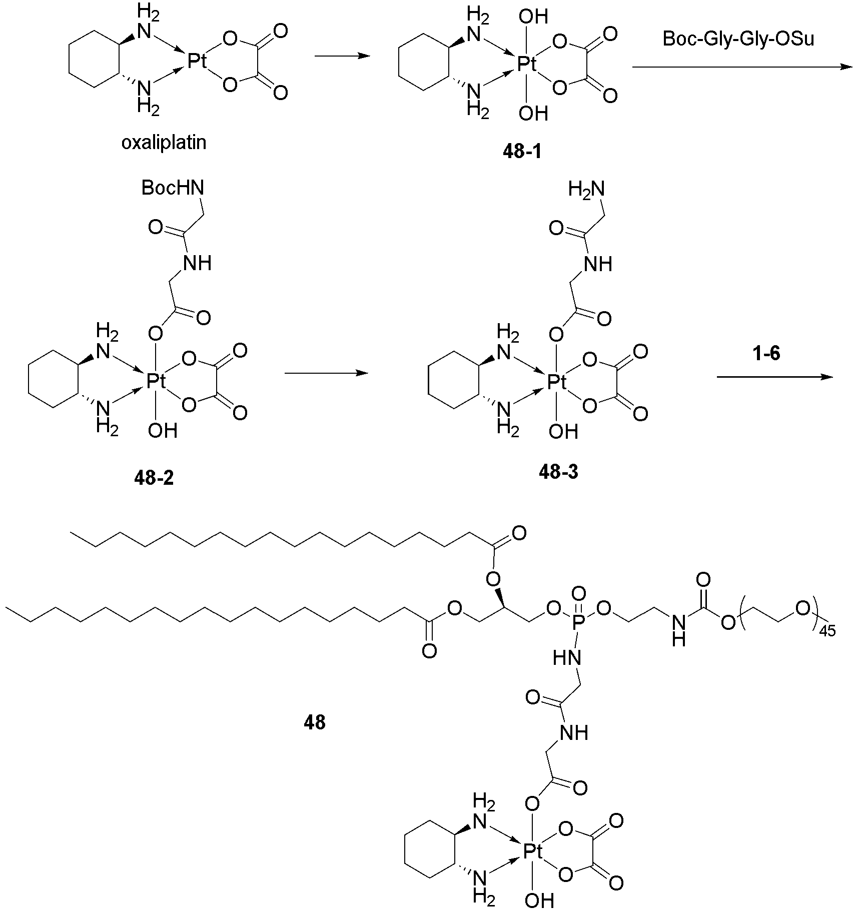

### Synthesis of Compound 48-1

Oxaliplatin (2.0 g, 5.03 mmol) was added to water (50 mL), followed by 30% hydrogen peroxide (50 mL). The mixture was heated to 50 °C and reacted for 1 h. The reaction mixture was concentrated, then 50 mL of ethanol was added, precipitating a solid. The solid was filtered and dried under vacuum to yield compound 48-1 as a white solid, 2.0 g, with a yield of 92%.

MS (ESI), m/z, 395.8 [M+H-2H₂O]⁺.

### Synthesis of Compound 48-2

**48-1** (1.0 g, 2.32 mmol, 1.0 eq) and Boc-Gly-Gly-Osu (0.76 g, 2.32 mmol, 1.0 eq) were added to DMSO (10 mL). The mixture was heated to 60 °C and reacted for 12 h. The reaction mixture was then added dropwise to 100 mL of ice-cold MTBE. The solid was collected by centrifugation and dried under vacuum to yield crude product compound **48-2,** 1.2 g, with a yield of 80%.

MS (ESI), m/z, 590.2 [M-55]⁺.

### Synthesis of Compound 48-3

Trifluoroacetic acid (5 mL) was added to a solution of compound **48-2** (1.0 g) in DCM (10 mL). The reaction mixture was stirred at room temperature for 1 h, concentrated, washed twice with MTBE, and the solid was dried under vacuum to yield compound **48-3** (TFA salt), 0.7 g, with a yield of 78%.

MS (ESI), m/z, 546.2 [M+H]⁺.

### Synthesis of Compound 48

At room temperature (25 °C) under N₂ protection, crude product **1-6** (500 mg, 0.18 mmol, 1.0 eq) was dissolved in CH₂Cl₂ (10 mL). The temperature was lowered to 0 °C, and a DCM solution (2 mL) of **48-3** (0.49 g, 0.89 mmol, 5.0 eq) and triethylamine (180 mg, 1.78 mmol, 10.0 eq) were added dropwise sequentially to the reaction mixture. After the dropwise addition, the ice-water bath was removed, and the mixture was stirred at room temperature for 6 h. The reaction mixture was concentrated to 5 mL and added dropwise to MTBE (100 mL). The solid was collected by centrifugation to yield 460 mg of crude product, which could be further purified by Sephadex LH-20.

MS (ESI), m/z, 607.0 [DAG-OH]⁺. 699.8 [M-DAG+4Na]⁴⁺. DAG (Diglyceride) is a diglyceride fragment.

### Evaluation Example 1: Drug Release Study

### PBS Release Study:

Experimental Method: At room temperature, 2.0 mg of the phospholipid conjugate was weighed and dissolved in 20 mL of distilled water. 1.0 mL of the above solution was pipetted into a 50 mL volumetric flask and diluted to volume (50 mL) with PBS buffer (pH=5.0). This solution was placed in a 37 °C water bath constant temperature shaker. At regular intervals, 0.5 mL samples were taken and sent for HPLC detection of drug release content.

### Experimental Results:

**Table 1 Drug Release Test of the Compounds of the Present Invention in PBS**

| Compound | SN38 Release Rate % (pH = 5.0 PBS, 6 h) | SN38 Release Rate % (pH = 5.0 PBS, 24 h) |
|---|---|---|
| 1 | 6.5 | 13.1 |
| 5 | 7.5 | 19.6 |
| 7 | 7.0 | 15.6 |
| 15 | <1 | <1 |
| 16 | <1 | <1 |

### Protease Release Study:

Papain Activation: 10 mg of papain was dissolved in 1.0 mL of PBS (10 mM, pH = 5.0), and 1.5 mL of a 30 mM DTT/15 mM EDTA solution was added. The mixture was shaken in a 37 °C water bath for 30 minutes and then cooled to room temperature.

At room temperature, 4 mg each of phospholipid conjugate Examples **5, 9, 15,** and **16** were accurately weighed and dissolved in 10 mM sodium acetate buffer pH 5.0 (1.0 mL each). 0.5 mL of each solution was taken, and 0.1 mL of the activated papain solution was added to each. The mixtures were shaken in a 37 °C water bath for 6 h and 24 h, respectively. At the time points, 1.4 mL of 0.05% TFA in acetonitrile was added to each, diluted to 2.0 mL with acetonitrile, filtered, and analyzed by HPLC. The experimental results showed that under these conditions, compound **5** had SN38 release rates of 25% and 65% at 6 h and 24 h, respectively; **9** had SN38 release rates of 45% and 95% at 6 h and 24 h, respectively; and compounds **15** and **16** had release rates <1%.

### DTT Release Study:

At room temperature, 2 mg each of phospholipid conjugate Examples **10, 15,** and **16** were accurately weighed and dissolved in 10 mM phosphate buffer pH 7.0 (0.5 mL each). 0.5 mL of a 20 mM DTT solution was added to each (final DTT concentration 10 mM). The mixtures were shaken in a 37 °C water bath for 6 h, then 1.4 mL of 0.05% TFA in acetonitrile was added, diluted to 2.0 mL with acetonitrile, filtered, and analyzed by HPLC. The experimental results showed that under these conditions, compound **10** had an SN38 release rate of 100%, while compounds **15** and **16** had release rates <1%.

As can be seen from the results in Table 1, the example compounds containing stimuli-responsive linkers, **1, 5,** and **7,** released the drug SN38 in PBS at pH=5.0 and 37 °C, whereas the compounds without stimuli-responsive linkers, **15** and **16,** hardly released any drug under these conditions. Simultaneously, the protease-responsive compound **9** and the reduction-responsive compound **10,** both released the drug SN38 under their respective responsive release conditions, while the compounds without responsive linkers, **15** and **16,** similarly did not release the drug.

### Evaluation Example 2: Particle Size Test of Example 1

Sample Preparation: Approximately 10 mg of the compound from Example 1 was weighed, dissolved in 1 mL of the above-mentioned distilled water, and filtered through a 0.22 µm aqueous phase filter membrane for use.

Sample Detection: The above sample solution was slowly transferred to a cuvette using a dropper for sample detection. The detection instrument was a Zeta sizer Pro Blue nano particle size and zeta potential analyzer.

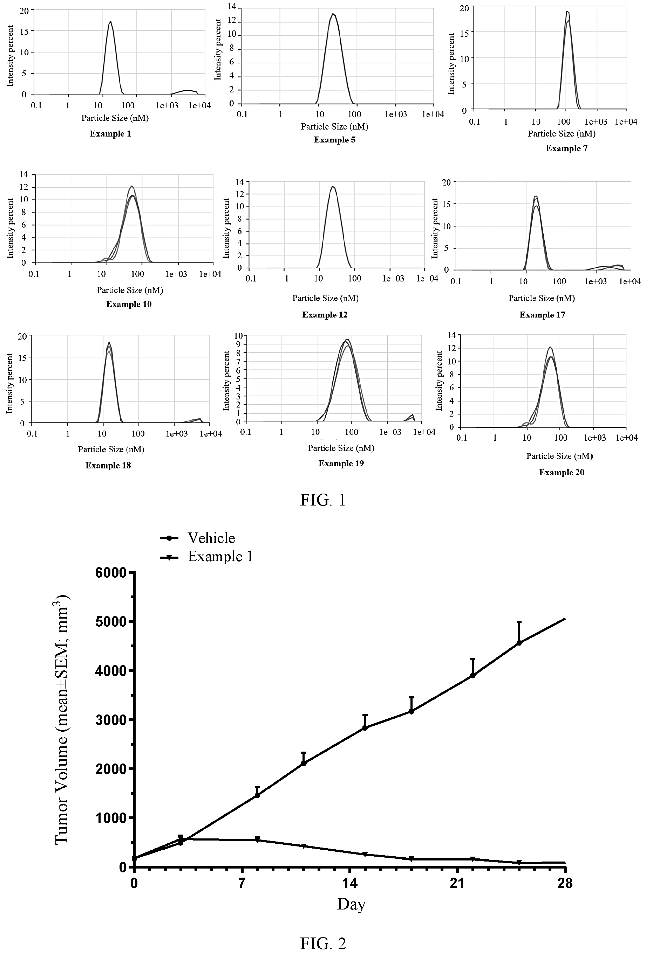

Detection Results: The nanoparticle size data for the compounds of the present invention are shown in FIG. 1 and Table 2.

**Table 2 Nanoparticle Size Data of the Compounds of the Present Invention**

| Example | Average Particle Size (nM) | Polydispersity Index (PDI) |
|---|---|---|
| Example **1** | 18.03 | 0.22 |
| Example **5** | 27.30 | 0.20 |
| Example **7** | 80.06 | 0.26 |
| Example **10** | 28.22 | 0.26 |
| Example **12** | 42.47 | 0.26 |
| Example **13** | 20.86 | 0.24 |
| Example **17** | 18.99 | 0.22 |
| Example **18** | 15.38 | 0.20 |
| Example **19** | 60.92 | 0.27 |
| Example **20** | 42.38 | 0.22 |
| Example **21** | 42.38 | 0.22 |
| Example **22** | 25.62 | 0.23 |
| Example **23** | 18.65 | 0.18 |
| Example **25** | 25.23 | 0.20 |
| Example **34** | 16.21 | 0.15 |
| Example **37** | 18.28 | 0.14 |
| Example **38** | 26.32 | 0.18 |
| Example **40** | 15.62 | 0.11 |
| Example **42** | 21.35 | 0.17 |
| Example **45** | 38.21 | 0.21 |
| Example **46** | 46.22 | 0.23 |

The results in Table 2 show that the average particle sizes of the compounds of the present invention are all within the range of 10-200 nM, with the nano-size varying depending on the different drugs and linkage methods.

### Evaluation Example 3: Pharmacodynamic Study of Example 1 in a Human Hepatocellular Carcinoma HepG2 Xenograft Mouse Model

### Experimental Animals and Inoculation Method:

BALB/c nude mice, female, 4-5 weeks old, weighing 20-25 g, were acclimated in the experimental environment for one week after arrival. HepG2 tumor fragments were subcutaneously inoculated into the right back of the nude mice. When the tumor volume reached ~170 mm³, the mice were grouped for administration, with 6 mice per group.

### Preparation of Test Samples:

An appropriate amount of the compound was weighed, a certain volume of physiological saline was added, and the mixture was shaken to dissolve, then filtered through a 0.2 µM filter membrane for use.

### Administration of Test Drugs:

The administration doses and regimens are shown in Table 2. The tumor volume under the skin of the nude mice was measured 2-3 times per week (the tumor volume was calculated using the formula: V = 0.5a × b², where a and b represent the long and short diameters of the tumor, respectively). The mouse body weight was measured, and data were recorded.

**Table 3**

| No. | Group | Dose (mg/kg) | Administration Route | Number of Animals | Administration Cycle |
|---|---|---|---|---|---|
| 1 | Blank Control Group | - | i.v | 6 | qw × 3 |
| 2 | Example **1** | 10 | i.v | 6 | qw × 3 |

| | | | | | |
|---|---|---|---|---|---|
| Note: The administration volume was 10 mg/mL. | | | | | |

### Analysis and Evaluation:

Experimental evaluation index: Evaluated using the tumor growth inhibition rate TGI (%) or the relative tumor proliferation rate T/C (%), where T is the experimental group and C is the control group.

Calculation of the relative tumor proliferation rate T/C (%): If T > T₀, T/C (%) = (T - T₀) / (C - C₀) × 100%; if T < T₀, T/C (%) = (T - T₀) / T₀ × 100%, where T and C are the tumor volumes at the end of the experiment; T₀ and C₀ are the tumor volumes at the beginning of the experiment.

Calculation of the tumor growth inhibition rate TGI (%): TGI (%) = (1 - T/C) × 100%.

Evaluation criteria: T/C (%) > 40 (i.e., TGI (%) < 60%) is considered ineffective; T/C (%) ≤ 40 (i.e., TGI (%) ≥ 60%) is considered effective, and statistical significance .of P < 0.05 is required for effectiveness.

### Pharmacodynamic Results:

The tumor inhibitory effect of Example **1** on the human hepatocellular carcinoma HepG2 tumor model is shown in FIG. 2.

The results indicate that the compound of Example **1** of the present invention has a very strong inhibitory effect on tumor growth in the HepG2 cell nude mouse model, with a tumor inhibition rate of 99%, and the tumors almost completely regressed.

Evaluation Example 4: Pharmacodynamic Study of Examples 5, 7, 15, and 16 in a Human Hepatocellular Carcinoma HepG2 Xenograft Mouse Model

### Experimental Animals and Inoculation Method:

BALB/c nude mice, female, 4-5 weeks old, weighing 20-25 g, were acclimated in the experimental environment for one week after arrival. HepG2 tumor fragments were subcutaneously inoculated into the right back of the nude mice. When the tumor volume reached ~170 mm³, the mice were grouped for administration, with 6 mice per group.

### Preparation of Test Samples:

An appropriate amount of the compound was weighed, a certain volume of physiological saline was added, and the mixture was shaken to dissolve, then filtered through a 0.2 µM filter membrane for use.

### Administration of Test Drugs:

The administration doses and regimens are shown in Table 3. The tumor volume under the skin of the nude mice was measured 2-3 times per week (the tumor volume was calculated using the formula: V = 0.5a × b², where a and b represent the long and short diameters of the tumor, respectively). The mouse body weight was measured, and data were recorded.

**Table 4**

| No. | Group | Dose (mg/kg) | Administration Route | Number of Animals | Administration Cycle |
|---|---|---|---|---|---|
| 1 | Vehicle | - | i.v | 6 | qw × 3 |
| 2 | Example **5** | 10 | i.v | 6 | qw × 3 |
| 3 | Example **7** | 10 | i.v | 6 | qw × 3 |
| 4 | Comparative Example **15** | 10 | i.v | 6 | qw × 3 |
| 5 | Comparative Example **16** | 10 | i.v | 6 | qw × 3 |

| | | | | | |
|---|---|---|---|---|---|
| Note: The administration volume was 10 mg/mL. | | | | | |

### Analysis and Evaluation:

The analysis and evaluation indicators, calculation of the relative tumor proliferation rate T/C (%), calculation of the tumor growth inhibition rate TGI (%), and evaluation criteria are the same as in Example 3.

### Pharmacodynamic Results:

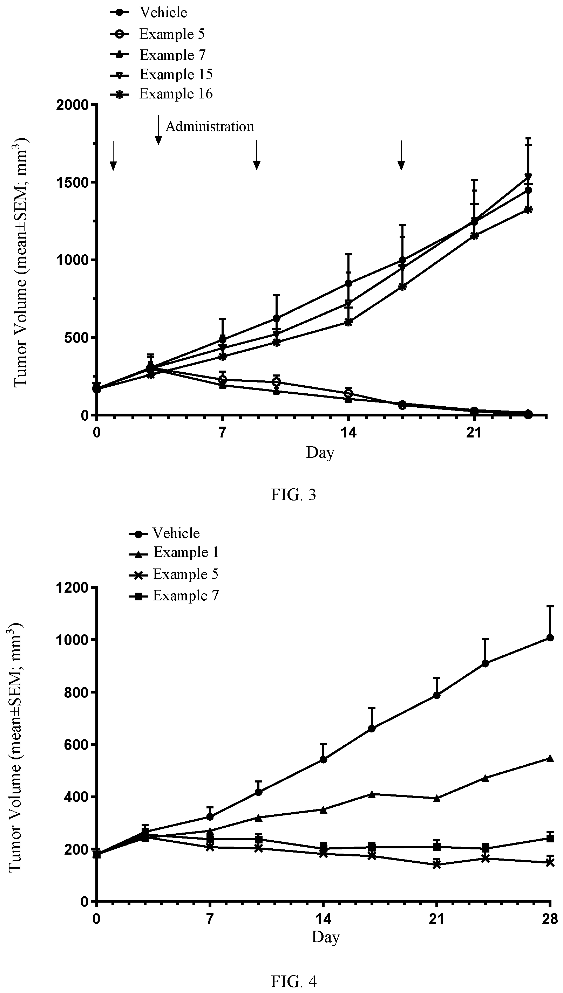

The tumor inhibitory effects of Examples **5, 7, 15,** and **16** on the human hepatocellular carcinoma HepG2 tumor model are shown in FIG. 3.

The results indicate that the example compounds **5** and **7,** which contain environmentally sensitive releasable linkers, have a very strong inhibitory effect on tumor growth in the HepG2 cell nude mouse model. Among them, Examples **5** and **7** caused complete tumor regression, with tumor inhibition rates reaching 99%. In contrast, the compounds **15** and **16** without sensitive linkers showed very poor tumor inhibition effects (tumor inhibition rates of 0% and 6%, respectively). This demonstrates that the presence of a sensitive linker has a significant impact on the pharmacodynamic outcome of the compounds.

Evaluation Example 5: Pharmacodynamic Study of Examples 1, 5, and 7 in a Human Gastric Cancer Cell NCI-N87 Xenograft Mouse Model

### Experimental Animals and Inoculation Method:

BALB/c nude mice, female, 4-5 weeks old, weighing 20-25 g, were acclimated in the experimental environment for one week after arrival. NCI-N87 cancer cells were subcutaneously inoculated into the right back of the nude mice. When the tumor volume reached ~180 mm³, the mice were grouped for administration, with 6 mice per group.

### Preparation of Test Samples:

An appropriate amount of the compound was weighed, a certain volume of physiological saline was added, and the mixture was shaken to dissolve, then filtered through a 0.2 µM filter membrane for use.

### Administration of Test Drugs:

The administration doses and regimens are shown in Table 2. The tumor volume under the skin of the nude mice was measured 2-3 times per week (the tumor volume was calculated using the formula: V = 0.5a × b², where a and b represent the long and short diameters of the tumor, respectively). The mouse body weight was measured, and data were recorded.

**Table 5**

| No. | Group | Dose (mg/kg) | Administrati on Route | Number of Animals | Administration Cycle |
|---|---|---|---|---|---|
| 1 | Vehicle | - | i.v | 6 | qw × 3 |
| 2 | Example **1** | 10 | i.v | 6 | qw × 3 |
| 3 | Example **5** | 10 | i.v | 6 | qw × 3 |
| 4 | Example **7** | 10 | i.v | 6 | qw × 3 |

| | | | | | |
|---|---|---|---|---|---|
| Note: The administration volume was 10 mg/mL. | | | | | |

### Analysis and Evaluation:

The analysis and evaluation indicators, calculation of the relative tumor proliferation rate T/C (%), calculation of the tumor growth inhibition rate TGI (%), and evaluation criteria are the same as in Example 3.

### Pharmacodynamic Results:

The tumor inhibitory effects of Examples **1, 5,** and **7** on the human gastric cancer NCI-N87 tumor model are shown in FIG. 4.

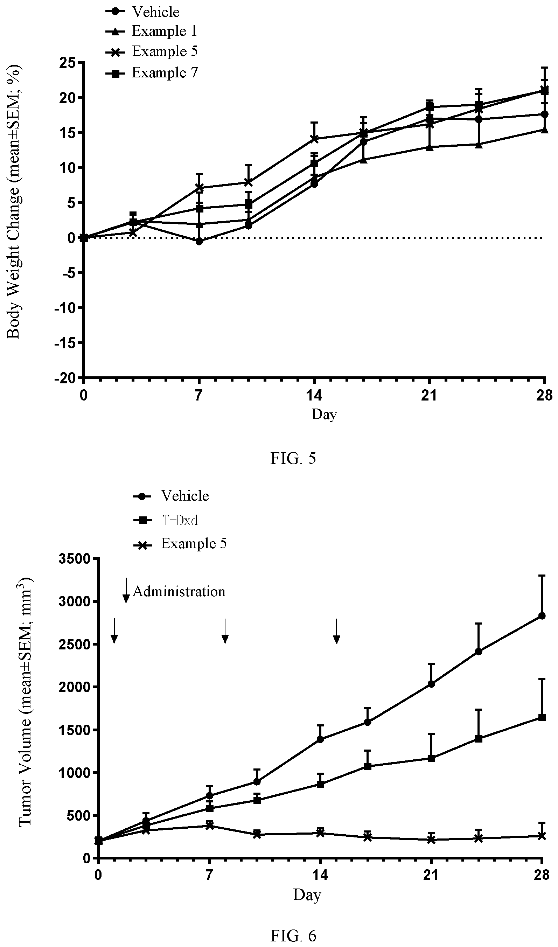

The changes in animal body weight during the pharmacodynamic evaluation for Examples 1, 5, and 7 are shown in FIG. 5.

The results indicate that the compounds of Examples **1, 5,** and **7** of the present invention have a very strong inhibitory effect on the human gastric cancer NCI-N87 tumor model. Among them, the 10 mg/kg dose groups of Examples **5** and **7** showed tumor volume inhibition rates of 86% and 77%, respectively. At the end of the experiment, the body weight of animals in all administration groups increased, showing no significant difference compared to the control group.

Evaluation Example 6: Pharmacodynamic Study of Example 5 and T-Dxd in a Human Colon Cancer Cell HT-29 Xenograft Mouse Model

### Experimental Animals and Inoculation Method:

BALB/c nude mice, female, 4-5 weeks old, weighing 20-25 g, were acclimated in the experimental environment for one week after arrival. HT-29 cancer cells were subcutaneously inoculated into the right back of the nude mice. When the tumor volume reached ~180 mm³, the mice were grouped for administration, with 6 mice per group.

### Preparation of Test Samples:

An appropriate amount of the compound of this example was weighed, a certain volume of physiological saline was added, and the mixture was shaken to dissolve, then filtered through a 0.2 µM filter membrane for use.

### Administration of Test Drugs:

The administration doses and regimens are shown in Table 2. The tumor volume under the skin of the nude mice was measured 2-3 times per week (the tumor volume was calculated using the formula: V = 0.5a × b², where a and b represent the long and short diameters of the tumor, respectively). The mouse body weight was measured, and data were recorded.

**Table 6**

| No. | Group | Dose (mg/kg) | Administrati on Route | Number of Animals | Administration Cycle |
|---|---|---|---|---|---|
| 1 | Vehicle | - | i.v | 6 | qw × 3 |
| 2 | Example **5** | 10 | i.v | 6 | qw × 3 |
| 3 | **T-Dxd** | 10 | i.v | 6 | qw × 3 |

| | | | | | |
|---|---|---|---|---|---|
| Note: The administration volume was 10 mg/mL. | | | | | |

### Analysis and Evaluation:

The analysis and evaluation indicators, calculation of the relative tumor proliferation rate T/C (%), calculation of the tumor growth inhibition rate TGI (%), and evaluation criteria are the same as in Example 3.

### Pharmacodynamic Results:

The tumor inhibitory effects of Example **5** and **T-Dxd** on the human colon cancer cell HT-29 tumor model are shown in FIG. 6.

The results indicate that the compound of Example **5** of the present invention has a significantly superior tumor inhibitory effect on human colon cancer cell HT-29 tumors compared to the ADC drug T-Dxd, with tumor inhibition rates of 77% and 49% for the 10 mg/kg dose groups, respectively.

### Evaluation Example 7: Pharmacodynamic Study of Example 5 in a Human Fibrosarcoma Cell HT-1080 Xenograft Mouse Model

### Experimental Animals and Inoculation Method:

BALB/c nude mice, female, 4-5 weeks old, weighing 20-25 g, were acclimated in the experimental environment for one week after arrival. HT-1080 cancer cells were subcutaneously inoculated into the right back of the nude mice. When the tumor volume reached ~400 mm³, the mice were grouped for administration, with 6 mice per group.

### Preparation of Test Samples:

An appropriate amount of the compound of this example was weighed, a certain volume of physiological saline was added, and the mixture was shaken to dissolve, then filtered through a 0.2 µM filter membrane for use.

### Administration of Test Drugs:

The administration doses and regimens are shown in Table 2. The tumor volume under the skin of the nude mice was measured 2-3 times per week (the tumor volume was calculated using the formula: V = 0.5a × b², where a and b represent the long and short diameters of the tumor, respectively). The mouse body weight was measured, and data were recorded.

**Table 7**

| No. | Group | Dose (mg/kg) | Administrati on Route | Number of Animals | Administration Cycle |
|---|---|---|---|---|---|
| 1 | Vehicle | - | i.v | 6 | qw × 3 |
| 2 | Example **5** | 10 | i.v | 6 | qw × 3 |

| | | | | | |
|---|---|---|---|---|---|
| Note: The administration volume was 10 mg/mL. | | | | | |

### Analysis and Evaluation:

The analysis and evaluation indicators, calculation of the relative tumor proliferation rate T/C (%), calculation of the tumor growth inhibition rate TGI (%), and evaluation criteria are the same as in Example 3.

### Pharmacodynamic Results:

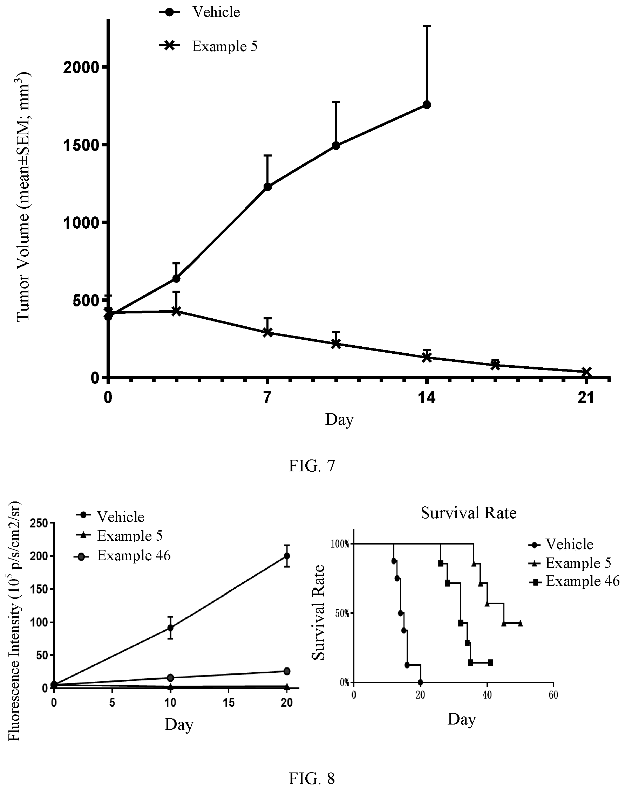

The tumor inhibitory effect of Example **5** on the human fibrosarcoma cell HT-1080 tumor model is shown in FIG. 7.

The results indicate that the compound of Example **5** of the present invention has a significant tumor inhibitory effect on human fibrosarcoma cell HT-1080 tumors.

### Evaluation Example 8: Pharmacodynamic Study of Examples 5 and 46 in a U87-MG Glioblastoma Orthotopic Mouse Model

### Experimental Animals and Inoculation Method:

BALB/c nude mice, female, 6-8 weeks old, weighing 18-20 g, were acclimated in the experimental environment for one week after arrival. The cultured U87MG-Luc2 cell suspension at a concentration of 2.5×10⁷ cells/mL, totaling 2 mL, was pipetted until uniform and placed on ice. After removing the body hair at the inoculation site, the mouse inoculation site was disinfected with an iodophor cotton ball. The animal was fixed on a mouse brain stereotaxic instrument, and 2 µL of the cell suspension (5×10⁴ cells/point) was inoculated into the mouse brain using a microsyringe. On day 8 post-inoculation, body weight was measured, and in vivo animal imaging was performed. The U87MG-Luc2 model animals were randomly grouped based on body weight and fluorescence imaging data, with 8 animals per group. The grouping day was considered experimental Day 0, and administration was performed after grouping.

### Preparation of Test Samples:

An appropriate amount of the compound of the present invention was weighed, a certain volume of physiological saline was added, and the mixture was shaken to dissolve, then filtered through a 0.2 µM filter membrane for use.

### Administration of Test Drugs:

The administration doses and regimens are shown in Table 8. The tumor volume under the skin of the nude mice was measured 2-3 times per week (the tumor volume was calculated using the formula: V = 0.5a × b², where a and b represent the long and short diameters of the tumor, respectively). The mouse body weight was measured, and data were recorded.

**Table 8**

| No. | Group | Dose (mg/kg) | Administrati on Route | Number of Animals | Administration Cycle |
|---|---|---|---|---|---|
| 1 | Vehicle | - | i.v | 6 | qw × 3 |
| 2 | Example **5** | 10 | i.v | 6 | qw × 3 |
| 3 | Example **46** | 30 | i.v | 6 | qw × 3 |

| | | | | | |
|---|---|---|---|---|---|
| Note: The administration volume was 10 mg/mL. | | | | | |

### Analysis and Evaluation:

Detection indicators: Starting from administration, mouse body weight was measured twice a week, and in vivo imaging and quantitative analysis (TGI%) of the fluorescent cell line model were performed twice a week. Before in vivo imaging, D-Luciferin was administered intraperitoneally to the experimental animals at a concentration of 15 mg/mL and a volume of 0.2 mL. In vivo imaging was performed 10 minutes after D-Luciferin injection.

Animals were checked daily, and the time of death was recorded. Once an animal showed deteriorating health, was moribund (significant weight loss, weight decrease >20%), could not eat or drink normally, or had difficulty moving or was paralyzed, it was considered dead and immediately euthanized with CO₂. The mean survival time (days, MST) for each group of animals was calculated.

### Pharmacodynamic Results:

The tumor inhibitory effects and survival curves of Example **5** and Example **46** on the U87-MG glioblastoma orthotopic mouse model are shown in FIG. 8.

The results indicate that the compounds of Example **5** and **46** of the present invention exhibit very strong tumor inhibitory effects in the U87-MG glioblastoma orthotopic mouse model, showing significant differences compared to the blank control group. The survival times were increased by 15 days and 30 days, respectively. This demonstrates that the conjugate drugs of Example **5** and **46** of the present invention can cross the blood-brain barrier, accumulate in brain tumor tissue, and have potential application value in the field of brain tumor therapy.

Although specific embodiments of the present invention have been described above, those skilled in the art should understand that these are merely illustrative. Various changes or modifications can be made to these embodiments without departing from the principle and essence of the present invention. Therefore, the scope of protection of the present invention is defined by the appended claims.

## Claims

1. A phospholipid drug conjugate represented by formula (I), 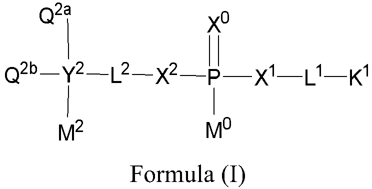
or a stereoisomer, a prodrug, a pharmaceutically acceptable salt, or a pharmaceutically acceptable solvate of said phospholipid drug conjugate,
wherein:
X⁰ is O or S;
X¹ is O, S, or NR^{1a};
X² is O, S, or NR^{2a};
L¹ and L² are absent or are linking units;
M⁰ is OR^{0A}, SR^{0b}, NR^{0c}R^{0d}, or T⁰-(L-D)ₙ;
K¹ is P¹ or 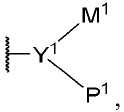 Y¹ is a branching center, P¹ is a pharmacokinetic modulating moiety unit, M¹ is T¹-(L-D)ₙ;
Y² is a branching center;
M² is absent or is T²-(L-D)ₙ;
at least one of M⁰, M¹, and M² contains an L-D fragment;
Q^{2a} is T^{2a}-R¹ or is absent, Q^{2b} is T^{2b}-R² or is absent, and at least one of Q^{2a} and Q^{2b} is present;
T⁰, T¹, and T² are each independently selected from absent or an extension moiety;
T^{2a} and T^{2b} are absent or are linking units;
L is L³-L^{s}, L³ is an environment-responsive linking unit selected from enzyme-responsive linkers, pH-responsive linkers, light-responsive linkers, and redox-responsive linkers; L^{s} is present or absent, and when present, L^{s} is a self-immolative linker portion;
D is a bioactive molecule fragment, and when the conjugate contains multiple D, D may be the same or different;
R^{1a} and R^{2a} are each independently selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or an amino protecting group;
R^{0a} is each independently selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or a hydroxyl protecting group;
R^{0b} is selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or a thiol protecting group;
R^{0c} and R^{0d} are each independently selected from hydrogen, C₁-C₁₀ alkyl, C₁-C₁₀ alkoxy, C₃-C₁₀ alkenyl, C₃-C₁₀ alkynyl, C₃-C₈ cycloalkyl, C₂-C₈ heterocycloalkyl, C₆-C₁₀ aryl, C₅-C₁₀ heteroaryl, or an amino protecting group;
R¹ and R² are each independently selected from saturated or unsaturated C₁-C₅₀ alkyl, cholesterol analogs, retinoids, and tocopherol analogs, wherein said C₁-C₅₀ alkyl, cholesterol analogs, retinoids, and tocopherol analogs are optionally substituted with one or more substituents selected from hydroxyl, halogen, C₁₋₁₀ alkyl, C₁₋₁₀ alkoxy, C₃₋₁₂ cycloalkyl, and 3-12 membered heterocycloalkyl, and said C₁₋₁₀ alkyl, C₁₋₁₀ alkoxy, C₃₋₁₂ cycloalkyl, and 3-12 membered heterocycloalkyl are optionally substituted with one or more substituents selected from hydroxyl, halogen, C₁₋₃ alkyl, C₁₋₃ alkoxy, and C₃₋₆ cycloalkyl;
n is an integer from 1 to 10, and when n> 1, the L-D may be the same or different;
in the compound represented by formula (I), one or more hydrogens bonded to carbon may be substituted by deuterium;
in the compound represented by formula (I), one or more hydrogens bonded to carbon may be substituted by any substituent;
phospholipid isomers include compounds with phosphorus as the chiral center, selected from: 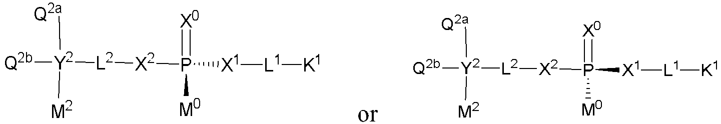 or chiral mixtures thereof.

2. The phospholipid drug conjugate according to claim 1, or a stereoisomer, prodrug, pharmaceutically acceptable salt, or pharmaceutically acceptable solvate thereof, wherein the phospholipid core 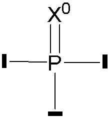 in formula (I) satisfies one or more of the following conditions:
(1) 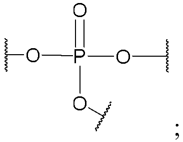
(2) 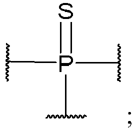
(3) 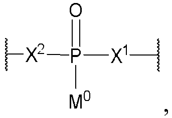 wherein at least one of the atoms connecting X², X¹, and M⁰ to P is not an oxygen atom.

3. The phospholipid drug conjugate according to any one of claims 1-2, or a stereoisomer, prodrug, pharmaceutically acceptable salt, or pharmaceutically acceptable solvate thereof, wherein said phospholipid drug conjugate has nano-size; preferably, said nano-size is an average nanoparticle size ranging from 1 to 500 nanometers; more preferably, the average particle size ranges from 5 to 100 nanometers.

4. The phospholipid drug conjugate according to any one of claims 1-3, or a stereoisomer, prodrug, pharmaceutically acceptable salt, or pharmaceutically acceptable solvate thereof, wherein it satisfies one or more of the following conditions:
(1) L¹ and L² are absent or present, and when present, L¹ and L² are each independently alkane, heterocycloalkane, aromatic hydrocarbon, heterocycloalkane, substituted or unsubstituted amino acids or polypeptides composed thereof, or various combinations thereof, wherein the functional group connecting portions at the two ends of L¹ and L² are each independently selected from: 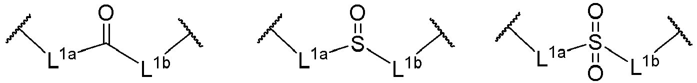 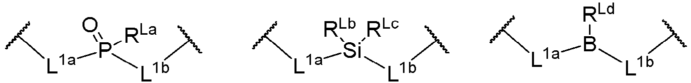 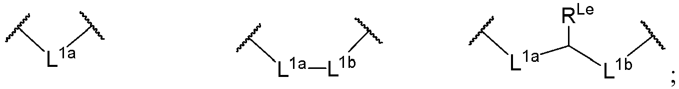
wherein, R^{La}, R^{Lb}, R^{Lc}, and R^{Ld} are each independently selected from hydroxyl, C1-C5 alkyl, or C1-C5 alkoxy;
R^{Le} is hydrogen, hydroxyl, C1-C5 alkyl, or C1-C5 alkoxy;
L^{1a} and L^{1b} are each independently selected from O, NR^{Lf}, S, S-S, 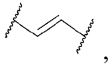 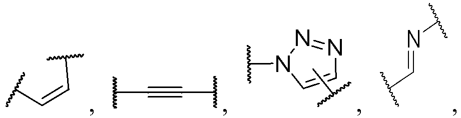 or directly bonded (absent), wherein R^{Lf} is hydrogen or C1-C5 alkyl;
(2) T⁰, T¹, and T² are each independently absent or present, and when present, T⁰, T¹, and T² are each independently alkane, heterocycloalkane, aromatic hydrocarbon, heterocycloalkane, substituted or unsubstituted amino acids or polypeptides composed thereof, or various combinations thereof, wherein the functional groups at the two ends of T⁰, T¹, and T² are each independently selected from: 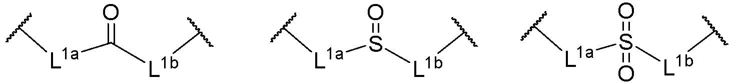 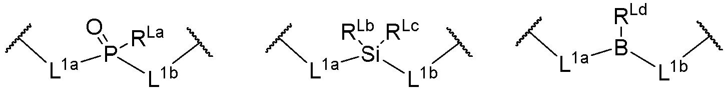 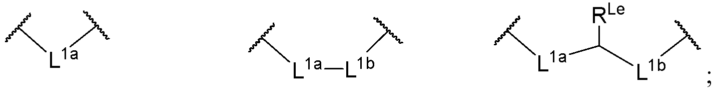
wherein, R^{La}, R^{Lb}, R^{Lc}, and R^{Ld} are each independently selected from hydroxyl, C1-C5 alkyl, or C1-C5 alkoxy;
R^{Le} is hydrogen, hydroxyl, C1-C5 alkyl, or C1-C5 alkoxy;
L^{1a} and L^{1b} are each independently selected from O, NR^{Lf}, S, S-S, 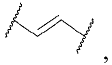 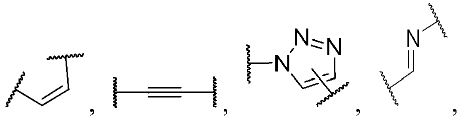 or directly bonded (absent), wherein R^{Lf} is hydrogen or C1-C5 alkyl;
(3) T^{2a} and T^{2b} are absent or present, and when present, T^{2a} and T^{2b} are each independently alkane, heterocycloalkane, aromatic hydrocarbon, heterocycloalkane, substituted or unsubstituted amino acids or polypeptides composed thereof, or various combinations thereof, wherein the functional groups at the two ends of T^{2a} and T^{2b} are each independently selected from: 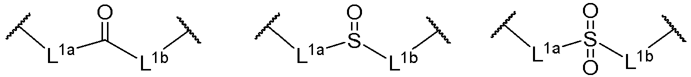 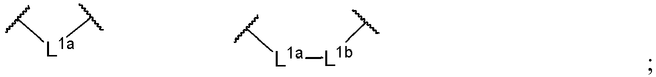 wherein, L^{1a} and L^{1b} are each independently selected from O, NR^{Lf}, S, S-S, 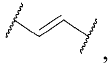 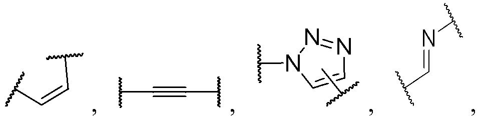 or directly bonded (absent), and R^{Lf} is hydrogen or C1-C5 alkyl;
(4) L³ is selected from an enzyme-responsive linker, a pH-responsive linker, a photo-responsive linker, and a redox-responsive linker, preferably selected from an enzyme-responsive linker, a pH-responsive linker, and a redox-responsive linker, more preferably an enzyme-responsive linker;
(5) Y¹ and Y² are at least trifunctional branching centers, selected from trifunctional amino acids, polypeptides, or the following structures: 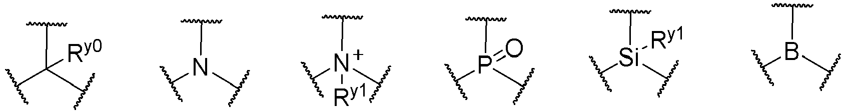 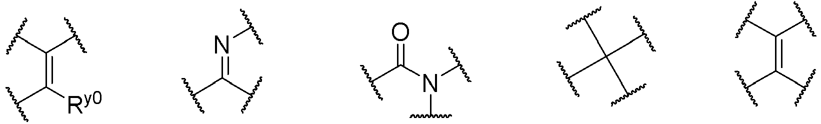 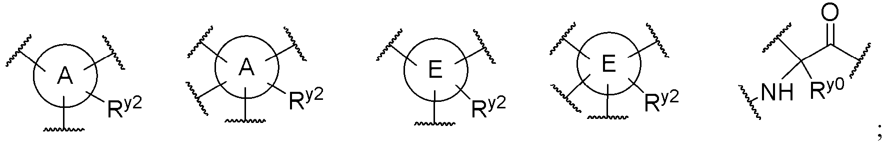
wherein, R^{y0} is selected from hydrogen, deuterium, halogen, nitro, cyano, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or a group containing primary amine, secondary amine, tertiary amine, hydroxyl, thiol, carboxyl, ester, amide, boronic acid, boronic ester, phosphoric acid, sulfonic acid, sulfoxide, aldehyde, or ketone functional groups;
R^{y1} is selected from C1-C10 alkyl, C1-C10 alkoxy, C3-C8 cycloalkyl, and C2-C8 heterocycloalkyl;
R^{y2} is selected from hydrogen, deuterium, halogen, hydroxyl, amino, nitro, cyano, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or a group containing primary amine, secondary amine, tertiary amine, hydroxyl, thiol, carboxyl, ester, amide, boronic acid, boronic ester, phosphoric acid, sulfonic acid, sulfoxide, aldehyde, ketone functional groups;
ring A is C₆-C₂₀ aryl or C₅-C₂₀ heteroaryl; the heteroatom(s) in said C₅-C₂₀ heteroaryl are selected from O, S, or N, the number of heteroatoms is one or more, and when multiple heteroatoms are present, they may be the same or different;
ring E is C2-C8 heterocycloalkyl; the heteroatom(s) in said heterocycloalkyl are selected from O, S, or N, the number of heteroatoms is one or more, and when multiple heteroatoms are present, they may be the same or different;
(6) P¹ is a pharmacokinetic modulating moiety unit, selected from polyethylene glycol, sugars, oligosaccharides, and polysaccharides (such as hyaluronic acid, chondroitin sulfate, chitosan, heparin, tea polysaccharide, glucan, polysialic acid, sodium alginate, cellulose, polysucrose, cyclodextrin), polysarcosine, polyphosphoester, polyglycerol, polyvinylpyrrolidone, polyoxazoline, polyamino acids, polyacrylic acid residues, polymethacrylic acid residues, quaternary ammonium salt derivatives, carboxyl-containing derivatives, sulfonic acid-containing derivatives, phosphoric acid-containing derivatives, zwitterionic derivatives, or zwitterionic polymers.

5. The phospholipid drug conjugate according to any one of claims 1-4, or a stereoisomer, prodrug, pharmaceutically acceptable salt, or pharmaceutically acceptable solvate thereof, wherein it satisfies one or more of the following conditions:
(1) when said L³ is an enzyme-responsive linker, it can be cleaved by one or more of the following enzymes: secretory phospholipase A2, acid phosphatase, serum alkaline phosphatase, cytochrome P450, sulfatase, prostate-specific antigen, phospholipase A1, phospholipase A2, phospholipase B, phospholipase C, phospholipase D, neutrophil elastase, caspase-3, cathepsin, matrix metalloproteinase, β-glucuronidase, β-galactosidase, nitroreductase, NADPH, aminopeptidase N, carboxylesterase, quinone reductase, histone deacetylase, legumain, urokinase-type plasminogen activator, urokinase-type plasminogen activator receptor, collagenase; preferably cleaved by one or more of the following enzymes: caspase-3, cathepsin, matrix metalloproteinase, elastase, or β-glucuronidase;
(2) when said L³ is a pH-responsive linker, it comprises one or more of the following structures: hydrazone, imine, oxime, carboxylic ester, thioester, sulfate, sulfonate, orthoester, carbonate, carbamate, substituted carbamate, ketal, acetal, silyl ether, phosphate, boronic ester, phosphoramidate, or cis-aconitic group;
(3) when said L³ is a photo-responsive linker, it comprises one or more of the following structures: o-nitrobenzyl, coumarin, benzoin, BODIPY, or cyanine group;
(4) when said L³ is a redox-responsive linker, it comprises one or more of the following structures: thioketal, phenylboronic ester, phenylboronic acid, oxalate, vinyl ether, thioether, aminoacrylate, disulfide, diselenide, 2,4-dinitrobenzenesulfonate, 2-azidomethylbenzoate, 4-azidobenzyl, unsaturated acid ester, or azobenzene group.

6. The phospholipid drug conjugate according to any one of claims 1-5, or a stereoisomer, prodrug, pharmaceutically acceptable salt, or pharmaceutically acceptable solvate thereof, wherein it satisfies one or more of the following conditions:
(1) when L³ is an enzyme-responsive linker, it is selected from short peptides composed of 2-10 amino acid residues Val, D-Val, Cit, Phe, Lys, Leu, Gly, Ala, Asn, Asp, Arg, Pro, Tyr, Ile, His, Ser, Gln, Glu, Met, preferably short peptides composed of 2-10 amino acids selected from Val, Cit, Phe, Lys, Ala, Leu, Gly, Asn, Asp; preferably, L³ is selected from the following structures: 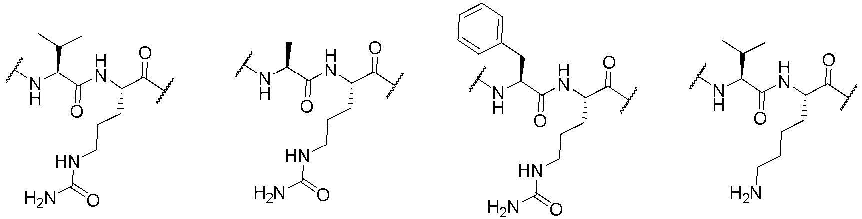 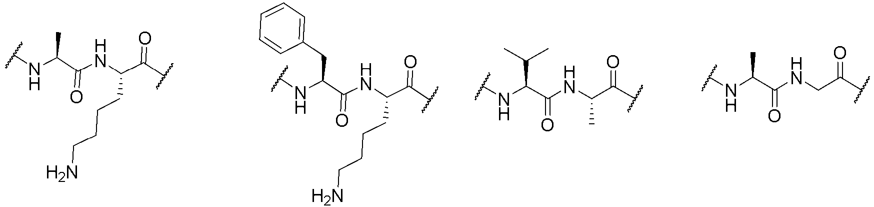 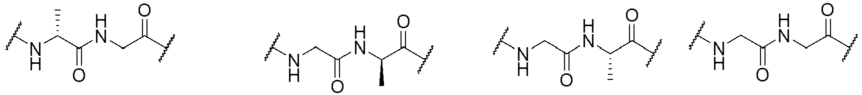 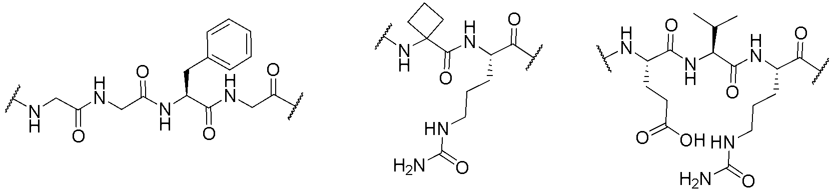 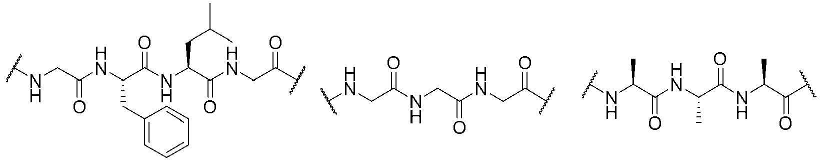 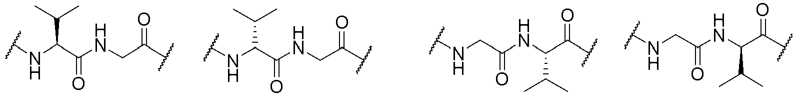 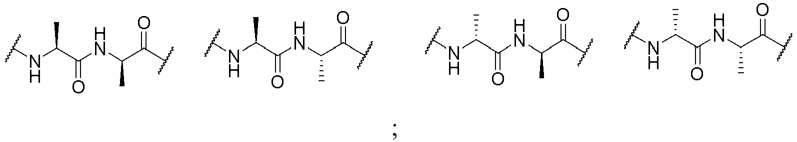
(2) when L^{s} is present, it is selected from the following structures: 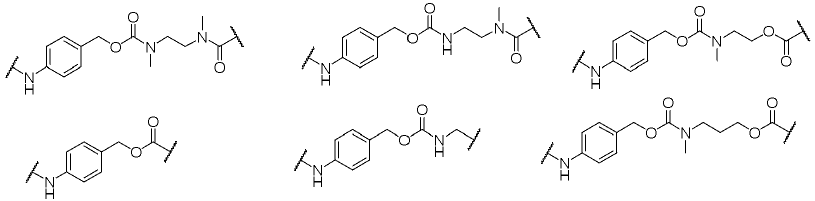 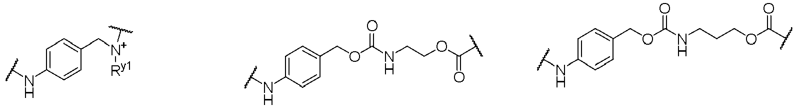 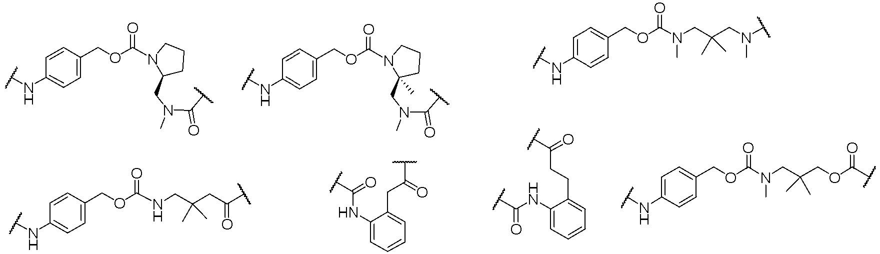 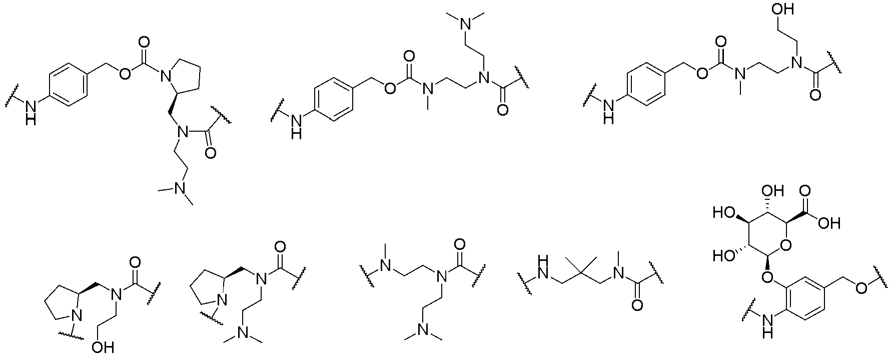 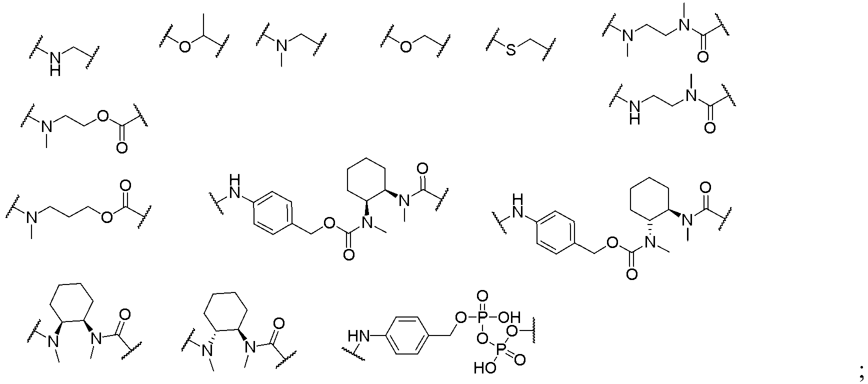 preferably, L^{s} is selected from the following structures: 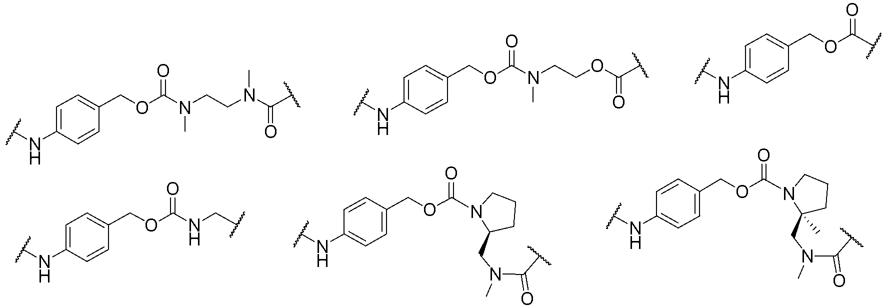 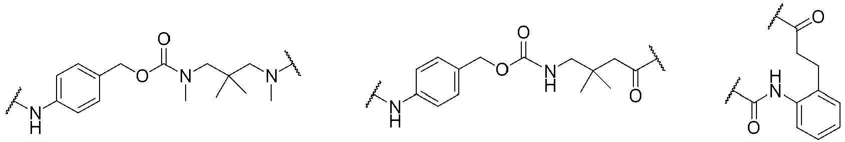 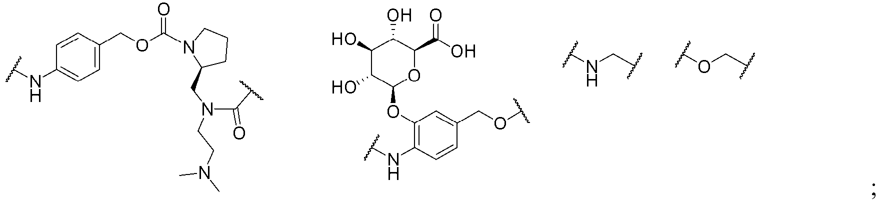
(3) when L³ is a pH-responsive linker, it is selected from the following structures: 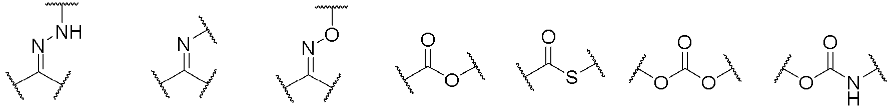 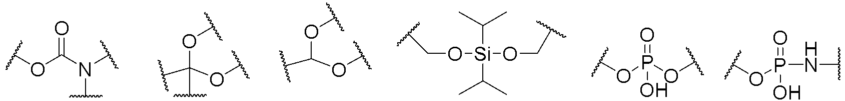 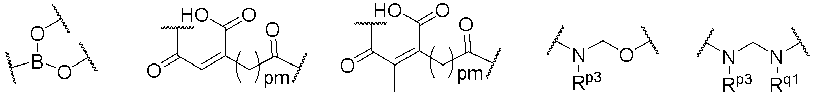 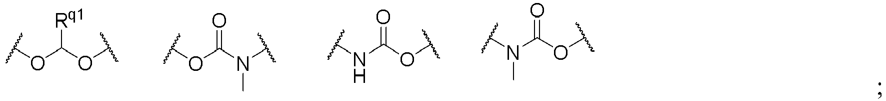
wherein, pm = 0-4; R^{p3} and R^{q1} are selected from H, substituted or unsubstituted C1-C10 alkyl;
preferably, L³ is selected from the following structures: 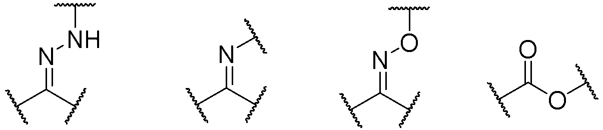 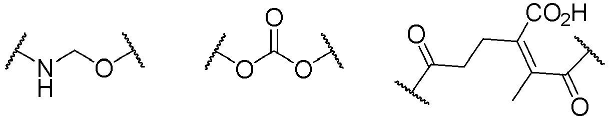 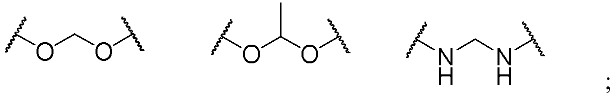
(4) when said L³ is a photo-responsive linker, it is selected from the following structures: 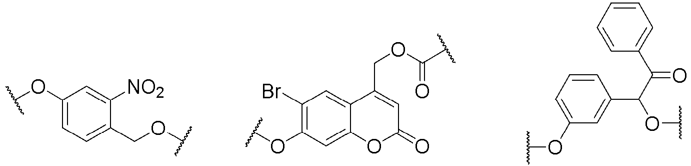 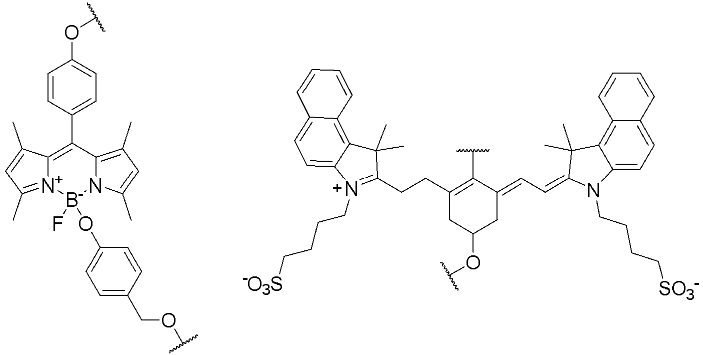 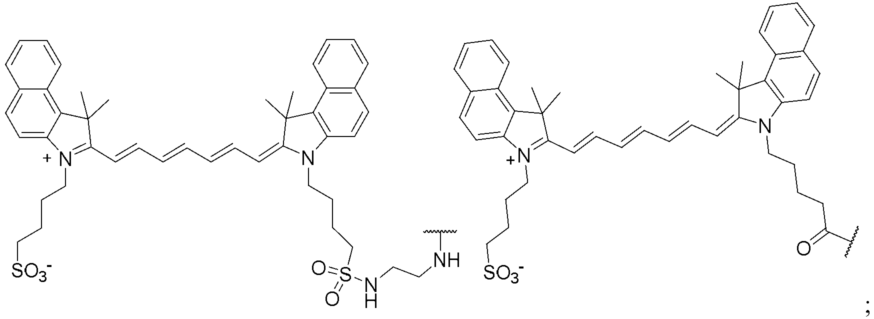
(5) when L³ is a redox-responsive linker, it comprises the following structure:

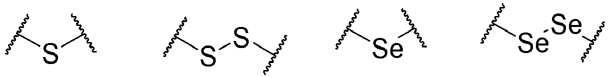

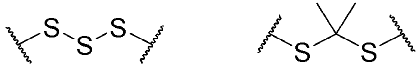

7. The phospholipid drug conjugate according to any one of claims 1-4, or a stereoisomer, prodrug, pharmaceutically acceptable salt, or pharmaceutically acceptable solvate thereof, wherein P1 is selected from:
(1) polyethylene glycol derivative residues are selected from the following structures: 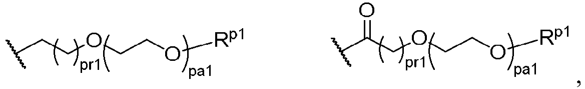
wherein, pa1 is an integer from 5 to 250, pr1 is an integer from 0 to 8, and R^{p1} is hydrogen, C1-C10 alkyl, C1-C10 heteroalkyl, C3-C10 cycloalkyl, C3-C10 alkenyl, C3-C10 alkynyl, or a hydroxyl protecting group;
preferably, pa1 is an integer from 5 to 150, pr1 is an integer from 0 to 8, and R^{p1} is hydrogen, C1-C10 alkyl, or a hydroxyl protecting group;
more preferably, pa1 is an integer from 10 to 60, pr1 is an integer from 1 to 2, and R^{p1} is C1-C10 alkyl;
(2) polysarcosine derivative residues are selected from the following structures: 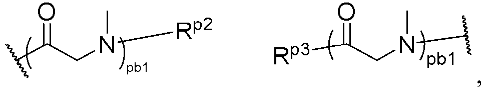
wherein, pb1 is an integer from 5 to 250, R^{p2} is selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or an amino protecting group; R^{p3} is selected from OR^{p3a} or NR^{p3b}R^{p3c}, wherein R^{p3a} is selected from hydrogen, C1-C10 alkyl, C3-C10 alkenyl, C3-C10 alkynyl, C3-C8 cycloalkyl, C2-C8 heterocycloalkyl, C6-C10 aryl, or C5-C10 heteroaryl; R^{p3b} and R^{p3c} are each independently selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, an amino protecting group, or R^{p3b} and R^{p3c} together with the nitrogen atom to which they are attached form a C3-C10 cyclic structure;
preferably, pb1 is an integer from 5 to 150;
(3) quaternary ammonium salt derivative residues are selected from the following structures: 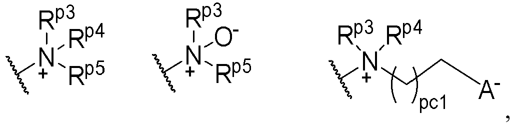 wherein, pc1 is an integer from 0 to 10, A⁻ is selected from 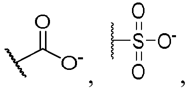 or 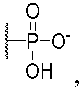 and R^{p3}, R^{p4}, and R^{p5} are each independently selected from C1-C10 alkyl, C1-C10 alkoxy, C3-C8 cycloalkyl, or C2-C8 heterocycloalkyl;
(4) zwitterionic derivatives or zwitterionic polymers: 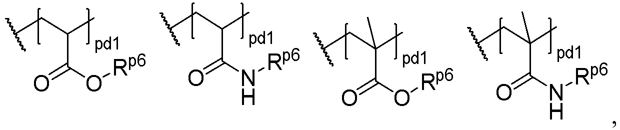 wherein,
pd1 is an integer from 5 to 250;
R^{p6} is selected from: 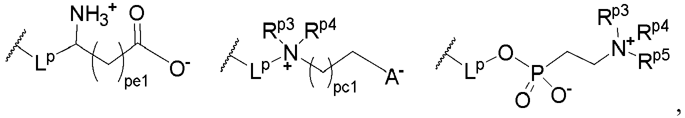
pc1 and pe1 are integers from 0 to 10, A⁻ is selected from 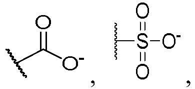 or 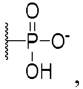 R^{p3}, R^{p4}, and R^{p5} are each independently selected from C1-C10 alkyl, C1-C10 alkoxy, C3-C8 cycloalkyl, or C2-C8 heterocycloalkyl; L^{p} is absent or a linking unit, selected from alkane, heterocycloalkane, aromatic hydrocarbon, heterocycloalkane, or various combinations thereof;
(5) polysaccharide polymers: 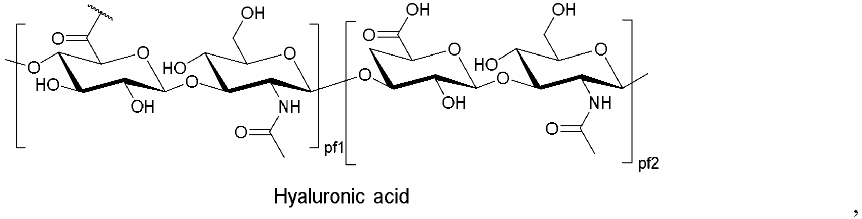 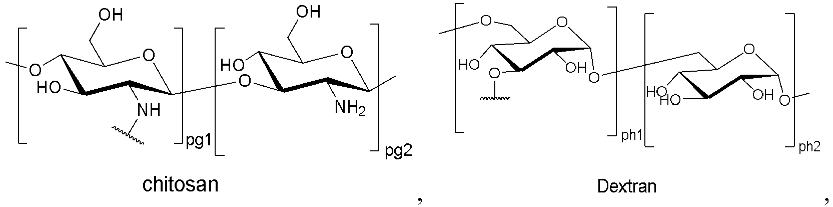 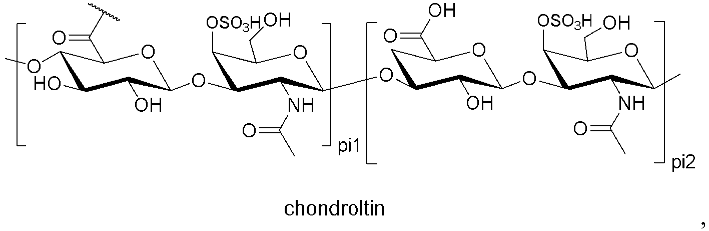 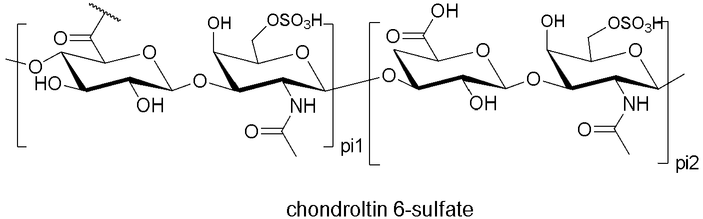 and 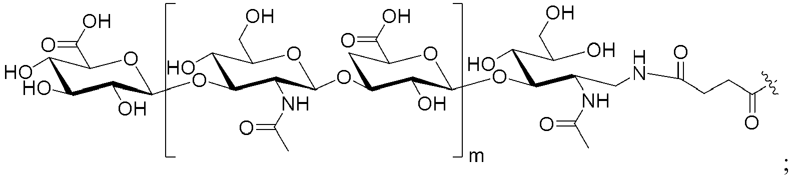 wherein, pf1, pf2, pg1, pg2, ph1, ph2, pi1, pi2, and m are integers from 1 to 250, preferably integers from 1 to 50, for example integers from 1 to 5, integers from 4 to 24, or integers from 5 to 25.

8. The phospholipid drug conjugate according to any one of claims 1-4, or a stereoisomer, prodrug, pharmaceutically acceptable salt, or pharmaceutically acceptable solvate thereof, wherein the bioactive molecular fragment D is selected from: small molecule drugs, peptides, proteins, nucleic acids, sugars, including anesthetics, ophthalmic drugs, central nervous system drugs, pain therapeutics, anti-arthritic agents, anticonvulsants, antihistamines, anti-ulcer drugs, behavioral modification drugs, respiratory drugs, antispasmodics, muscle relaxants, anti-inflammatory drugs, appetite suppressants, growth promoters, hemostatic agents, immunostimulants, immunomodulators, muscle relaxant drugs, obesity therapeutics, osteoporosis drugs, sedatives, tranquilizers, migraine therapeutics, muscle contractants, anti-infectives, antirheumatics, antimalarials, antiemetics, bronchodilators, antithrombotics, antihypertensives, cardiovascular drugs, antiarrhythmics, antioxidants, antiasthmatics, diuretics, lipid regulators, antiandrogens, antiparasitics, anticoagulants, antineoplastic agents, hypoglycemic agents, nutritional agents, growth supplements, anti-enteritis agents, vaccines, antibodies, radionuclides, diagnostic agents, and contrast agents; preferably, selected from small molecule drugs or peptide drugs; more preferably, selected from antineoplastic drugs, such as cytotoxic drugs, small molecule targeted drugs, hormones, biological response modifiers (e.g., immunomodulators), tumor adjuvant drugs, and radionuclide drugs.

9. The phospholipid drug conjugate according to any one of claims 1-8, or a stereoisomer, prodrug, pharmaceutically acceptable salt, or pharmaceutically acceptable solvate thereof, wherein said phospholipid drug conjugate has the structure represented by formula (II): 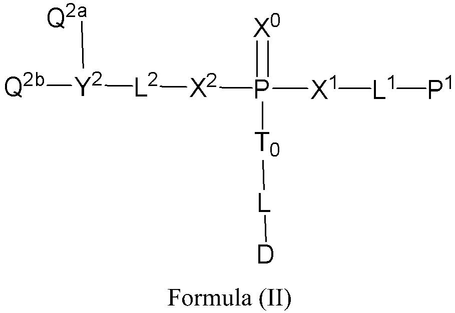
wherein , X⁰, X¹ , L¹, P¹ , X², L², Y², Q^{2a}, Q^{2b}, Y², and D are as defined in any one of claims 1-7;
wherein T⁰ is present or absent, and when present, the connection to the P end is selected from 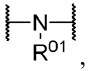 wherein R⁰¹ is hydrogen, C1-C10 alkyl, C1-C10 alkoxy, cycloalkyl, heterocycloalkyl, aryl, or heteroaryl;
L is L³-L^{s}, wherein L³ is an environment-responsive linking unit, and L^{s} is present or absent, and when present, it is a self-immolative linking moiety; when T⁰ is absent, L³ is directly bonded to the P end, and the functional group at the connection point is a nitrogen atom.

10. The phospholipid drug conjugate according to claim 9, or a stereoisomer, prodrug, pharmaceutically acceptable salt, or pharmaceutically acceptable solvate thereof, wherein:
X⁰, X¹, X² are O;
L¹ and L² are selected from absent or present, and when present, are selected from: 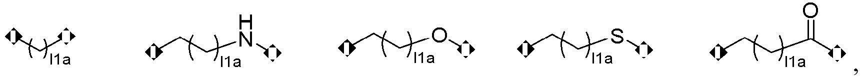 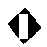 is the connection point to the P end, 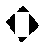 is the connection point to P¹ or Y²; 11a is an integer from 0 to 10, preferably an integer from 1 to 5;
Y² is selected from 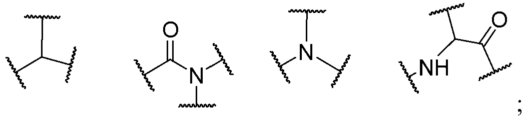
preferably, Y² is selected from 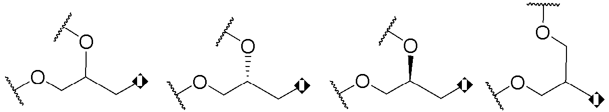 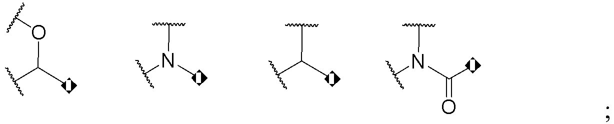
more preferably, Y² is selected from 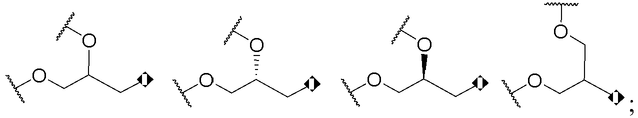 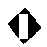 is the connection point to L²;
P¹ is selected from a polyethylene glycol derivative residue with a repeating unit number of pa1 and an end group R^{p1}, wherein pa1 is an integer from 10 to 60, preferably an integer from 15 to 50, for example 21, 22, 44, or 45; R^{p1} is C1-C6 alkyl, preferably C1-C3 alkyl, for example methyl, ethyl, n-propyl, or isopropyl;
or P¹ is selected from a polysarcosine derivative residue with a repeating unit number of pb1 and an end group R^{p2}: 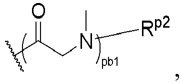
wherein, pb1 is an integer from 10 to 60, preferably an integer from 15 to 50; R^{p2} is selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, or an amino protecting group;
Q^{2a} is T^{2a}-R¹, Q^{2b} is T^{2b}-R², T^{2a} and T^{2b} are as defined in claim 1, and R¹ and R² are selected from: 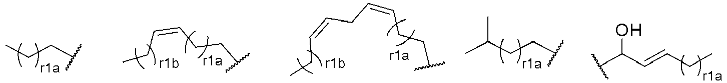 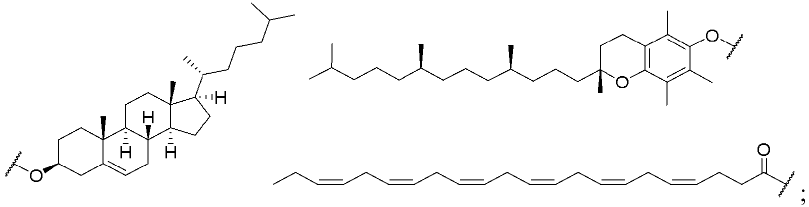
wherein, rla and r1b are integers from 1 to 30.

11. The phospholipid drug conjugate according to any one of claims 1-3 or 8, or a stereoisomer, prodrug, pharmaceutically acceptable salt, or pharmaceutically acceptable solvate thereof, wherein the bioactive molecular fragment D is selected from:
(1) cytotoxic drugs:
topoisomerase inhibitor camptothecins as shown in formula (III): 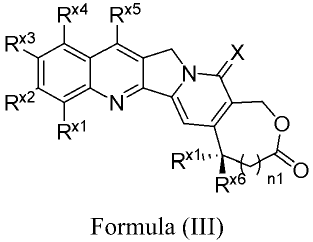
wherein, **R^{x1}** is selected from C1-C4 alkyl, C1-C4 haloalkyl, C1-C4 cycloalkyl, C1-C4 deuterated alkyl, C2-C4 alkynyl, or C2-C4 alkenyl;
**R^{x2} R^{x3}**, **R^{x4}**, and **R^{x5}** are each independently selected from hydrogen atom, deuterium atom, halogen, cyano, hydroxy, mercapto, sulfonyl, sulfinyl, substituted or unsubstituted amino, nitro, alkynyl, alkenyl, alkyl, haloalkyl, cycloalkyl, alkoxy, alkylthio, heterocycloalkyl, deuterated alkyl, aryl, or heteroaryl;
or, **R^{x1}** and **R^{x2}**, **R^{x2}** and **R^{x3}**, **R^{x3}** and **R^{x4}**, **R^{x4}** and **R^{x5}** together with the carbon atom to which they are attached form a 5-7 membered cycloalkyl or heterocycloalkyl;
X is selected from O or S;
**Z** is selected from OH or F;
**n1** is selected from 0 or 1;
preferably, selected from the following structures: 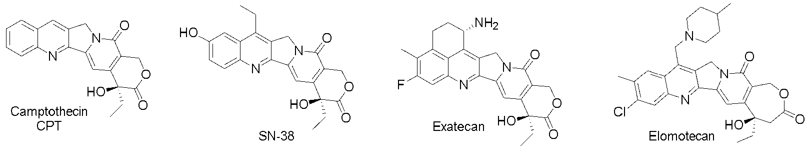 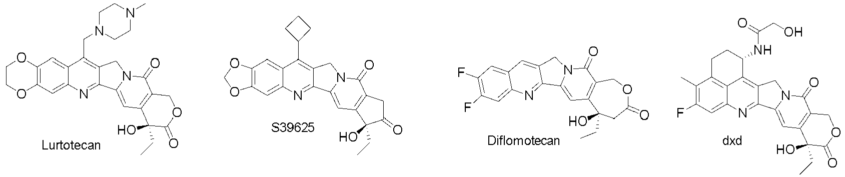 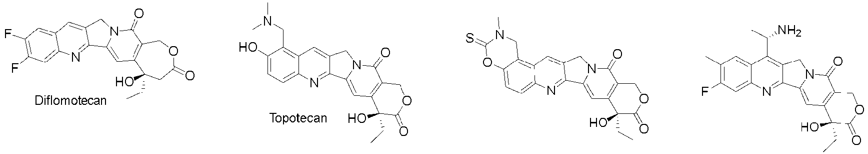 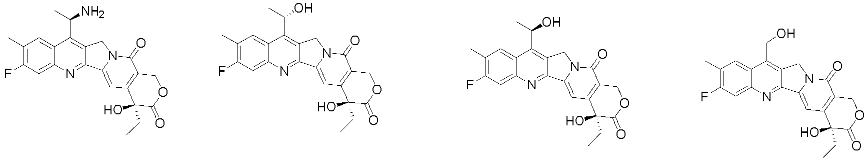 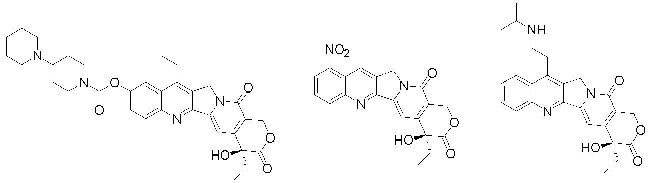 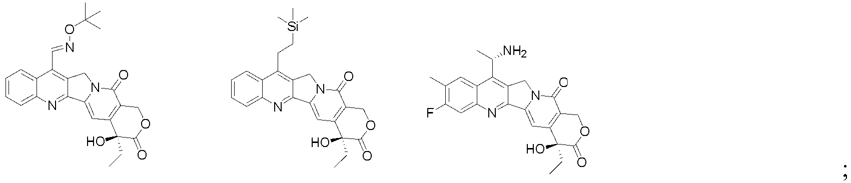
tubulin inhibitors, preferably selected from: 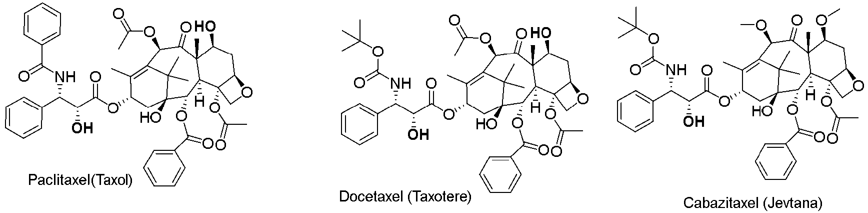 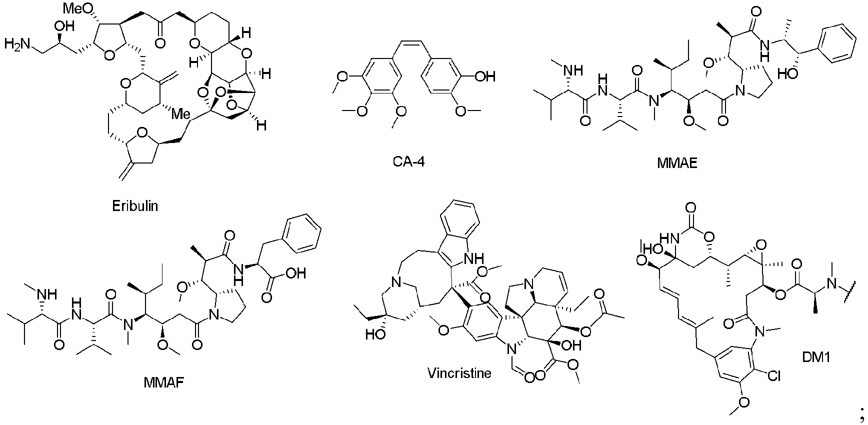
nucleic acid transcription inhibitors, preferably selected from: 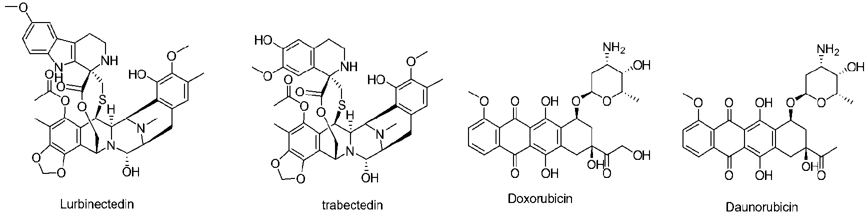 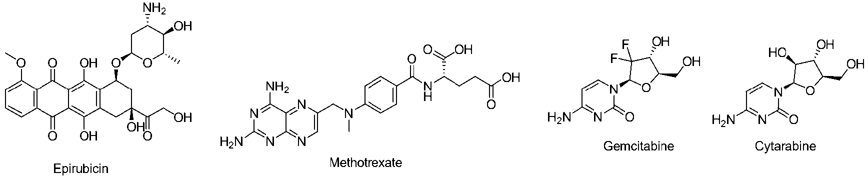 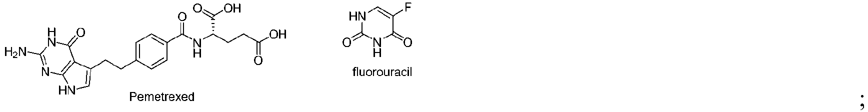
drugs acting on DNA structure, preferably selected from: 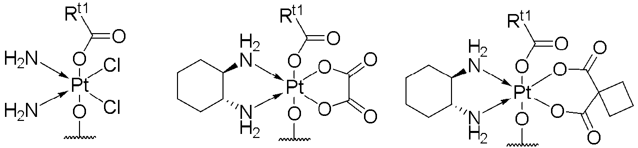 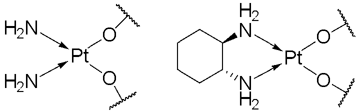 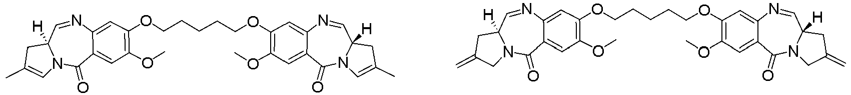 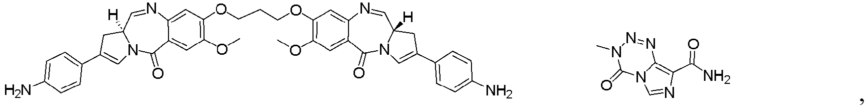
wherein R^{t1} is selected from hydrogen, alkyl, alkoxy, heteroalkyl, aryl, or heteroaryl;
(2) small molecule targeted drugs:
EGFR inhibitors, for example: gefitinib, erlotinib, icotinib, afatinib, or osimertinib;
HER2 inhibitors, for example: lapatinib, pyrotinib, neratinib, or tucatinib;
ALK inhibitors, for example: crizotinib, alectinib, ceritinib, lorlatinib, or brigatinib;
MEK inhibitors, for example: trametinib, cabozantinib, or binimetinib;
MET inhibitors, for example: savolitinib, volitinib, or capmatinib;
BRAF inhibitors, for example: sorafenib, dabrafenib, vemurafenib, or encorafenib;
CDK4/6 inhibitors, for example: palbociclib, ribociclib, abemaciclib, or 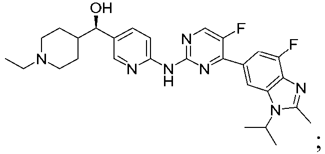
PARP inhibitors, for example: olaparib, niraparib, rucaparib, or talazoparib;
BTK inhibitors, for example: acalabrutinib, ibrutinib, tirabrutinib, or zanubrutinib;
protease inhibitors, for example: bortezomib, carfilzomib, or ixazomib;
mTOR inhibitors, for example: temsirolimus or everolimus;
JAK inhibitors, for example: ruxolitinib, tofacitinib, upadacitinib, baricitinib, deucravacitinib, peficitinib, filgotinib, abrocitinib, pacritinib, or deuterated ruxolitinib;
PI3K inhibitors, for example: idelalisib, copanlisib, duvelisib, or umbralisib;
VEGFR inhibitors, for example: sorafenib, axitinib, apatinib, sunitinib, regorafenib, vandetanib, pazopanib, lenvatinib, cabozantinib, ponatinib, aflibercept, or fruquintinib;
PDGFR, c-Kit inhibitors, for example: imatinib, nilotinib, or avapritinib;
BCL inhibitors, for example: venetoclax;
HDAC inhibitors, for example: vorinostat, romidepsin, belinostat, panobinostat, or chidamide;
FLT3 inhibitors, for example: midostaurin, gilteritinib, or quizartinib;
RET inhibitors, for example: selpercatinib or pralsetinib;
KRAS inhibitors, for example: sotorasib or adagrasib;
IDH inhibitors, for example: enasidenib, ivosidenib, or olutasidenib;
BCR-ABL inhibitors, for example: imatinib, dasatinib, nilotinib, ponatinib, or olverembatinib;
(3) hormone molecules, for example: fulvestrant, abiraterone, prednisone, dexamethasone, bicalutamide, enzalutamide, tamoxifen, toremifene, letrozole, exemestane, goserelin, or leuprolide;
(4) immunotherapeutic molecules, for example: small molecule PD-L1 inhibitors, TLR agonists, stimulator of interferon genes (STING) agonists, or indoleamine-2,3-dioxygenase (IDO) inhibitors;
(5) other small molecules and polypeptides, for example: lenalidomide, thalidomide, pomalidomide, iberdomide, octreotide, lanreotide, or pasireotide.

12. The phospholipid drug conjugate according to any one of claims 1-11, or a stereoisomer, prodrug, pharmaceutically acceptable salt, or pharmaceutically acceptable solvate thereof, wherein L-D is selected from any one of the following conditions:
(1) when the drug molecule D is the following structure, wherein the connection site to L is indicated by 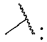 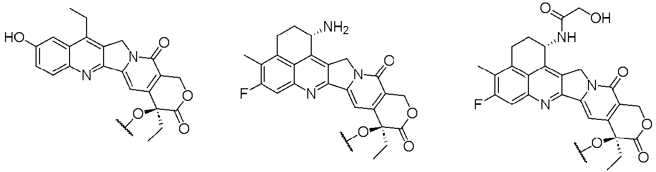 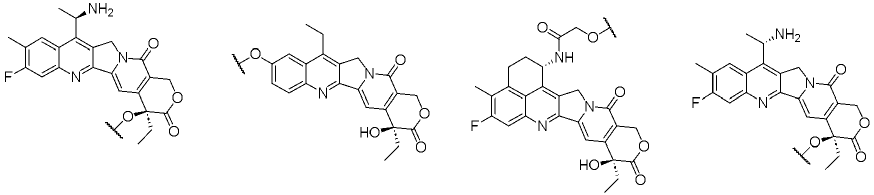 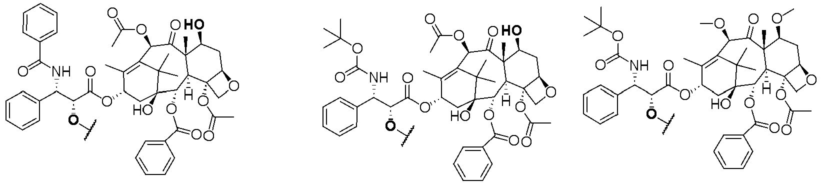 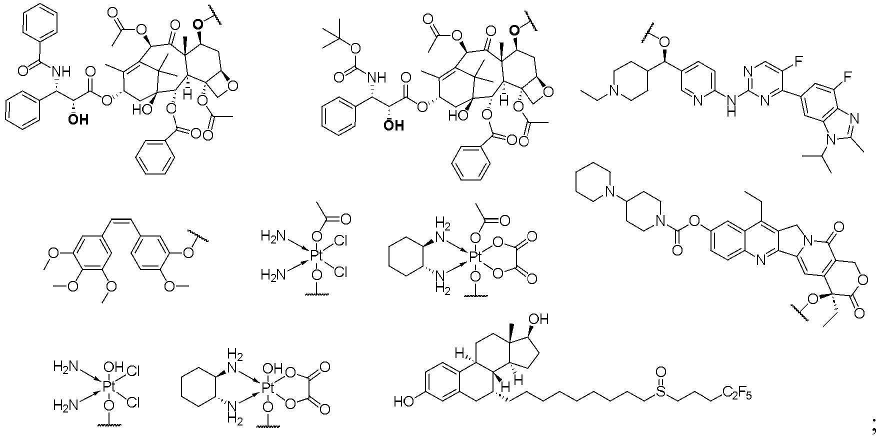 L is selected from the following structures, wherein the connection site to drug D is indicated by 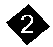 : 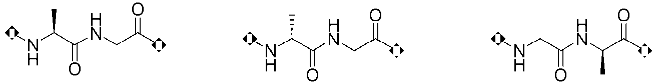 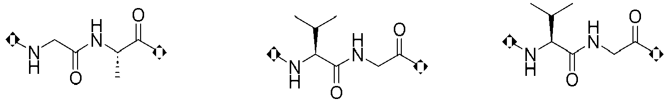 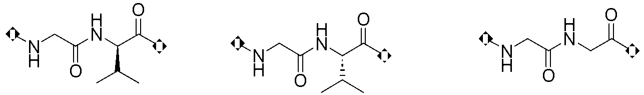 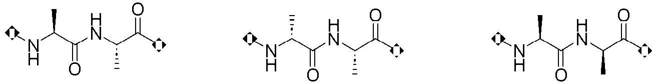 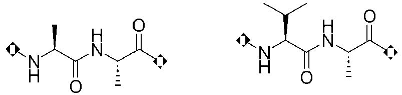 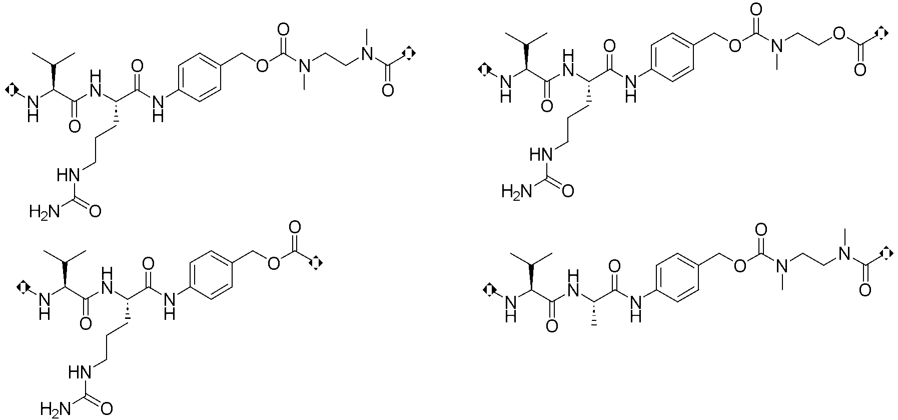 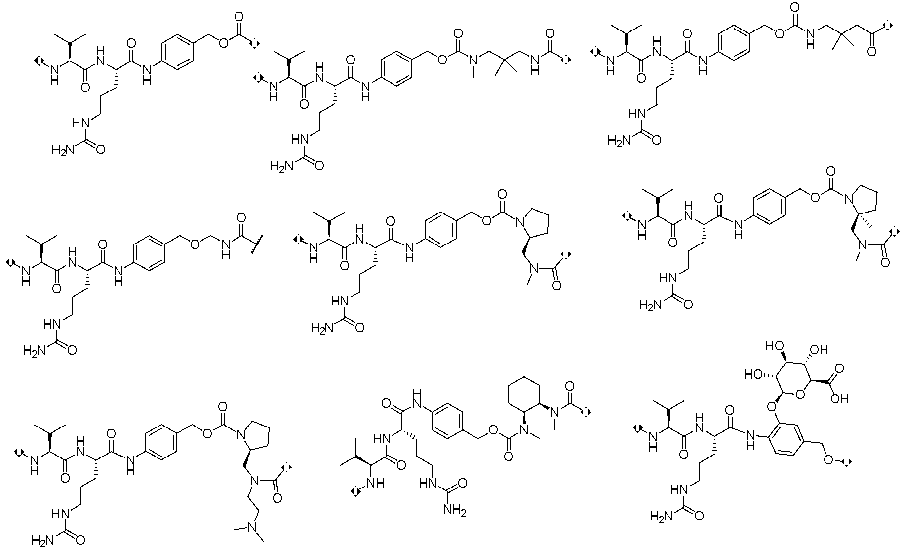 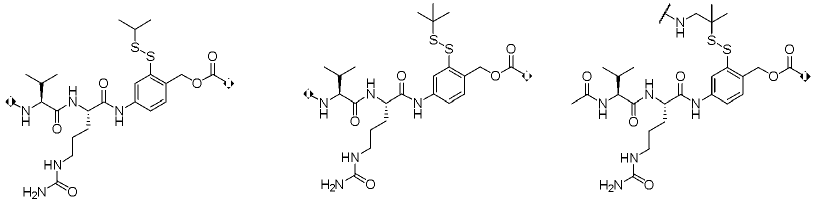 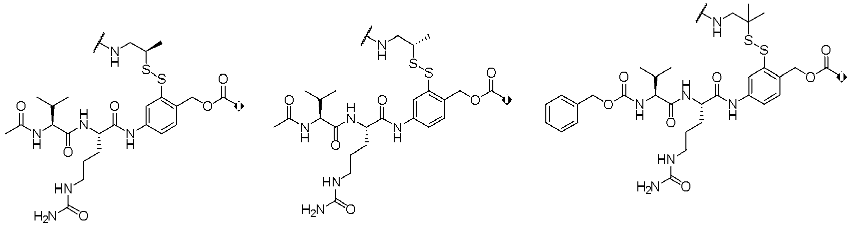 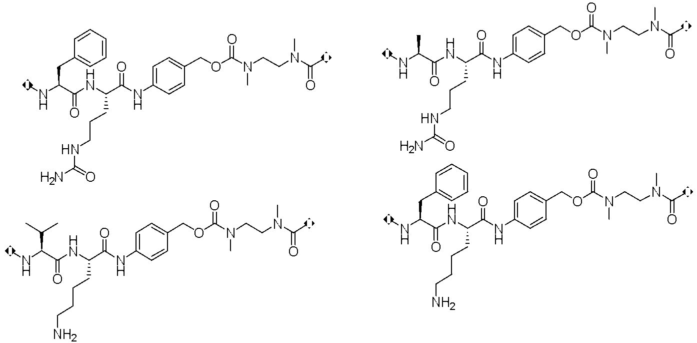 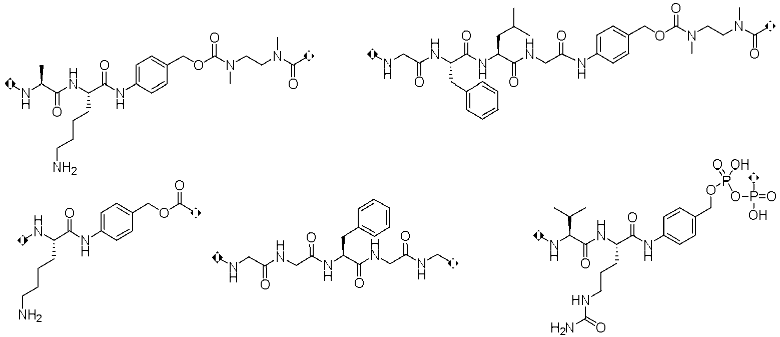 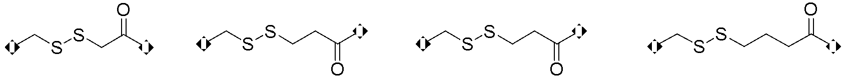 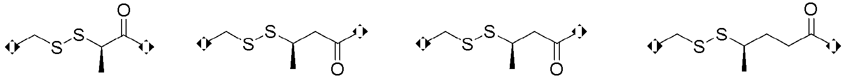 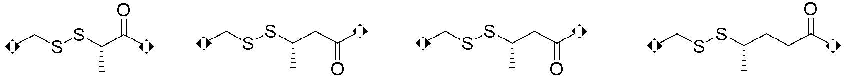 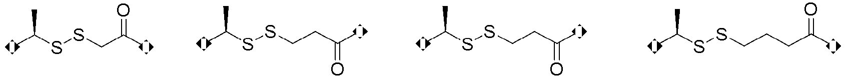 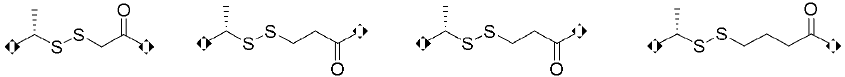 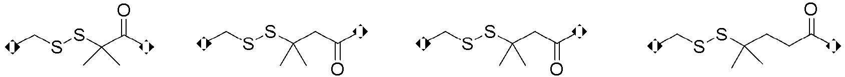 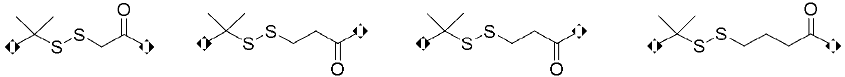 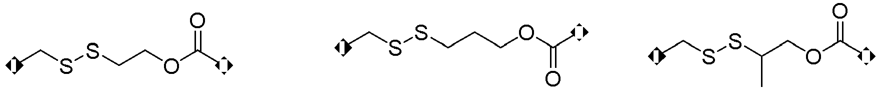 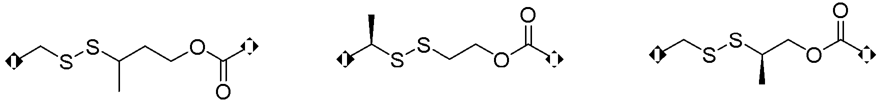 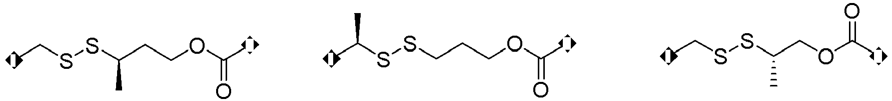 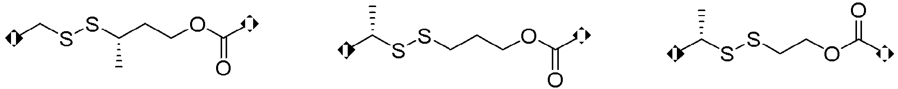 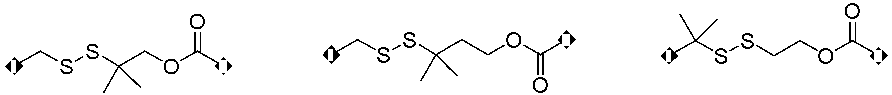 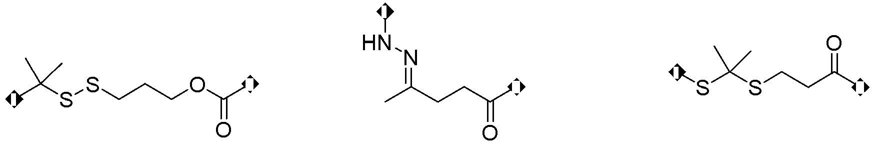 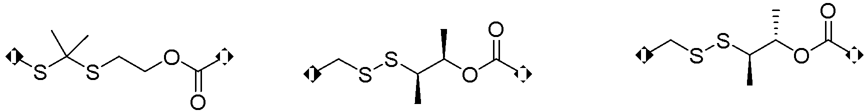 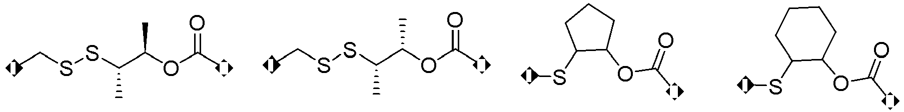 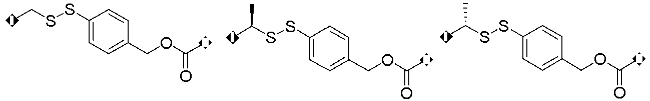 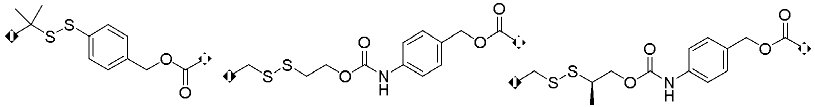 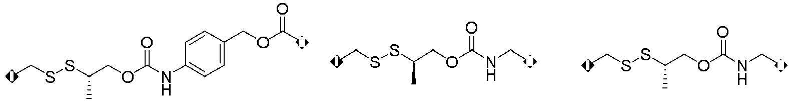 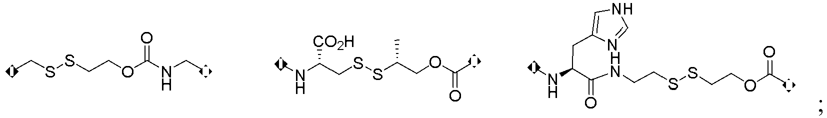
(2) when the drug molecule D is the following structure, wherein the connection site to L is indicated by 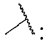 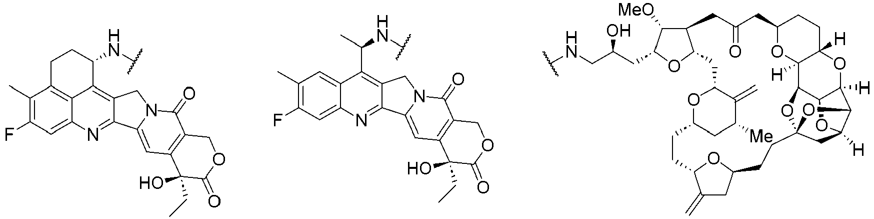 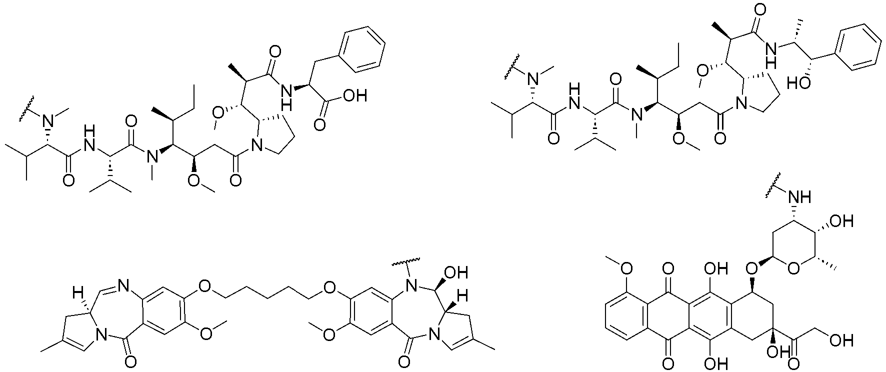 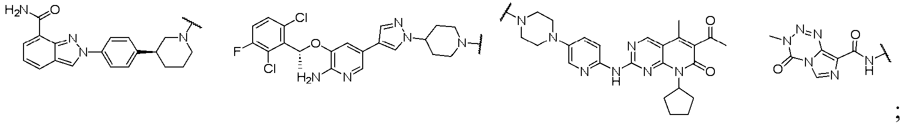 L is selected from the following structures, wherein the connection site to drug D is indicated by 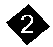 :

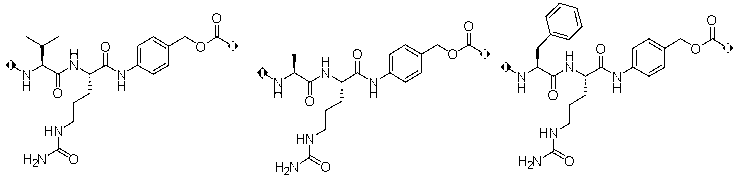

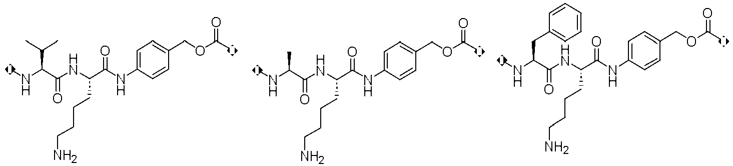

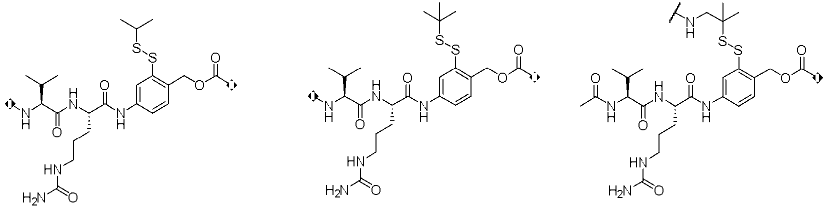

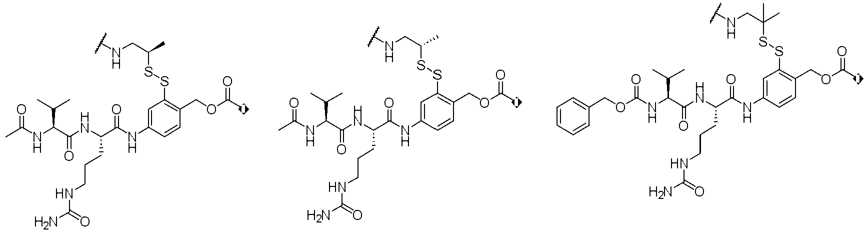

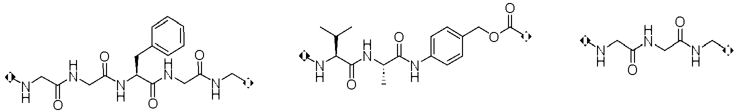

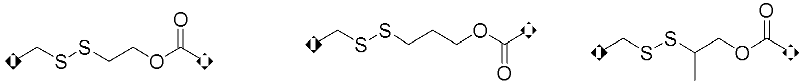

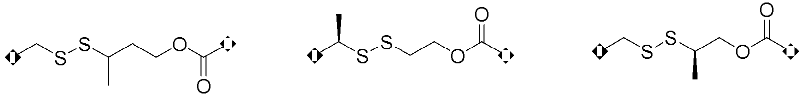

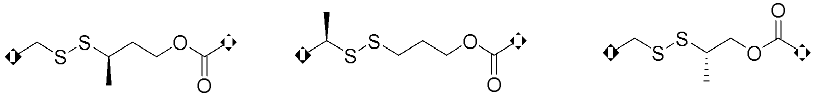

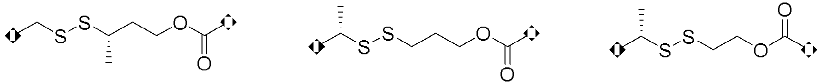

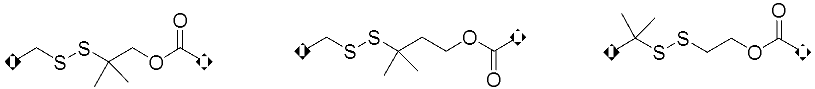

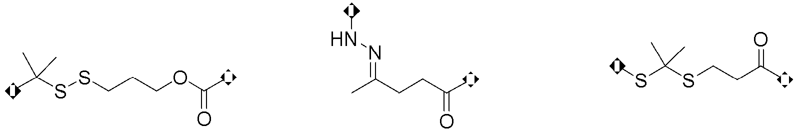

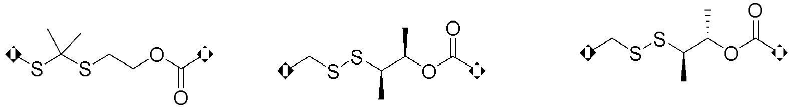

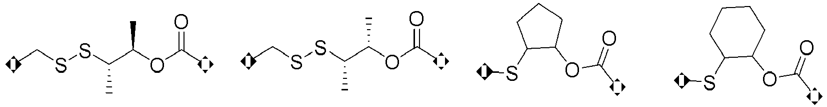

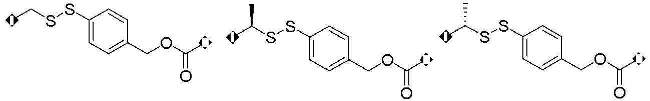

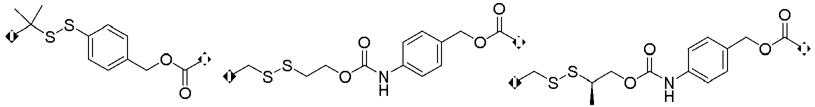

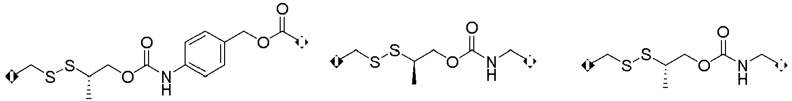

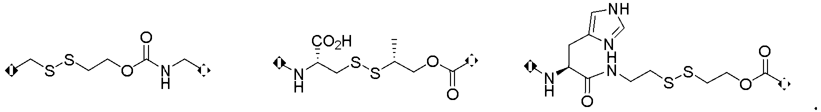

13. The phospholipid drug conjugate according to any one of claims 9-12, or a stereoisomer, prodrug, pharmaceutically acceptable salt, or pharmaceutically acceptable solvate thereof, wherein said phospholipid drug conjugate has the structure represented by formula (IV): 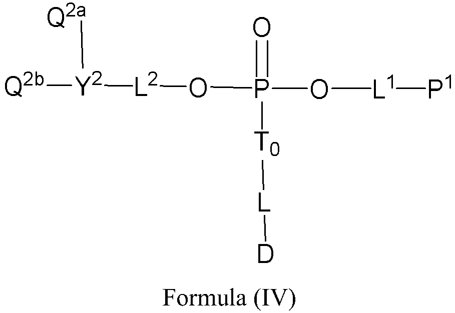 wherein,
(1) 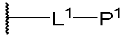 is selected from the following structures: 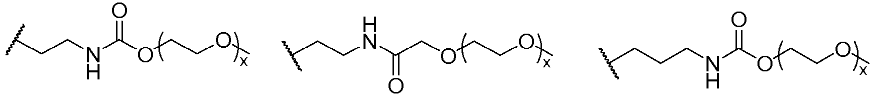 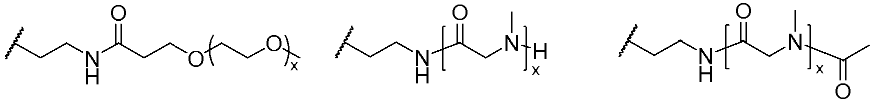 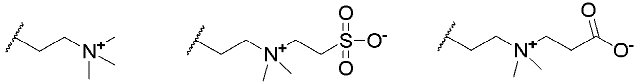 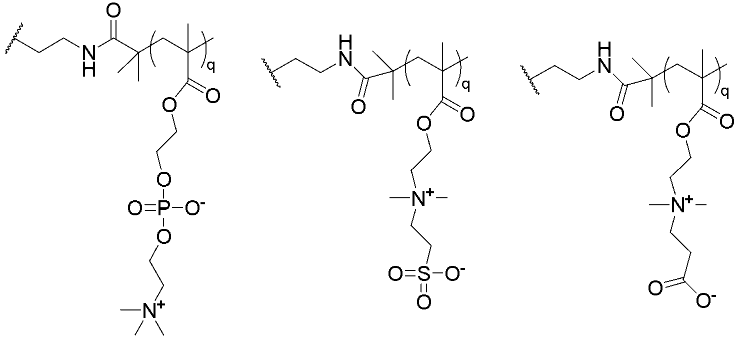 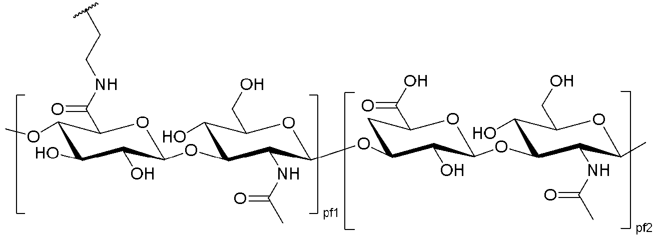 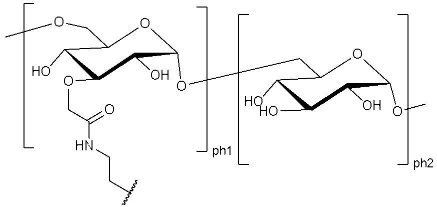 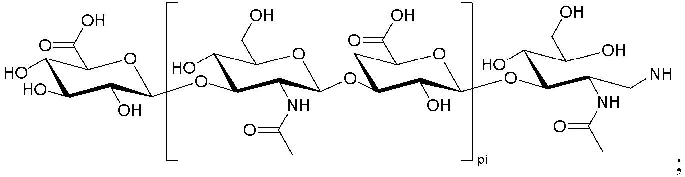 wherein, x, q are integers from 10 to 60; pf1, pf2, ph1, ph2, pi are integers from 1 to 100;
(2) 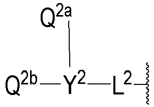 is selected from the following structures: 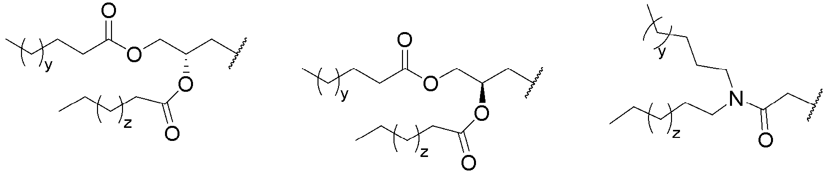 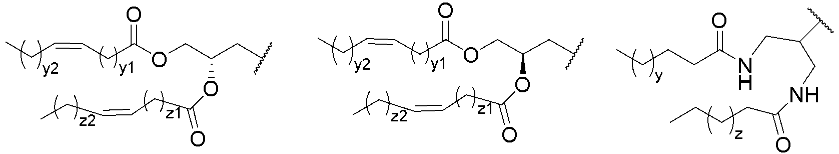 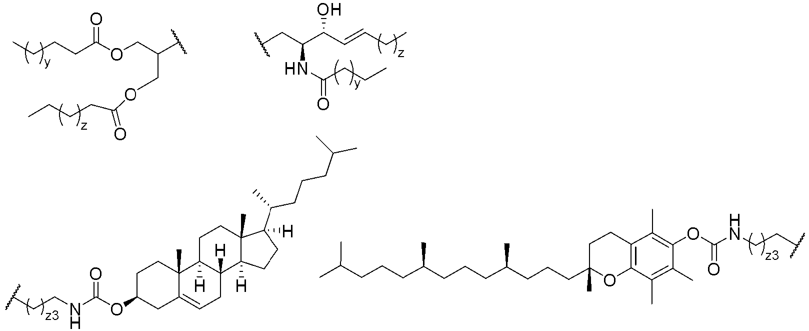 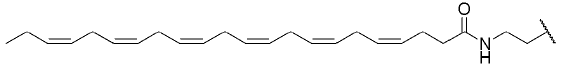 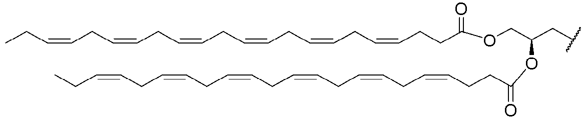 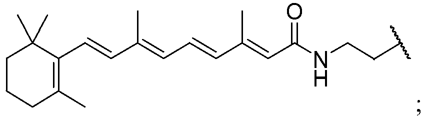 wherein, y, z, y1, y2, z1, z2, z3 are integers from 1 to 20;
(3) 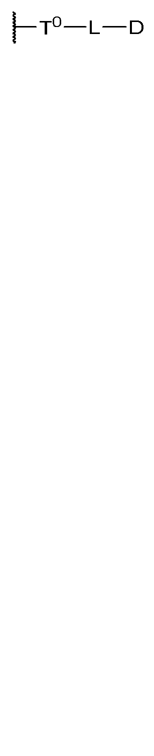 is selected from the following structures:

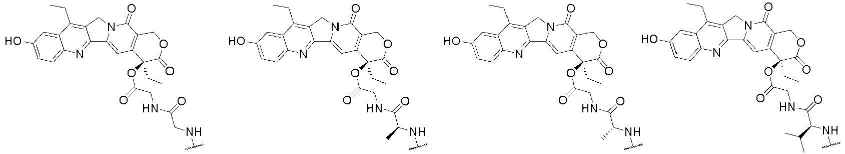

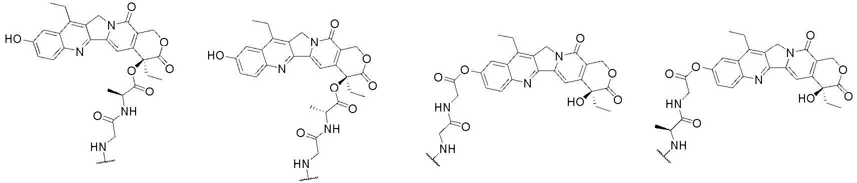

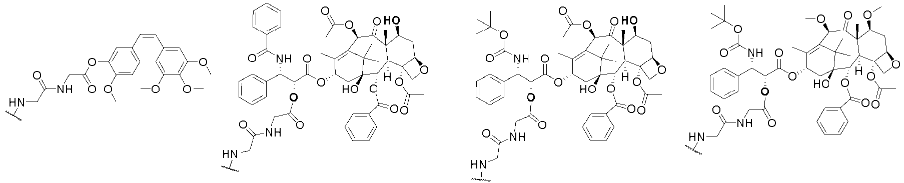

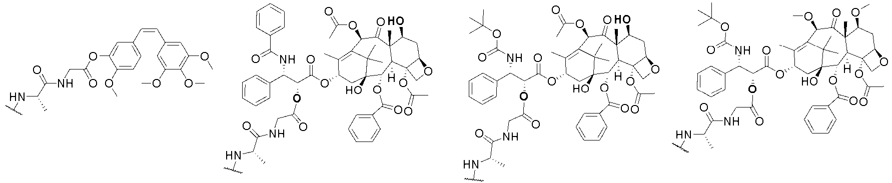

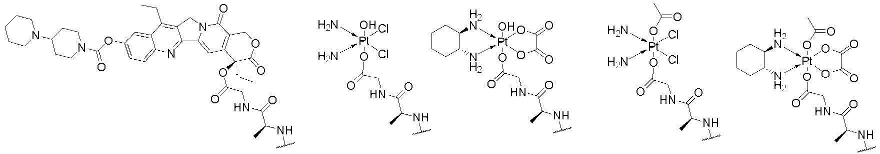

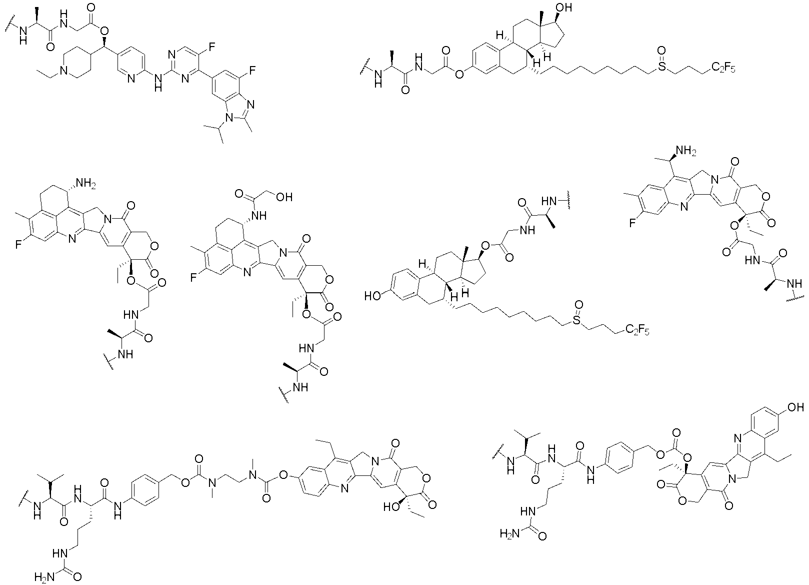

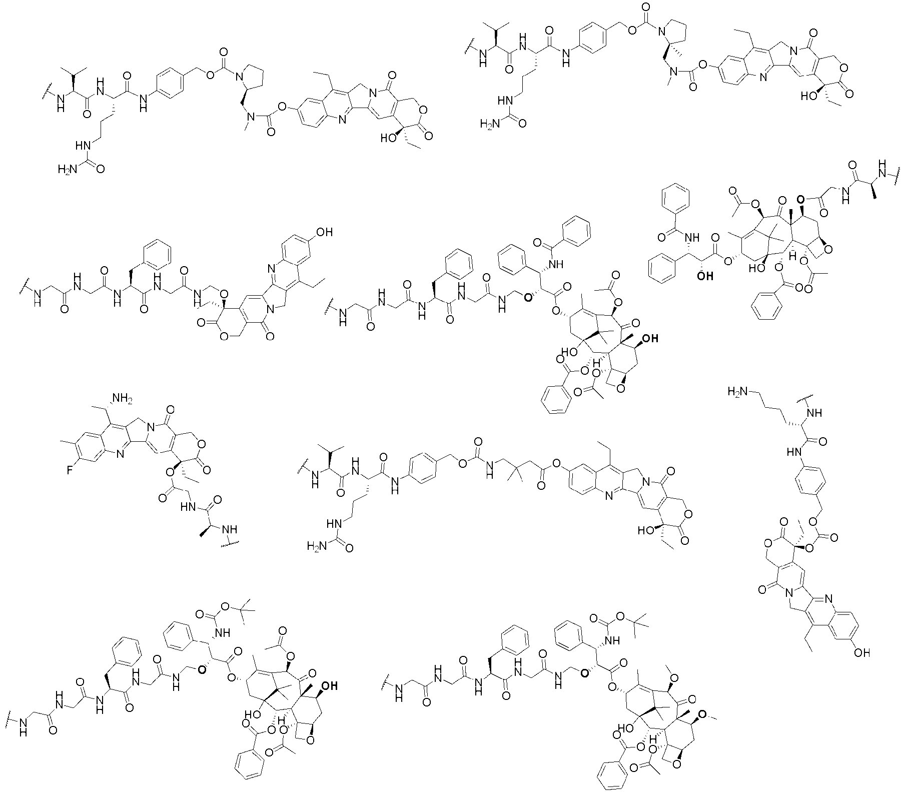

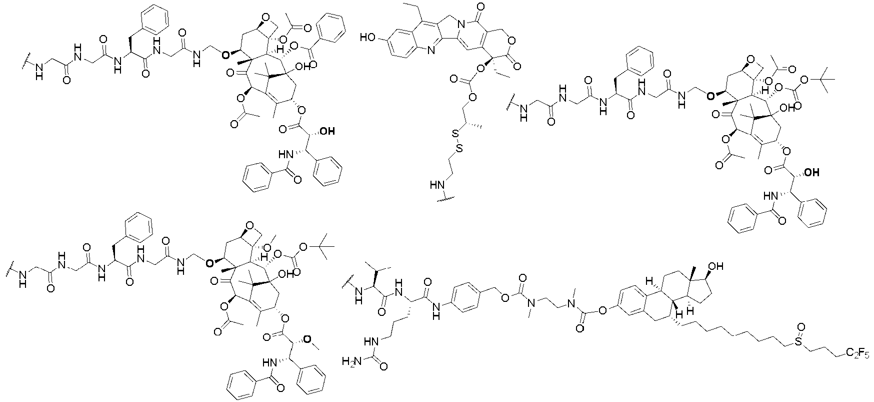

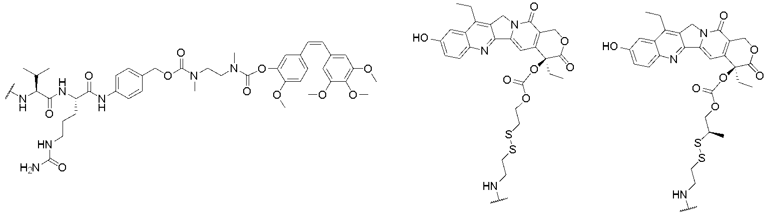

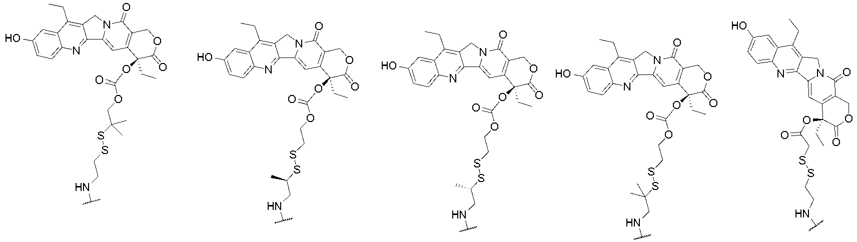

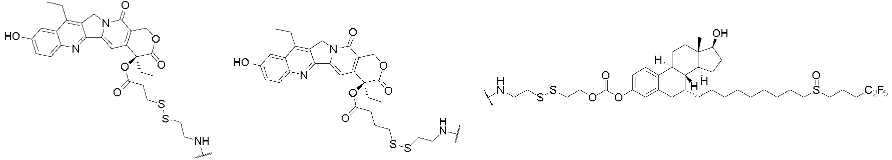

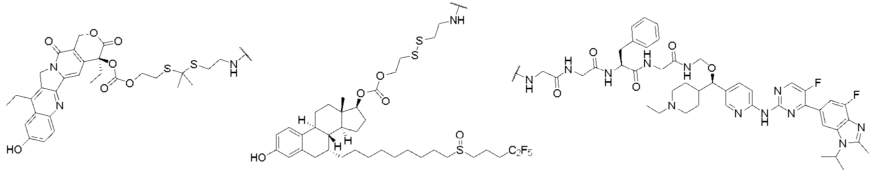

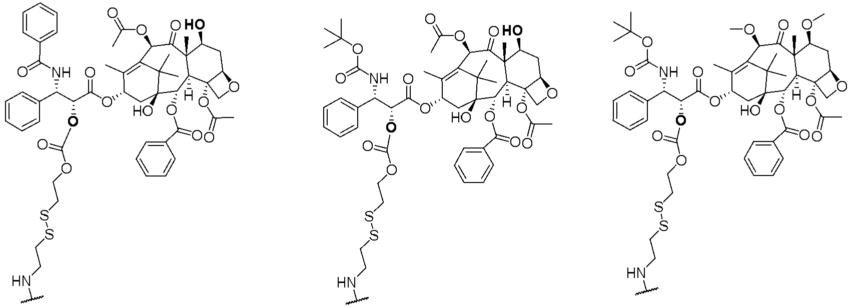

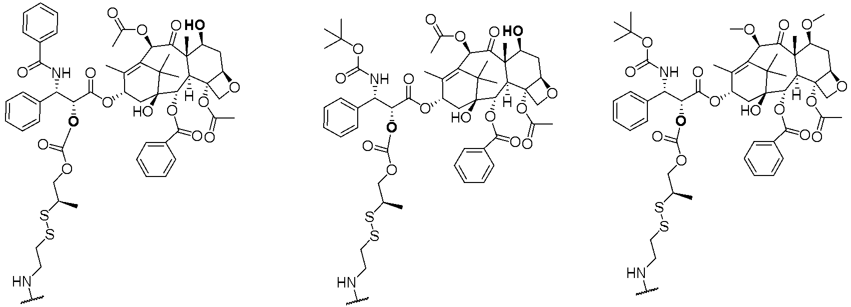

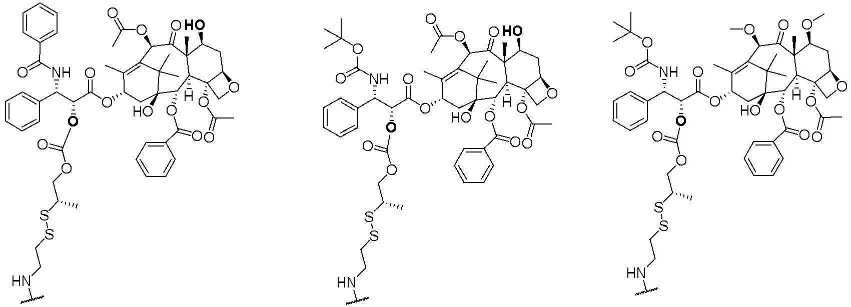

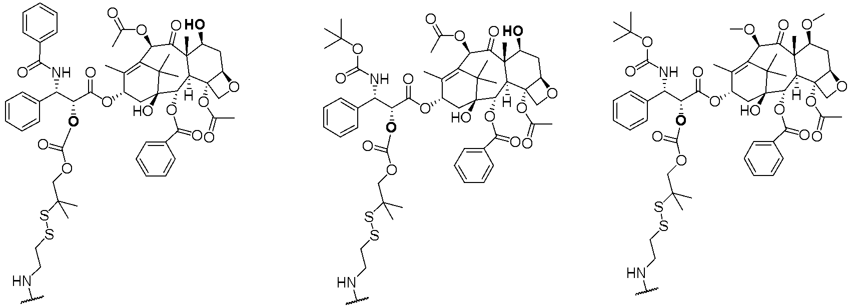

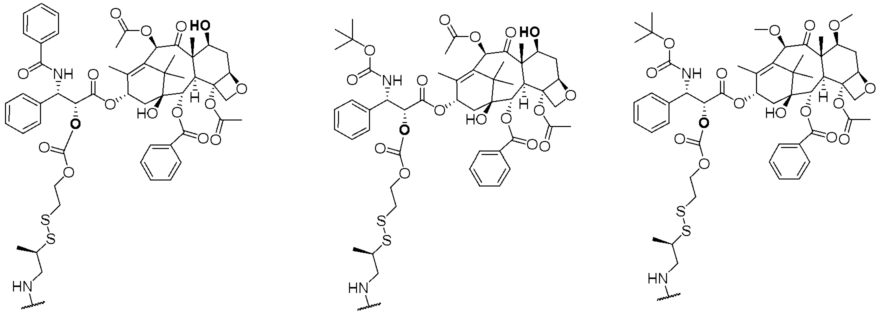

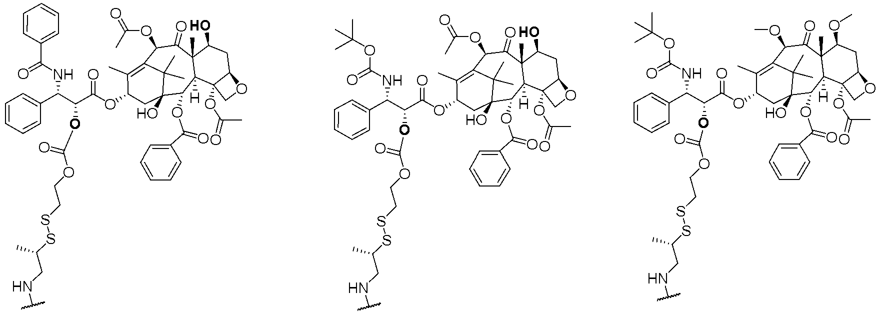

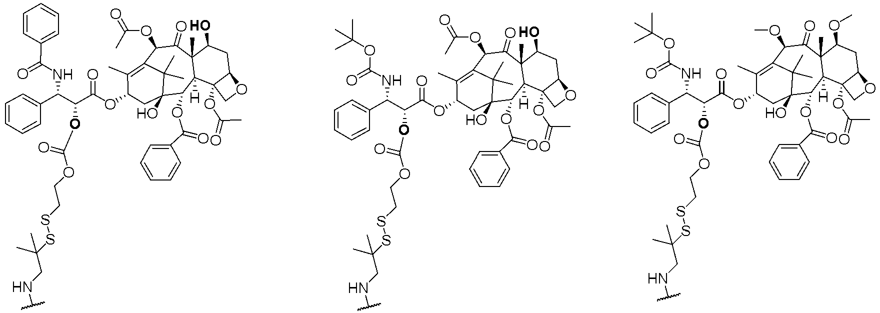

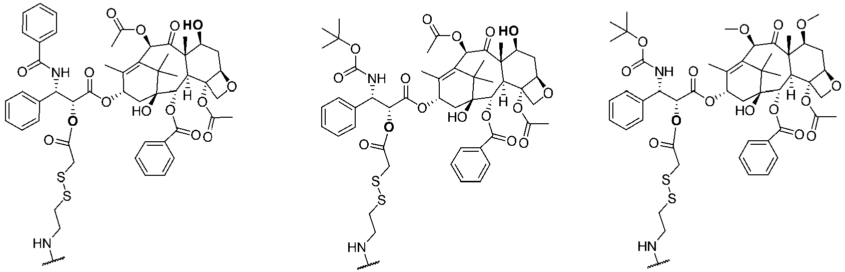

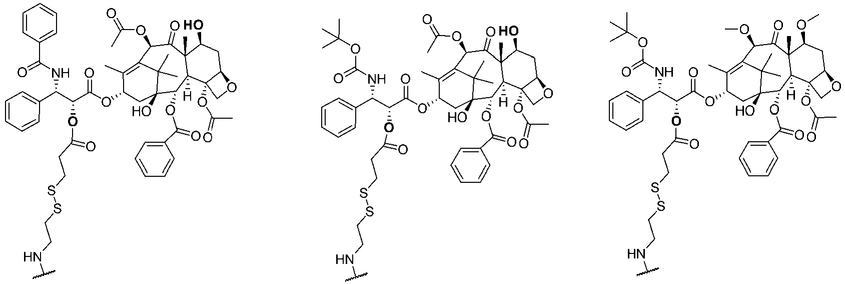

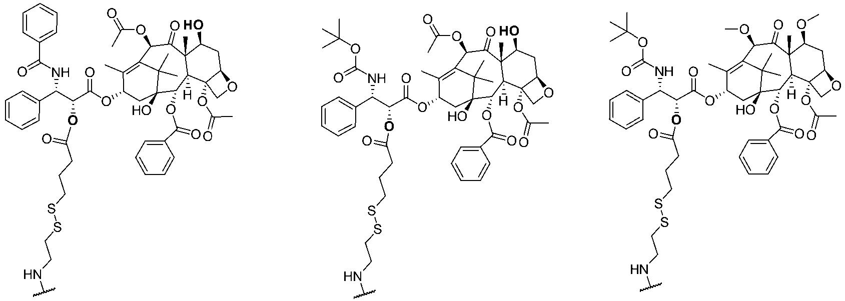

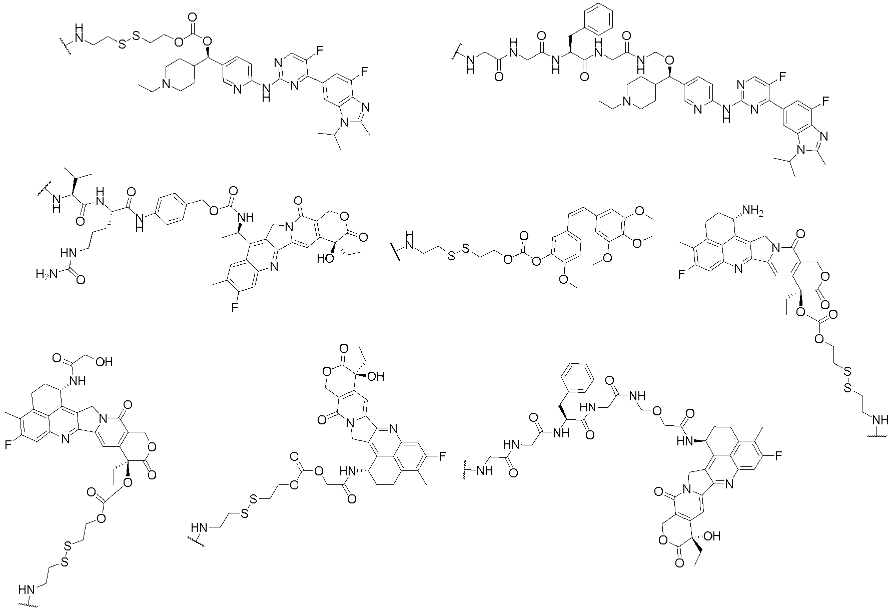

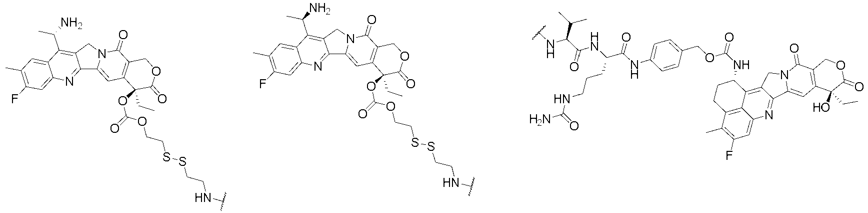

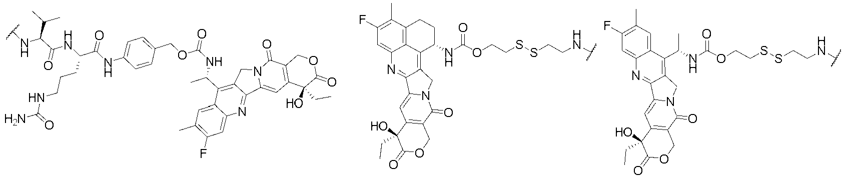

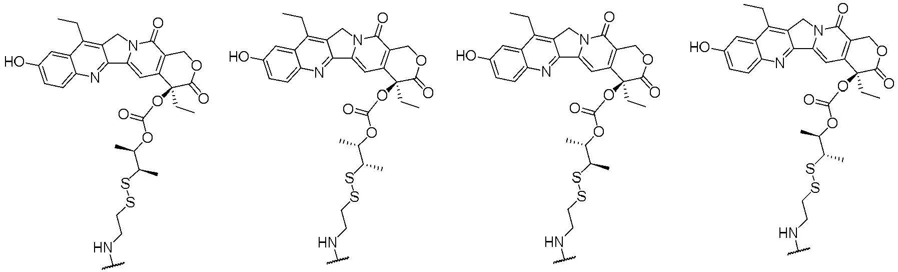

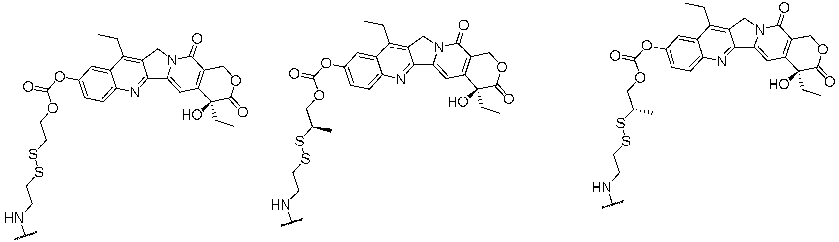

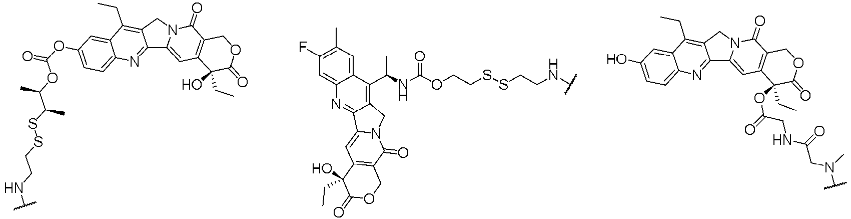

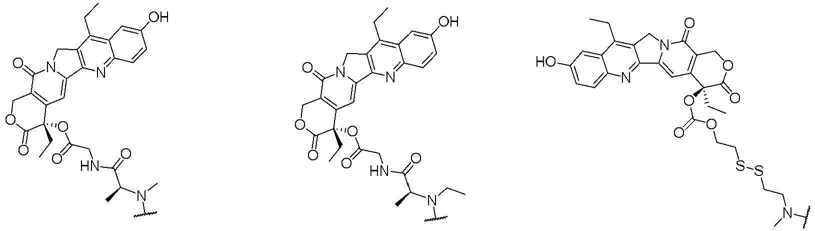

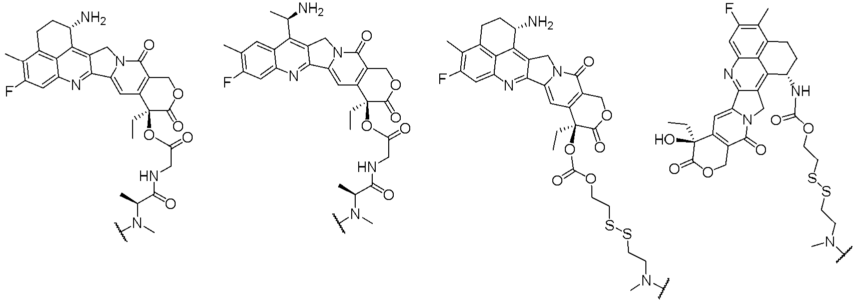

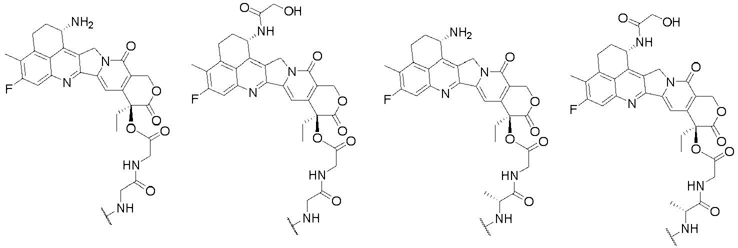

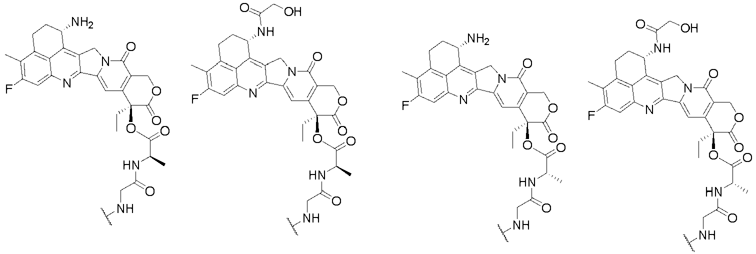

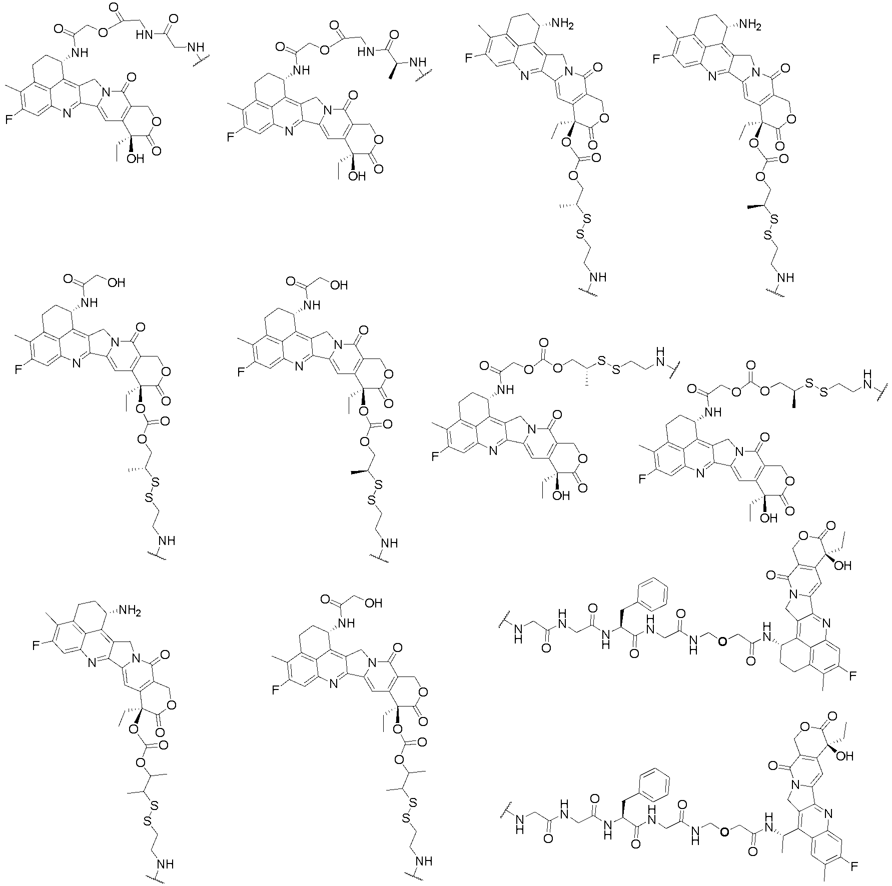

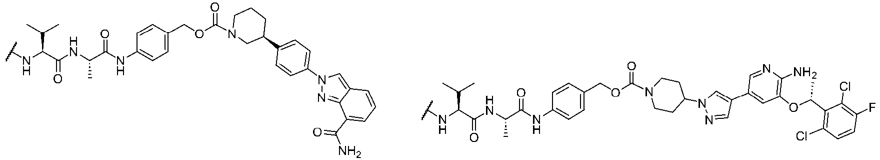

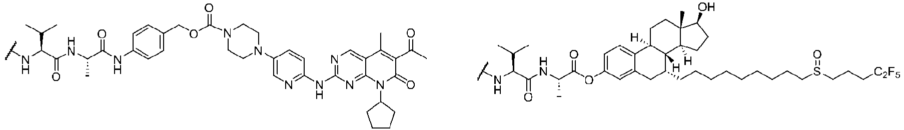

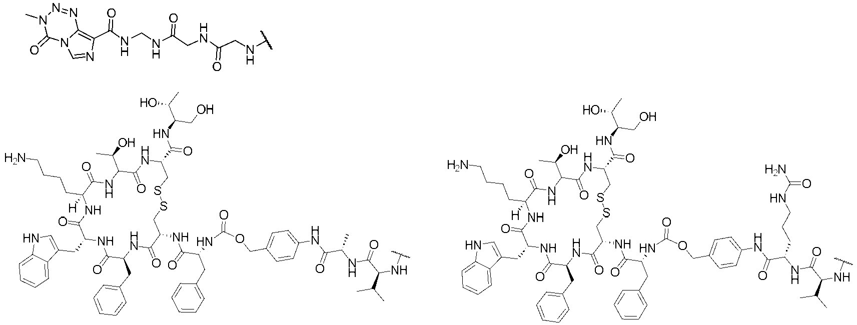

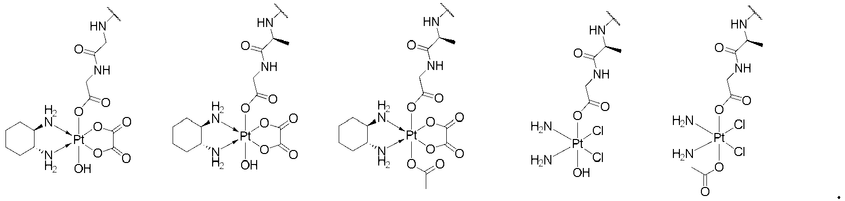

14. The phospholipid drug conjugate according to claim 1, or a stereoisomer, prodrug, pharmaceutically acceptable salt, or pharmaceutically acceptable solvate thereof, wherein it satisfies one or more of the following conditions:
(1) X⁰ is O;
(2) X¹ is O;
(3) X² is O;
(4) M⁰ is -T⁰-(L-D)ₙ, preferably -L-D, and n is preferably 1;
(5) T⁰ is absent or present, and when present, T⁰ is preferably selected from -NHCH₂- and 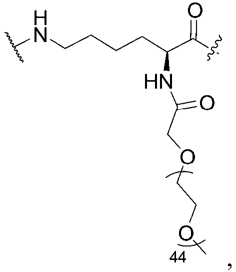 wherein the C end is preferably connected to L;
(6) M² is absent;
(7) L¹ is 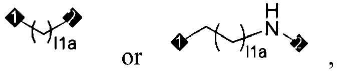 preferably 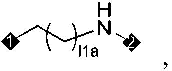 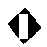 is connected to X¹, 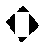 is connected to P¹, and 11a is an integer from 0 to 10, preferably an integer from 0 to 5, for example 1 or 2;
(8) L² is 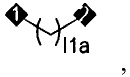 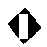 is connected to X¹, 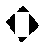 is connected to P¹; and 11a is an integer from 0 to 10, preferably an integer from 0 to 5, for example 0 or 2;
(9) K¹ is P¹, and P¹ is a pharmacokinetic modulating unit, selected from polyethylene glycol derivative residues, polysarcosine derivative residues, quaternary ammonium salt derivative residues, zwitterionic derivatives or zwitterionic polymers, and polysaccharide polymers, preferably selected from polyethylene glycol derivative residues, polysarcosine derivative residues, quaternary ammonium salt derivative residues, and polysaccharide polymers, more preferably polyethylene glycol derivative residues; said polyethylene glycol derivative residue is preferably 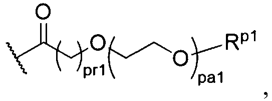 wherein pa1 is an integer from 5 to 250, preferably an integer from 5 to 150 (for example 22, 45, or 113), more preferably an integer from 10 to 60 (for example 45); pr1 is an integer from 0 to 8, preferably an integer from 0 to 2, for example 0; R^{p1} is hydrogen, C1-C10 alkyl, C1-C10 heteroalkyl, C3-C10 cycloalkyl, C3-C10 alkenyl, C3-C10 alkynyl, or a hydroxyl protecting group, preferably hydrogen, C1-C10 alkyl, or a hydroxyl protecting group, more preferably C1-C10 alkyl; said polysarcosine derivative residue is preferably 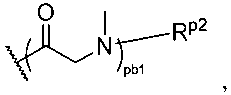 wherein pb1 is an integer from 5 to 250, preferably an integer from 5 to 150, more preferably an integer from 10 to 50, for example 28; R^{p2} is selected from hydrogen, C1-C10 alkyl, C1-C10 alkoxy, C3-C10 alkenyl, C3-C10 alkynyl, C3-C8 cycloalkyl, C2-C8 heterocycloalkyl, C6-C10 aryl, C5-C10 heteroaryl, and an amino protecting group, preferably an amino protecting group, for example -C(O)CH₃; said quaternary ammonium salt derivative residue is preferably 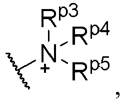 wherein R^{p3}, R^{p4}, and R^{p5} are each independently selected from C1-C10 alkyl, C1-C10 alkoxy, C3-C8 cycloalkyl, or C2-C8 heterocycloalkyl, preferably C1-C10 alkyl, for example methyl; said polysaccharide polymer is preferably selected from: 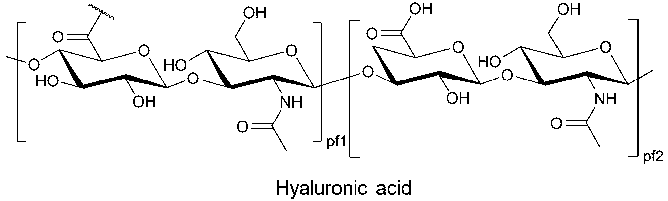 and 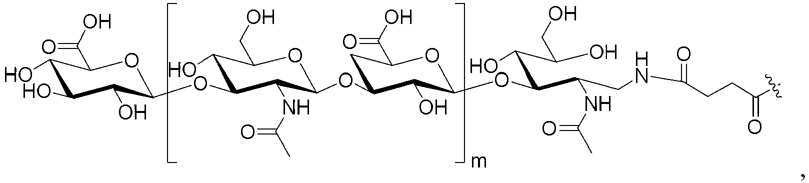 wherein pf1, pf2, and m are each independently selected from integers between 1 and 250, preferably integers between 1 and 50, for example integers between 1 and 5, integers between 4 and 24, or integers between 5 and 25;
(10) Y² is a branching center, selected from 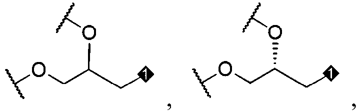 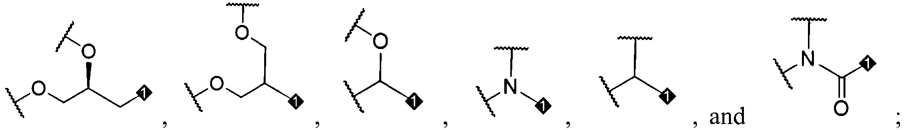 preferably selected from 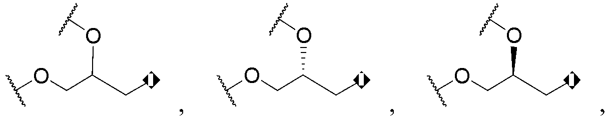 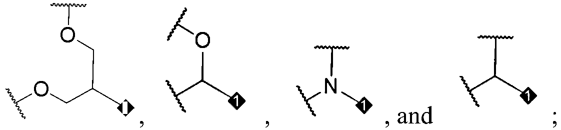 more preferably 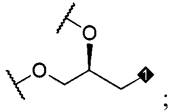 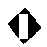 is the connection site for the L² end;
(11) T^{2a} and T^{2b} are absent or present, and when present, T^{2a} and T^{2b} are each independently selected from -C(O)-;
(12) for R¹ and R², said C₁-C₅₀ alkyl groups are each independently C₈-C₅₀ alkyl, more preferably C₁₀-C₃₀ alkyl, further preferably C₁₀-C₂₀ alkyl; preferably, R¹ and R² are each independently selected from 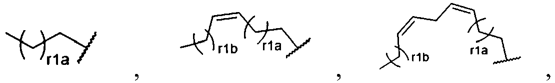 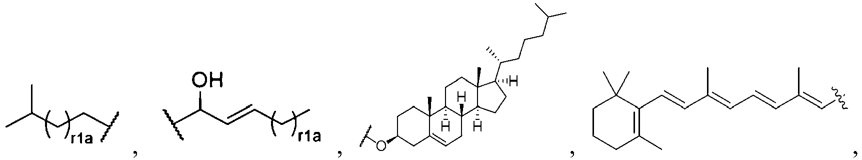 and 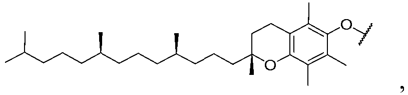 preferably selected from 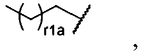 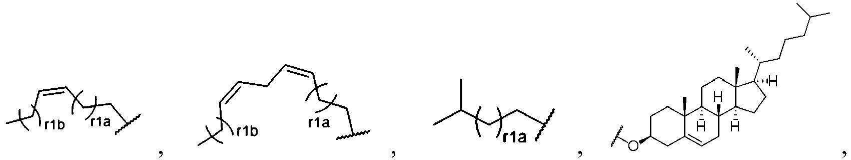 and 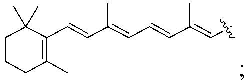 wherein, rla, r1b are integers from 1 to 30, and said 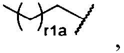 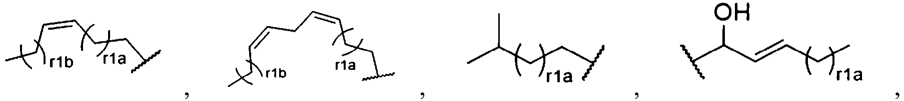 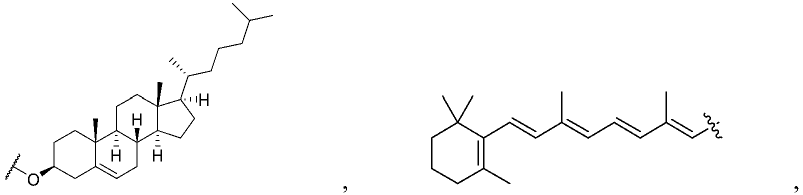 and 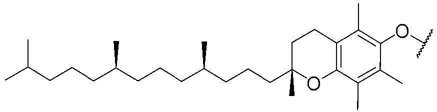 are optionally substituted by one or more substituents selected from hydroxyl, halogen, C₁₋₁₀ alkyl, C₁₋₁₀ alkoxy, C₃₋₁₂ cycloalkyl, and 3-12 membered heterocycloalkyl, and said C₁₋₁₀ alkyl, C₁₋₁₀ alkoxy, C₃₋₁₂ cycloalkyl, and 3-12 membered heterocycloalkyl are optionally substituted by one or more substituents selected from hydroxyl, halogen, C₁₋₃ alkyl, C₁₋₃ alkoxy, and C₃₋₆ cycloalkyl; alternatively, R¹ and R² are each independently preferably selected from 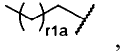 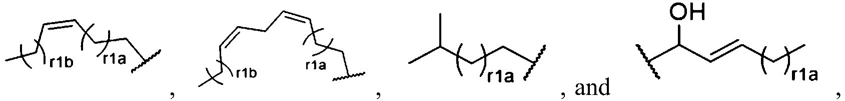 more preferably selected from 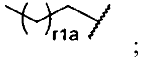 wherein, rla, r1b are integers from 1 to 30, and said 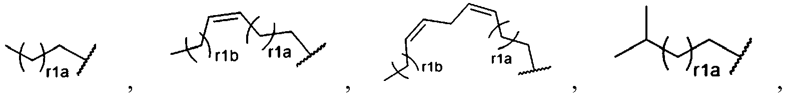 and 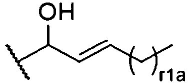 are optionally substituted by one or more substituents selected from hydroxyl, halogen, C₁₋₁₀ alkyl, C₁₋₁₀ alkoxy, C₃₋₁₂ cycloalkyl, and 3-12 membered heterocycloalkyl, and said C₁₋₁₀ alkyl, C₁₋₁₀ alkoxy, C₃₋₁₂ cycloalkyl, and 3-12 membered heterocycloalkyl are optionally substituted by one or more substituents selected from hydroxyl, halogen, C₁₋₃ alkyl, C₁₋₃ alkoxy, and C₃₋₆ cycloalkyl;
(13) said bioactive molecular fragment D is preferably selected from cytotoxic drugs, small molecule targeted drugs, hormone molecules, and other small molecules and polypeptides;
said cytotoxic drugs are preferably selected from topoisomerase inhibitor camptothecins as shown in formula (III), tubulin inhibitors, and drugs acting on DNA structure, more preferably selected from topoisomerase inhibitor camptothecins as shown in formula (III) and tubulin inhibitors; said topoisomerase inhibitor camptothecins as shown in formula (III) are preferably selected from: 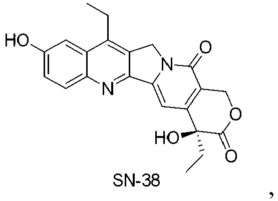 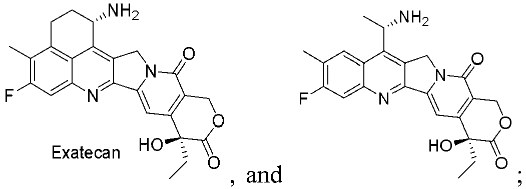 said tubulin inhibitors are preferably 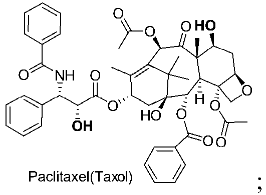 said drugs acting on DNA structure are preferably selected from 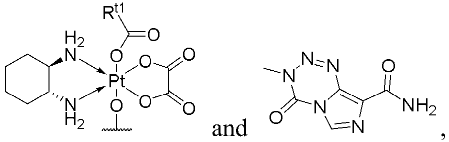 wherein Rt1 is selected from hydrogen, alkyl, alkoxy, heteroalkyl, aryl, and heteroaryl;
said small molecule targeted drugs are preferably selected from ALK inhibitors, CDK4/6 inhibitors, and PARP inhibitors; said ALK inhibitor is preferably crizotinib; said CDK4/6 inhibitors are preferably selected from palbociclib and 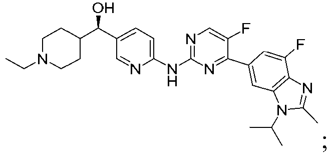 said PARP inhibitor is preferably niraparib;
said hormone molecule is preferably fulvestrant;
said other small molecules and polypeptides are preferably octreotide.

15. The phospholipid drug conjugate according to claim 1, or a stereoisomer, prodrug, pharmaceutically acceptable salt, or pharmaceutically acceptable solvate thereof, wherein it satisfies one or more of the following conditions:
(1) R¹ and R² are each independently selected from 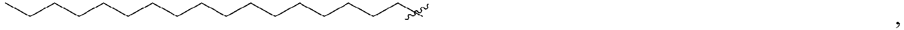 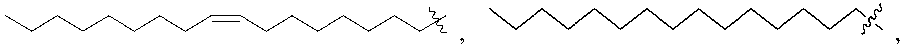 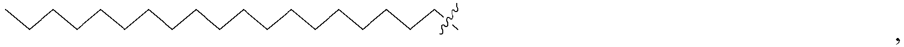 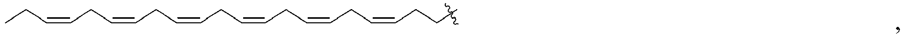 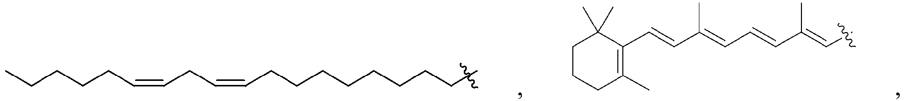 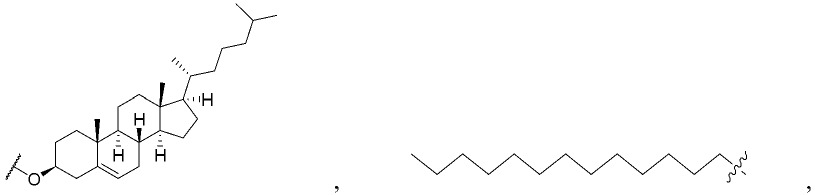 and 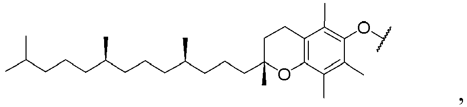 preferably selected from 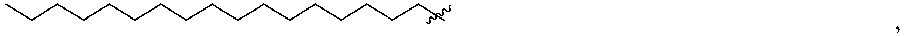 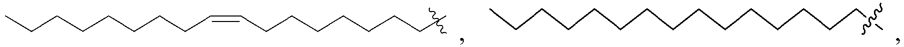 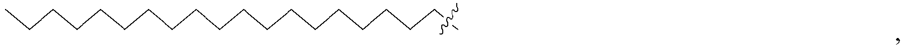 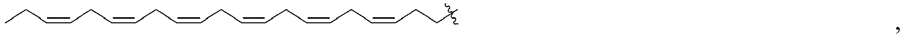 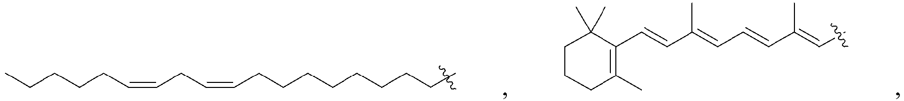 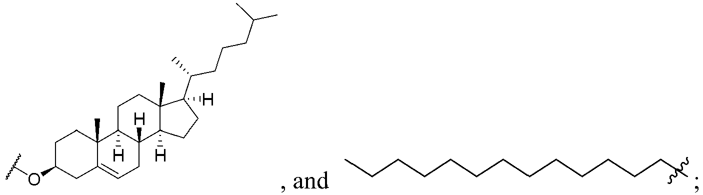 alternatively, R¹ and R² are each independently preferably selected from 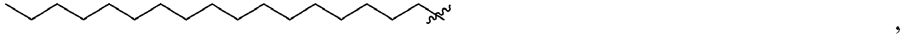 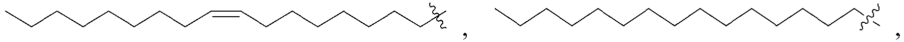 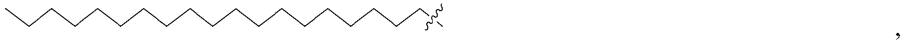 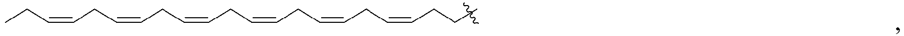 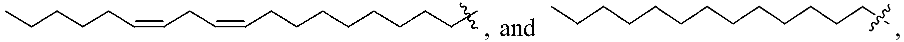 more preferably 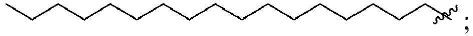
(2) Q^{2a} and Q^{2b} are each independently selected from 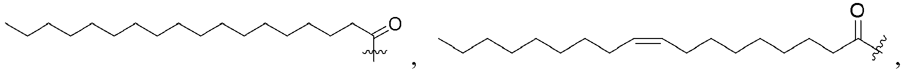 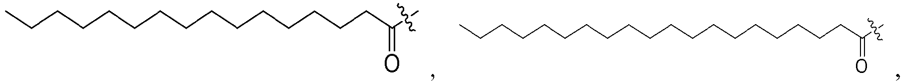 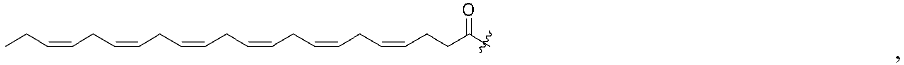 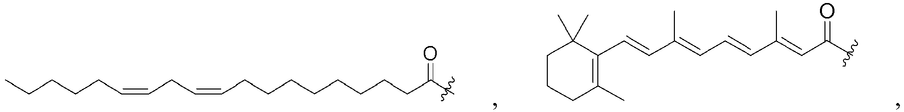 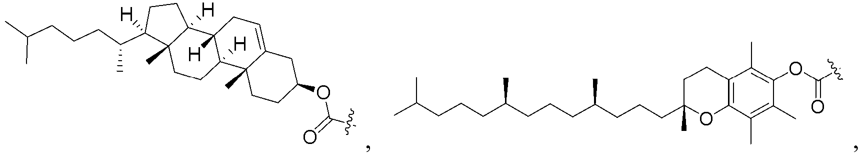 and 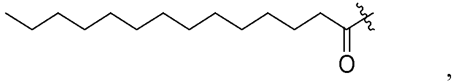 preferably selected from 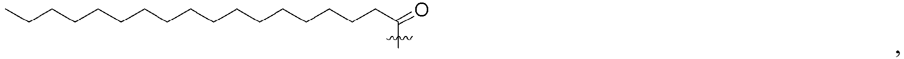 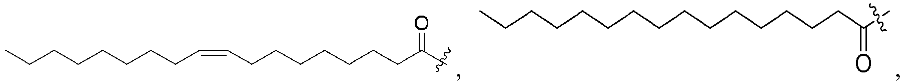 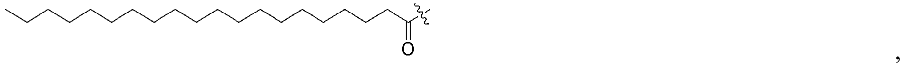 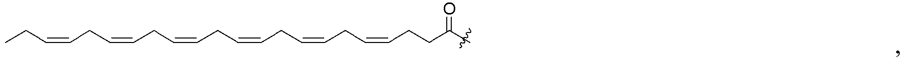 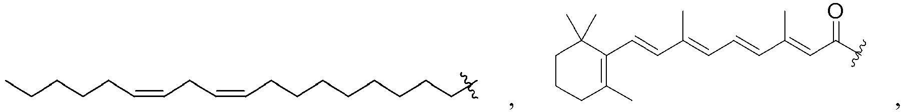 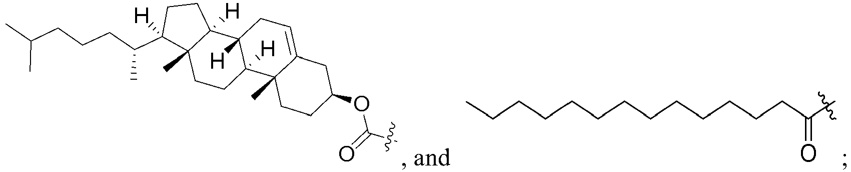 alternatively, Q^{2a} and Q^{2b} are each independently preferably selected from 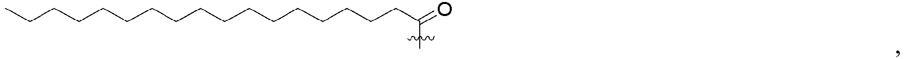 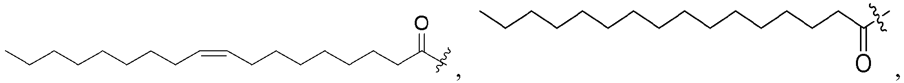 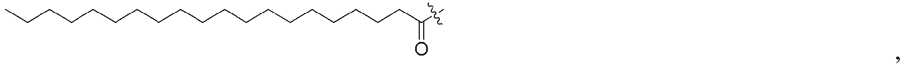 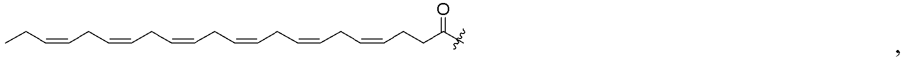 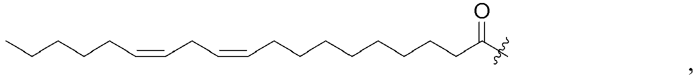 and 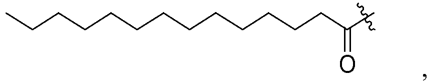 more preferably selected from 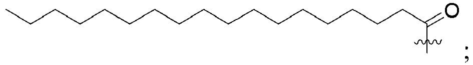
(3) D is selected from the following structures: 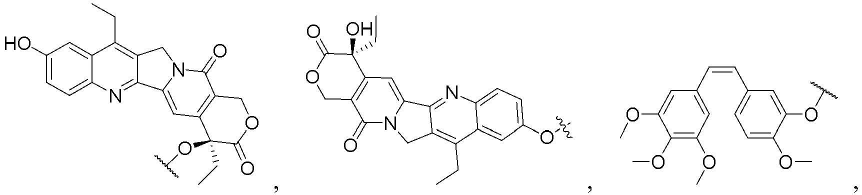 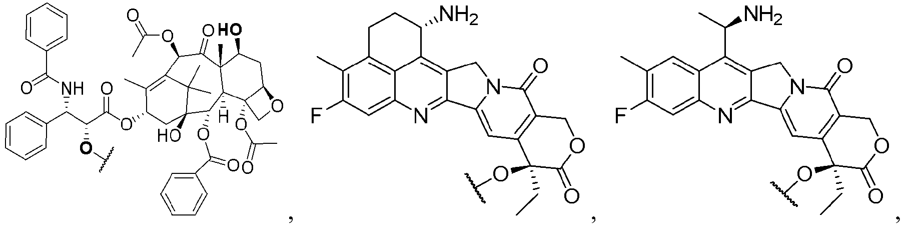 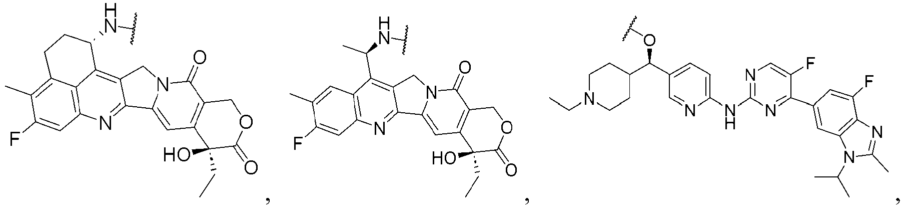 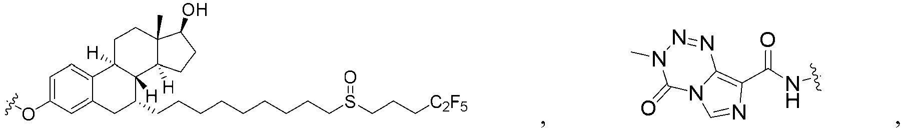 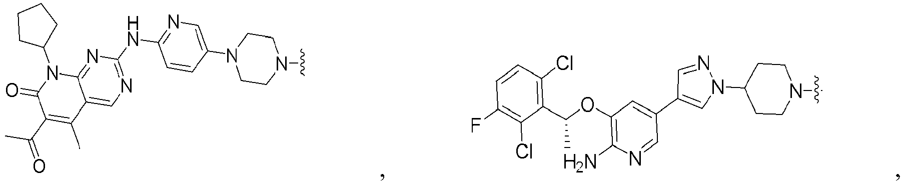 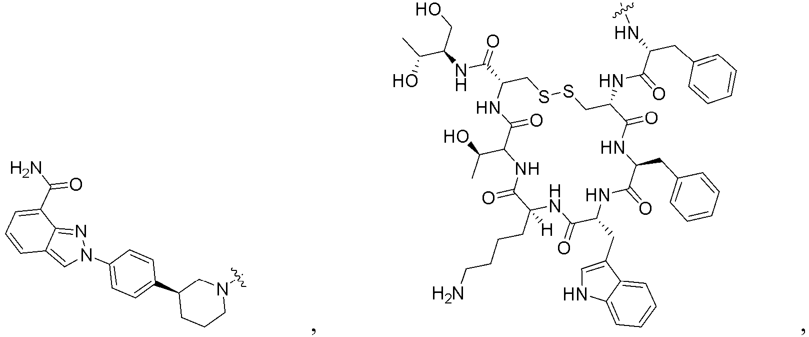 and 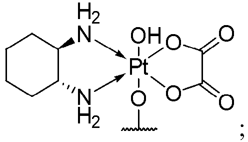
(4) L is selected from the following structures (wherein the connection site to D is indicated by 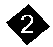 ): 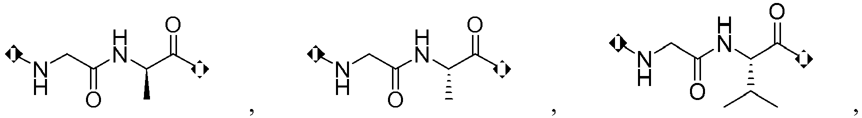 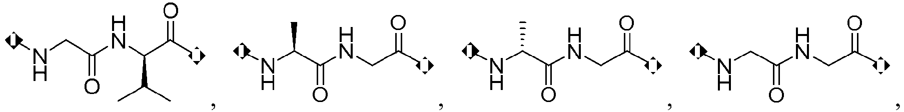 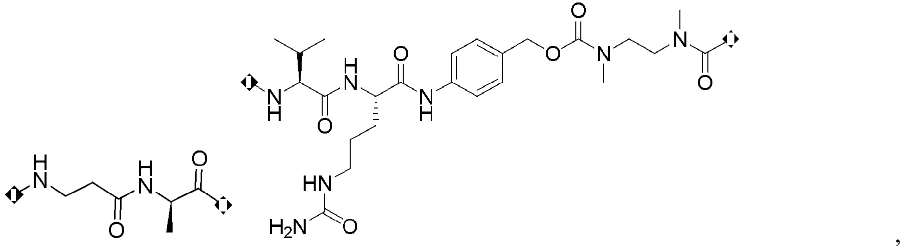 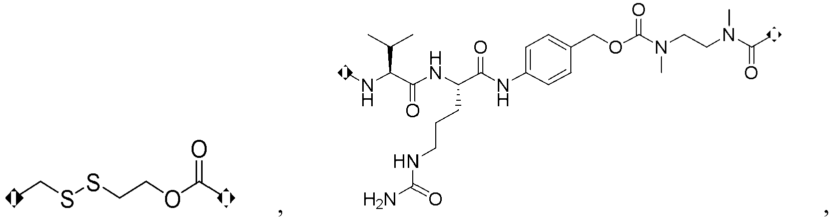 and 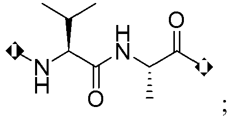 when L is 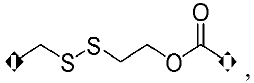 T⁰ is preferably -NHCH₂-, wherein the C end is connected to L; when L is 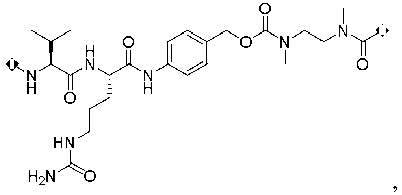 T⁰ is preferably 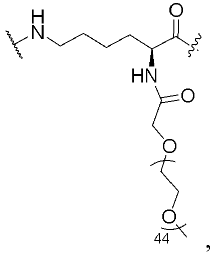 wherein the C end is connected to L; when L is another fragment, T⁰ is preferably absent.

16. The phospholipid drug conjugate according to claim 1, or a stereoisomer, prodrug, pharmaceutically acceptable salt, or pharmaceutically acceptable solvate thereof, wherein it satisfies one or more of the following conditions:
(1) 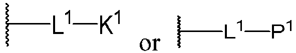 is selected from the following structures: 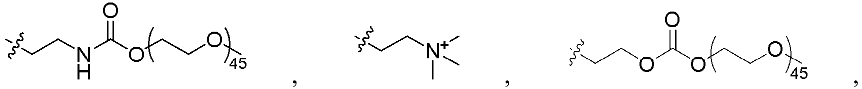 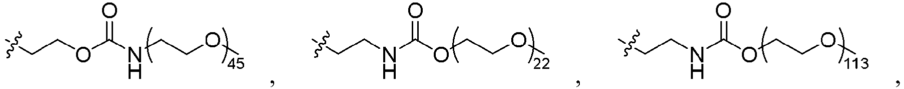 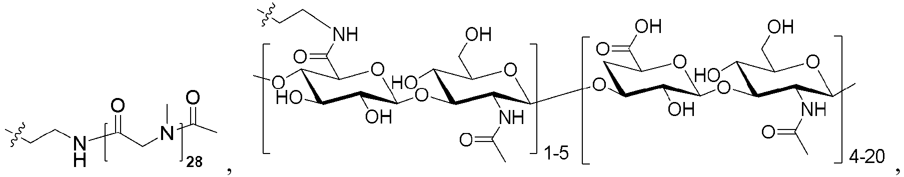 and 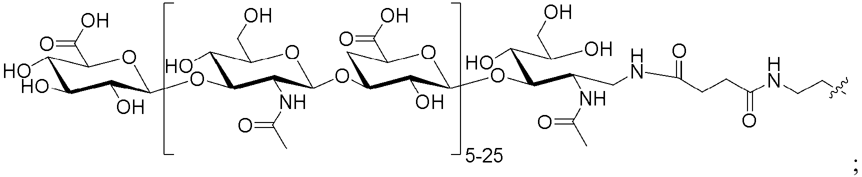
(2) 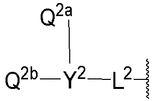 is selected from the following structures: 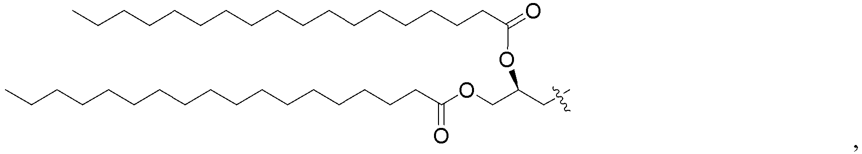 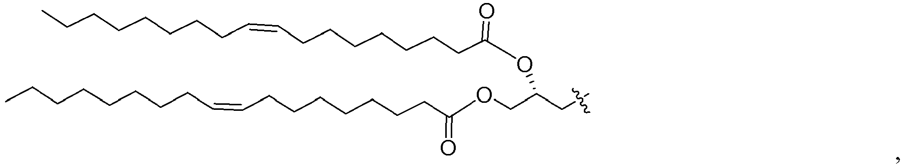 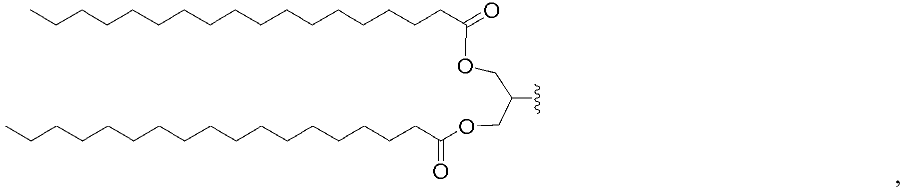 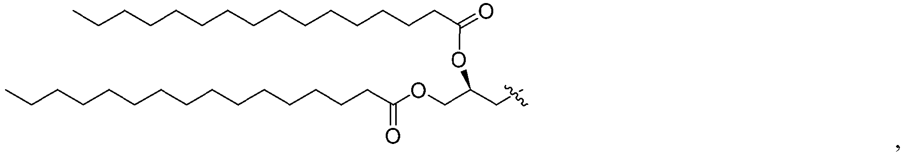 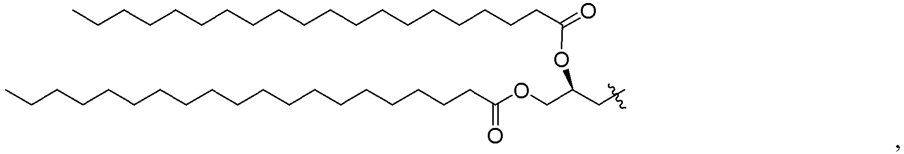 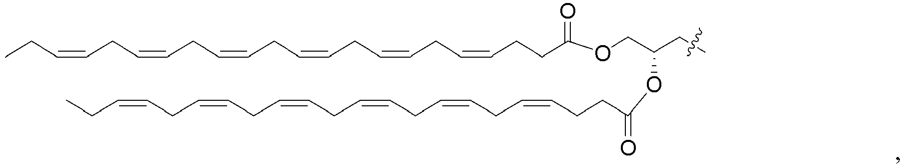 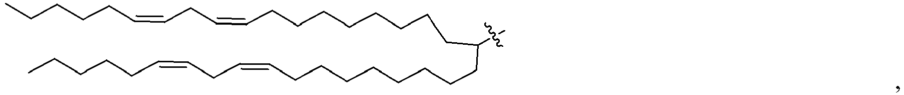 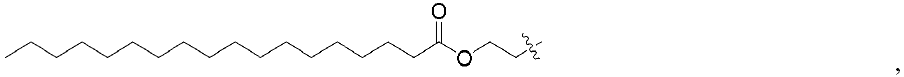 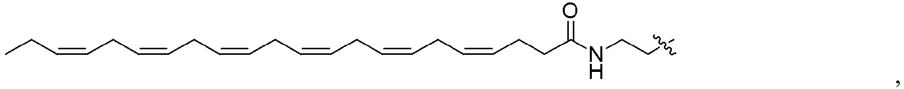 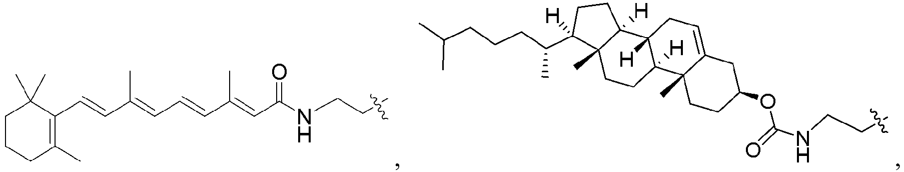 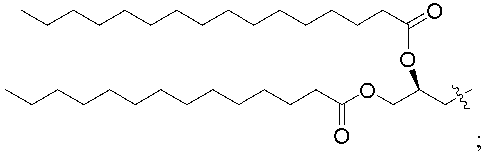
(3) 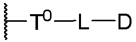 is selected from the following structures:

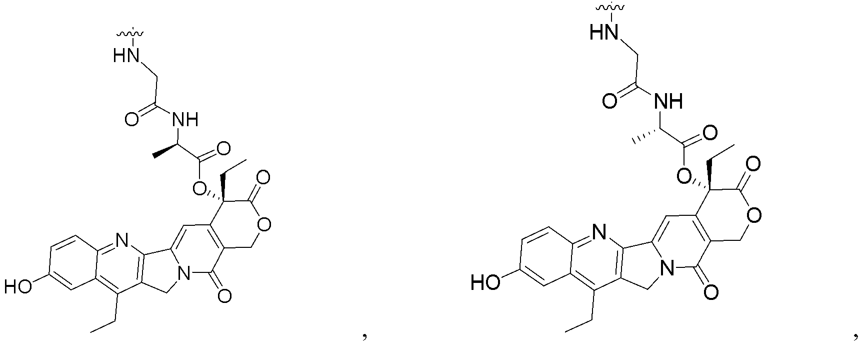

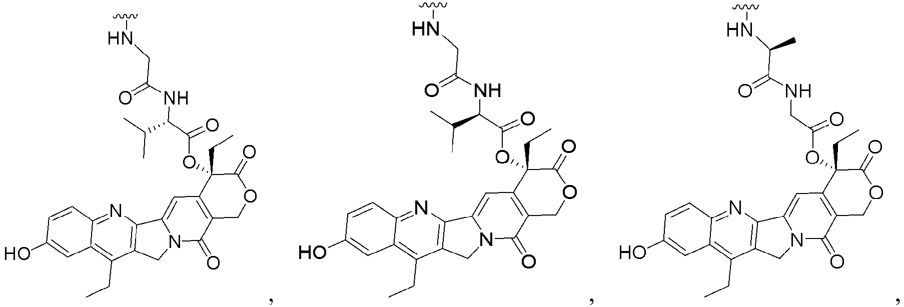

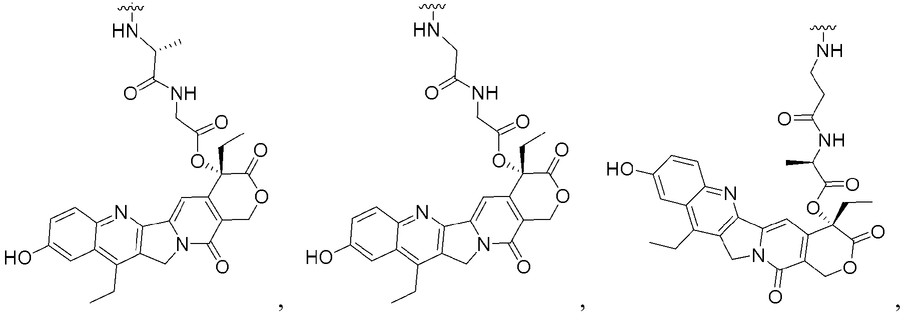

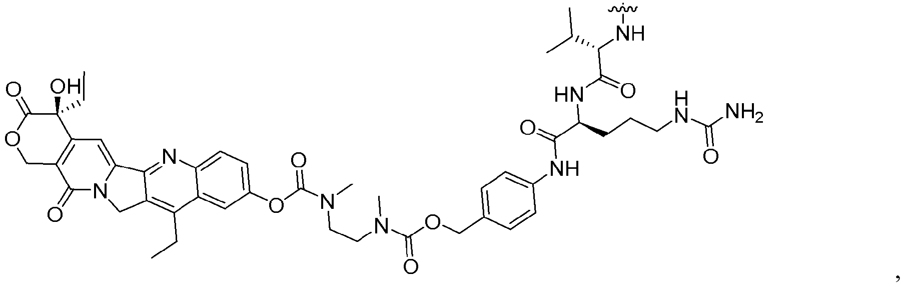

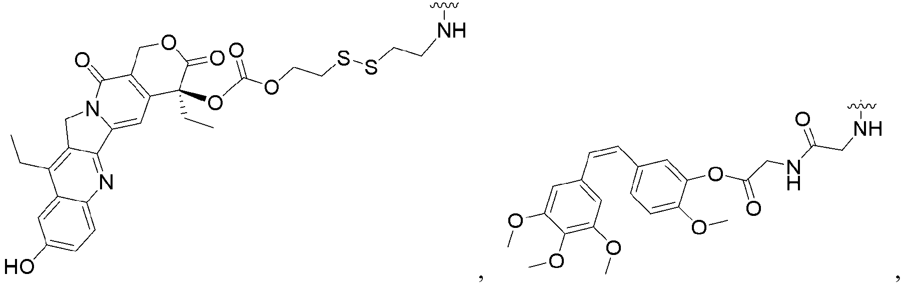

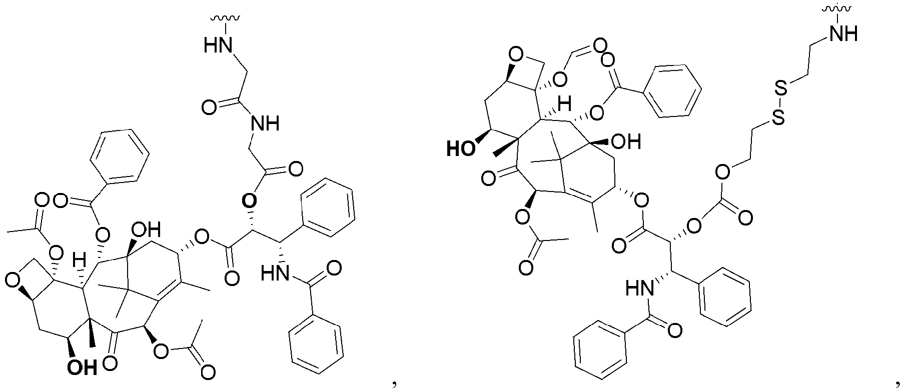

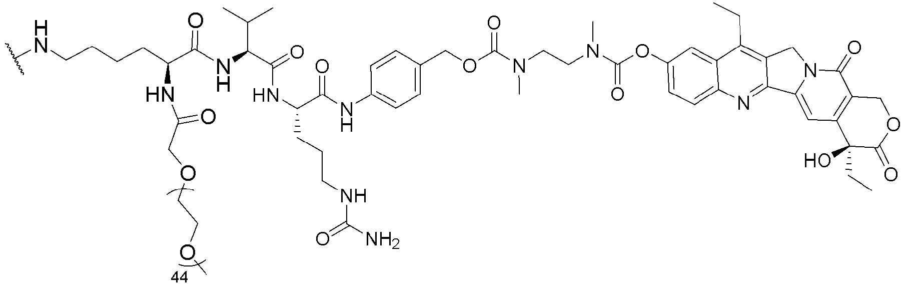

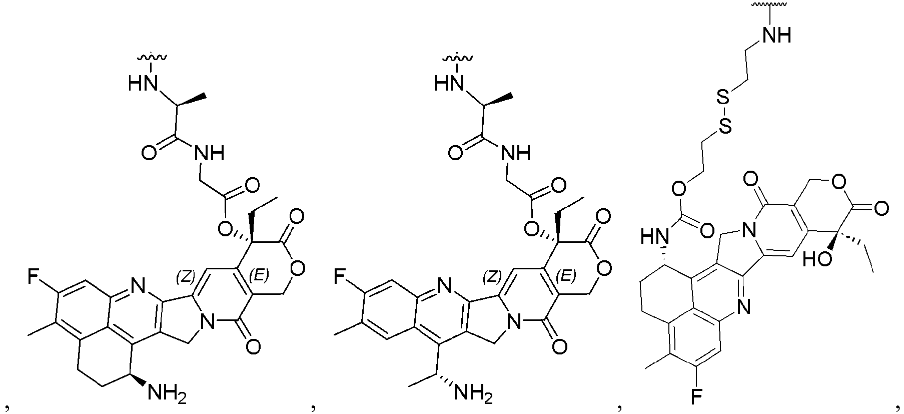

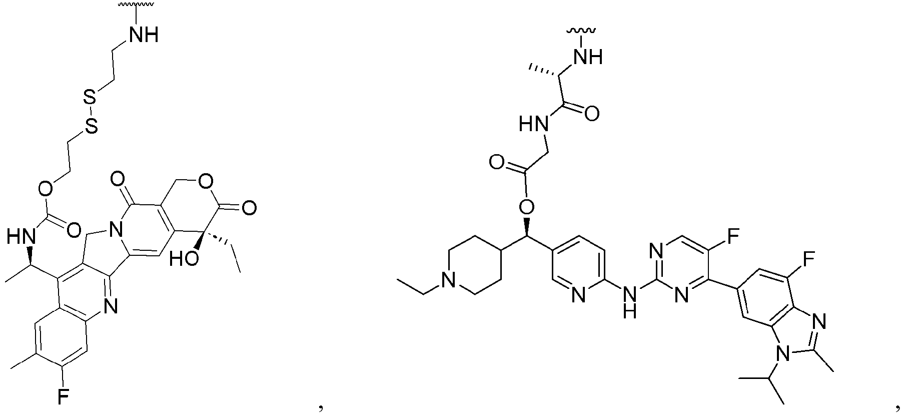

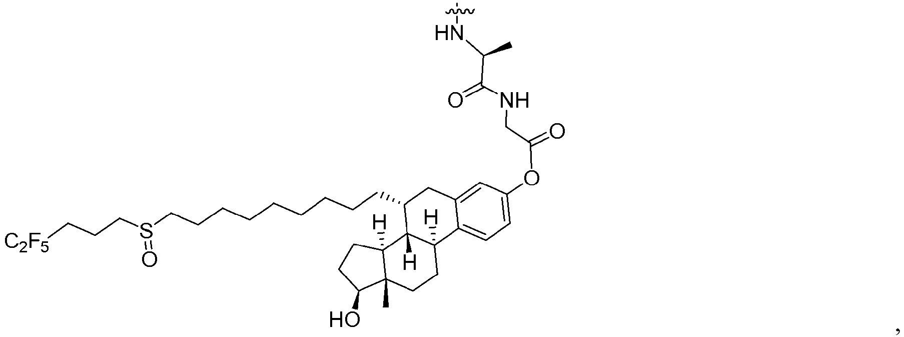

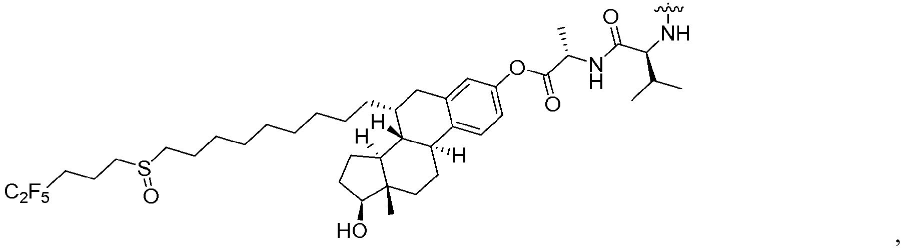

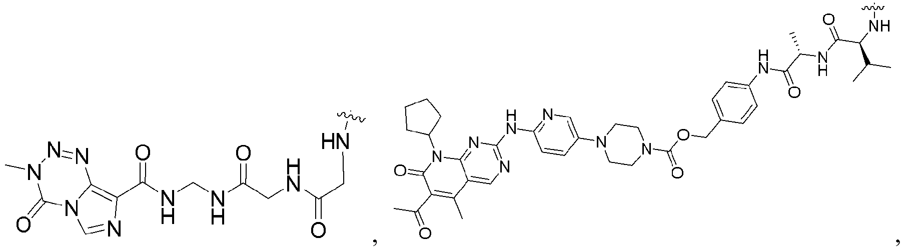

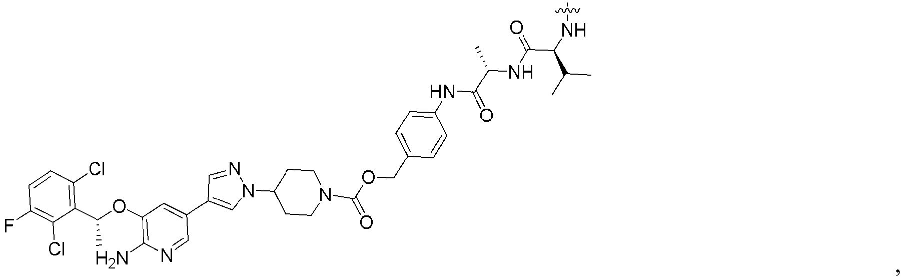

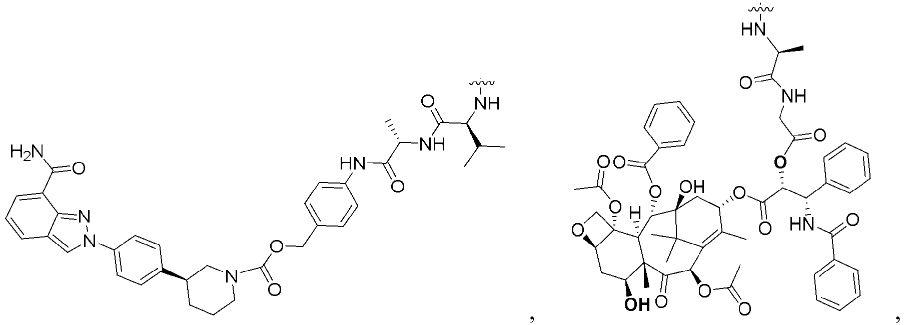

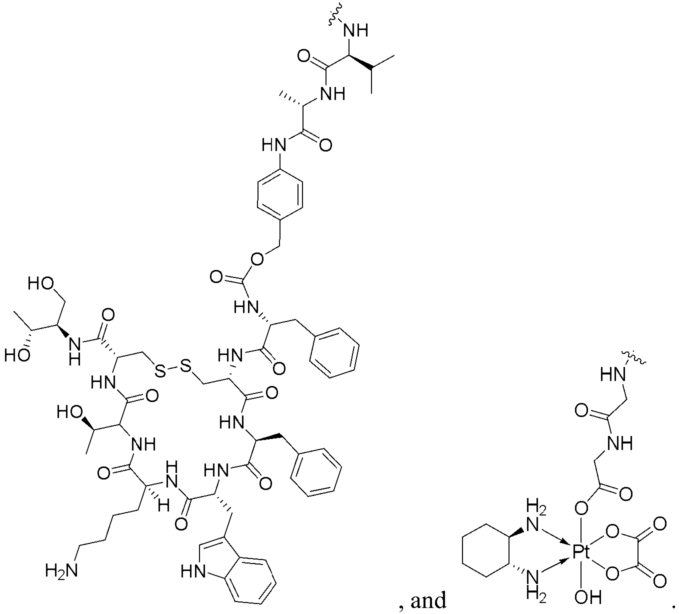

17. The phospholipid drug conjugate according to any one of claims 1-16, or a stereoisomer, prodrug, pharmaceutically acceptable salt, or pharmaceutically acceptable solvate thereof, wherein said phospholipid drug conjugate is selected from the following structures:

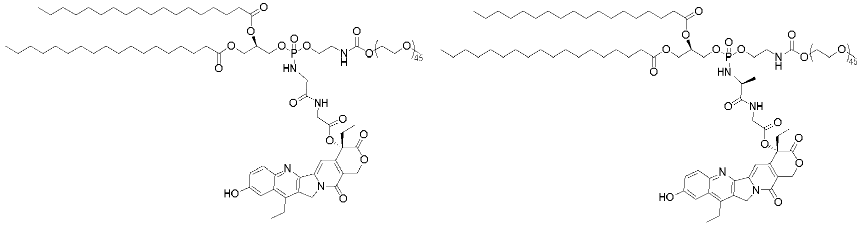

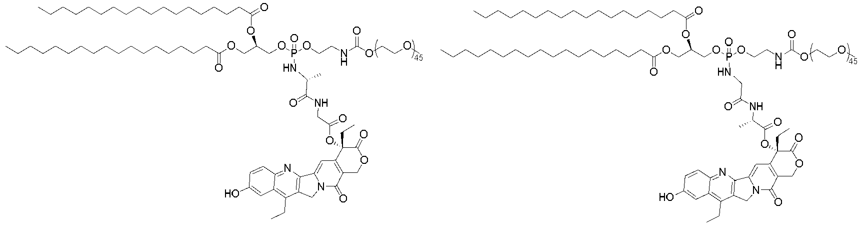

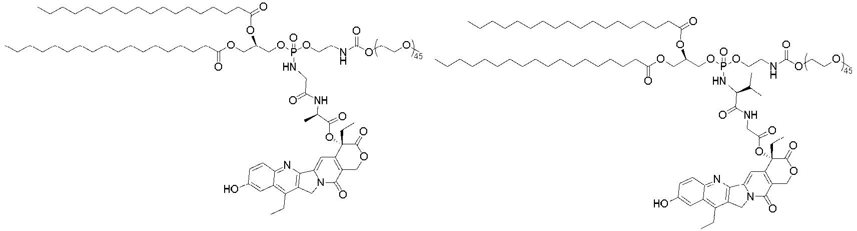

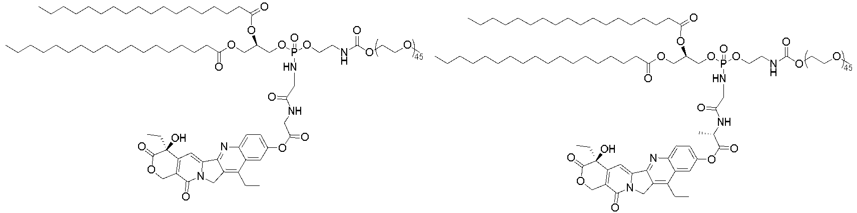

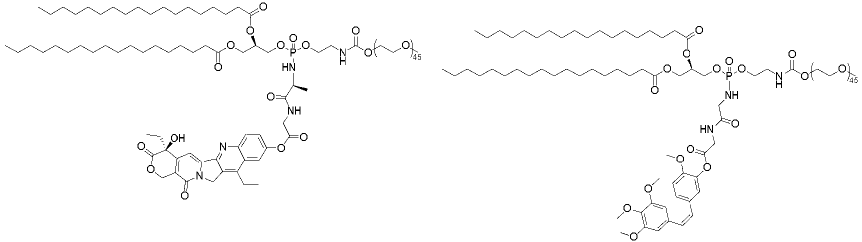

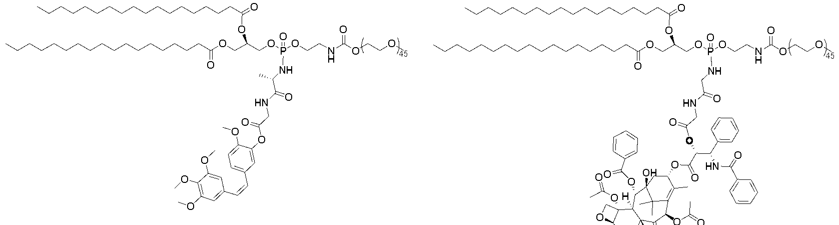

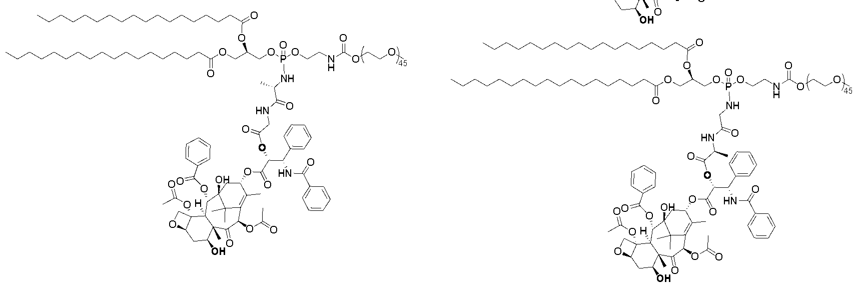

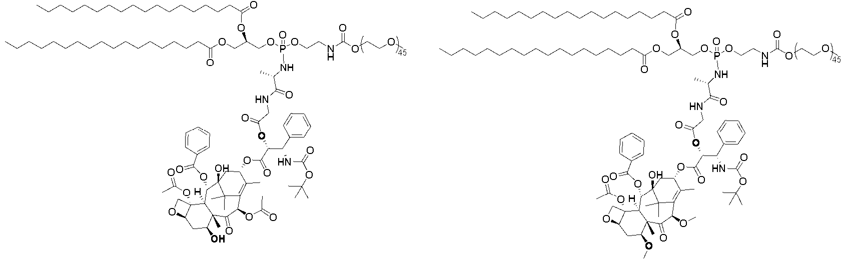

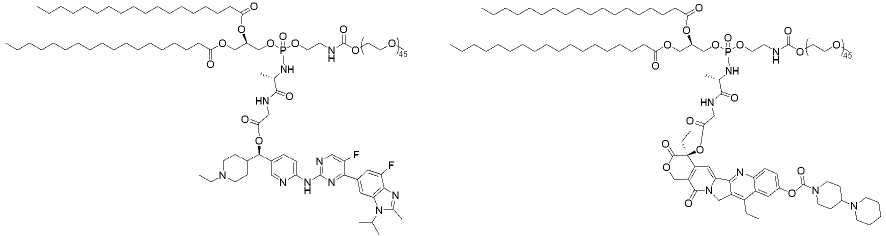

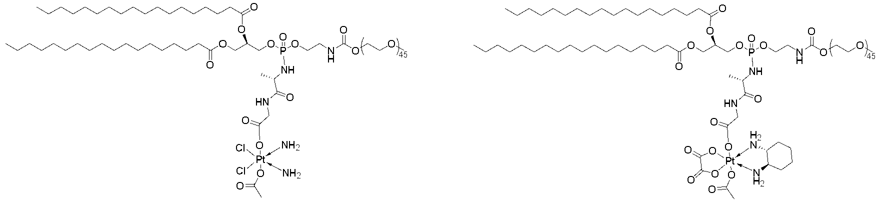

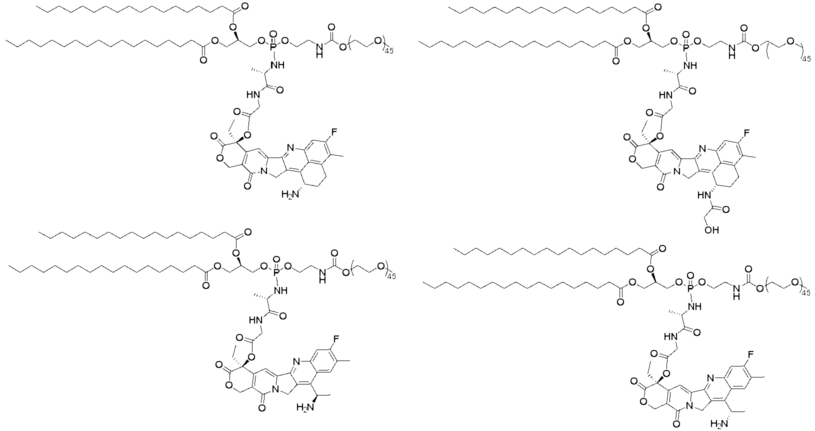

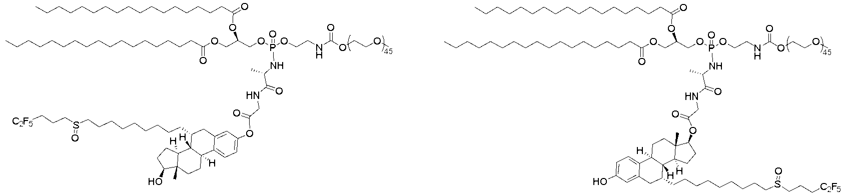

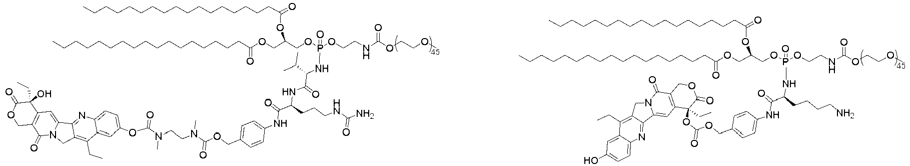

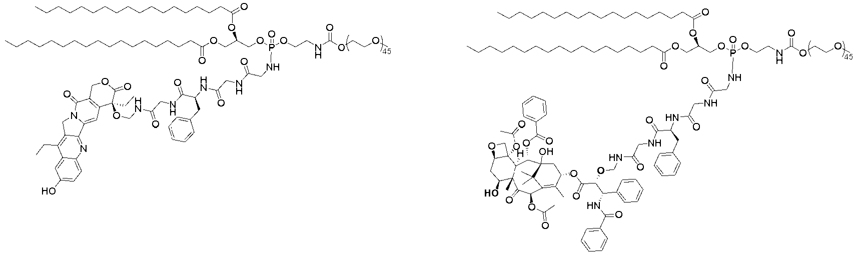

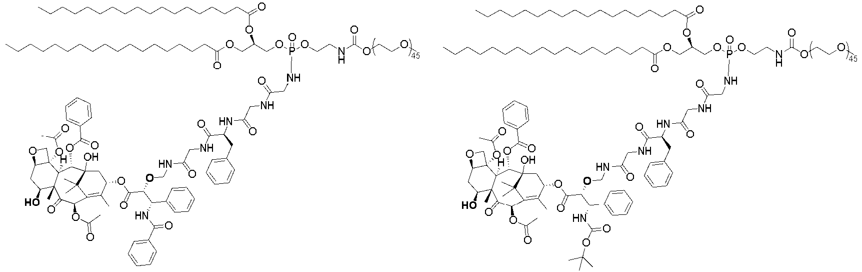

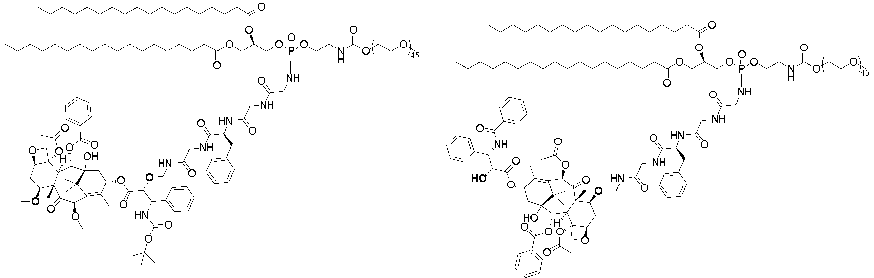

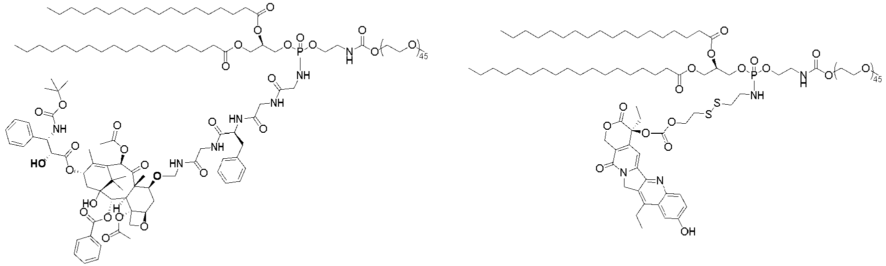

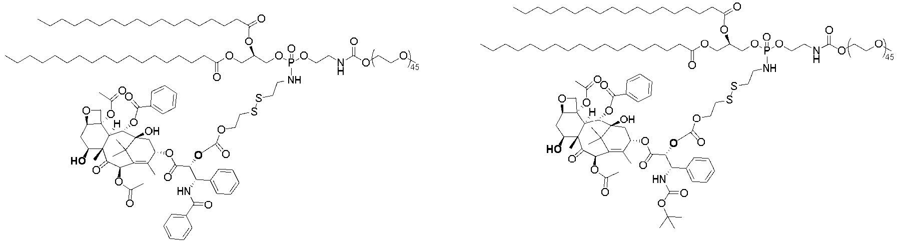

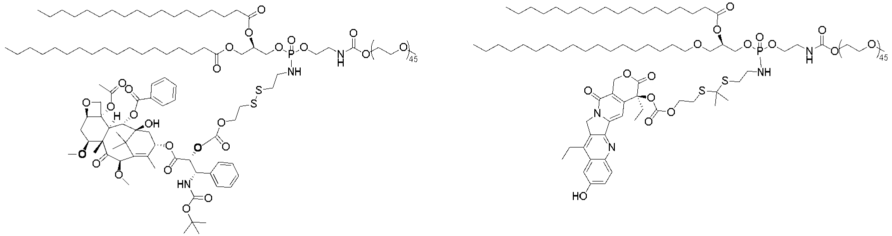

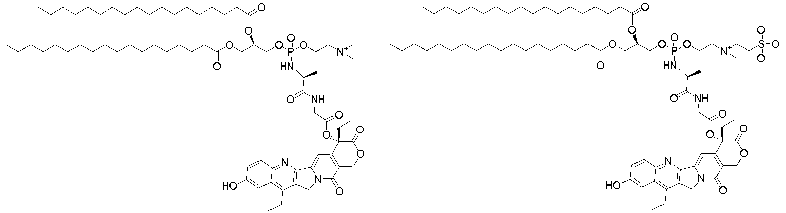

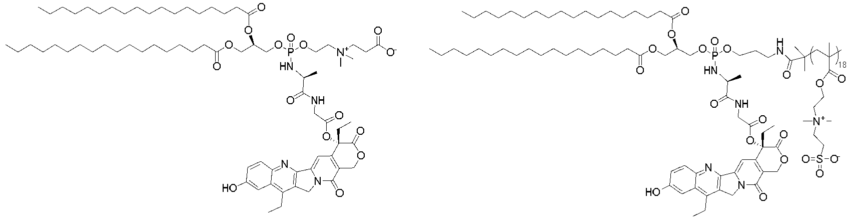

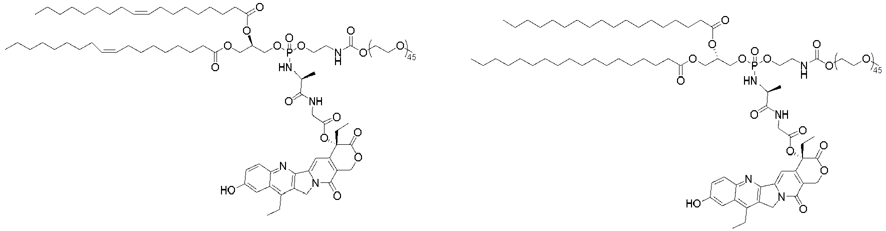

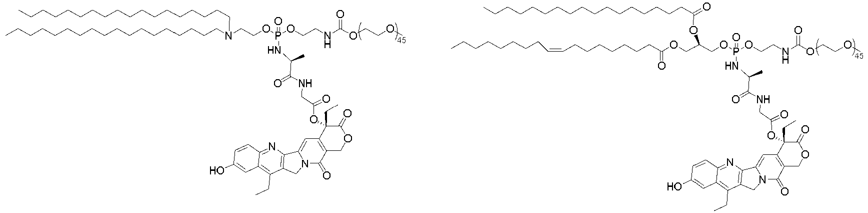

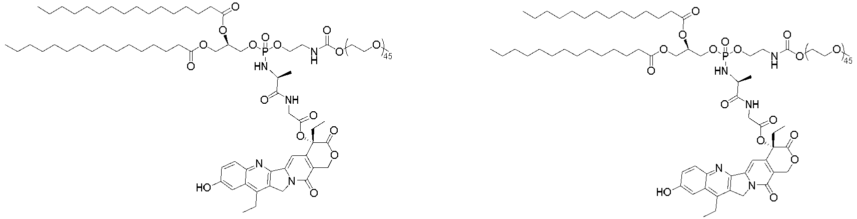

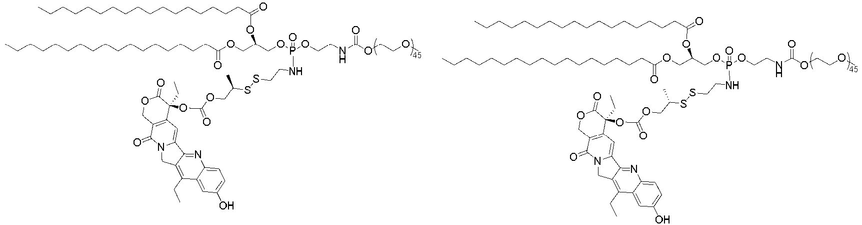

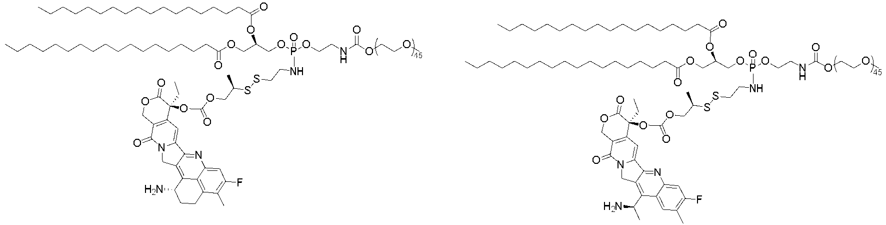

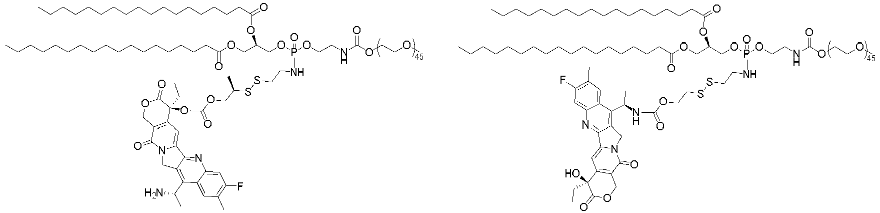

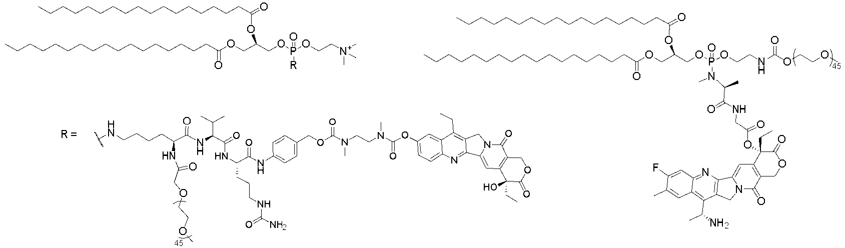

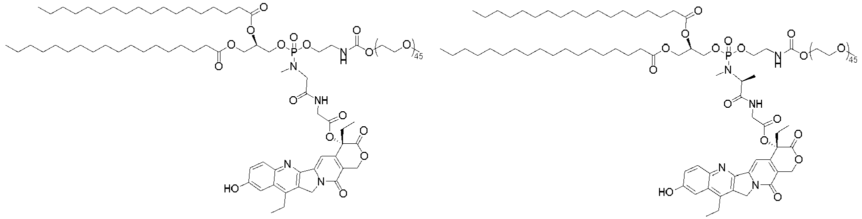

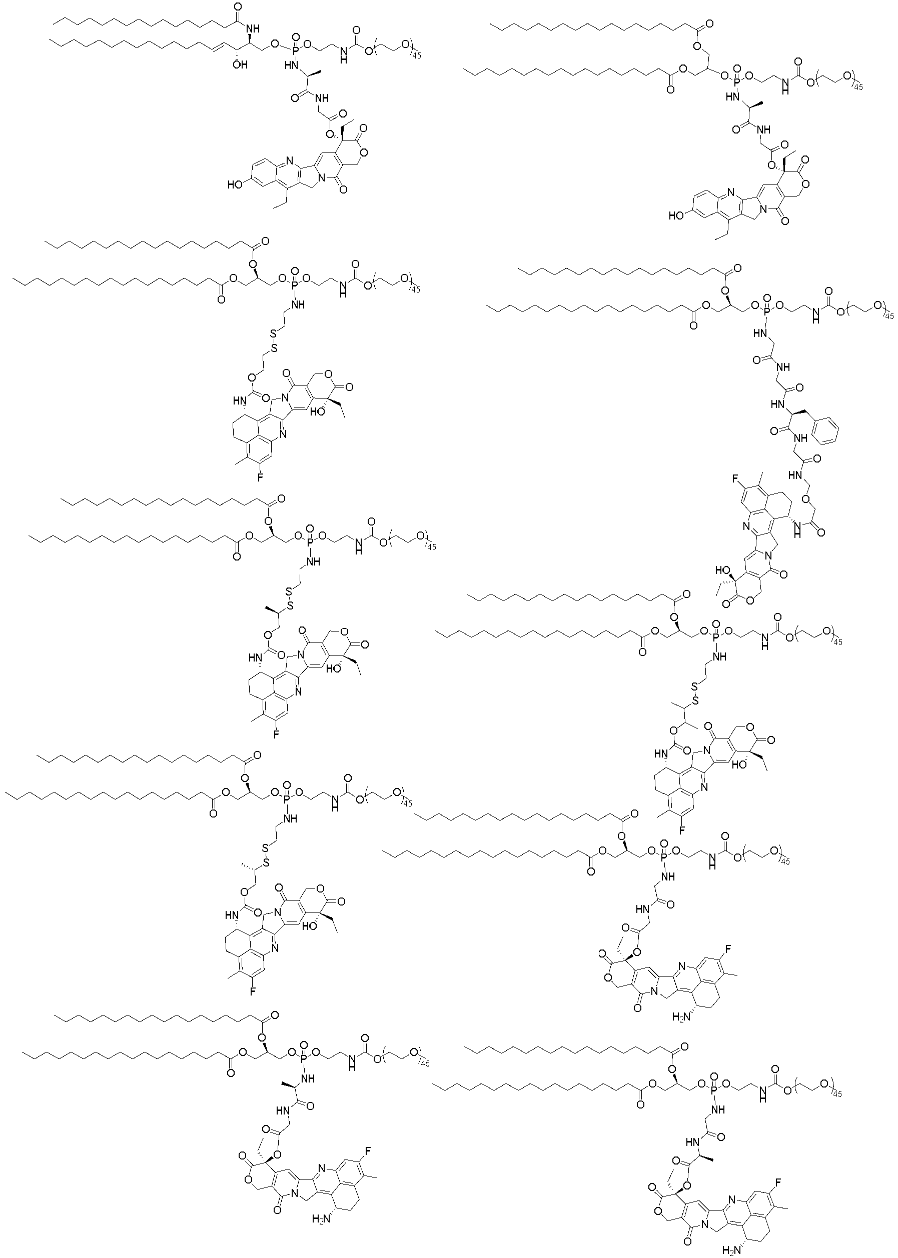

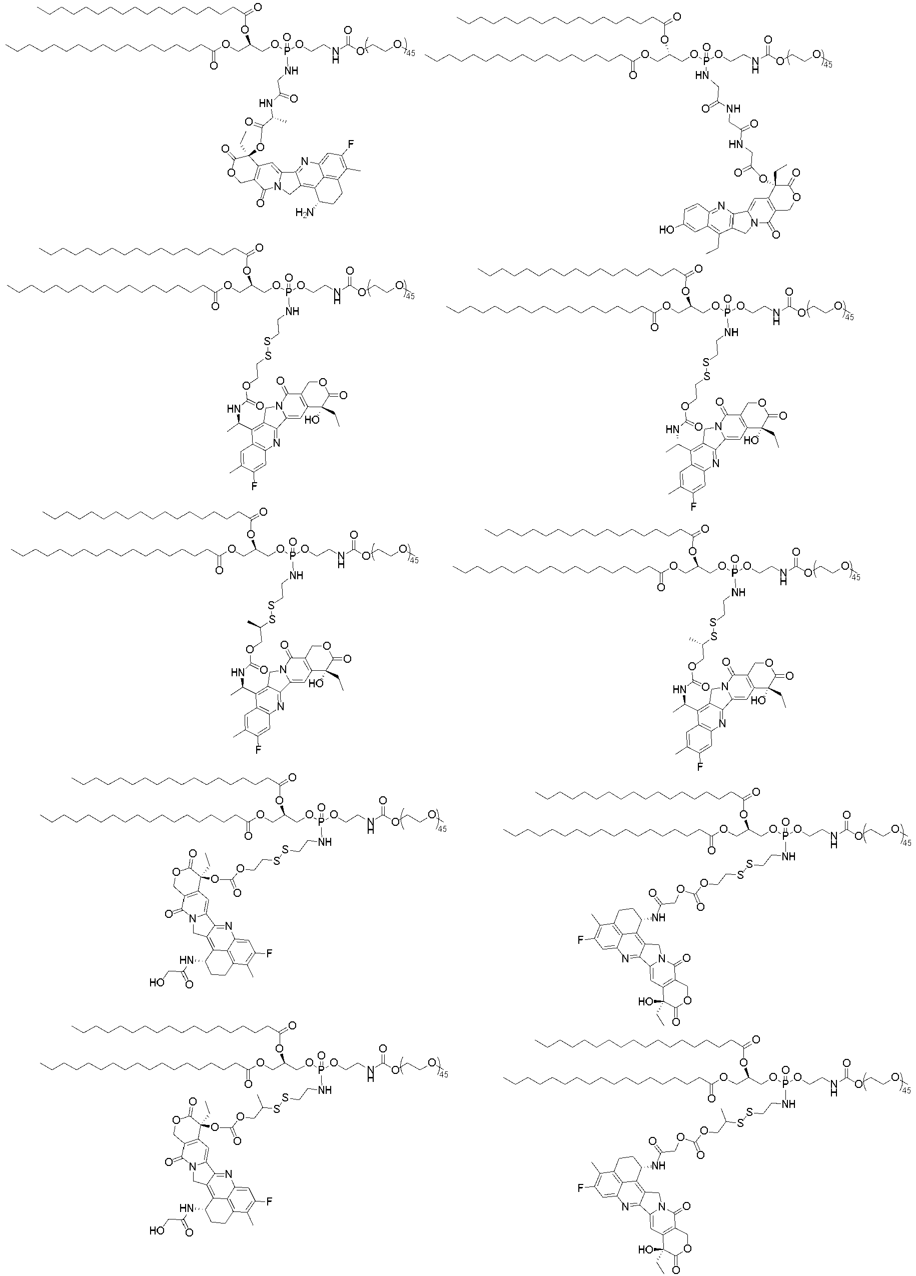

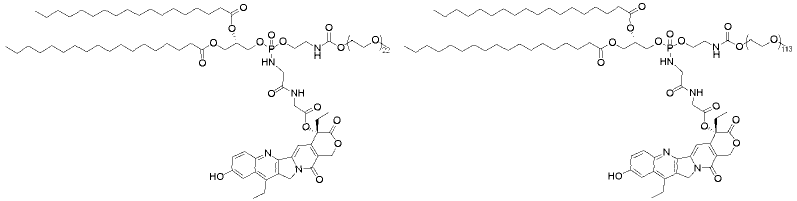

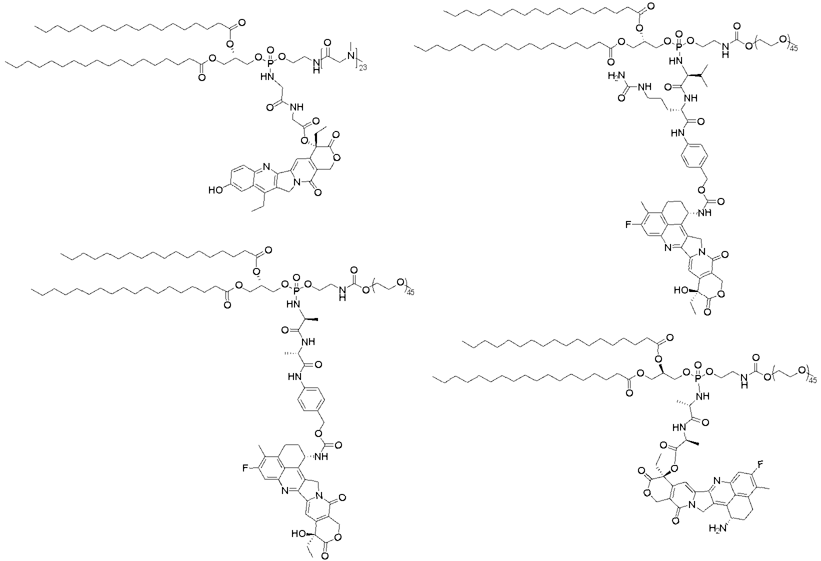

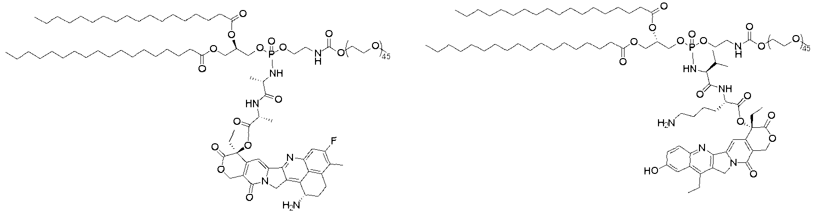

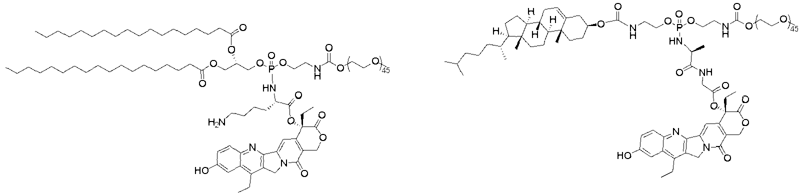

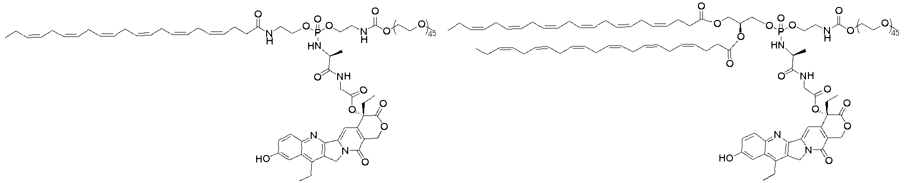

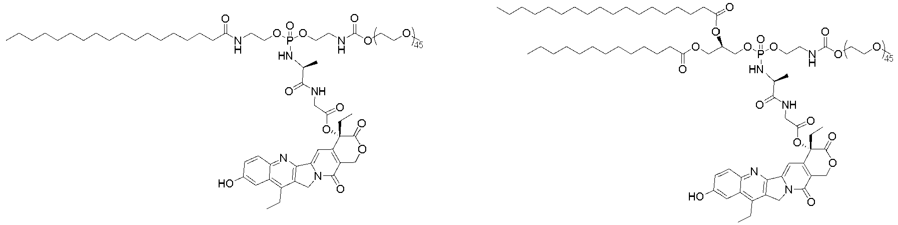

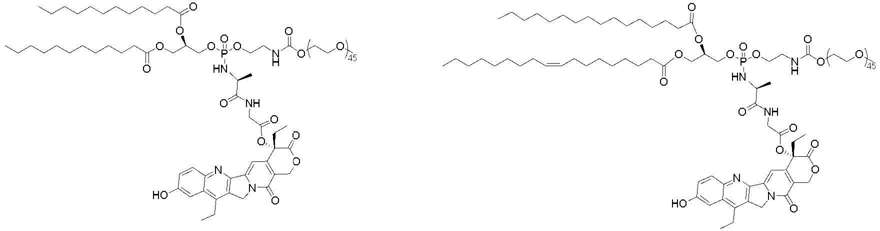

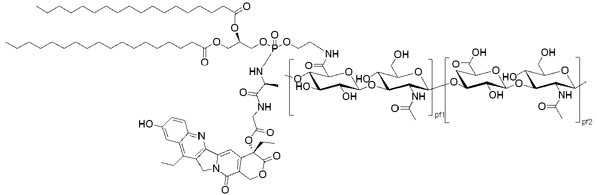

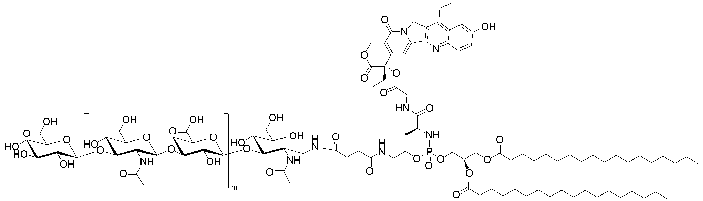

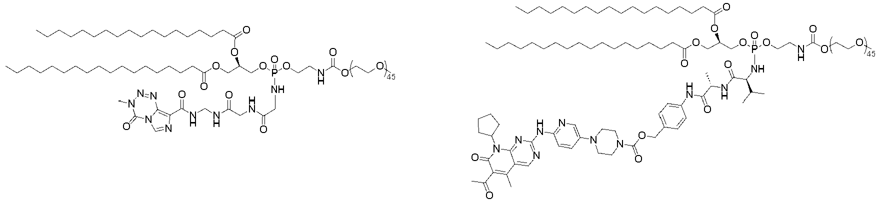

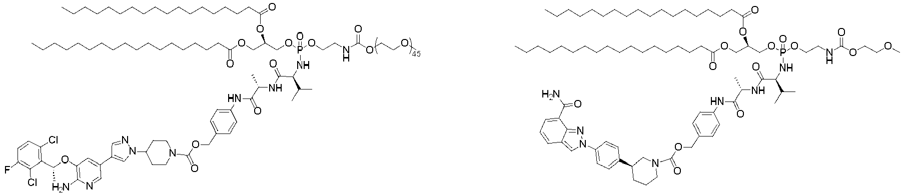

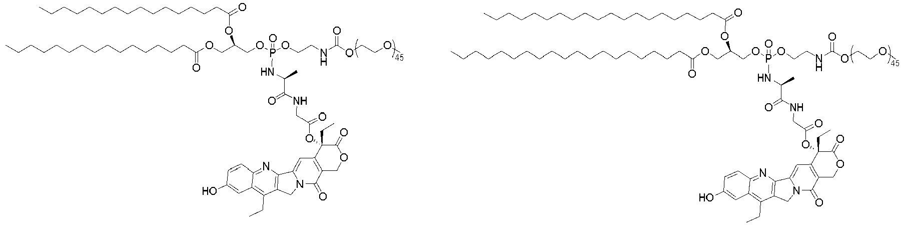

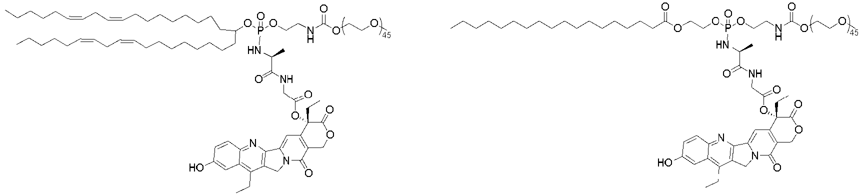

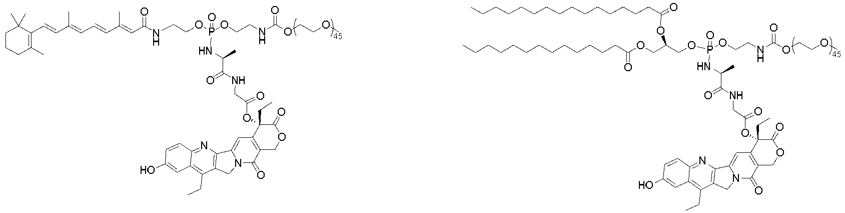

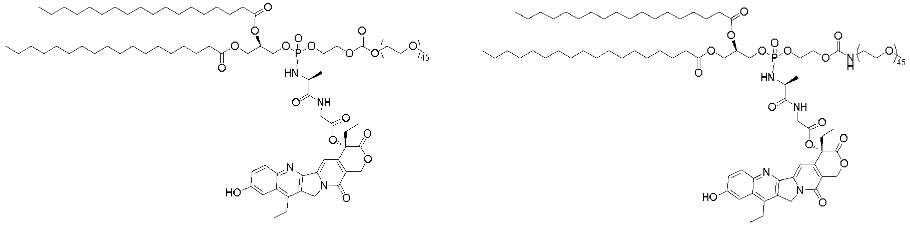

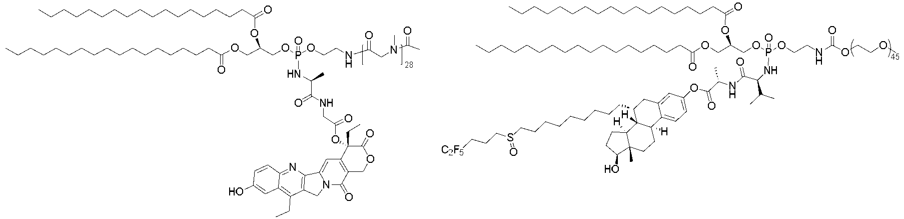

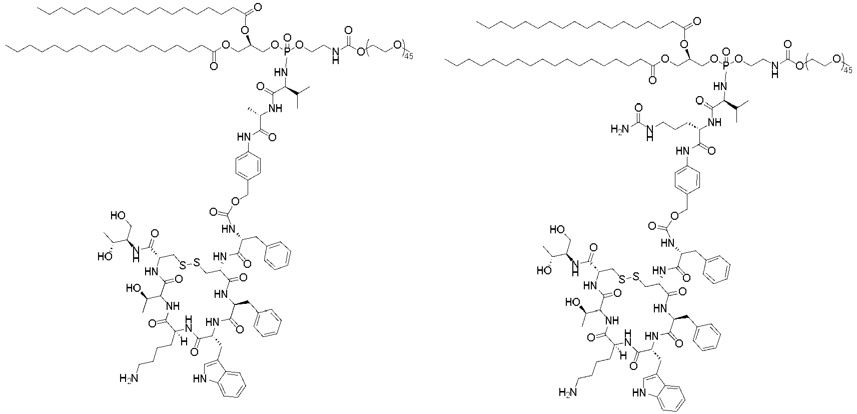

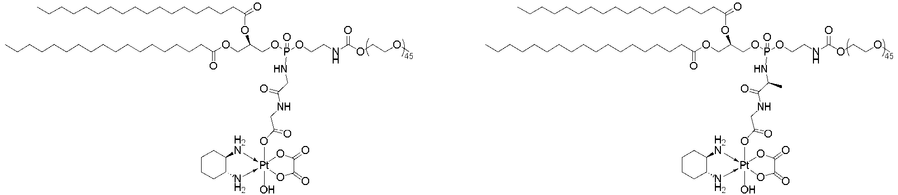

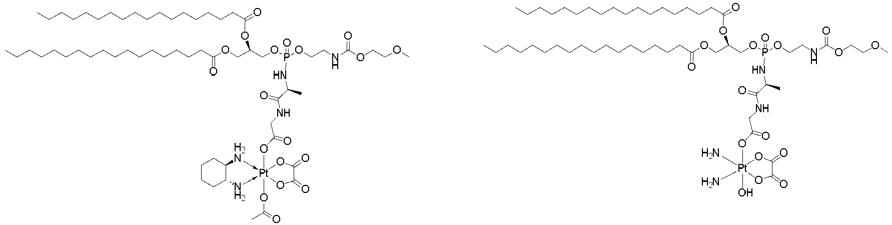

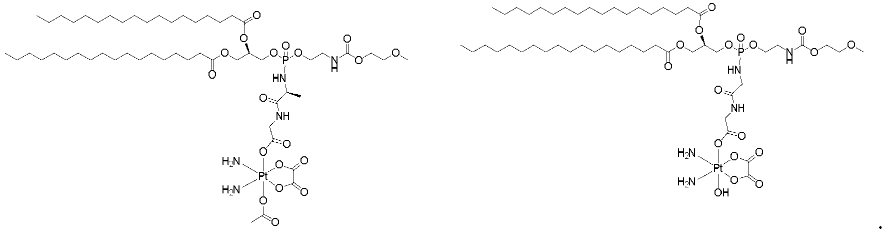

18. Use of the phospholipid drug conjugate according to any one of claims 1-17, or a stereoisomer, prodrug, pharmaceutically acceptable salt, or pharmaceutically acceptable solvate thereof, or said pharmaceutical composition, in the manufacture of a medicament for preventing, alleviating and/or treating abnormal cell proliferation, infection (e.g., viral infection, such as HIV, HBV), inflammatory disease (e.g., rheumatoid arthritis), autoimmune disease (e.g., psoriasis, lupus erythematosus, diabetes), cardiovascular disease (e.g., stroke, myocardial infarction, atherosclerosis), ocular disease, or neurodegenerative disease (e.g., Alzheimer's disease, Parkinson's disease), wherein the abnormal cell proliferative disease may be cancer; said cancer preferably includes breast cancer, ovarian cancer, brain cancer, prostate cancer, melanoma, nasopharyngeal carcinoma, esophageal cancer, gastric cancer, liver cancer, pancreatic cancer, colorectal cancer (e.g., colon cancer, rectal cancer), lung cancer (e.g., small cell lung cancer, non-small cell lung cancer, squamous cell carcinoma, undifferentiated carcinoma), kidney cancer, skin cancer, neuroblastoma, sarcoma, liposarcoma, osteochondroma, bone cancer, osteosarcoma, seminoma, testicular tumor, uterine tumor (e.g., cervical cancer, endometrial cancer), head and neck tumor (e.g., laryngeal cancer, pharyngeal cancer, tongue cancer), multiple myeloma, malignant lymphoma (e.g., reticulum cell sarcoma, lymphosarcoma, Hodgkin's lymphoma, mantle cell lymphoma), polycythemia vera, leukemia (e.g., acute myelogenous leukemia, chronic myelogenous leukemia, acute lymphoblastic leukemia, chronic lymphocytic leukemia), thyroid tumor, ureteral tumor, bladder tumor, gallbladder cancer, bile duct cancer, choriocarcinoma, or pediatric tumor (e.g., neuroblastoma, embryonal carcinoma of testis, retinoblastoma).
